# EUROPEAN PATENT APPLICATION

(11) **EP 1 798 224 A1**
(43) Date of publication of application: **20.06.2007**
(21) Application number: 05790508.5
(22) Date of filing: 04.10.2005
(51) Int. Cl.: C07D 235/06

(54) **BIARYL DERIVATIVE**

(30) Priority: 05.10.2004 JP 2004292506
(71) Applicant: Shionogi Co., Ltd., Osaka-shi, Osaka 541-0045 (JP)
(72) Inventor: KUGIMIYA, Akira, Shionogi & Co., Ltd., Osaka-shi, Osaka, 553-0002 (JP); HAGA, Nobuhiro, Shionogi & Co., Ltd., Osaka-shi, Osaka, 553-0002 (JP); KOJIMA, Eiichi, Shionogi & Co., Ltd., Osaka-shi, Osaka, 553-0002 (JP)
(74) Representative: Vossius & Partner
(86) International application number: PCT/JP2005/018326
(87) International publication number: WO 2006/038606

(57) **Abstract**

The present invention provides a compound represented by the formula (I): 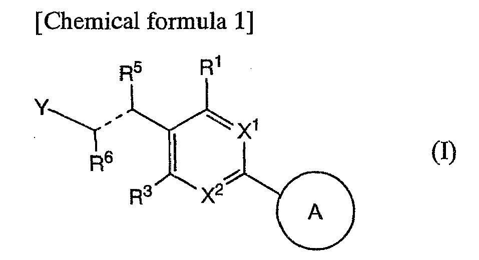 wherein X¹ is N or CR², X² is N or CR⁴, R¹ to R⁶ are each independently hydrogen, halogen, lower alkyl, lower alkoxy etc., a broken line is a single bond or a double bond etc., Y is COOR^{A}, CONHB(CR^{C}R^{D})_{P}COOR^{A}, CONR^{E}R^{F}, CONR^{B}(CR^{C}R^{D})ₚCONR^{E}R^{F} etc., R^{A} to R^{F}, R^{H} and R^{J} are each independently hydrogen, lower alkyl etc., R^{G} is lower alkyl, aryl heterocycle etc., p is 1 or 2, ring A is optionally substituted phenyl, optionally substituted indolyl etc., X³ is O, S or NR¹³, X⁴ is CR⁷ or N, X⁵ is CR⁸ or N, X⁶ is CR⁹ or N, X⁷ is CR¹⁰ or N, R⁷ to R¹² are each independently hydrogen, halogen, lower alkyl etc., R¹³ and R¹⁴ are each independently hydrogen, lower alkyl etc., W is hydrogen, lower alkyl, NR¹⁵R¹⁶ etc., and R¹⁵ to R²¹ are each independently hydrogen, lower alkyl etc.),
or a pharmaceutically acceptable salt or a solvate thereof, and a pharmaceutical composition containing them.

## Description

### Technical Field

The present invention relates to a novel biaryl derivative and a pharmaceutical composition containing the same, particularly an antibody production suppressing agent, and/or a dihydroorotate dehydrogenase inhibitor.

### Background Art

A serious problem of transplantation operation of a tissue, or an internal organ which is frequently performed in recent years is a rejection reaction for excluding a transplanted part after operation. Avoidance of this becomes very important for successful transplantation operation.
Various immunosuppressing agents such as azathioprine, corticoid, cyclosporin A and tacrolimus have been developed and put into practice, and are used in preventing or treating a rejection reaction against internal organ or tissue transplantation, or a graft versus host reaction which occurs by bone marrow transplantation. However, these are not necessarily satisfactory in respect of the effect or the side effect.
On the other hand, an allergic disease such as atopic dermatitis, allergic rhinitis, bronchial asthma, allergic conjunctivitis and the like tends to increase worldwide in recent years, and has become a serious problem. The existing anti-allergie agent is an agent for suppressing release of a chemical transmitter from a mast cell, an agent for inhibiting a receptor for a released chemical transmitter, or an agent for suppressing an allergic inflammatory reaction, but all of them are drugs for symptomatic treatment, and are not for fundamental treatment of an allergic disease.

Dihydroorotate dehydrogenase (hereinafter, DHODH) is an enzyme catalyzing an oxidation-reduction step which is the fourth step of pyrimidine biosynthesis. For rapid cell proliferation, synthesis of DNA and RNA, glycosylation of proteins, biosynthesis of membrane lipids, and new pyrimidine biosynthesis for repairing nucleic acid chain are necessary, and the step which is catalyzed by DHODH is a rate-determining step of pyrimidine biosynthesis.
DHODH is known to be associated with rheumatoid arthritis, systemic erythematosus, multiple sclerosis, inflammatory bowl disease, uveitis, myasthenia gravis, bronchial asthma, atopic dermatitis, psoriasis, virus infectious disease, parasite infectious disease, cancer, a rejection reaction in transplantation, and a graft versus host disease, and a DHODH inhibitor is useful for treating or preventing the aforementioned diseases (see Non-Patent Literatures 1-5).

Patent Literature 1 describes compounds which can be used in treatment of a dermatosis condition associated with a keratinization disease, a dermatosis condition having an inflammation and/or immune allergic component, and a degeneration disease of a connective tissue, and have the anti-tumor activity. However, all of specifically disclosed compounds have an adamantylphenylnaphthilic acid structure or a phenylpropenylbenzoic acid structure, and the compounds of the present invention are not described nor suggested.
In addition, compounds having a structure similar to those of the compounds of the present invention are described in Patent Literature 2, Patent Literature 3 and Non-Patent Literatures 6 to 8, but all of those compounds are not described to have the antibody production suppressing activity, the DHODH inhibitory activity, the immunosuppression activity or the anti-allergic activity.
As the DHODH inhibitors, Non-Patent Literature 9 discloses a terphenyl derivatives having a carboxyl group, Non-Patent Literature 10 and Patent Literature 4 disclose quinolinecarboxylic derivatives, and Patent Literature 5 discloses compounds having a biphenylcarbamoyl group, but none of those Literatures describe or suggest the compounds of the present invention.
Patent Literature 6 describes retinoid derivatives having the anti-cancer activity and the anti-inflammation activity, but all of compounds which are specifically shown to have the activity are compounds having a feature of an adamantyl group, and the compounds of the present invention are not described or suggested.

[Patent Literature 1] International Publication WO92/19583
[Patent Literature 2] International Publication WO92/06099
[Patent Literature 3] Japanese Patent Application Laid-Open (JP-A) No.2-244059
[Patent Literature 4] JP-A No.60-42367
[Patent Literature 5] US2003/0203951
[Patent Literature 6] International Publication WO03/011808
[Non-Patent Literature 1] Immunopharmacology, 2000, vol.47, p.63-83
[Non-Patent Literature 2] Immunopharmacology, 2000, vol.47, p.273-289
[Non-Patent Literature 3] Cancer Research, 1986, vol.46, p.5014-5019
[Non-Patent Literature 4] Biochemistry, 1994, vol.33, p.5268-5274
[Non-Patent Literature 5] Biochemical Pharmacology, 2000, vol.60, p.339-342
[Non-Patent Literature 6] Heterocycles, 1999, vol.51, No.12, p.2915-2930
[Non-Patent Literature 7] Helvetica Chimica Acta, 1975, vol.58, No.1, p.268-78
[Non-Patent Literature 8] Symposia of the Faraday Society, 1971, vol. p.54-67
[Non-Patent Literature 9] Tetrahedron Letters, 2001, vol.42, p.547-551
[Non-Patent Literature 10] Biochemical Pharmacology, 1990, vol.40, No.4, p.709-714

### Disclosure of The Invention

### Problems to be Solved by The Invention

An object of the present invention is to provide a novel biaryl derivative and a pharmaceutical composition containing the same, an antibody production suppressing agent and a DHODH inhibitor.

### Means to Solve The Problems

The present invention provides:
1) A compound represented by the formula (I): 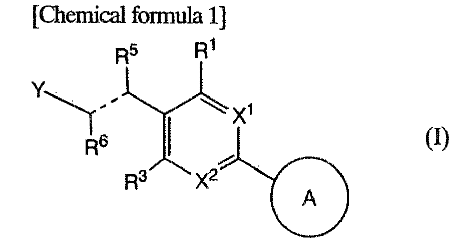
wherein X¹ is N or CR², X² is N or CR⁴,
R¹, R², R³ and R⁴ are each independently hydrogen, halogen, optionally substituted lower alkyl, optionally substituted lower alkoxy, optionally substituted acyl, carboxy, or optionally substituted lower alkoxycarbonyl, provided that all of R¹ to R⁴ are not simultaneously hydrogen, 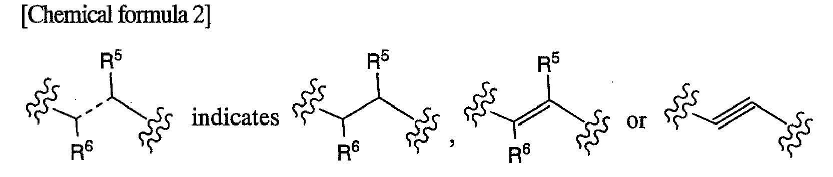 (hereinafter referred to as "a broken line means a single bond", "a broken line means a double bond" and "a broken line means a triple bond")
   wherein R⁵ and R⁶ are each independently hydrogen, halogen, hydroxy, optionally substituted lower alkyl, optionally substituted lower alkoxy, optionally substituted amino, or cyano, and when a broken line is a single bond, then R⁵ may be oxo,
Y is: 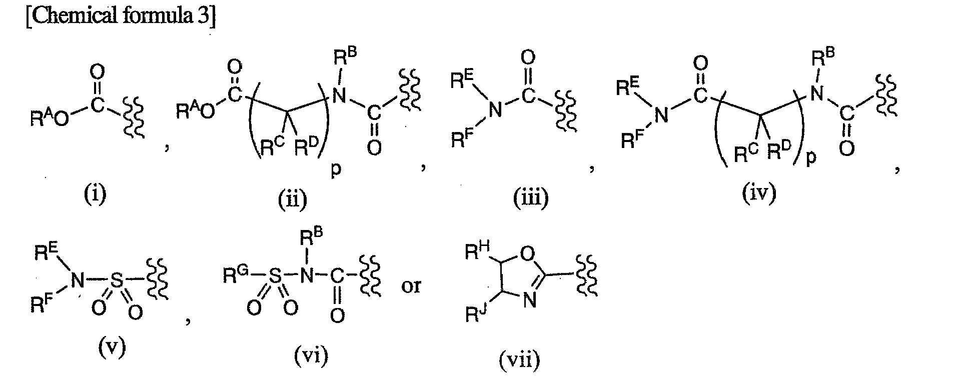 wherein R^{A}, R^{B} and R^{E} are each independently hydrogen or lower alkyl,
R^{C} and R^{D} are each independently hydrogen, optionally substituted lower alkyl, and R^{C} and R^{D} may be taken together to form a carbocycle containing an adjacent carbon atom,
R^{F} is hydrogen, optionally substituted lower alkyl, optionally substituted lower alkoxy, optionally substituted cycloalkyl, optionally substituted cycloalkenyl, optionally substituted amino, optionally substituted amidino, cyano, optionally substituted aryl, or optionally substituted heterocycle,
R^{G} is optionally substituted lower alkyl, optionally substituted aryl, or optionally substituted heterocycle,
R^{H} and R^{J} are each independently hydrogen, optionally substituted lower alkyl, carboxy, or optionally substituted lower alkoxycarbonyl,
p is 1 or 2,
ring A is: 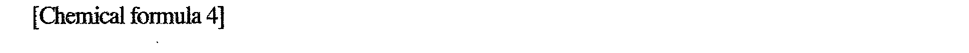 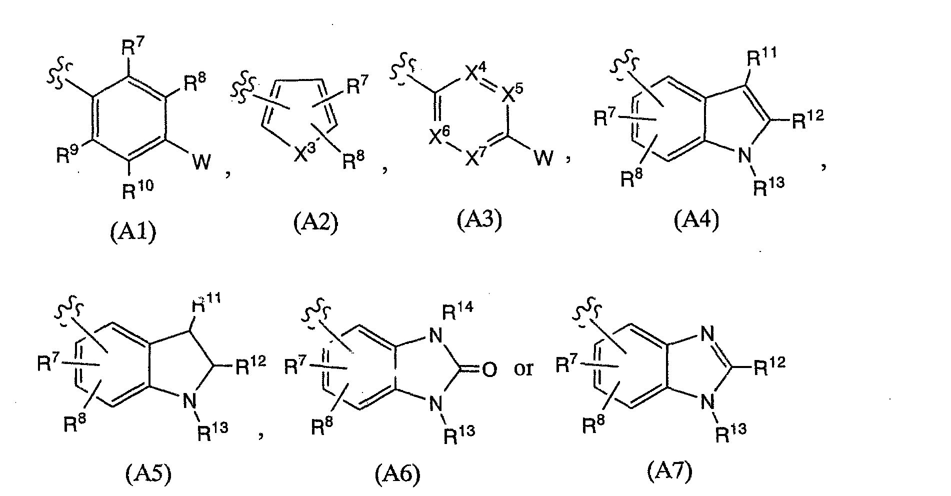 wherein X³ is O, S or NR¹³,
X⁴ is CR⁷ or N, X⁵ is CR⁸ or N, X⁶ is CR⁹ or N, X⁷ is CR¹⁰ or N, provided that at least one of X⁴ to
X⁷ is N, and at least one of X⁴ to X⁷ is other than N, R⁷ to R¹² are each independently hydrogen, halogen, hydroxy, lower alkyl, lower alkenyl, lower alkoxy, carboxy, lower alkoxycarbonyl, acyl, acyloxy, lower alkylsulfonyloxy or arylsulfonyloxy,
R¹³ and R¹⁴ are each independently hydrogen, lower alkyl, lower alkoxycarbonyl or aryl(lower)alkyl,
W is hydrogen, optionally substituted lower alkyl, NR¹⁵R¹⁶, OR¹⁷, SR¹⁸, COR¹⁹ or CONR²⁰R²¹, when ringAis (A1), and R⁵ is lower alkyl, then W is NR¹⁵R¹⁶, SR¹⁸, COR¹⁹ or CONR²⁰R²¹,
R¹⁵ and R¹⁶ are each independently hydrogen, optionally substituted lower alkyl, optionally substituted lower alkenyl, optionally substituted cycloalkyl, optionally substituted carbamoyl, optionally substituted lower alkylsulfonyl, optionally substituted arylsulfonyl, optionally substituted aryl, or optionally substituted heterocycle,
R¹⁷, R¹⁸, R¹⁹, R²⁰ and R²¹ are each independently hydrogen, optionally substituted lower alkyl, optionally substituted lower alkenyl, optionally substituted cycloalkyl, optionally substituted aryl,
or optionally substituted heterocycle,
or a pharmaceutically acceptable salt, or a solvate thereof.:

2) The compound according to the above 1, wherein ring A is: 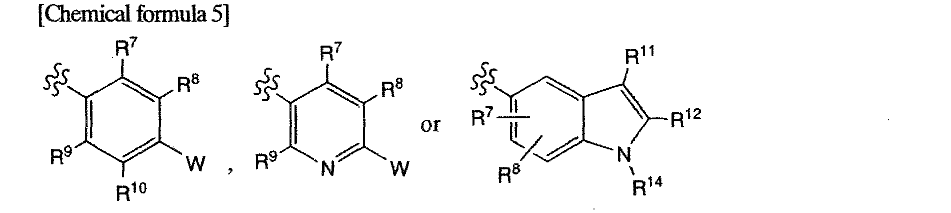 or a pharmaceutically acceptable salt, or a solvate thereof.
3) The compound according to the above 1, wherein ring A is: 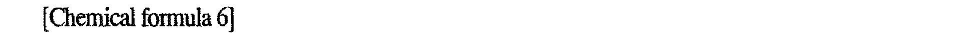 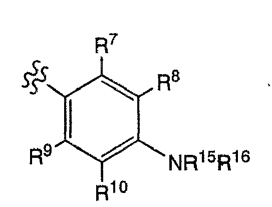 or a pharmaceutically acceptable salt, or a solvate thereof.
4) The compound according to any one of the above 1 to 3, wherein X¹ is CR², and X² is CR⁴, or a pharmaceutically acceptable salt, or a solvate thereof.
5) The compound according to any one of the above 1 to 4, wherein: 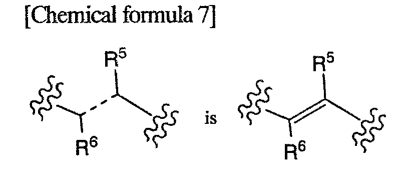 or a pharmaceutically acceptable salt, or a solvate thereof.
6) A compound represented by the formula (I'): 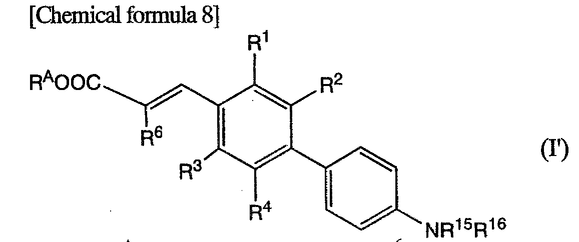
wherein R^{A} is hydrogen or lower alkyl, R⁶ is hydrogen, halogen or lower alkyl, R¹, R², R³ and R⁴ are each independently hydrogen, halogen, lower alkyl or lower alkoxy, and R¹⁵ and R¹⁶ are each independently hydrogen, optionally substituted lower alkyl or lower alkenyl,
or a pharmaceutically acceptable salt, or a solvate thereof.

7) The compound according to the above1, 4 or 5, wherein Y is (i), ring A is (A1) or (A3), and W is NR¹⁵R¹⁶, or a pharmaceutically acceptable salt, or a solvate thereof.
8) The compound according to the above 6, wherein R^{A} is a hydrogen, ring A is (A1), R⁵ and R⁶ are each independently hydrogen, halogen or lower alkyl, R¹, R², R³ and R⁴ are each independently hydrogen, lower alkyl or lower alkoxy, R⁷, R⁸, R⁹ and R¹⁰ are each independently hydrogen or halogen, and R¹⁵ and R¹⁶ are each independently hydrogen, optionally substituted lower alkyl, lower alkenyl or cycloalkyl, or a pharmaceutically acceptable salt, or a solvate thereof.
9) The compound according to the above 1, 4 or 5, wherein Y is (i), ring A is (A4), (A5), (A6), or (A7), both of R⁷ and R⁸ are hydrogen, R¹¹ and R¹² are each independently hydrogen or lower alkyl, and R¹³ and R¹⁴ are each independently hydrogen, lower alkyl or lower alkoxycarbonyl, or a pharmaceutically acceptable salt, or a solvate thereof.

10) The compound according to the above 1, 4 or 5, wherein Y is (ii), ringAis (A1) or (A3), W is NR¹⁵R¹⁶, and R¹⁵ is optionally substituted lower alkyl, lower alkenyl or cycloalkyl, or a pharmaceutically acceptable salt, or a solvate thereof.
11) The compound according to the above 10, wherein ring A is (A1), and R⁷, R⁸, R⁹ and R¹⁰ are each independently hydrogen, halogen, lower alkyl, or lower alkoxy, or a pharmaceutically acceptable salt, or a solvate thereof.
12) The compound according to the above 1, 4 or 5, wherein Y is (ii), ring A is (A4), (A5) or (A⁷), both of R⁷ and R⁸ are hydrogen, R¹¹ and R¹² are each independently hydrogen or lower alkyl, and R²³ is hydrogen or lower alkoxycarbonyl, or a pharmaceutically acceptable salt, or a solvate thereof.

13) The compound according to the above 1, 4 or 5, wherein Y is (iii), ring A is (A1) or (A3), and W is NR¹⁵R¹⁶, or a pharmaceutically acceptable salt, or a solvate thereof.
14) The compound according to the above 13, wherein R^{F} is hydrogen, optionally substituted lower alkyl, optionally substituted lower alkoxy, optionally substituted cycloalkyl, cyano, optionally substituted amino, optionally substituted aryl, or optionally substituted heterocycle, or a pharmaceutically acceptable salt, or a solvate thereof.
15) The compound according to the above 13, wherein R^{F} is optionally substituted lower alkyl, or optionally substituted cycloalkyl, or a pharmaceutically acceptable salt, or a solvate thereof.
16) The compound according to the above 13, wherein R¹⁵ is hydrogen, optionally substituted lower alkyl, lower alkenyl, cycloalkyl, lower alkylcarbamoyl, lower alkylsulfonyl or a heterocycle, or a pharmaceutically acceptable salt, or a solvate thereof.
17) The compound according to the above 13, wherein R¹⁵ is lower alkyl, lower alkenyl or cycloalkyl, or a pharmaceutically acceptable salt, or a solvate thereof.
18) The compound according to the above 1, 4 or 5, wherein Y is (iii), ring A is(A2), (A4), (A5), (A6) or (A7), and R^{F} is optionally substituted lower alkyl, optionally substituted lower alkoxy, optionally substituted amino, optionally substituted cycloalkyl, or optionally substituted heterocycle, or a pharmaceutically acceptable salt, or a solvate thereof.
19) The compound according to the above 18, wherein ring A is (A4), all of R⁷, R⁸ and R¹³ are hydrogen, and R¹¹ and R¹² are each independently hydrogen or lower alkyl, or a pharmaceutically acceptable salt, or a solvate thereof.
20) The compound according to the above 18, wherein ring A is (A4), and R^{F} is optionally substituted lower alkyl, or optionally substituted cycloalkyl, or a pharmaceutically acceptable salt, or a solvate thereof.

21) The compound according to the above 1, 4 or 5, wherein Y is (iv), and ring A is (A1), (A4) or (A7), or a pharmaceutically acceptable salt, or a solvate thereof.
22) The compound according to the above 21, wherein ringAis (A1), and W is NR¹⁵R¹⁶, or a pharmaceutically acceptable salt, or a solvate thereof.
23) The compound according to the above 1, 4 or 5, wherein Y is (v), ring A is (A1) or (A4), and R^{F} is optionally substituted lower alkyl, or optionally substituted cycloalkyl, or a pharmaceutically acceptable salt, or a solvate thereof.
24) The compound according to the above 1, 4 or 5, wherein Y is (vi), ring A is (A1), (A3) or (A4), and R^{G} is optionally substituted lower alkyl, or optionally substituted aryl, or a pharmaceutically acceptable salt, or a solvate thereof.
25) The compound according to the above 24, wherein ring A is (A1), and W is NR¹⁵R¹⁶, or a pharmaceutically acceptable salt, or a solvate thereof.
26) The compound according to the above 1, 4 or 5, wherein Y is (vii), and ring A is (A1), or a pharmaceutically acceptable salt, or a solvate thereof.

27) The compound according to the above 1, wherein Y is (i), (ii), or (iii), R^{F} is optionally substituted lower alkyl, or optionally substituted cycloalkyl, 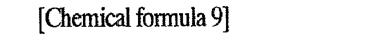 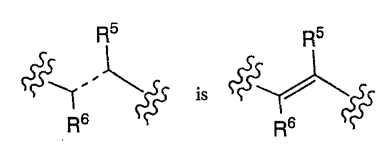 X is CR², X² is CR⁴, R¹ to R⁴ are each independently hydrogen, fluoro, methyl or methoxy (provided that the case where 3 or more selected from R¹ to R⁴ are hydrogen is excluded), ringAis (A1), and W is lower alkylamino, lower alkenylamino, cycloalkylamino, cycloalkyl(lower)alkylamino, or furyl(lower)alkyl optionally substituted with lower alkyl, or a pharmaceutically acceptable salt, or a solvate thereof.
28) The compound according to the above 1, wherein Y is (i), (iv), (v), or (vi), or Y is (ii) and p is 1, or Y is (iii) and R^{F} is optionally substituted lower alkyl, optionally substituted cycloalkyl, optionally substituted cycloalkenyl, optionally substituted aryl, or cyano, 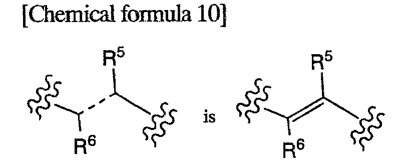 X¹ is CR², ring A is (A1), and W is NR¹⁵R¹⁶, or a pharmaceutically acceptable salt, or a solvate thereof.
29) The compound according to the above 1, wherein Y is (i), (ii), or (iii), R^{F} is optionally substituted lower alkyl, or optionally substituted cycloalkyl, 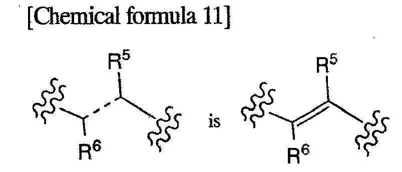 X¹ is CR², X² is CR⁴, R¹ to R⁴ are each independently hydrogen, fluoro, methyl or methoxy (provided that the case where 3 or more selected from R¹ to R⁴ are hydrogen is excluded), ring A is (A1), and W is lower alkylamino, lower alkenylamino, cycloalkylamino, cycloalkyl(lower)alkylamino, or furyl(lower)alkyl optionally substituted with lower alkyl, or a pharmaceutically acceptable salt, or a solvate thereof.
30) The compound according to the above 1, wherein Y is (i), (iv), (v) or (vi), or Y is (ii) and p is 1, or Y is (iii), and R^{F} is optionally substituted lower alkyl, optionally substituted cycloalkyl, optionally substituted cycloalkenyl, optionally substituted aryl, or cyano, 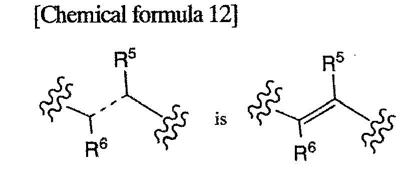 X¹ is CR², ring A is (A1), and W is NR¹⁵R¹⁶, or a pharmaceutically acceptable salt, or a solvate thereof.

31) A pharmaceutical composition comprising a compound as defined in any one of the above 1 to 30, or a pharmaceutically acceptable salt, or a solvate thereof.
32) A pharmaceutical composition for use as antibody production suppressor comprising a compound as defined in any one of the above 1 to 30, or a pharmaceutically acceptable salt, or a solvate thereof.
33) A pharmaceutical composition for use as dihydroorotate dehydrogenase inhibitor comprising a compound as defined in any one of the above 1 to 30, or a pharmaceutically acceptable salt, or a solvate thereof.
34) A pharmaceutical composition for use as immunosuppressing agent comprising a compound as defined in any one of the above 1 to 30, a pharmaceutically acceptable salt thereof or a solvate thereof,
35) A pharmaceutical composition for use as an and-allergic agent comprising a compound as defined in any one of the above 1 to 30, a pharmaceutically acceptable salt thereof or a solvate thereof,
36) A pharmaceutical composition for use as an and-cancer agent comprising a compound as defined in any one of the above 1 to 30, a pharmaceutically acceptable salt thereof or a solvate thereof.

As another embodiment, the present application provides a method of suppressing an immune reaction, a method of suppressing antibody production, as well as a method of treating and/or a method of preventing an allergic disease and/or a tumor, comprising administering the compound (I).
As a further another embodiment, the present application provides use of the compound (I) for producing a medicament for suppressing an immune reaction, suppressing antibody production, as well as treating and/or preventing an allergic disease and/or a tumor.

### Effect of The Invention

The compound of the present invention exhibits the strong antibody production suppressing activity and/or DHODH inhibitory activity, and is useful as an immunosuppressing agent, an anti-allergic agent and/or an anti-tumor agent.

### Best Mode for Carrying out The Invention

As used herein, the "halogen" includes fluorine, chlorine, bromine and iodine. Particularly, fluorine or chlorine is preferable.
The "lower alkyl" includes straight or branched alkyl of a carbon number of 1 to 10, preferably a carbon number of 1 to 6, further preferably a carbon number of 1 to 3, and examples include methyl, ethyl, n-propyl, isopropyl, n-butyl, isobutyl, sec-butyl, tert-butyl, n-pentyl, isopentyl, neapentyl, hexyl, isohexyl, n-heptyl, isoheptyl, n-octyl, isooctyl, n-nonyl and n-decyl.

Examples of a substituent of the "optionally substituted lower alkyl" include halogen; hydroxy; lower alkoxy; lower alkenyloxy; lower alkenylthio; lower alkynyloxy; lower alkynylthio; acyl; acyloxy; carboxy; lower alkoxycarbonyl; mercapto; lower alkylthio; amino; lower alkylamino; acylamino; lower alkoxycarbonylamino; amidino; hydroxyamidino; imino; carbamoyl; lower alkylcarbamoyl; arylcarbamoyl; thiocarbamoyl; lower alkylthiocarbamoyl; arylthiocarbamoyl; cycloalkyl optionally substituted with lower alkyl or lower alkoxy; cycloalkenyl optionally substituted with lower alkyl or lower alkoxy; cyano; nitro; lower alkylsulfonyloxy; arylsulfonyloxy; phenyl optionally substituted with one or more groups selected from the group of halogen, hydroxy, lower alkyl, lower alkoxy, acyl, carboxy and lower alkoxycarbonyl; and heterocycle (particularly, furan ring, thiophene ring, tetrahydropyran ring, dioxane ring etc.) which may be substituted with one or more groups selected from halogen, hydroxy, lower alkyl, lower alkoxy, acyl, carboxy and lower alkoxycarbonyl, and may be fused with a benzene ring. They are preferably halogen; hydroxy; acyl; carboxy; amino; acylamino; lower alkoxycarbonylamino; hydroxyamidino; lower alkylcarbamoyl; cycloalkyl; cyano; phenyl optionally substituted with hydroxy; and heterocycle optionally substituted with lower alkyl. An arbitrary position may be substituted with one or more these substituents.
Preferable examples of a substituent of the "optionally substituted lower alkyl" in R^{F} include one or two groups selected from the group of hydroxy, phenyl, acylamino, lower alkoxycarbonylamino, acyl, cyano and hydroxyamidino.

A lower alkyl part of the "lower alkoxy", the "lower alkoxycarbonyl", the "lower alkoxycarbonylamino", the "lower alkylsulfonyl", the "lower alkylsulfonyloxy", the "lower alkylthio", the "lower alkylamino", the "lower alkylcarbamoyl", the "lower alkylthiocarbamoyl", the "lower alkylsulfamoyl", the "aryl(lower)alkyl", the "lower alkoxy(lower)alkyl" and the "lower alkylenedioxy" is the same as the aforementioned "lower alkyl".
A substituent of the "optionally substituted lower alkoxy", the "optionally substituted lower alkoxycarbonyl", the "optionally substituted lower alkylsulfonyl" and the "optionally substituted lower alkylsulfonyloxy" is the same as the aforementioned substituent of the "optionally substituted lower alkyl".

The "lower alkenyl" includes straight or branched alkenyl of a carbon number of 2 to 10, preferably a carbon number of 2 to 8, further preferably a carbon number of 3 to 6, having one or more double bonds at an arbitrary position. Specifically, the "lower alkenyl" includes vinyl, propenyl, isopropenyl, butenyl, isobutenyl, prenyl, butadienyl, pentenyl, isopentenyl, pentadienyl, hexenyl, isohexenyl, hexadienyl, heptenyl, octenyl, nonenyl and decenyl.
A substituent of the "optionally substituted lower alkenyl" is the same as the aforementioned substituent of the "optionally substituted lower alkyl". An unsubstituted lower alkenyl is preferable.
A lower alkenyl part of the "lower alkenyloxy", the "lower alkenylthio" and the "lower alkenyloxycarbonyl" is the same as the aforementioned "lower alkenyl".

The "lower alkynyl" includes straight or branched alkynyl of a carbon number of 2 to 10, preferably a carbon number of 2 to 8, further preferably a carbon number of 3 to 6, and specific examples include ethynyl, propynyl, butynyl, pentynyl, hexynyl, heptynyl, octynyl, nonynyl and decynyl. These have one or more triple bonds at an arbitrary position, and may further have a double bond.
A lower alkynyl part of the "lower alkynyloxy" and the "lower alkynylthio" is the same as the aforementioned "lower alkynyl".

The "acyl" includes straight or branched chain aliphatic acyl of a carbon number of 1 to 10, preferably a carbon number of 1 to 6, further preferably a carbon number of 1 to 4, and cyclic aliphatic acyl of a carbon number of 4 to 9, preferably a carbon number of 4 to 7, and aroyl. Specific examples include formyl, acetyl, propionyl, butyryl, isobutyryl, valeryl, pivaloyl, hexanoyl, acryloyl, propioloyl, methacryloyl, crotonoyl, cyclopropylcarbonyl, cyclohexylcarbonyl, cyclooctylcarbonyl and benzoyl, and preferably acetyl.
A substituent of the "optionally substituted acryl" is the same as the aforementioned substituent of the "optionally substituted lower alkyl", and aroyl may have lower alkyl as a substituent. Preferable is unsubstituted acyl.
An acyl part of the "acylamino" and the "acyloxy" is the same as the aforementioned "acyl".

The "cycloalkyl" is a carbocycle of a carbon number of 3 to 8, preferably a carbon number of 3 to 6, and includes, for example, cyclopropyl, cyclobutyl, cyclopentyl, cyclohexyl, cycloheptyl and cyclooctyl.
Examples of a substituent of the "optionally substituted cycloalkyl" include lower alkyl, and the same substituent as the aforementioned substituent of the "optionally substituted lower alkyl", and one or more arbitrary positions may be substituted. Preferable are lower alkyl, halogen and hydroxy.
The "cycloalkenyl" includes a group having one or more double bonds at an arbitrary position in the aforementioned cycloalkyl ring, and specific examples include cyclopropenyl, cyclobutenyl, cyclopentenyl, cyclohexenyl, cycloheptynyl, cyclooctynyl and cyclohexadienyl.
A substituent of the "optionally substituted cycloalkenyl" is the same as the aforementioned substituent of the "optionally substituted cycloalkyl".

Examples of a substituent of the "optionally substituted amino" include hydroxy; lower alkyl; lower alkenyl; lower alkynyl; acyl; cycloalkyl; carbamoyl; lower alkylcarbamoyl; aryl optionally substituted with lower alkyl, carboxy, acyl or lower alkoxycarbonyl; sulfamoyl; lower alkylsulfamoyl; lower alkoxycarbonyl; lower alkylsulfonyl; and cyano. Preferable are lower alkyl, acyl, carbamoyl, lower alkoxycarbonyl and lower alkylsulfonyl.
A substituent of the "optionally substituted amidino" is the same as the aforementioned substituent of the "optionally substituted amino". Preferable are unsubstituted amidino, hydroxy-substituted amidino and cyano-substituted amidino.
The "optionally substituted carbamoyl" includes carbamoyl optionally substituted with lower alkyl, lower alkenyl, lower alkynyl, cycloalkyl, aryl or the like.

The "aryl" includes phenyl, naphthyl, anthryl, phenanthryl and indenyl, and phenyl is particularly preferable.
Examples of a substituent of the "optionally substituted aryl" include halogen; hydroxy; lower alkyl, lower alkoxy, lower alkylthio, lower alkenyl, lower alkenyloxy, lower alkenylthio, lower alkynyl, lower alkynyloxy, lower alkynylthio, cycloalkyl, cycloalkyloxy, acyl, acyloxy, lower alkoxycarbonyl, lower alkenyloxycarbonyl, acyl, amino, lower alkylsulfonyl, lower alkylsulfonyloxy, each being optionally substituted with one or more groups selected from a substituent group α described later, carboxy; amidino; guanidino; nitro; arylsulfonyl optionally substituted with one or more groups selected from a substituent group α and/or lower alkyl; arylsulfonyloxy optionally substituted with one or more groups selected from a substituent group α and/or lower alkyl; aryl optionally substituted with one or more groups selected from a substituent group α and/or lower alkyl; heterocycle optionally substituted with one or more groups selected from a substituent group α and/or lower alkyl; and lower alkylenedioxy, and one or more arbitrary positions may be substituted with these substituents. Preferable are unsubstituted phenyl and amino phenyl.
Herein, the substituent group α is a group consisting of halogen, hydroxy, lower alkoxy, acyl, carboxy, lower alkoxycarbonyl, cycloalkyl and phenyl.
An aryl part of the "arylsulfonyl", the "arylsulfonyloxy", the "aryl(lower)alkyl", the "arylcarbamoyl" and the "arylthiocarbamoyl" is the same as the aforementioned "aryl".
A substituent of the "optionally substituted arylsulfonyl" is the same as the aforementioned substituent of the "optionally substituted aryl".

The "heterocycle" includes a 5-membered or 6-membered heterocycle having one or more heteroatoms optionally selected from O, S and N in a ring, and specific examples include aromatic heterocycles such as a pyrrole ring, an imidazole ring, a pyrazole ring, a pyridine ring (4-pyridyl etc.), a pyridazine ring, a pyrimidine ring, a pyrazine ring, a triazole ring, a tetrazole ring, a triazine ring, an isoxazole ring, an oxazole ring, an oxadiazole ring, an isothiazole ring, a thiazole ring, a thiadiazole ring, a furan ring (2-furyl, 3-furyl etc.) and a thiophene ring (3-thienyl etc.), and alicyclic heterocycles such as a tetrahydropyran ring, a dihydropyridine ring (1,2-dihydropyridyl etc.), a dihydropyridazine ring (2,3-dihydropyridazinyl etc.), a dihydropyrazine ring (1,2-dihydropyrazinyl etc.), a dioxane ring, an oxathiolane ring, a thiane ring, a pyrrolidine ring, a pyrroline ring, an imidazolidine ring, an imidazoline ring, a pyrazolidine ring, a pyrazoline ring, a piperidine ring, a piperazine ring and a morpholine ring.
A substituent of the "optionally substituted heterocycle" is the same as the aforementioned substituent of the "optionally substituted aryl", and preferable are lower alkyl, lower alkoxy(lower)alkyl and carboxy

"R^{C} and R^{D} may be taken together to form a carbocycle containing an adjacent carbon atom" includes the case where R^{C} and R^{D} are taken together with a carbon atom to which they bind, to form cyclopropane, cyclobutane, cyclopentane, cyclohexane, cycloheptane or the like.
When a broken line indicates a double bond in the formula (I), R⁵ and R⁶ may take any configuration of cis and trans.

In the present specification, the "compound (I)" includes a pharmaceutically acceptable salt of each compound, which can be produced. Examples of the "pharmaceutically acceptable salt" include salts of mineral acids such as hydrochloric acid, sulfuric acid, nitric acid, phosphoric acid, hydrofluoric acid, hydrobromic acid and the like; salts of organic acids such as formic acid, acetic acid, tartaric acid, lactic acid, citric acid, fumaric acid, maleic acid, succinic acid and the like; salts of organic bases such as ammonium, trimethylammonium, triethylammonium and the like; salts of alkaline metals such as sodium, potassium and the like, and the salts of alkaline earth metals such as calcium, magnesium and the like.
The compound of the present invention includes a solvate (preferably, hydrate) thereof. Examples of the solvate include solvates with an organic solvent and/or water. When a hydrate is formed, an arbitrary number of water molecules may be coordinated.
The present compound includes all isomers (keto-enol isomer, diastereoisomer, optical isomer, rotation isomer etc.) thereof.

A process for producing the compound (I) will be explained below.

### Process A

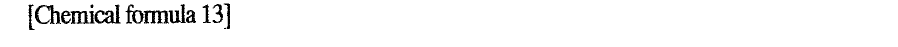

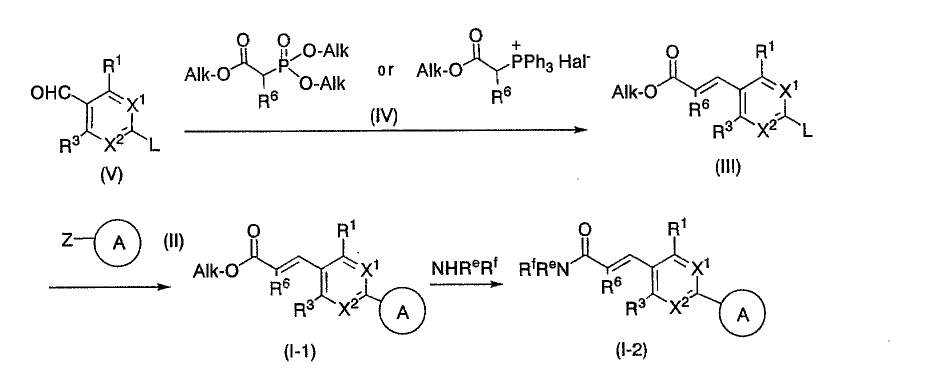

wherein Alks are each independently lower alkyl, Hal is halogen, NR^{e}R^{f} is 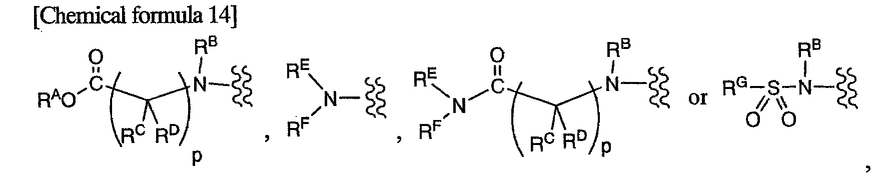 one of Land Z is dihydroxyboryl, di-(lower)alkylboryl or di-(lower)alkoxyboryl, and the other is halogen or -OSO₂(C_{q}F_{2q+1})(q is an integer of 1 to 4), and other symbols are as defined above.

### (First step)

Compound (V) which is the known compound or obtained from the known compound by a conventional method, and phosphonic acid ester or triphenylphosphonium salt represented by the formula (IV) are reacted to obtain Compound (III).
The present reaction may be performed according to the condition of the Wittig reaction or the Homer-Emmons reaction. For example, those compounds are reacted at about -80 °C to about 100°C, preferably about -20°C to about 30°C for about 5 minutes to about 24 hours, preferably about 0.5 hour to about 2 hours in the presence of a base (e.g. sodium hydride, alkyllithium, KOt-Bu, lithiumhexamethyldisilazane etc.) in an appropriate solvent (e.g. N, N-dimethylformamide, dimethyl sulfoxide, tetrahydrofuran, toluene etc.) to obtain the objective Compound (III).

### (Second step)

The resulting Compound (ID) is reacted with Compound (II) to obtain Compound (1-i).
The present reaction may be performed according to the usual condition of the Suzuki reaction. For example, Compound (III) and Compound (II) are reacted at room temperature to under heating, preferably at about 0°C to about 180°C for about 5 minutes to about 48 hours, preferably about 0.5 hour to about 18 hours in a mixed system of an appropriate solvent (e.g. benzene, toluene, N, N-dimethylformamide, dimethoxyethane, tetrahydrofuran, dioxane, ethanol or methanol) and water, or in a non-aqueous system in the presence of a palladium catalyst (e.g. Pd(PPh₃)₄, PdCl₂(PPh₃)₂, PdCl₂(OAc)₂, PdCl₂(CH₃CN)₂ or the like, preferably Pd(PPh₃)₄), under the basic condition (as a base, for example, K₃PO₄, NaHCO₃, N aOEt, Na₂CO₃, K₂CO₃, Ba(OH)₂, Cs₂CO₃, CsF, NaOH, Ag₂CO₃ or the like) to obtain the present Compound (I-i, R^{A}= lower alkyl).
As one of substituents L and Z in compounds which are to be reacted, any substituent may be used as far as it is a boryl group which is applicable to the Suzuki reaction ( Chemical Communication 1979, 866, J, Synth. Org. Chem. Jpn, 1993, vol.51, No.11, p.91-p.100), preferably dihydroxyboryl. The other may be any substituent as far as it is a leaving group which is applicable to the Suzuki reaction and, for example, halogen or OSO₂(C_{q}F_{2q+1}) (wherein q is an integer of 1 to 4) can be used. Particularly, halogen or trifluoromethanesulfonyloxy is preferable, most preferably bromine, iodine or OTf.
A substituent other than L and Z may be any substituent as far as it is a group which does not adversely influence on the Suzuki reaction, for example, a group other than halogen and -OSO₂(C_{q}F_{2q+1}) (wherein q is an integer of 1 to 4).
Even when a substituent other than L and Z is a halogen, if reactivity of a substituent L with a substituent Z is higher than that of the halogen with L or Z, the present reaction can be proceeded without any disorder.
In addition, even when a substituent other than L and Z is hydroxy, the reaction is possible, and in that case, preferably, the hydroxy is protected with an ordinary hydroxy protecting group (e.g. methoxymethyl, benzyl, t-butyldimethylsilyl, methanesulfonyl, p-toluenesulfonyl or the like), and the protected compound is subjected to the reaction and, thereafter, an usual deprotecting reaction is performed.
Thus obtained present compound (I-1, R^{A}=lower alkyl) is hydrolyzed by a conventional method to obtain the present Compound (I-1, R^{A}=hydrogen).

### (Third step)

The present Compound (I-2) is obtained by amidation of the resulting Compound (I-1).
The present reaction may be performed at about -20°C to about 100°C, preferably about -5°C to about 30°C for about 1 minute to 24 hours, preferably about 0.5 hour to 2 hours in an appropriate solvent (e.g. dichloromethane, diethyl ether, tetrahydrofuran, acetonitrile, N, N-dimethylformamide, dimethyl sulfoxide, ethyl acetate, dioxane etc.) in the presence of a base (triethylamine, pyridine, N-methylmorpholine etc.) and a condensing agent (diethylphosphoric acid cyanide, N, N'-dicyclohexylcarbodiimide, N-dimethylaminopropyl-N'-ethylcarbodiimide, diethylphosphoric acid cyanide diphenylphosphoric acid azide, diisopropylcarbodiimide etc.).

### Process B

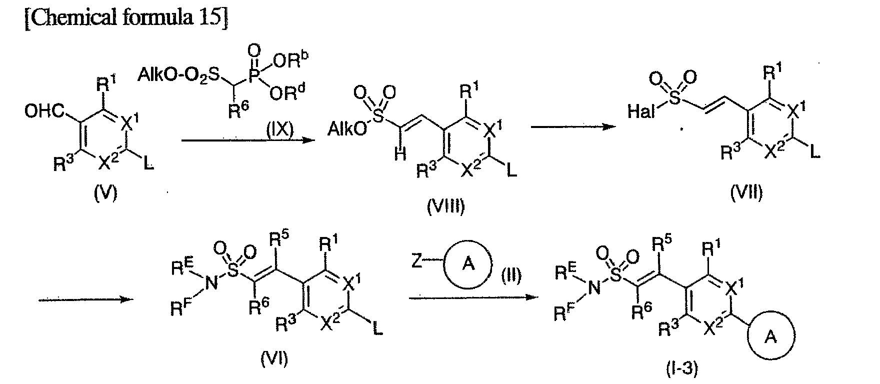

wherein each symbol is as defined above

### (First step)

Compound (V) which is the known compound, or obtained from the known compound by a conventional method is reacted with phosphonic acid ester (IX) as in the Process A first step, thereby, a compound represented by the formula (VIII) can be obtained.

### (Second step)

Thus obtained Compound (VIII) is halogenated by a conventional method to obtain a compound represented by the formula (VII).
In the present reaction, Compound (VII) may be reacted at about -20°C to about 100°C, preferably about -5°C to about 30°C for about 1 minute to 24 hours, preferably about 0.5 hour to about 2 hours in an appropriate solvent (e.g. N, N-dimethylformamide, dimethyl sulfoxide, dichloromethane, diethyl ether, tetrahydrofuran, acetonitrile, ethyl acetate, dioxane, acetone etc.) using a halogenating agent which is usually used (e.g. thionyl halide such as thionyl chloride, thionyl bromide etc., sulfuryl halide such as sulfuryl chloride etc., phosphoryl halide such as phosphoryl chloride, phosphoryl bromide etc., phosphorus halide such as phosphorus pentachloride, phosphorus trichloride, phosphorus pentabromide, phosphorus tribromide, etc., phosgene, oxalyl halide such as oxalyl chloride etc., triphenylphosphine/carbon tetrachloride, triphenylphosphine/carbon tetrabromide, triphenylphosphine/sulfuryl halide, or halogen such as bromine, chlorine, iodine etc.). If necessary, a phase transfer catalyst such as tetrabutylammonium halide and the like may be used.

### (Third step)

The resulting Compound (VII) can be amidated as in the Process A third step to obtain Compound (VI).

### (Fourth step)

The resulting Compound (VI) can be subjected to the Suzuki reaction as in the Process A second step to obtain objective Compound (I-3).

### Process C

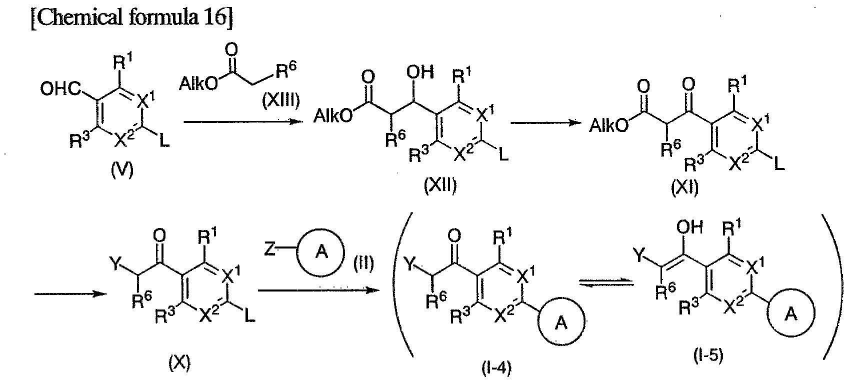

wherein respective symbols are as defined above

### (First step)

Compound (V) which is the known compound or is obtained from the known compound by a conventional method is reacted with Compound (XIII) at -80°C to room temperature, preferably about -80°C to about 10°C for 5 minutes to 24 hours, preferably 0.5 hour to 2 hours in an appropriate solvent (e.g. tetrahydrofuran, N, N-dimethylformamide, diethyl ether, hexane etc.) in the presence of a base (e.g. lithium diisopropylamide, lithium bistrimethylsilylamide, lithium 2,2,6,6-tetrahydromethylpiperide, butyl lithium, potassium t-butoxide, sodium hydride, sodium amide etc.) to obtain Compound (XII).

### (Second step)

The resulting Compound (XII) is oxidized to obtain Compound (XI). For example, when the compound is oxidized by a conventional method such as Swem oxidation, Jones oxidation and the like, objective Compound (XI) is obtained.

### (Third step)

A substituent of the resulting Compound (XI) is converted into a substituent Y by the known method.
For example, when an objective substituent Y is a group represented by (ii), (iii) or (iv), Compound (XI) may be reacted with an amino compound having an objective substituent. The reaction may be performed at about -40°C to about 150°C, preferably about 100 to about 150°C for 5 minutes to 48 hours, preferably 6 hours to 24 hours in an appropriate solvent (toluene, diethyl ether, tetrahydrofuran, acetonitrile, N, N-dimethylformamide, dimethyl sulfoxide, dichloromethane, ethyl acetate, xylene, ethanol, methanol or water), or without solvent, in the presence of a base (pyridine, triethylamine, dimethylaminopyridine, potassium carbonate, sodium carbonate, potassium bicarbonate, sodium bicarbonate etc.).

### (Fourth step)

The resulting Compound (X) is reacted as in the Process A second step to obtain objective Compound (I-4) or (I-5).

### Process D

A compound in wherein a broken line is a single bond can be obtained by hydrogenation of Compound (I) wherein a broken line is a double bond and which can be obtained by the aforementioned process, in an appropriate solvent (e.g. methanol, ethanol, ethyl acetate, tetrahydrofuran etc.) using a catalyst such as pafladium-carbon, palladium hydroxide-carbon, platinum-carbon, rhodium-carbon, ruthenium-carbon and the like.

### Process E

A compound wherein a broken line is a triple bond can be obtained by the following process. 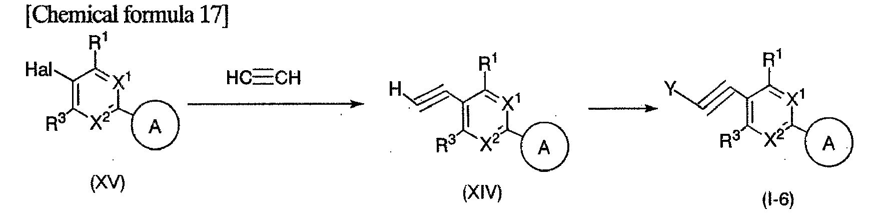 wherein respective symbols are as defined above.
First, Compound (XVI) which is the known compound or obtained from the known compound by a conventional method, and acetylene are subjected to an alkylating reaction by a conventional method, to obtain Compound (XV). For example, the compound is reacted at about -80°C to about 100°C, preferably about -20°C to about 30°C about for 5 minutes to about 24 hours, preferably about 0.5 hour to about 2 hours in an appropriate solvent (e.g. N, N-dimethylformamide, dimethyl sulfoxide, tetrahydrofuran, toluene, dioxane, N-methylpyrrolidone etc.) in the presence of a base (e.g. triethylamine, pyridine, N-methylmorpholine, piperidine, dialkylamine, monoalkylamine etc.) to obtain objective Compound (XIV).
Thus obtained Compound (XIV) can be subjected to the known substituent introducing reaction to obtain objective Compound (I-6).
As acetylene, trimethylsilylacetylene may be used so that operation becomes simple, or acetylene substituted with a substituent which dose not become a disorder of the first step may be used so that a reaction of introducing a substituent in the second step becomes simple.

Among the present compounds, particularly, the following compounds are preferable.
1) a compound wherein X¹ is CR^{2,} and X² is CR^{4,}
2) a compound wherein R¹ to R⁴ are each independently hydrogen, halogen, lower alkyl or lower alkoxy (provided that, all of R¹ to R⁴ are not simultaneously hydrogen) (hereinafter, R¹ to R⁴ are R-a),
   a compound wherein R¹ to R⁴ are each independently hydrogen, fluoro, chloro, methyl or methoxy (provided that all of R¹ to R⁴ are not simultaneously hydrogen),(hereinafter, R¹ to R⁴ are R-b),
   a compound wherein R¹ to R⁴ are each independently halogen, lower alkyl or lower alkoxy (hereinafter, R¹ to R⁴ are R-c),
   a compound wherein two or more groups of R¹ to R⁴ are halogen, lower alkyl or lower alkoxy, and other groups are hydrogen (hereinafter, R¹ to R⁴ are R-d),
   a compound wherein two or more groups of R¹ to R⁴ are fluoro, chloro, methyl or methoxy, and other groups are hydrogen (hereinafter, R¹ to R⁴ are R-e),
3) a compound wherein R⁵ and R⁶ are each independently hydrogen, halogen or lower alkyl (hereinafter, R⁵ and R⁶ are R-f),
   a compound wherein R⁵ and R⁶ are each independently hydrogen, fluoro or C1 to C3 alkyl (hereinafter, R⁵ and R⁶ are R-g),
   a compound wherein R⁵ is hydrogen, and R⁶ is lower alkyl or fluoro (hereinafter, R⁵ and R⁶ are R-h),

4) a compound wherein Y is (i), and R^{A} is hydrogen (hereinafter, Y is Y-a),
   a compound wherein Y is (ii), and all of R^{A} R^{B}, R^{C} and R^{D} are hydrogen (hereinafter, Y is Y-b),
   a compound wherein Y is (ii), both of R^{A} and R^{B} are hydrogen, and one of R^{C} and R^{D} is C1-C3 alkyl, and the other is hydrogen (hereinafter, Y is Y-c),

a compound wherein Y is (iii), R^{E} is hydrogen, and R^{F} is hydrogen, a lower alkyl, cycloalkyl, cyano or phenyl, (hereinafter, Y is Y-d),
a compound wherein Y is (iii), R^{E} is hydrogen, and R^{F} is hydrogen, lower alkyl or cycloalkyl (hereinafter, Y is Y-e),
a compound wherein Y is (iii), R^{E} is hydrogen, and R^{F} is hydrogen, C3-C6 alkyl, cyclopropyl, cyclopentyl or cyclohexyl (Y is Y-f),

a compound wherein Y is (iv), R^{B} is hydrogen, both of R^{C} and R^{D} are hydrogen, or one of R^{C} and
R^{D} is methyl, and the other is hydrogen, R^{E} is hydrogen, and R^{F} is hydrogen, lower alkyl or cycloalkyl (hereinafter, Y is Y-g),
a compound wherein Y is (v), R^{E} is hydrogen, and R^{F} is hydrogen, lower alkyl or cycloalkyl (hereinafter, Y is Y-h),
a compound wherein Y is (vi), R^{B} is hydrogen, and R^{G} is hydrogen, lower alkyl, cycloalkyl or phenyl (hereinafter, Y is Y-i),
a compound wherein Y is (vii), and R^{J} is lower alkoxycarbonyl, lower alkyl or hydroxy(lower)alkyl (hereinafter, Y is Y-j),

5) a compound wherein ring A is a group represented by A1, R⁷, R⁸, R⁹ and R¹⁰ are each independently hydrogen, halogen, lower alkyl or lower alkoxy, W is NR¹⁵R¹⁶, R¹⁵ is lower alkyl, cycloalkyl(lower)alkyl optionally substituted with lower alkyl, heterocyclic(lower)alkyl optionally substituted with lower alkyl, aryl(lower)alkyl optionally substituted with lower alkyl, cycloalkyl optionally substituted with lower alkyl, or lower alkenyl, and R¹⁶ is hydrogen (hereinafter, ringAis A-a),
   a compound wherein ring A is A1, R⁷, R⁸, R⁹ and R¹⁰ are each independently hydrogen or halogen, W is NR¹⁵R¹⁶, R¹⁵ is lower alkyl, cycloalkyl(lower)alkyl, lower alkylcycloalkyl(lower)alkyl, heterocyclic(lower)alkyl, lower alkyl heterocyclic(lower)alkyl, aryl(lower)alkyl, lower alkyl aryl(lower)alkyl, cycloalkyl, lower alkyl cycloalkyl or lower alkenyl, and R¹⁶ is hydrogen (hereinafter, ring A is A-b),
   a compound wherein ring A is A1, R⁷, R⁸, R⁹ and R¹⁰ are each independently hydrogen or halogen, W is NR¹⁵R¹⁶, R¹⁵ is C3-C6 alkyl, cycloalkyl(C1-C3)alkyl, furyl(C1-C3)alkyl, C1-C3 alkyl furyl(C1-C3)alkyl, thienyl(C1-C3)alkyl, C1-C3 alkyl thienyl(C1-C3)alkyl, phenyl(C1-C3)alkyl, cyclopentyl, cyclohexyl or C3-C6 alkenyl, and R¹⁶ is hydrogen (hereinafter, ring A is A-c),

a compound wherein ring A is a group represented by A2, X³ is S or NH, and R⁷ and R⁸ are both hydrogen (hereinafter, ring A is A-d),
a compound wherein ring A is a group represented by A3, X⁷ is N, X⁴, X⁵ and X⁶ are CR⁷, CR⁸ and CR⁹, respectively (hereinafter, ring A is A-e),
a compound wherein ring A is a group represented by A3, X⁷ is N, X⁴, X⁵ and X⁶ are CR⁷, CR⁸ and CR⁹, respectively and R⁷, R⁸ and R⁹ are each independently hydrogen, halogen, hydroxy or lower alkyl (hereinafter, ring A is A-f),
a compound wherein ring A is a group represented by A3, X⁷ is N, and all of X⁴, X⁵ and X⁶ are CH (hereinafter, ring A is A-g),
a compound wherein ring A is a group represented by A4, R⁷, R⁸, R¹¹ and R¹² are each independently hydrogen, halogen, hydroxy or lower alkyl, and R¹³ is hydrogen, lower alkyl or lower alkoxycarbonyl (hereinafter, ring A is A-h),
a compound wherein ringAis a group represented byA4, and R⁷, R⁸, R¹¹, R¹² and R¹³ are each independently hydrogen or lower alkyl (hereinafter, ring A is A-i),
a compound wherein ring A is a group represented by A4, and all of R⁷, R⁸, R¹¹, R¹² and R¹³ are hydrogen (hereinafter, ring A is A-j),
a compound wherein ring A is a group represented by A5, R⁷, R⁸, R¹¹ and R¹² are each independently hydrogen, halogen, hydroxy or lower alkyl, and R¹³ is hydrogen, a lower alkyl or lower alkoxycarbonyl (hereinafter, ring A is A-k),
a compound wherein ring A is a group represented by A5, R⁷, R⁸, R¹¹ and R¹² are hydrogen, and R¹³ is hydrogen or lower alkoxycarbonyl (hereinafter, ring A is A-1),
a compound wherein ring A is a group represented by A5, and all of R⁷, R⁸, R¹¹, R¹² and R¹³ are hydrogen (hereinafter, ring A is A-m),

a compound wherein ring A is a group represented by A6, and R⁷, R⁸, R¹¹ and R¹² are each independently hydrogen, halogen, hydroxy or lower alkyl (hereinafter, ring A is A-n),
a compound wherein ring A is a group represented by A6, and all of R⁷, R⁸, R¹¹ and R¹² are hydrogen (hereinafter, ring A is A-o),
a compound wherein ring A is a group represented by A7, and R⁷, R⁸, R¹² and R¹³ are each independently hydrogen or lower alkyl (hereinafter, ring A is A-p),
a compound wherein ring A is a group represented by A7, and all of R⁷, R⁸, R¹² and R¹³ are hydrogen (hereinafter, ring A is A-q),

a compound wherein X¹ is CR², X² is CR⁴, and a combination of R¹ to R⁴, R⁵ and R⁶, Y, and ring A (R¹-R⁴, R⁵ and R⁶, Y, ring A) is as follows.
(R¹-R⁴, R⁵ and R⁶, Y, ring A) =
(R-a,R-f,Y-a,A-a),(R-a,R-f,Y-a,A-b),(R-a,R-f,Y-a,A-d),(R-a,R-f,Y-a,A-e),(R-a,R-f,Y-a,A-f),(R-a,R-f,Y-a,A-h),(R-a,R-f,Y-a,A-i),(R-a,R-f,Y-a,A-k),(R-a,R-f,Y-a,A-l),(R-a,R-f,Y-a,A-n),(R-a,R-f,Y-a,A -p),(R-a,R-f,Y-b,A-a),(R-a,R-f,Y-b,A-b),(R-a,R-f,Y-b,A-d),(R-a,R-f,Y-b,A-e),(R-a,R-f,Y-b,A-f),(R-a,R-f,Y-b,A-h),(R-a,R-f,Y-b,A-i),(R-a,R-f,Y-b,A-k),(R-a,R-f,Y-b,A-l),(R-a,R-f,Y-b,A-n),(R-a,R-f,Y -b,A-p),(R-a,R-f,Y-c,A-a),(R-a,R-f,Y-c,A-b),(R-a,R-f,Y-c,A-d),(R-a,R-f,Y-c,A-e),(R-a,R-f,Y-c,A-f) ,(R-a,R-f,Y-c,A-h),(R-a,R-f,Y-c,A-i),(R-a,R-f,Y-c,A-k),(R-a,R-f,Y-c,A-l),(R-a,R-f,Y-c,A-n),(R-a,R -f,Y-c,A-p),(R-a,R-f,Y-d,A-a),(R-a,R-f,Y-d,A-b),(R-a,R-f,Y-d,A-d),(R-a,R-f,Y-d,A-e),(R-a,R-f,Y-d, A-f),(R-a,R-f,Y-d,A-h),(R-a,R-f,Y-d,A-i),(R-a,R-f,Y-d,A-k),(R-a,R-f,Y-d,A-l),(R-a,R-f,Y-d,A-n),( R-a,R-f,Y-d,A-p),(R-a,R-f,Y-e,A-a),(R-a,R-f,Y-e,A-b),(R-a,R-f,Y-e,A-d),(R-a,R-f,Y-e,A-e),(R-a,R-f,Y-e,A-f),(R-a,R-f,Y-e,A-h),(R-a,R-f,Y-e,A-i),(R-a,R-f,Y-e,A-k),(R-a,R-f,Y-e,A-l),(R-a,R-f,Y-e,An),(R-a,R-f,Y-e,A-p),(R-a,R-f,Y-f,A-a),(R-a,R-f,Y-f,A-b),(R-a,R-f,Y-f,A-d),(R-a,R-f,Y-f,A-e),(R-a, R-f,Y-f,A-f),(R-a,R-f,Y-f,A-h),(R-a,R-f,Y-f,A-i),(R-a,R-f,Y-f,A-k),(R-a,R-f,Y-f,A-l),(R-a,R-f,Y-f,A -n),(R-a,R-f,Y-f,A-p),(R-a,R-f,Y-g,A-a),(R-a,R-f,Y-g,A-b),(R-a,R-f,Y-g,A-d),(R-a,R-f,Y-g,A-e),(R-a,R-f,Y-g,A-f),(R-a,R-f,Y-g,A-h),(R-a,R-f,Y-g,A-i),(R-a,R-f,Y-g,A-k),(R-a,R-f,Y-g,A-l),(R-a,R-f,Y -g,A-n),(R-a,R-f,Y-g,A-p),(R-a,R-f,Y-h,A-a),(R-a,R-f,Y-h,A-b),(R-a,R-f,Y-h,A-d),(R-a,R-f,Y-h,A-e),(R-a,R-f,Y-h,A-f),(R-a,R-f,Y-h,A-h),(R-a,R-f,Y-h,A-i),(R-a,R-f,Y-h,A-k),(R-a,R-f,Y-h,A-l),(R-a, R-f,Y-h,A-n),(R-a,R-f,Y-h,A-p),(R-a,R-f,Y-i,A-a),(R-a,R-f,Y-i,A-b),(R-a,R-f,Y-i,A-d),(R-a,R-f,Y-i, A-e),(R-a,R-f,Y-i,A-f),(R-a,R-f,Y-i,A-h),(R-a,R-f,Y-i,A-i),(R-a,R-f,Y-i,A-k),(R-a,R-f,Y-i,A-l),(R-a, R-f,Y-i,A-n),(R-a,R-f,Y-i,A-p),(R-a,R-f,Y-k,A-a),(R-a,R-f,Y-k,A-b),(R-a,R-f,Y-k,A-d),(R-a,R-f,Y-k,A-e),(R-a,R-f,Y-k,A-f),(R-a,R-f,Y-k,A-h),(R-a,R-f,Y-k,A-i),(R-a,R-f,Y-k,A-k),(R-a,R-f,Y-k,A-l), (R-a,R-f,Y-k,A-n),(R-a,R-f,Y-k,A-p),(R-a,R-f,Y-l,A-a),(R-a,R-f,Y-l,A-b),(R-a,R-f,Y-l,A-d),(R-a,R-f,Y-l,A-e),(R-a,R-f,Y-l,A-f),(R-a,R-f,Y-l,A-h),(R-a,R-f,Y-l,A-i),(R-a,R-f,Y-l,A-k),(R-a,R-f,Y-l,A-l), (R-a,R-f,Y-l,A-n),(R-a,R-f,Y-l,A-p),(R-a,R-g,Y-a,A-a),(R-a,R-g,Y-a,A-b),(R-a,R-g,Y-a,A-d),(R-a, R-g,Y-a,A-e),(R-a,R-g,Y-a,A-f),(R-a,R-g,Y-a,A-h),(R-a,R-g,Y-a,A-i),(R-a,R-g,Y-a,A-k),(R-a,R-g, Y-a,A-l),(R-a,R-g,Y-a,A-n),(R-a,R-g,Y-a,A-p),(R-a,R-g,Y-b,A-a),(R-a,R-g,Y-b,A-b),(R-a,R-g,Y-b, A-d),(R-a,R-g,Y-b,A-e),(R-a,R-g,Y-b,A-f),(R-a,R-g,Y-b,A-h),(R-a,R-g,Y-b,A-i),(R-a,R-g,Y-b,A-k) ,(R-a,R-g,Y-b,A-l),(R-a,R-g,Y-b,A-n),(R-a,R-g,Y-b,A-p),(R-a,R-g,Y-c,A-a),(R-a,R-g,Y-c,A-b),(R-a,R-g,Y-c,A-d),(R-a,R-g,Y-c,A-e),(R-a,R-g,Y-c,A-f),(R-a,R-g,Y-c,A-h),(R-a,R-g,Y-c,A-i),(R-a,R-g ,Y-c,A-k),(R-a,R-g,Y-c,A-l),(R-a,R-g,Y-c,A-n),(R-a,R-g,Y-c,A-p),(R-a,R-g,Y-d,A-a),(R-a,R-g,Y-d, A-b),(R-a,R-g,Y-d,A-d),(R-a,R-g,Y-d,A-e),(R-a,R-g,Y-d,A-f),(R-a,R-g,Y-d,A-h),(R-a,R-g,Y-d,A-i) ,(R-a,R-g,Y-d,A-k),(R-a,R-g,Y-d,A-l),(R-a,R-g,Y-d,A-n),(R-a,R-g,Y-d,A-p),(R-a,R-g,Y-e,A-a),(R-a,R-g,Y-e,A-b),(R-a,R-g,Y-e,A-d),(R-a,R-g,Y-e,A-e),(R-a,R-g,Y-e,A-f),(R-a,R-g,Y-e,A-h),(R-a,-g,Y-e,A-i),(R-a,R-g,Y-e,A-k),(R-a,R-g,Y-e,A-l),(R-a,R-g,Y-e,A-n),(R-a,R-g,Y-e,A-p),(R-a,R-g,Y-f, A-a),(R-a,R-g,Y-f,A-b),(R-a,R-g,Y-f,A-d),(R-a,R-g,Y-f,A-e),(R-a,R-g,Y-f,A-t),(R-a,R-g,Y-f,A-h),( R-a,R-g,Y-f,A-i),(R-a,R-g,Y-f,A-k),(R-a,R-g,Y-f,A-l),(R-a,R-g,Y-f,A-n),(R-a,R-g,Y-f,A-p),(R-a,R-g,Y-g,A-a),(R-a,R-g,Y-g,A-b),(R-a,R-g,Y-g,A-d),(R-a,R-g,Y-g,A-e),(R-a,R-g,Y-g,A-f),(R-a,R-g,Y-g,A-h),(R-a,R-g,Y-g,A-i),(R-a,R-g,Y-g,A-k),(R-a,R-g,Y-g,A-l),(R-a,R-g,Y-g,A-n),(R-a,R-g,Y-g,A-p),(R-a,R-g,Y-h,A-a),(R-a,R-g,Y-h,A-b),(R-a,R-g,Y-h,A-d),(R-a,R-g,Y-h,A-e),(R-a,R-g,Y-h,A-f),( R-a,R-g,Y-h,A-h),(R-a,R-g,Y-h,A-i),(R-a,R-g,Y-h,A-k),(R-a,R-g,Y-h,A-l),(R-a,R-g,Y-h,A-n),(R-a, R-g,Y-h,A-p),(R-a,R-g,Y-i,A-a),(R-a,R-g,Y-i,A-b),(R-a,R-g,Y-i,A-d),(R-a,R-g,Y-i,A-e),(R-a,R-g,Y -i,A-f),(R-a,R-g,Y-i,A-h),(R-a,R-g,Y-i,A-i),(R-a,R-g,Y-i,A-k),(R-a,R-g,Y-i,A-l),(R-a,R-g,Y-i,A-n),( R-a,R-g,Y-i,A-p),(R-a,R-g,Y-k,A-a),(R-a,R-g,Y-k,A-b),(R-a,R-g,Y-k,A-d),(R-a,R-g,Y-k,A-e),(R-a, R-g,Y-k,A-f),(R-a,R-g,Y-k,A-h),(R-a,R-g,Y-k,A-i),(R-a,R-g,Y-k,A-k),(R-a,R-g,Y-k,A-l),(R-a,R-g, Y-k,A-n),(R-a,R-g,Y-k,A-p),(R-a,R-g,Y-l,A-a),(R-a,R-g,Y-l,A-b),(R-a,R-g,Y-l,A-d),(R-a,R-g,Y-l,A -e),(R-a,R-g,Y-l,A-f),(R-a,R-g,Y-l,A-h),(R-a,R-g,Y-l,A-i),(R-a,R-g,Y-l,A-k),(R-a,R-g,Y-l,A-l),(R-a, R-g,Y-l,A-n),(R-a,R-g,Y-l,A-p),(R-a,R-h,Y-a,A-a),(R-a,R-h,Y-a,A-b),(R-a,R-h,Y-a,A-d),(R-a,R-h, Y-a,A-e),(R-a,R-h,Y-a,A-f),(R-a,R-h,Y-a,A-h),(R-a,R-h,Y-a,A-i),(R-a,R-h,Y-a,A-k),(R-a,R-h,Y-a, A-l),(R-a,R-h,Y-a,A-n),(R-a,R-h,Y-a,A-p),(R-a,R-h,Y-b,A-a),(R-a,R-h,Y-b,A-b),(R-a,R-h,Y-b,A-d) ,(R-a,R-h,Y-b,A-e),(R-a,R-h,Y-b,A-f),(R-a,R-h,Y-b,A-h),(R-a,R-h,Y-b,A-i),(R-a,R-h,Y-b,A-k),(R-a,R-h,Y-b,A-l),(R-a,R-h,Y-b,A-n),(R-a,R-h,Y-b,A-p),(R-a,R-h,Y-c,A-a),(R-a,R-h,Y-c,A-b),(R-a,R-h,Y-c,A-d),(R-a,R-h,Y-c,A-e),(R-a,R-h,Y-c,A-f),(R-a,R-h,Y-c,A-h),(R-a,R-h,Y-c,A-i),(R-a,R-h,Y-c ,A-k),(R-a,R-h,Y-c,A-l),(R-a,R-h,Y-c,A-n),(R-a,R-h,Y-c,A-p),(R-a,R-h,Y-d,A-a),(R-a,R-h,Y-d,A-b ),(R-a,R-h,Y-d,A-d),(R-a,R-b,Y-d,A-e),(R-a,R-h,Y-d,A-f),(R-a,R-h,Y-d,A-h),(R-a,R-b,Y-d,A-i),(R-a,R-h,Y-d,A-k),(R-a,R-h,Y-d,A-l),(R-a,R-h,Y-d,A-n),(R-a,R-h,Y-d,A-p),(R-a,R-h,Y-e,A-a),(R-a,R-h,Y-e,A-b),(R-a,R-h,Y-e,A-d),(R-a,R-b,Y-e,A-e),(R-a,R-h,Y-e,A-f),(R-a,R-h,Y-e,A-h),(R-a,R-h,Ye,A-i),(R-a,R-h,Y-e,A-k),(R-a,R-h,Y-e,A-l),(R-a,R-h,Y-e,A-n),(R-a,R-h,Y-e,A-p),(R-a,R-h,Y-f,A-a ),(R-a,R-h,Y-f,A-b),(R-a,R-h,Y-f,A-d),(R-a,R-h,Y-f,A-e),(R-a,R-h,Y-f,A-f),(R-a,R-h,Y-f,A-h),(R-a, R-h,Y-f,A-i),(R-a,R-h,Y-f,A-k),(R-a,R-h,Y-f,A-l),(R-a,R-h,Y-f,A-n),(R-a,R-h,Y-f,A-p),(R-a,R-h,Y-g,A-a),(R-a,R-h,Y-g,A-b),(R-a,R-h,Y-g,A-d),(R-a,R-h,Y-g,A-e),(R-a,R-h,Y-g,A-f),(R-a,R-h,Y-g,A -h),(R-a,R-h,Y-g,A-i),(R-a,R-h,Y-g,A-k),(R-a,R-h,Y-g,A-l),(R-a,R-h,Y-g,A-n),(R-a,R-h,Y-g,A-p),( R-a,R-h,Y-h,A-a),(R-a,R-h,Y-h,A-b),(R-a,R-h,Y-h,A-d),(R-a,R-h,Y-h,A-e),(R-a,R-h,Y-h,A-f),(R-a, R-h,Y-h,A-h),(R-a,R-h,Y-h,A-i),(R-a,R-h,Y-h,A-k),(R-a,R-h,Y-h,A-l),(R-a,R-h,Y-h,A-n),(R-a,R-h, Y-h,A-P),(R-a,R-h,Y-i,A-a),(R-a,R-h,Y-i,A-b),(R-a,R-h,Y-i,A-d),(R-a,R-h,Y-i,A-e),(R-a,R-h,Y-i,A-f),(R-a,R-h,Y-i,A-h),(R-a,R-h,Y-i,A-i),(R-a,R-h,Y-i,A-k),(R-a,R-h,Y-i,A-l),(R-a,R-h,Y-i,A-n),(R-a, R-h,Y-i,A-p),(R-a,R-h,Y-k,A-a),(R-a,R-h,Y-k,A-b),(R-a,R-h,Y-k,A-d),(R-a,R-h,Y-k,A-e),(R-a,R-h, Y-k,A-f),(R-a,R-h,Y-k,A-h),(R-a,R-h,Y-k,A-i),(R-a,R-h,Y-k,A-k),(R-a,R-h,Y-k,A-l),(R-a,R-h,Y-k, A-n),(R-a,R-h,Y-k,A-p),(R-a,R-h,Y-l,A-a),(R-a,R-h,Y-l,A-b),(R-a,R-h,Y-l,A-d),(R-a,R-h,Y-l,A-e),( R-a,R-h,Y-l,A-f),(R-a,R-h,Y-l,A-h),(R-a,R-h,Y-l,A-i),(R-a,R-h,Y-l,A-k),(R-a,R-h,Y-l,A-l),(R-a,R-h ,Y-l,A-n),(R-a,R-h,Y-l,A-p),

(R-c,R-f,Y-a,A-a),(R-c,R-f,Y-a,A-b),(R-c,R-f,Y-a,A-d),(R-c,R-f,Y-a,A-e),(R-c,R-f,Y-a,A-f),(R-c,R-f,Y-a,A-h),(R-c,R-f,Y-a,A-i),(R-c,R-f,Y-a,A-k),(R-c,R-f,Y-a,A-l),(R-c,R-f,Y-a,A-n),(R-c,R-f,Y-a,A -p),(R-c,R-f,Y-b,A-a),(R-c,R-f,Y-b,A-b),(R-c,R-f,Y-b,A-d),(R-c,R-f,Y-b,A-e),(R-c,R-f,Y-b,A-f),(R-c,R-f,Y-b,A-h),(R-c,R-f,Y-b,A-i),(R-c,R-f,Y-b,A-k),(R-c,R-f,Y-b,A-l),(R-c,R-f,Y-b,A-n),(R-c,R-t,Y -b,A-p),(R-c,R-f,Y-c,A-a),(R-c,R-f,Y-c,A-b),(R-c,R-f,Y-c,A-d),(R-c,R-f,Y-c,A-e),(R-c,R-f,Y-c,A-f) ,(R-c,R-f,Y-c,A-h),(R-c,R-f,Y-c,A-i),(R-c,R-f,Y-c,A-k),(R-c,R-f,Y-c,A-l),(R-c,R-f,Y-c,A-n),(R-c,R -f,Y-c,A-p),(R-c,R-f,Y-d,A-a),(R-c,R-f,Y-d,A-b),(R-c,R-f,Y-d,A-d),(R-c,R-f,Y-d,A-e),(R-c,R-f,Y-d, A-f),(R-c,R-f,Y-d,A-h),(R-c,R-f,Y-d,A-i),(R-c,R-f,Y-d,A-k),(R-c,R-f,Y-d,A-l),(R-c,R-f,Y-d,A-n),( R-c,R-f,Y-d,A-p),(R-c,R-f,Y-e,A-a),(R-c,R-f,Y-e,A-b),(R-c,R-f,Y-e,A-d),(R-c,R-f,Y-e,A-e),(R-c,R-f,Y-e,A-f),(R-c,R-f,Y-e,A-h),(R-c,R-f,Y-e,A-i),(R-c,R-f,Y-e,A-k),(R-c,R-f,Y-e,A,-l),(R-c,R-f,Y-e,An),(R-c,R-f,Y-e,A-p),(R-c,R-f,Y-f,A-a),(R-c,R-f,Y-f,A-b),(R-c,R-f,Y-f,A-d),(R-c,R-f,Y-f,A-e),(R-c, R-f,Y-f,A-f),(R-c,R-f,Y-f,A-h),(R-c,R-f,Y-f,A-i),(R-c,R-f,Y-f,A-k),(R-c,R-f,Y-f,A-l),(R-c,R-f,Y-f,A -n),(R-c,R-f,Y-f,A-p),(R-c,R-f,Y-g,A-a),(R-c,R-f,Y-g,A-b),(R-c,R-f,Y-g,A-d),(R-c,R-f,Y-g,A-e),(R-c,R-f,Y-g,A-f),(R-c,R-f,Y-g,A-h),(R-c,R-f,Y-g,A-i),(R-c,R-f,Y-g,A-k),(R-c,R-f,Y-g,A-l),(R-c,R-f,Y -g,A-n),(R-c,R-f,Y-g,A-p),(R-c,R-f,Y-h,A-a),(R-c,R-f,Y-h,A-b),(R-c,R-f,Y-h,A-d),(R-c,R-f,Y-h,A-e),(R-c,R-f,Y-h,A-f),(R-c,R-f,Y-h,A-h),(R-c,R-f,Y-h,A-i),(R-c,R-f,Y-h,A-k),(R-c,R-f,Y-h,A-l),(R-c, R-f,Y-h,A-n),(R-c,R-f,Y-h,A-p),(R-c,R-f,Y-i,A-a),(R-c,R-f,Y-i,A-b),(R-c,R-f,Y-i,A-d),(R-c,R-f,Y-i, A-e),(R-c,R-f,Y-i,A-f),(R-c,R-f,Y-i,A-h),(R-c,R-f,Y-i,A-i),(R-c,R-f,Y-i,A-k),(R-c,R-f,Y-i,A-l),(R-c, R-f,Y-i,A-n),(R-c,R-f,Y-i,A-p),(R-c,R-f,Y-k,A-a),(R-c,R-f,Y-k,A-b),(R-c,R-f,Y-k,A-d),(R-c,R-f,Y-k,A-e),(R-c,R-f,Y-k,A-f),(R-c,R-f,Y-k,A-h),(R-c,R-f,Y-k,A-i),(R-c,R-f,Y-k,A-k),(R-c,R-f,Y-k,A-l), (R-c,R-f,Y-k,A-n),(R-c,R-f,Y-k,A-p),(R-c,R-f,Y-l,A-a),(R-c,R-f,Y-l,A-b),(R-c,R-f,Y-l,A-d),(R-c,R-f,Y-l,A-e),(R-c,R-f,Y-l,A-f),(R-c,R-f,Y-l,A-h),(R-c,R-f,Y-l,A-i),(R-c,R-f,Y-l,A-k),(R-c,R-f,Y-l,A-l), (R-c,R-f,Y-l,A-n),(R-c,R-f,Y-l,A-p),(R-c,R-g,Y-a,A-a),(R-c,R-g,Y-a,A-b),(R-c,R-g,Y-a,A-d),(R-c, R-g,Y-a,A-e),(R-c,R-g,Y-a,A-f),(R-c,R-g,Y-a,A-h),(R-c,R-g,Y-a,A-i),(R-c,R-g,Y-a,A-k),(R-c,R-g, Y-a,A-l),(R-c,R-g,Y-a,A-n),(R-c,R-g,Y-a,A-p),(R-c,R-g,Y-b,A-a),(R-c,R-g,Y-b,A-b),(R-c,R-g,Y-b, A-d),(R-c,R-g,Y-b,A-e),(R-c,R-g,Y-b,A-f),(R-c,R-g,Y-b,A-h),(R-c,R-g,Y-b,A-i),(R-c,R-g,Y-b,A-k) ,(R-c,R-g,Y-b,A-l),(R-c,R-g,Y-b,A-n),(R-c,R-g,Y-b,A-p),(R-c,R-g,Y-c,A-a),(R-c,R-g,Y-c,A-b),(R-c,R-g,Y-c,A-d),(R-c,R-g,Y-c,A-e),(R-c,R-g,Y-c,A-f),(R-c,R-g,Y-c,A-h),(R-c,R-g,Y-c,A-i),(R-c,R-g ,Y-c,A-k),(R-c,R-g,Y-c,A-l),(R-c,R-g,Y-c,A-n),(R-c,R-g,Y-c,A-p),(R-c,R-g,Y-d,A-a),(R-c,R-g,Y-d, A-b),(R-c,R-g,Y-d,A-d),(R-c,R-g,Y-d,A-e),(R-c,R-g,Y-d,A-f),(R-c,R-g,Y-d,A-h),(R-c,R-g,Y-d,A-i) ,(R-c,R-g,Y-d,A-k),(R-c,R-g,Y-d,A-l),(R-c,R-g,Y-d,A-n),(R-c,R-g,Y-d,A-p),(R-c,R-g,Y-e,A-a),(R-c,R-g,Y-e,A-b),(R-c,R-g,Y-e,A-d),(R-c,R-g,Y-e,A-e),(R-c,R-g,Y-e,A-f),(R-c,R-g,Y-e,A-h),(R-c,R-g,Y-e,A-i),(R-c,R-g,Y-e,A-k),(R-c,R-g,Y-e,A-l),(R-c,R-g,Y-e,A-n),(R-c,R-g,Y-e,A-p),(R-c,R-g,Y-f, A-a),(R-c,R-g,Y-f,A-b),(R-c,R-g,Y-f,A-d),(R-c,R-g,Y-f,A-e),(R-c,R-g,Y-f,A-f),(R-c,R-g,Y-f,A-h),( R-c,R-g,Y-f,A-i),(R-c,R-g,Y-f,A-k),(R-c,R-g,Y-f,A-l),(R-c,R-g,Y-f,A-n),(R-c,R-g,Y-f,A-p),(R-c,R-g,Y-g,A-a),(R-c,R-g,Y-g,A-b),(R-c,R-g,Y-g,A-d),(R-c,R-g,Y-g,A-e),(R-c,R-g,Y-g,A-f),(R-c,R-g,Y-g,A-h),(R-c,R-g,Y-g,A-i),(R-c,R-g,Y-g,A-k),(R-c,R-g,Y-g,A-l),(R-c,R-g,Y-g,A-n),(R-c,R-g,Y-g,A-p),(R-c,R-g,Y-h,A-a),(R-c,R-g,Y-h,A-b),(R-c,R-g,Y-h,A-d),(R-c,R-g,Y-h,A-e),(R-c,R-g,Y-h,A-f),( R-c,R-g,Y-h,A-h),(R-c,R-g,Y-h,A-i),(R-c,R-g,Y-h,A-k),(R-c,R-g,Y-h,A-l),(R-c,R-g,Y-h,A-n),(R-c, R-g,Y-h,A-p),(R-c,R-g,Y-i,A-a),(R-c,R-g,Y-i,A-b),(R-c,R-g,Y-i,A-d),(R-c,R-g,Y-i,A-e),(R-c,R-g,Y -i,A-f),(R-c,R-g,Y-i,A-h),(R-c,A-g,Y-i,A-i),(R-c,R-g,Y-i,A-k),(R-c,R-g,Y-i,A-l),(R-c,R-g,Y-i,A-n),( R-c,R-g,Y-i,A-p),(R-c,R-g,Y-k,A-a),(R-c,R-g,Y-k,A-b),(R-c,R-g,Y-k,A-d),(R-c,R-g,Y-k,A-e),(R-c, R-g,Y-k,A-f),(R-c,R-g,Y-k,A-h),(R-c,R-g,Y-k,A-i),(R-c,R-g,Y-k,A-k),(R-c,R-g,Y-k,A-l),(R-c,R-g, Y-k,A-n),(R-c,R-g,Y-k,A-p),(R-c,R-g,Y-l,A-a),(R-c,R-g,Y-l,A-b),(R-c,R-g,Y-l,A-d),(R-c,R-g,Y-l,A -e),(R-c,R-g,Y-l,A-f),(R-c,R-g,Y-l,A-h),(R-c,R-g,Y-l,A-i),(R-c,R-g,Y-l,A-k),(R-c,R-g,Y-l,A-l),(R-c, R-g,Y-l,A-n),(R-c,R-g,Y-l,A-p),(R-c,R-h,Y-a,A-a),(R-c,R-h,Y-a,A-b),(R-c,R-h,Y-a,A-d),(R-c,R-h, Y-a,A-e),(R-c,R-h,Y-a,A-f),(R-c,R-h,Y-a,A-h),(R-c,R-h,Y-a,A-i),(R-c,R-h,Y-a,A-k),(R-c,R-h,Y-a, A-1),(R-c,A-h,Y-a,A-n),(R-c,R-h,Y-a,A-p),(R-c,R-h,Y-b,A-a),(R-c,R-h,Y-b,A-b),(R-c,R-h,Y-b,A-d) ,(R-c,R-h,Y-b,A-e),(R-c,R-h,Y-b,A-f),(R-c,R-h,Y-b,A-h),(R-c,R-h,Y-b,A-i),(R-c,R-h,Y-b,A-k),(R-c,R-h,Y-b,A-l),(R-c,R-h,Y-b,A-n),(R-c-,R-b,Y-b,A-p),(R-c,R-h,Y-c,A-a),(R-c,R-h,Y-c,A-b),(R-c,R-h,Y-c,A-d),(R-c,R-h,Y-c,A-e),(R-c,R-h,Y-c,A-f),(R-c,R-h,Y-c,A-h),(R-c,R-h,Y-c,A-i),(R-c,R-h,Y-c ,A-k),(R-c,R-h,Y-c,A-l),(R-c,R-h,Y-c,A-n),(R-c,R-h,Y-c,A-p),(R-c,R-h,Y-d,A-a),(R-c,R-h,Y-d,A-b ),(R-c,R-h,Y-d,A-d),(R-c,R-h,Y-d,A-e),(R-c,R-h,Y-d,A-f),(R-c,R-h,Y-d,A-h),(R-c,R-h,Y-d,A-i),(R-c,R-h,Y-d,A-k),(R-c,R-h,Y-d,A-l),(R-c,R-h,Y-d,A-n),(R-c,R-h,Y-d,A-p),(R-c,R-h,Y-e,A-a),(R-c,R-h,Y-e,A-b),(R-c,R-h,Y-e,A-d),(R-c,R-h,Y-e,A-e),(R-c,R-h,Y-e,A-f),(R-c,R-h,Y-e,A-h),(R-c,R-h,Ye,A-i),(R-c,R-h,Y-e,A-k),(R-c,R-h,Y-e,A-l),(R-c,R-h,Y-e,A-n),(R-c,R-h,Y-e,A-p),(R-c,R-h,Y-f,A-a ),(R-c,R-h,Y-f,A-b),(R-c,R-h,Y-f,A-d),(R-c,R-h,Y-f,A-e),(R-c,R-h,Y-f,A-f),(R-c,R-h,Y-f,A-h),(R-c, R-h,Y-f,A-i),(R-c,R-h,Y-f,A-k),(R-c,R-h,Y-f,A-l),(R-c,R-h,Y-f,A-n),(R-c,R-h,Y-t,A-p),(R-c,R-h,Y-g,A-a),(R-c,R-h,Y-g,A-b),(R-c,R-h,Y-g,A-d),(R-c,R-h,Y-g,A-e),(R-c,R-h,Y-g,A-f),(R-c,R-h,Y-g,A -h),(R-c,R-h,Y-g,A-i),(R-c,R-h,Y-g,A-k),(R-c,R-h,Y-g,A-l),(R-c,R-h,Y-g,A-n),(R-c,R-h,Y-g,A-p),( R-c,R-h,Y-h,A-a),(R-c,R-h,Y-h,A-b),(R-c,R-h,Y-h,A-d),(R-c,R-h,Y-h,A-e),(R-c,R-h,Y-h,A-f),(R-c, R-h,Y-h,A-h),(R-c,R-h,Y-h,A-i),(R-c,R-h,Y-h,A-k),(R-c,R-h,Y-h,A-l),(R-c,R-h,Y-h,A-n),(R-c,R-h, Y-h,A-p),(R-c,R-h,Y-i,A-a),(R-c,R-h,Y-i,A-b),(R-c,R-h,Y-i,A-d),(R-c,R-h,Y-i,A-e),(R-c,R-h,Y-i,A-f),(R-c,R-h,Y-i,A-h),(R-c,R-h,Y-i,A-i),(R-c,R-h,Y-i,A-k),(R-c,R-h,Y-i,A-l),(R-c,R-h,Y-i,A-n),(R-c, R-h,Y-i,A-p),(R-c,R-h,Y-k,A-a),(R-c,R-h,Y-k,A-b),(R-c,R-h,Y-k,A-d),(R-c,R-h,Y-k,A-e),(R-c,R-h, Y-k,A-f),(R-c,R-h,Y-k,A-h),(R-c,R-h,Y-k,A-i),(R-c,R-h,Y-k,A-k),(R-c,R-h,Y-k,A-l),(R-c,R-h,Y-k, A-n),(R-c,R-h,Y-k,A-p),(R-c,R-h,Y-l,A-a),(R-c,R-h,Y-l,A-b),(R-c,R-h,Y-l,A-d),(R-c,R-h,Y-l,A-e),( R-c,R-h,Y-l,A-f),(R-c,R-h,Y-l,A-h),(R-c,R-h,Y-l,A-i),(R-c,R-h,Y-l,A-k),(R-c,R-h,Y-l,A-l),(R-c,R-h ,Y-l,A-n),(R-c,R-h,Y-l,A-p),

(R-d,R-f,Y-a,A-a),(R-d,R-f,Y-a,A-b),(R-d,R-f,Y-a,A-d),(R-d,R-f,Y-a,A-e),(R-d,R-f,Y-a,A-f),(R-d, R-f,Y-a,A-h),(R-d,R-f,Y-a,A-i),(R-d,R-f,Y-a,A-k),(R-d,R-f,Y-a,A-l),(R-d,R-f,Y-a,A-n),(R-d,R-f,Y-a,A-p),(R-d,R-f,Y-b,A-a),(R-d,R-f,Y-b,A-b),(R-d,R-f,Y-b,A-d),(R-d,R-f,Y-b,A-e),(R-d,R-f,Y-b,A-f ),(R-d,R-f,Y-b,A-h),(R-d,R-f,Y-b,A-i),(R-d,R-f,Y-b,A-k),(R-d,R-f,Y-b,Al),(R-d,R-f,Y-b,A-n),(R-d ,R-f,Y-b,A-p),(R-d,R-f,Y-c,A-a),(R-d,R-f,Y-c,A-b),(R-d,R-f,Y-c,A-d),(R-d,R-f,Y-c,A-e),(R-d,R-f,Y -c,A-f),(R-d,R-f,Y-c,A-h),(R-d,R-f,Y-c,A-i),(R-d,R-f,Y-c,A-k),(R-d,R-f,Y-c,A-l),(R-d,R-f,Y-c,A-n) ,(R-d,R-f,Y-c,A-p),(R-d,R-f,Y-d,A-a),(R-d,R-f,Y-d,A-b),(R-d,R-f,Y-d,A-d),(R-d,R-f,Y-d,A-e),(R-d ,R-f,Y-d,A-f),(R-d,R-f,Y-d,A-h),(R-d,R-f,Y-d,A-i),(R-d,R-f,Y-d,A-k),(R-d,R-f,Y-d,A-l),(R-d,R-f,Y-d,A-n),(R-d,R-f,Y-d,A-p),(R-d,R-f,Y-e,A-a),(R-d,R-f,Y-e,A-b),(R-d,R-f,Y-e,A-d),(R-d,R-f,Y-e,A-e ),(R-d,R-f,Y-e,A-f),(R-d,R-f,Y-e,A-h),(R-d,R-f,Y-e,A-i),(R-d,R-f,Y-e,A-k),(R-d,R-f,Y-e,A-l),(R-d, R-f,Y-e,A-n),(R-d,R-f,Y-e,A-p),(R-d,R-f,Y-f,A-a),(R-d,R-f,Y-f,A-b),(R-d,R-f,Y-f,A-d),(R-d,R-f,Y-f ,A-e),(R-d,R-f,Y-f,A-f),(R-d,R-f,Y-f,A-h),(R-d,R-f,Y-f,A-i),(R-d,R-f,Y-f,A-k),(R-d,R-f,Y-f,A-l),(R-d,R-f,Y-f,A-n),(R-d,R-f,Y-f,A-p),(R-d,R-f,Y-g,A-a),(R-d,R-f,Y-g,A-b),(R-d,R-f,Y-g,A-d),(R-d,R-f, Y-g,A-e),(R-d,R-f,Y-g,A-f),(R-d,R-f,Y-g,A-h),(R-d,R-f,Y-g,A-i),(R-d,R-f,Y-g,A-k),(R-d,R-f,Y-g,A -1),(R-d,R-f,Y-g,A-n),(R-d,R-f,Y-g,A-p),(R-d,R-f,Y-h,A-a),(R-d,R-f,Y-h,A-b),(R-d,R-f,Y-h,A-d),(R -d,R-f,Y-h,A-c),(R-d,R-f,Y-h,A-f),(R-d,R-f,Y-h,A-h),(R-d,R-f,Y-h,A-i),(R-d,R-f,Y-h,A-k),(R-d,R-f ,Y-h,A-l),(R-d,R-f,Y-h,A-n),(R-d,R-f,Y-h,A-p),(R-d,R-f,Y-i,A-a),(R-d,R-f,Y-i,A-b),(R-d,R-f,Y-i,A-d),(R-d,R-f,Y-i,A-e),(R-d,R-f,Y-i,A-f),(R-d,R-f,Y-i,A-h),(R-d,R-f,Y-i,A-i),(R-d,R-f,Y-i,A-k),(R-d,R -f,Y-i,A-l),(R-d,R-f,Y-i,A-n),(R-d,R-f,Y-i,A-p),(R-d,R-f,Y-k,A-a),(R-d,R-f,Y-k,A-b),(R-d,R-f,Y-k, A-d),(R-d,R-f,Y-k,A-e),(R-d,R-f,Y-k,A-f),(R-d,R-f,Y-k,A-h),(R-d,R-f,Y-k,A-i),(R-d,R-f,Y-k,A-k),( R-d,R-f,Y-k,A-l),(R-d,R-f,Y-k,A-n),(R-d,R-f,Y-k,A-p),(R-d,R-f,Y-l,A-a),(R-d,R-f,Y-l,A-b),(R-d,R-f,Y-l,A-d),(R-d,R-f,Y-l,A-e),(R-d,R-f,Y-l,A-f),(R-d,R-f,Y-l,A-h),(R-d,R-f,Y-l,A-i),(R-d,R-f,Y-l,A-k ),(R-d,R-f,Y-l,A-l),(R-d,R-f,Y-l,A-n),(R-d,R-f,Y-l,A-p),(R-d,R-g,Y-a,A-a),(R-d,R-g,Y-a,A-b),(R-d, R-g,Y-a,A-d),(R-d,R-g,Y-a,A-e),(R-d,R-g,Y-a,A-f),(R-d,R-g,Y-a,A-h),(R-d,R-g,Y-a,A-i),(R-d,R-g, Y-a,A-k),(R-d,R-g,Y-a,A-l),(R-d,R-g,Y-a,A-n),(R-d,R-g,Y-a,A-p),(R-d,R-g,Y-a,A-a),(R-d,R-g,Y-b, A-b),(R-d,R-g,Y-b,A-d),(R-d,R-g,Y-b,A-e),(R-d,R-g,Y-b,A-f),(R-d,R-g,Y-b,A-h),(R-d,R-g,Y-b,A-i ),(R-d,R-g,Y-b,A-k),(R-d,R-g,Y-b,A-l),(R-d,R-g,Y-b,A-n),(R-d,R-g,Y-b,A-p),(R-d,R-g,Y-c,A-a),( R-d,R-g,Y-c,A-b),(R-d,R-g,Y-c,A-d),(R-d,R-g,Y-c,A-e),(R-d,R-g,Y-c,A-f),(R-d,R-g,Y-c,A-h),(R-d ,R-g,Y-c,A-i),(R-d,R-g,Y-c,A-k),(R-d,R-g,Y-c,A-l),(R-d,R-g,Y-c,A-n),(R-d,R-g,Y-c,A-p),(R-d,R-g, Y-d,A-a),(R-d,R-g,Y-d,A-b),(R-d,R-g,Y-d,A-d),(R-d,R-g,Y-d,A-e),(R-d,R-g,Y-d,A-f),(R-d,R-g,Y-d ,A-h),(R-d,R-g,Y-d,A-i),(R-d,R-g,Y-d,A-k),(R-d,R-g,Y-d,A-l),(R-d,R-g,Y-d,A-n),(R-d,R-g,Y-d,A-p),(R-d,R-g,Y-e,A-a),(R-d,R-g,Y-c,A-b),(R-d,R-g,Y-e,A-d),(R-d,R-g,Y-e,A-e),(R-d,R-g,Y-e,A-f),( R-d,R-g,Y-e,A-h),(R-d,R-g,Y-c,A-i),(R-d,R-g,Y-c,A-k),(R-d,R-g,Y-e,A-l),(R-d,R-g,Y-e,A-n),(R-d, R-g,Y-e,A-p),(R-d,R-g,Y-f,A-a),(R-d,R-g,Y-f,A-b),(R-d,R-g,Y-f,A-d),(R-d,R-g,Y-f,A-e),(R-d,R-g, Y-f,A-f),(R-d,R-g,Y-f,A-h),(R-d,R-g,Y-f,A-i),(R-d,R-g,Y-f,A-k),(R-d,R-g,Y-f,A-l),(R-d,R-g,Y-f,An),(R-d,R-g,Y-f,A-p),(R-d,R-g,Y-g,A-a),(R-d,R-g,Y-g,A-b),(R-d,R-g,Y-g,A-d),(R-d,R-g,Y-g,A-e),( R-d,R-g,Y-g,A-t),(R-d,R-g,Y-g,A-h),(R-d,R-g,Y-g,A-i),(R-d,R-g,Y-g,A-k),(R-d,R-g,Y-g,A-l),(R-d, R-g,Y-g,A-n),(R-d,R-g,Y-g,A-p),(R-d,R-g,Y-h,A-a),(R-d,R-g,Y-h,A-b),(R-d,R-g,Y-h,A-d),(R-d,R-g,Y-h,A-e),(R-d,R-g,Y-h,A-f),(R-d,R-g,Y-h,A-h),(R-d,R-g,Y-h,A-i),(R-d,A-g,Y-h,A-k),(R-d,R-g,Y -h,A-l),(R-d,R-g,Y-h,A-n),(R-d,R-g,Y-h,A-p),(R-d,R-g,Y-i,A-a),(R-d,R-g,Y-i,A-b),(R-d,R-g,Y-i,A-d),(R-d,R-g,Y-i,A-e),(R-d,R-g,Y-i,A-f),(R-d,R-g,Y-i,A-h),(R-d,R-g,Y-i,A-i),(R-d,R-g,Y-i,A-k),(R-d ,R-g,Y-i,A-l),(R-d,R-g,Y-i,A-n),(R-d,R-g,Y-i,A-p),(R-d,R-g,Y-k,A-a),(R-d,R-g,Y-k,A-b),(R-d,R-g, Y-k,A-d),(R-d,R-g,Y-k,A-e),(R-d,R-g,Y-k,A-f),(R-d,R-g,Y-k,A-h),(R-d,R-g,Y-k,A-i),(R-d,R-g,Y-k ,A-k),(R-d,R-g,Y-k,A-l),(R-d,R-g,Y-k,A-n),(R-d,R-g,Y-k,A-p),(R-d,R-g,Y-l,A-a),(R-d,R-g,Y-l,A-b ),(R-d,R-g,Y-l,A-d),(R-d,R-g,Y-l,A-e),(R-d,R-g,Y-l,A-f),(R-d,R-g,Y-l,A-h),(R-d,R-g,Y-l,A-i),(R-d, R-g,Y-l,A-k),(R-d,R-g,Y-l,A-l),(R-d,R-g,Y-l,A-n),(R-d,R-g,Y-l,A-n),(R-d,R-h,Y-a,A-a),(R-d,R-h,Y -a,A-b),(R-d,R-h,Y-a,A-c),(R-d,R-h,Y-a,A-d),(R-d,R-h,Y-a,A-e),(R-d,R-h,Y-a,A-f),(R-d,R-h,Y-a,A -h),(R-d,R-h,Y-a,A-i),(R-d,R-h,Y-a,A-k),(R-d,R-h,Y-a,A-l),(R-d,R-h,Y-a,A-n),(R-d,R-h,Y-a,A-p),( R-d,R-h,Y-b,A-a),(R-d,R-h,Y-b,A-b),(R-d,R-h,Y-b,A-d),(R-d,R-h,Y-b,A-e),(R-d,R-h,Y-b,A-f),(R-d,R-h,Y-b,A-h),(R-d,R-h,Y-b,A-i),(R-d,R-h,Y-b,A-k),(R-d,R-h,Y-b,A-l),(R-d,R-h,Y-b,A-n),(R-d,R -h,Y-b,A-p),(R-d,R-h,Y-c,A-a),(R-d,R-h,Y-c,A-b),(R-d,R-h,Y-c,A-d),(R-d,R-h,Y-c,A-e),(R-d,R-h, Y-c,A-f),(R-d,R-h,Y-c,A-h),(R-d,R-h,Y-c,A-i),(R-d,R-h,Y-c,A-k),(R-d,R-h,Y-c,A-l),(R-d,R-h,Y-c, A-n),(R-d,R-h,Y-c,A-p),(R-d,R-h,Y-d,A-a),(R-d,R-h,Y-d,A-b),(R-d,R-h,Y-d,A-d),(R-d,R-h,Y-d,A-e),(R-d,R-h,Y-d,A-f),(R-d,R-h,Y-d,A-h),(R-d,R-h,Y-d,A-i),(R-d,R-h,Y-d,A-k),(R-d,R-h,Y-d,A-l),( R-d,R-h,Y-d,A-n),(R-d,R-h,Y-d,A-p),(R-d,R-h,Y-e,A-a),(R-d,R-h,Y-e,A-b),(R-d,R-h,Y-e,A-d),(R-d,R-h,Y-e,A-e),(R-d,R-h,Y-e,A-f),(R-d,R-h,Y-e,A-h),(R-d,R-h,Y-e,A-i),(R-d,R-h,Y-e,A-k),(R-d,R-h,Y-e,A-l),(R-d,R-h,Y-e,A-n),(R-d,R-h,Y-e,A-p),(R-d,R-h,Y-f,A-a),(R-d,R-h,Y-f,A-b),(R-d,R-h,Y-f ,A-d),(R-d,R-h,Y-f,A-e),(R-d,R-h,Y-f,A-f),(R-d,R-h,Y-f,A-h),(R-d,R-h,Y-f,A-i),(R-d,R-h,Y-f,A-k), (R-d,R-h,Y-f,A-l),(R-d,R-h,Y-f,A-n),(R-d,R-h,Y-f,A-p),(R-d,R-h,Y-g,A-a),(R-d,R-h,Y-g,A-b),(R-d ,R-h,Y-g,A-d),(R-d,R-h,Y-g,A-e),(R-d,R-h,Y-g,A-f),(R-d,R-h,Y-g,A-h),(R-d,R-h,Y-g,A-i),(R-d,R-h,Y-g,A-k),(R-d,R-h,Y-g,A-l),(R-d,R-h,Y-g,A-n),(R-d,R-h,Y-g,A-p),(R-d,R-h,Y-h,A-a),(R-d,R-h,Y -h,A-b),(R-d,R-h,Y-h,A-d),(R-d,R-h,Y-h,A-e),(R-d,R-h,Y-h,A-f),(R-d,R-h,Y-h,A-h),(R-d,R-h,Y-h, A-i),(R-d,R-h,Y-h,A-k),(R-d,R-h,Y-h,A-l),(R-d,R-h,Y-h,A-n),(R-d,R-h,Y-h,A-p),(R-d,R-h,Y-i,A-a) ,(R-d,R-h,Y-i,A-b),(R-d,R-h,Y-i,A-d),(R-d,R-h,Y-i,A-e),(R-d,R-h,Y-i,A-f),(R-d,R-h,Y-i,A-h),(R-d, R-h,Y-i,A-i),(R-d,R-h,Y-i,A-k),(R-d,R-h,Y-i,A-l),(R-d,R-h,Y-i,A-n),(R-d,R-h,Y-i,A-p),(R-d,R-h,Y-k,A-a),(R-d,R-h,Y-k,A-b),(R-d,R-h,Y-k,A-d),(R-d,R-h,Y-k,A-e),(R-d,R-h,Y-k,A-f),(R-d,R-h,Y-k, A-h),(R-d,R-h,Y-k,A-i),(R-d,R-h,Y-k,A-k),(R-d,R-h,Y-k,A-l),(R-d,R-h,Y-k,A-n),(R-d,R-h,Y-k,A-p ),(R-d,R-h,Y-l,A-a),(R-d,R-h,Y-l,A-b),(R-d,R-h,Y-l,A-d),(R-d,R-h,Y-l,A-e),(R-d,R-h,Y-l,A-f),(R-d, R-h,Y-l,A-h),(R-d,R-h,Y-l,A-i),(R-d,R-h,Y-l,A-k),(R-d,R-h,Y-l,A-l),(R-d,R-h,Y-l,A-n),(R-d,A-h,Y-1,A-p).

The compounds of the present invention have the antibody production suppressing activity, particularly, the IgE production suppressing activity, and is considered to be useful in preventing or treating a rejection reaction against internal organ or tissue transplantation, transplantation immunity (acute or chronic GVHD), autoimmune disease (particularly, organ-non-specific autoimmune disease), mixed-type connective tissue disease (MCTD), damage in ischemic reperfusion, ulcerative colitis, systemic erythematosus, myasthenia gravis, systemic progressive scleroderma, rheumatoid arthritis, interstitial cystitis, Hashimoto disease, Basedaw's disease, autoimmune hemolytic anemia, idiopathic thrombocytopenic purpura, Goodpasture's syndrome, atrophic gastritis, pernicious anemia, Addison's disease, pemphigus, pemphigoid, lens uveitis, sympathetic ophthalmia, primary biliary cirrhosis, active chronic hepatitis, Sjoegren's syndrome, multiple myositis, dermatomyositis, polyarteritis nodosa, rheumatic fever, glomerulonephritis (lupus nephritis, IgA nephropathy, membranous nephritis etc.), allergic encephalitis, atopic allergy disease (e.g. bronchial asthma, allergic rhinitis, allergic dermatitis, allergic conjunctivitis, pollinosis, urticaria, food allergy etc.), psoriasis, Omenn's syndrome, vernal conjunctivitis, hypereosinophilic syndrome and the like. Further, it is considered that an immunosuppressing agent can be used as a therapeutic agent for chronic renal insufficiency which is induced by a non-immunological mechanism as the trigger (Kidney International vol.54 (1998), pp. 1510-1519, Kidney International vol.55 (1999), pp. 945-955), and the compounds of the present invention can be a therapeutic agent for non-immune chronic renal insufficiency.
In addition, since the compounds of the present invention have the DHODH inhibitory activity, the compounds are considered to be useful in preventing or treating a disease associated with the action of DHODH, such as rheumatoid arthritis, systemic erythematosus, multiple sclerosis, inflammatory bowl disease, lens uveitis, myasthenia gravis, bronchial asthma, atopic dermatitis, psoriasis, virus infectious disease, bacterial infectious disease, parasite infectious disease, rejection reaction in transplantation, graft versus host disease, cancer (myeloma such as multiple myeloma, lymphoma, leukemia etc.) and the like.

The compounds of the present invention, when administered as an antibody production suppressing agent, a DHODH inhibitor, an anti-allergic agent, an immunosuppressing agent or an anti-cancer agent, can be administered by any of oral and parenteral methods. Oral administration may be performed by preparing the compound into a dosage form which is normally used, such as tablets, granules, powders, capsules, pills, liquid preparations, syrups, buccal and sublingual preparations according to a conventional method. Parenteral administration can be performed suitably by any dosage form which is normally used, such as injections (for intramuscular administration, intravenous administration, and the like), suppositories. transdermal absorbing agents, inhalants and the like. Particularly, oral administration is preferable.
An effective amount of the compounds of the present invention and, if necessary, various pharmaceutical additives such as excipients, binders, wetting agents, disintegrating agents, lubricants, diluents and the like which are suitable for a dosage form thereof, can be mixed to prepare a medical preparation. In the case of injections, the compounds of the present invention together with an appropriate carrier may be subjected to sterilization treatment to obtain a preparation.
Specifically, examples of excipients include lactose, sucrose, glucose, starch, calcium carbonate and crystalline cellulose, and examples of binders include methylcellulose, carboxymethylcellulose, hydroxypropylcellulose, gelatin and polyvinylpyrrolidone, examples of disintegrating agents include carboxymethylcellulose, carboxymethylcellulose sodium, starch, sodium alginate, agar powder and sodium laurylsulfate, and examples of lubricants include talc, magnesium stearate and macrogol. As a base for suppositories, cacao butter, macrogol, methylcellulose and the like can be used. When prepared as liquid preparations, or emulsion or suspension injections, then solubilizerrs, suspending agents, emulsifiers, stabilizers, preservatives, isotonics and the like which are normally used may be appropriately added and, in the case of oral administration, corrigents, flavors and the like may be added.

It is desirable that a dose of the compounds of the present invention as an antibody production suppressing agent, a DHODH inhibitor, an anti-allergic agent, an immunosuppressing agent or an anti-cancer agent is set in view of an age and a weight of a patient, a kind and an extent of a disease, an administration route and the like and, when orally administered to adult, the dose is usually 0.05 to 100 mg/kg/day, preferably 0.1 to 10 mg/kg/day. In the case of parenteral administration, a dose greatly differs depending on an administration route, and is usually 0.005 to 10 mg/kg/day, preferably 0.01 to 1 mg/kg/day. This may be administered by dividing into once to a few times per day
The present invention will be explained in more detail below by way of Examples, but these do not limit the present invention.

### Example

### Synthesis of Compound 73 and Compound 76

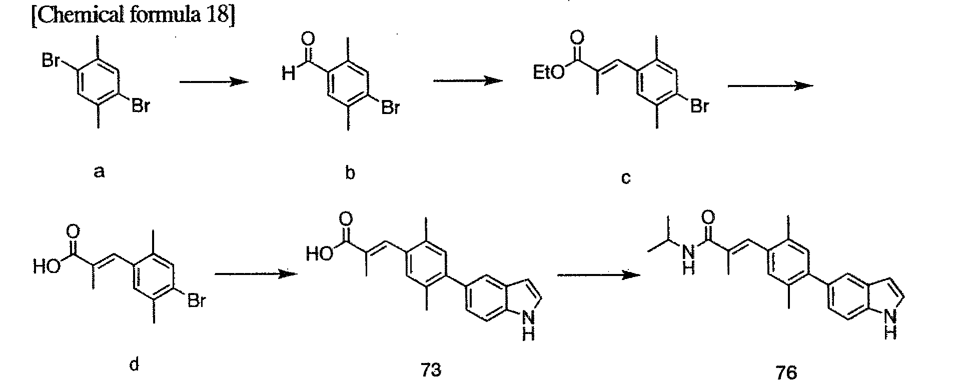

wherein Et is ethyl.

### (First step) Synthesis of Compound (b)

5.28 g (20mmol) of 1,4-dibromo-2,5-dimethylbenzene (a) was dissolved in 100ml of THF. After the reaction solution was cooled to -78°C, 24 ml (22 mmol) of butyl lithium (1.6 M) was added dropwise. After the reaction at -78°C for 0.5 hour, 7.8 ml (100 mmol) of dimethylformamide was added. After a temperature was raised to room temperature, 50 ml of 2N hydrochloric acid was added to stop the reaction, followed by extraction with ethyl acetate. The extract was washed with water and brine dried, and concentrated. The residue was purified by silica gel column chromatography (hexane-ethyl acetate 8:1) to obtain Compound (b) (3.9 g; yield 92%).

### (Second step) Synthesis of Compound (c)

5.0 g (21 mmol) of triethyl 2-phosphonopropionate was dissolved in 40 ml of dimethylformamide, and 0.88 g (22 mmol) of sodium hydride (60%) was added under ice-cooling, followed by stirring for 0.5 hour. To the reaction mixture was added 4.26 g (20 mmol) of Compound (b), to react them for 1 hour under ice-cooling. The reaction solution was poured into ice water to precipitate crystals. The resulting crystals were filtered, washed with water and dried to obtain Compound (c) (5.8 g; yield 97%).

### (Third step) Synthesis of Compound (d)

3.2 g (10.8 mmol) of Compound (c) was dissolved in 20 ml of methanol, and 12 ml of 2N sodium hydroxide was added, followed by stirring at 80°C for 1 hour. After the reaction solution was cooled in an ice bath, addition of 24 ml of 2N hydrochloric add to the reaction mixture gave a crystalline precipitate. The resulting precipitate were filtered, washed with water, and dried to obtain Compound (d) (2.86 g; yield 98%).

### (Fourth step) Synthesis of Compound (73)

1.0 g (3.7 mmol) of Compound (d) was dissolved in 1.2 ml of dimethylformamide, and 6 ml of water was added. Further,154 g (11.1 mmol) of potassium carbonate and 840mg (5.2 mmol) of indole 5-boronic acid were added, and 210 mg (0.18 mmol) of tetrakis(triphenylphosphine)palladium (0) was added under the argon atmosphere. This reaction suspension was heated at reflux for 4 hours under the argon atmosphere. After cooling, water was added, followed by extraction with ethyl acetate. The extract was washed sequentially with water and brine, dried, and concentrated. The residue was purified by silica gel column chromatography (hexane-ethyl acetate 1:1) to obtain Compound (73) (1.06 g; yield 93%).

### (Fifth step) Synthesis of Compound (76)

150 mg (0.49mmol) of Compound (73) was dissolved in 5 ml of dimethylformamide, and 0.084 ml of isopropylamine (0.98 mmol), and 0.27 ml of triethylamine (1.96 mmol) were added. Further, 0.11 ml (0.73 mmol) of diethyl cyanophosphate was added, followed by stirring for 1 hour. To the reaction solution was added water, followed by extraction with ethyl acetate. The extract was washed sequentially with water and an aqueous saturated sodium chloride solution, dried, and concentrated. The residue was purified by silica gel column chromatography (hexane-ethyl acetate 2: 1) to obtain Compound (76) (117 mg; yield 68%).

Other Compound (I) are synthesized similarly, and the structures of them are shown below. In Table, for example, in Compound 3, 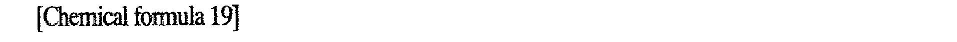 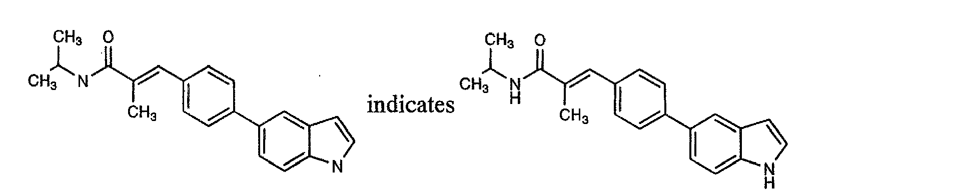 Other compounds are the same.

**[Table 1]**

| Compoun d No. | Structure | 1 H-NMR | mp |
|---|---|---|---|
| 1 | 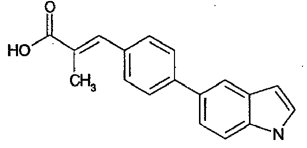 | | 195-198°C |
| 2 | 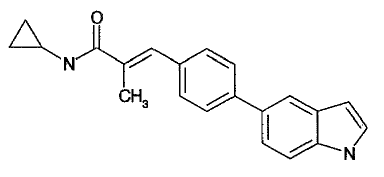 | 1H NMR (CDCl3) δ 0.60 (m, 2H), 0.85 (m, 2H), 2.15 (s, 3H), 2.85 (m, 1H), 5.99 (s, 1H), 6.62 (m, 1H), 7.25-7.47 (m, 6H), 7.87 (d, J = 8.1 Hz, 2H), 7.88 (s, 1 H), 8.24 (s, 1H) ppm | |
| 3 | 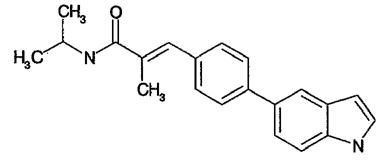 | 1H NMR (CDCl3) δ 1.25 (d, J = 6.6Hz, 6H), 2.16 (s, 3H), 4.20 (m, 1H), 5.67 (m, 1H), 6.62 (m, 1 H), 7.26-7.47 (m, 6H), 7.67 (d, J = 8.4Hz, 2H), 7.89 (s, 1H), 8.25 (s, 1H) ppm | |
| 4 | 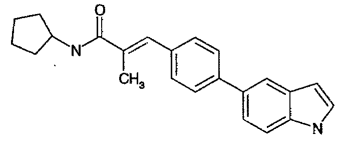 | | 154-157°C |
| 5 | 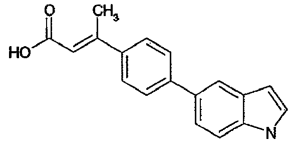 | | |
| 6 | 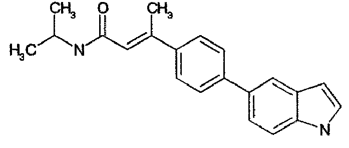 | | |
| 7 | 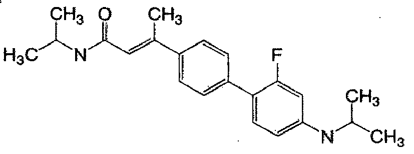 | | |

**[Table 2]**

| | | | |
|---|---|---|---|
| 8 | 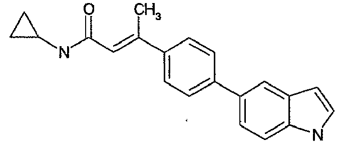 | | |
| 9 | 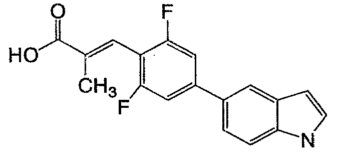 | | 175-180°C |
| 10 | 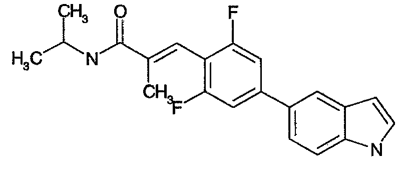 | 1H NMR (CDCl3) δ 1.25 (d, J = 6.6Hz, 6H), 1.95 (s, 3H), 4.20 (m, 1 H), 5.72 (m, 1H), 6.62 (m, 1 H), 7.08 (s, 1H), 7.18-7.29 (m, 3H), 7.39-7.50 (m, 2H), 7.83 (s, 1 H), 8.33 (s, 1 H) ppm | |
| 11 | 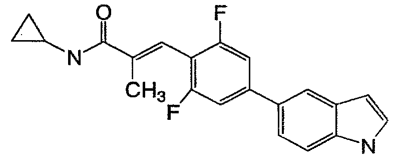 | 1 H NMR (CDCl3) δ 0.62 (m, 2H), 0.86 (m, 2H), 1.92 (s, 3H), 2.86 (m, 1 H), 6.62 (m, 1H), 7.07 (s, 1H), 7.20-7.29 (m, 3H), 7.39-7.49 (m, 2H), 7.85 (s, 1H), 8.32 (s, 1H) ppm | |
| 12 | 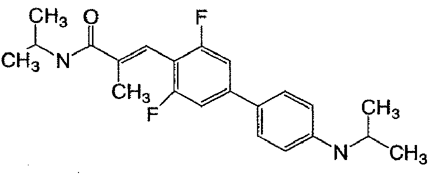 | 1H NMR (CDCl3) δ 1.25 (m, 12H), 1.92 (s, 3H), 3.82 (m, 1H), 4.20 (m, 1H), 5.70 (m, 1H), 6.69 (d, J = 8.1Hz, 2H), 7.05 (s, 1H), 7.08 (d, J = 9.0Hz, 2H), 7.40 (d, J = 8.7Hz, 2H) ppm | |
| 13 | 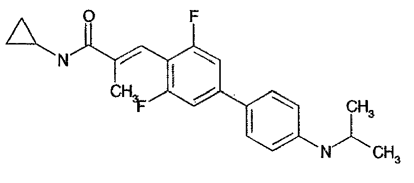 | 1 H NMR (CDCl3) δ 0.60 (m, 2H), 0.84 (m, 2H), 1.25 (d, J = 6.3Hz, 6H), 1.90 (s, 3H), 2.85 (m, 1H), 3.68 (m, 1H), 6.02 (s, 1H), 6.65 (d, J = 8.4Hz, 2H), 7.04 (s, 1 H), 7.08 (d, J = 9.0Hz, 2H), 7.41 (d, J = 8.7Hz, 2H) ppm | |
| 14 | 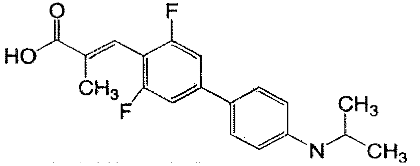 | 1 H NMR (DMSO-d6) δ 1.15 (d, J = 6.3Hz, 6H), 1.85 (s, 3H), 3.60 (m, 1H), 5.86 (s, 1H), 6.62 (d, J = 8.7Hz, 2H), 7.33-7.56 (m, 5H), 12.80 (s, 1H) ppm | |

**[Table 3]**

| | | | |
|---|---|---|---|
| 15 | 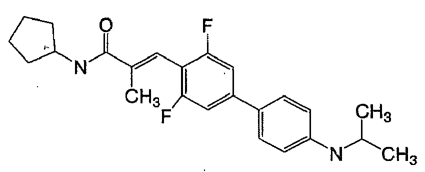 | | 160-162°C |
| 16 | 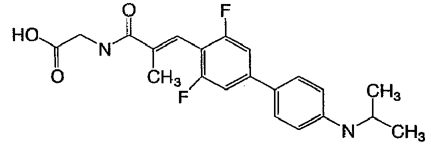 | | 165-167°C |
| 17 | 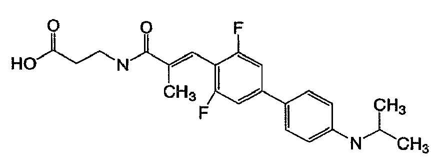 | | 165-167°C |
| 18 | 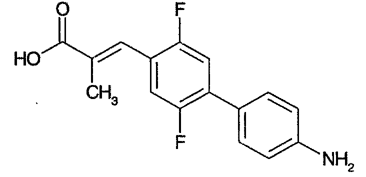 | | 222-225°C |
| 19 | 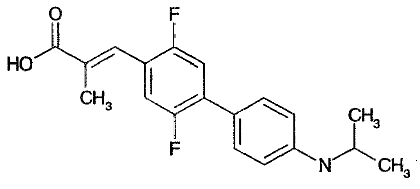 | | 283-185°C |
| 20 | 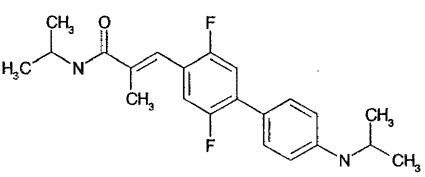 | | 136-138°C |

**[Table 4]**

| Compo und No. | Structure | 1H-NMR | mp |
|---|---|---|---|
| 21 | 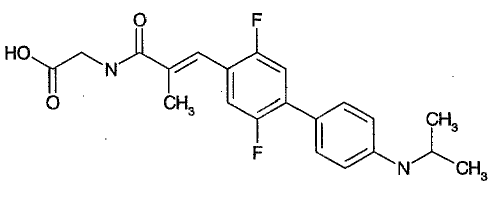 | | 156-158°C |
| 22 | 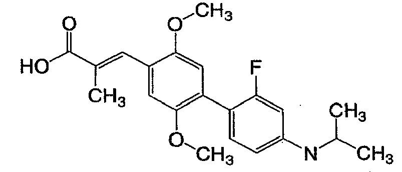 | | 213-216°C |
| 23 | 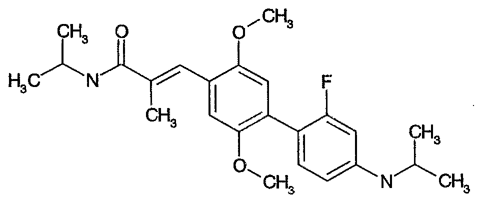 | 1H NMR (CDCl3) δ 1.25 (m, 12H), 2.09 (s, 3H), 3.62(m, 1 H), 3.74(s, 3H), 3.79(s, 3H), 4.21 (m, 1H), 5.54 (m, 1H), 6.36-6.44(m, 2H), 6.82(s, 1H), 6.87(s, 1 H), 7.18(t, J=8.1 Hz, 1 H), 7.33 (s, 1H)ppm | |
| 24 | 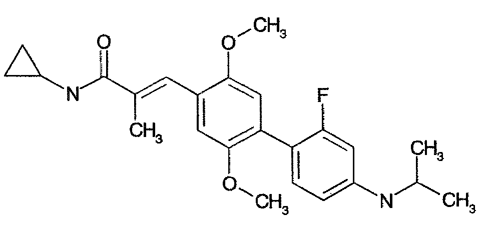 | 1H NMR (CDCl3) δ 0.59-0.62(m, 2H), 0.81-0.87(m, 2H), 1.24(s, 3H), 1.26(s, 3H), 2.08 (s, 3H), 2.83(m, 1H), 3.62(m, 1H), 3.73(s, 3H), 3.79(s, 3H), 6.04 (s,1H), 6.36-6.44(m, 2H), 6.82(s, 1H), 6.86(s, 1H), 7.17(t, J=8.4Hz, 1H), 7.31 (s, 1H)ppm | |
| 25 | 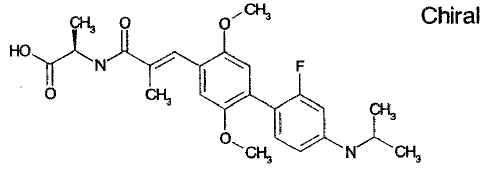 | 1H NMR (CDCl3) δ 1.25 (d, J = 6.6Hz, 6H), 2.09 (s, 3H), 3.62(m, 1H), 3.69(s, 3H), 3.76(s, 3H), 4.06 (m, 1 H), 4.33 (m, 1H), 6.35-6.45(m, 2H), 6.83(s, 1H), 6.94(s, 1 H), 7.09(t, J=7.2Hz, 1H), 7.34 (s, 1 H), 8.16 (brs, 1H)ppm | |

**[Table 5]**

| | | | |
|---|---|---|---|
| 26 | 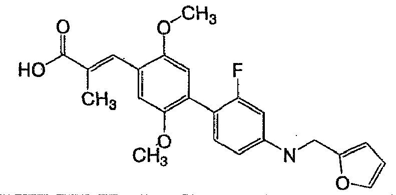 | | 178-180°C |
| 27 | 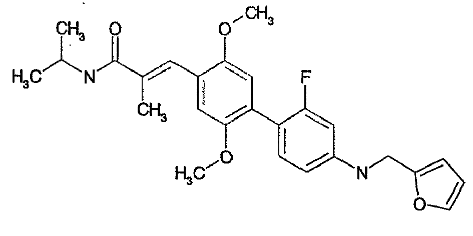 | 1H NMR (CDCl3) δ 1.25 (d, J = 6.6Hz, 6H), 2.09 (s, 3H), 3.62(m, 1 H), 3.74(s, 3H), 3.79(s, 3H), 4.18 (m, 1 H), 4.35 (s, 2H), 5.74 (m, 1 H), 6.29-6.55(m, 3H), 6.82(s, 1H), 6.87 (s, 1H), 7.21 (t, J=8.4Hz, 1 H), 7.26 (s, 1H), 7.39 (m, 1H)ppm | |
| 28 | 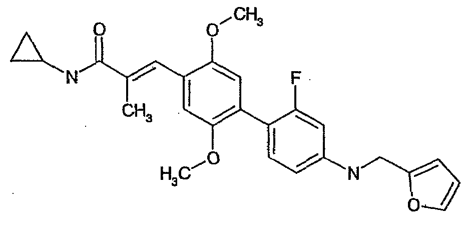 | 1 H NMR (CDCl3) δ 0.56-0.62(m, 2H), 0.81-0.86(m, 2H), 2.08 (s, 3H), 2.83(m, 1 H), 3.73(s, 3H), 3.79(s, 3H), 6.04 (s, 1H), 6.30(m, 1 H), 6.34(m, 1 H), 6.48-6.56(m, 2H), 6.81 (s, 1 H), 6.86(s, 1H), 7.20(t, J=8.1 Hz, 1H), 7.31 (s, 1 H), 7.38(m, 1H)ppm | |
| 29 | 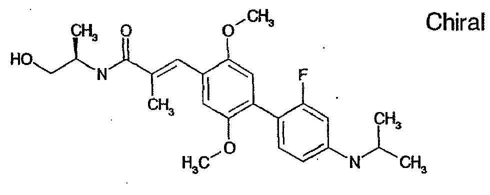 | 1H NMR (CDCl3) δ 1.25 (d, J = 6.6Hz, 6H), 2.10 (s, 3H), 3.62(m, 2H), 3.74(s, 3H), 3.79(s, 3H), 4.22 (m, 1 H), 6.06 (m,1 H), 6.36-6.44(m, 2H), 6.82(s, 1 H), 6.86(s, 1H), 7.18(t, J=8.1Hz, 1H), 7.42 (s, 1 H)ppm | |
| 30 | 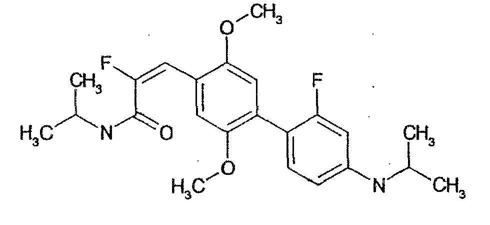 | 1H NMR (CDCl3) δ 1.43 (d, J = 6.6 Hz, 6H), 1.24 (d, J = 6.3 Hz, 6H), 3.57-3.66 (m, 1H), 3.89(s, 6H), 4.12-4.19 (m, 1H), 6.06 (s, 1 H), 6.33-6.41 (m, 2H), 6.79 (s, 1H), 6.95 (s, J = 27.3Hz, 1 H), 7.14-7.19 (m, 1 H), 7.68 (s, 1 H)ppm | |

**[Table 6]**

| | | | |
|---|---|---|---|
| 31 | 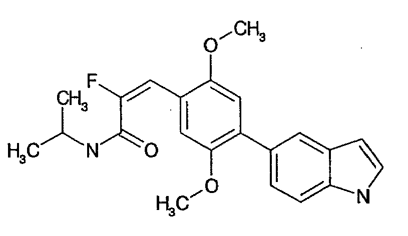 | 1H NMR (CDCl3) δ 1.20 (d, J = 6.6 Hz, 6H), 3.78(s, 3H), 3.84 (s, 3H), 4.14-4.21 (m, 1H), 6.10 (s, 1H), 6.58-6.66 (m, 1H), 6.91 (s, 1 H), 6.99 (d, J = 27.9 Hz, 1H), 7.21-7.24 (m, 1H), 7.38-7.45 (m, 2H), 7.73 (s, 1 H), 7.79 (s, 1H), 8.20 (s, 1 H)ppm | |
| 32 | 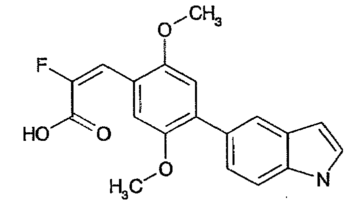 | 1H NMR (CDCl3) δ 3.75 (s, 3H), 3.84 (s, 3H), 6.56 (s, 1H), 6.91 (s, 1H), 7.11 (d, J = 23.7 Hz, 1 H), 7.23-7.24 (m, 1H), 7.32-7.44 (m, 4H), 7.78 (s, 1H), 9.18 (s, 1H)ppm | |
| 33 | 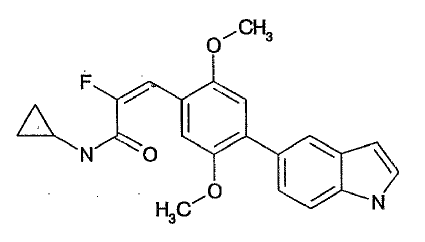 | 1H NMR (CDCl3) 0.56-0.59 (m, 2H), 0.83-0.88 (m, 2H), 3.82 (s, 3H), 3.84 (s, 3H), 4.11-4.14 (m, 1H), 6.36 (s, 1H), 6.60 (s, 1H), 6.91 (s, 1H), 7.01 (d, J = 28.2 Hz, 1 H), 7.23-7.27 (m, 2H), 7.41-7.45 (m, 2H), 7.91 (s, 1H), 8.20 (s, 1H)ppm | |
| 34 | 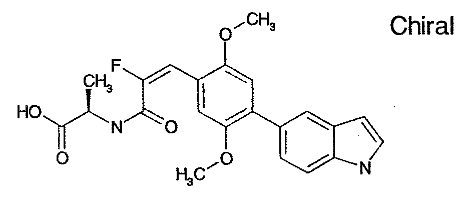 | 1 H NMR (CDCl3) δ 1.52 (d, J = 7.2 Hz, 3H), 3.78 (s, 3H), 3.84 (s, 3H), 4.07-4.17 (m, 1H), 4.67-4.72 (m, 1 H), 6.60 (s, 1H), 6.79 (s, 1H), 6.92 (s, 1H), 7.05 (d, J = 27.0 Hz, 1 H), 7.22-7.26 (m, 1 H), 7.40-7.45 (m, 2H), 7.66 (s, 1H), 7.79 (s, 1H), 8.21 (s, 1H)ppm | |
| 35 | 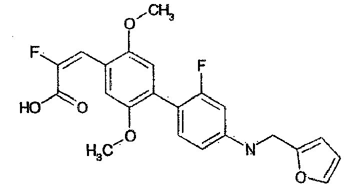 | 1 H NMR (CDCl3) δ 3.74 (s, 3H), 3.81 (s, 3H), 4.34 (s, 2H), 6.29-6.52 (m, 4H), 6.81 (s, 1H), 7.12-7.19 (m, 1H), 7.27 (s, 1H), 7.33 (s, 1H), 7.39 (s, 1H)ppm | |

**[Table 7]**

| | | | |
|---|---|---|---|
| 36 | 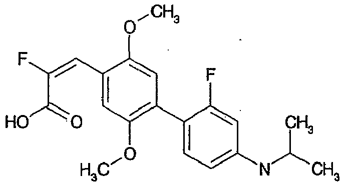 | 1H NMR (CDCl3) δ 1.23 (d, J = 6.3 Hz, 6H), 3.57-3.62 (m, 1H), 3.69 (s, 3H), 3.77 (s, 3H), 6.30-6.38 (m, 2H), 6.78 (s, 1H), 7.07-7.17 (m, 2H), 7.35 (s, 1 H)ppm | |
| 37 | 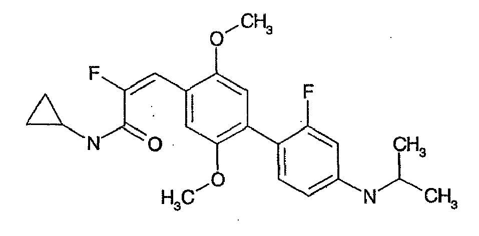 | 1H NMR (CDCl3) δ 0.56-0.57 (m, 2H), 0.80-0.86 (m, 2H), 1.26 (d, J = 6.6 Hz, 6H), 3.58-3.66 (m, 1 H), 3.79 (s, 3H), 3.80 (s, 3H), 6.33-6.42 (m, 3H), 6.78 (s, 1H), 6.97 (d, J = 28.2 Hz, 1H), 7.15-7.21 (m, 1 H), 7.73 (s, 1H)ppm | |
| 38 | 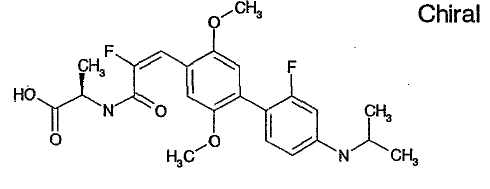 | 1H NMR (CDCl3) δ 1.24 (d, J = 6.3 Hz, 6H), 1.25 (d, J = 7.2 Hz, 3H), 3.77 (s, 3H), 3.80 (s, 3H), 4.65-4.70 (m, 1H), 6.34-6.43 (m, 2H), 6.76-6.83 (m, 2H), 7.00 (s, 1H), 7.14-7.20 (m, 1H), 7.61 (s, 1 H)ppm | |
| 39 | 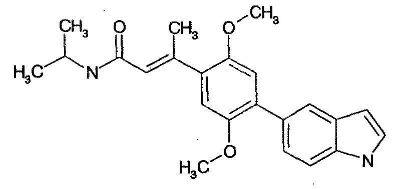 | | 178-180°C |
| 40 | 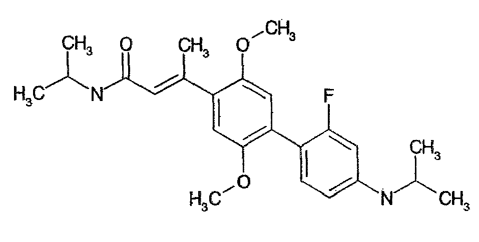 | (d, 6H, J = 6.6 Hz), 1.25 (d, 6H, J = 6.3 Hz), 2.49 (d, 3H, J = 1.2 Hz), 3.62 (1 H, m), 3.75 (s, 3H), 3.78 (s, 3H), 4.20 (1 H, m), 5.35 (d, 1 H, J = 7.8 Hz), 5.80 (m, 1H), 6.30-6.50 (br, 2H), 6.76 (s, 1H), 6.81 (s, 1H), 7.18 (t, 1H, J = 8.4 Hz) | |

**[Table 8]**

| | | | |
|---|---|---|---|
| 41 | 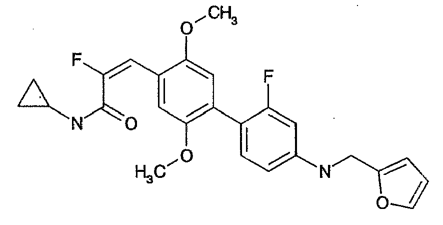 | 1 H NMR (CDCl3) δ 2.78-2.83 (m, 1H), 3.80 (s, 6H), 4.34 (s, 2H), 6.28-6.35 (m, 3H), 6.44-6.53 (m, 2H), 6.78 (s, 1H), 6.97 (d, J = 27.9 Hz, 1 H), 7.16-7.22 (m, 1H), 7.38 (s, 1H), 7.73 (s, 1H)ppm | |
| 42 | 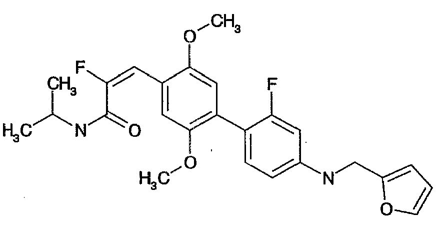 | 1H NMR (CDCl3) δ 1.18 (d, J = 6.6 Hz, 6H), 3.78 (s, 3H), 3.80 (s, 3H), 4.11-4.19 (m, 1H), 4.36 (s, 2H), 6,07 (s, 1H), 6.33 (s, 2H), 6.54-6.62 (m, 2H), 6.77 (s, 1 H), 6.95 (d, J = 27.3 Hz, 1H), 7.26 (s, 1H), 7.38 (s, 1 H), 7.69 (s, 1H)ppm | |
| 43 | 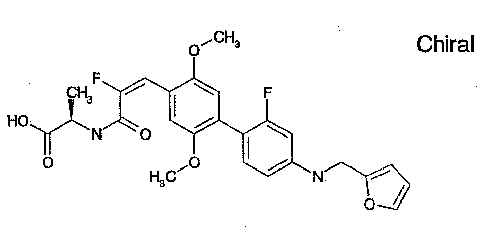 | 1H NMR (CDCl3) δ 1.50 (d, J = 7.2 Hz, 3H), 3.76 (s, 3H), 3.80 (s, 3H), 4.09-4.16 (m, 1 H), 4.65-4.70 (m, 1 H), 6.27-6.35 (m, 2H), 6.43-6.51 (m, 2H), 6.77-6.79 (m, 2H), 6.89 (d, J = 27.0 Hz, 1H), 7.16-7.21 (m, 1H), 7.38 (s, 1H), 7.61 (s, 1H)ppm | |
| 44 | 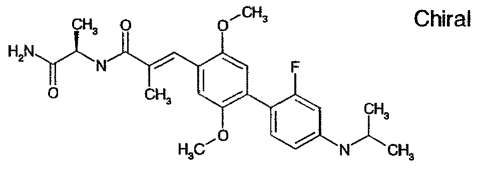 | | 156-157°C |
| 45 | 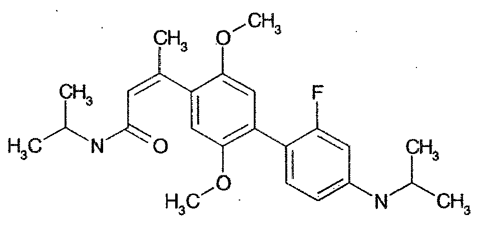 | 1 H NMR (CDCl3) δ 0.81 (d, 6H, J = 6.6 Hz), 1.25 (d, 6H, J = 6.3 Hz), 2.12 (d, 3H, J = 1.2 Hz), 3.62 (m, 1H), 3.72 (s, 3H), 3.81 (s,3H),3.85(m, 1H), 5.25 (d, 1H, J = 8.0 Hz), 5.98 (m, 1H), 6.30-6.50 (m, 2H), 6.68 (s, 1H), 6.85 (s, 1 H), 7.10-7.20 (m, 1H) | |

**[Table 9]**

| | | | |
|---|---|---|---|
| 46 | 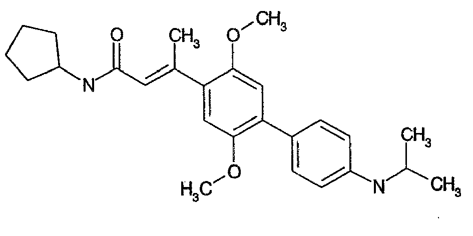 | 1H NMR (DMSO) δ 1.15 (d, 6H, J = 6.3 Hz), 1.30-2.00 (m, 8H), 2.39 (s, 3H), 3.58 (m, 1H), 3.69 (s, 3H), 3.75 (s, 3H), 4.05 (m, 1 H), 5.47 (d, 1H, J = 7.8 Hz), 5.88 (brs, 1H), 6.58 (d, 2H, J = 8.4 Hz), 6.82 (d, 2H, J = 19.5 Hz), 7.27 (d, 2H, J = 8.7 Hz), 7.86 (d, 1H, J = 7.5 (Hz) 1H NMR | |
| 47 | 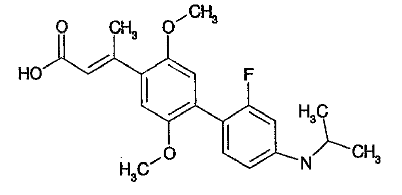 | (DMSO δ 1.14 (d, 6H, J = 6.0 Hz), 2.41 (d, 1 H, J = 1.5 Hz), 2.55-2.60 (m, 1H), 3.69 (s, 3H), 3.74 (s, 3H), 5.82 (d, 1H, J = 8.1 Hz), 5.86 (d, 1H, J = 1.2 Hz), 6.30-6.45 (m, 2H), 6.83 (s, 2H), 7.04 (t, 3H, J = 90HZ) 1H NMR (DMSO-D6) δ | |
| 48 | 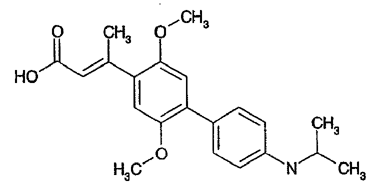 | 1.14 (d, 6H, J = 6.0 Hz), 2.40 (s, 3H), 3.71 (s, 3H), 3.77 (s, 3H), 5.50 (br, 1H), 5.86 (m, 1H), 6.58 (d, 2H, J = 8.7 Hz), 6.82 (s, 1 H), 6.88 (s, 3H), 7.28 (d, 2H, J = 8.7 Hz) | |
| 49 | 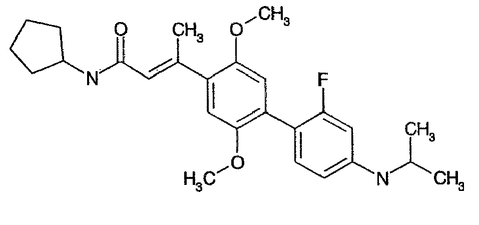 | 1H NMR (CDCl3) δ 1.25 (d, 6H, J = 6.3 Hz), 1.4-2.2 (m, 8H), 2.49 (d, 1H, J = 1.2 Hz), 3.58-3.70(m, 1H), 3.75 (s, 3H), 3.78 (s, 3H), 4.30 (m, 1 H), 5.45 (d, 1H, d = 7.8Hz), 5.92 (s, 1H), 6.35-6.50 (br, 2H), 6.76 (s, 1H), 6.80 (s, 1H), 7.18 (t, 1H, J = 9.0 Hz) | |
| 50 | 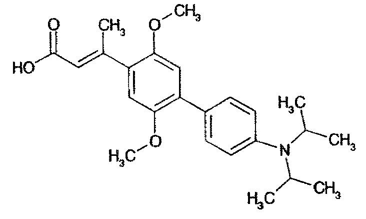 | | |

**[Table 10]**

| | | | |
|---|---|---|---|
| 51 | 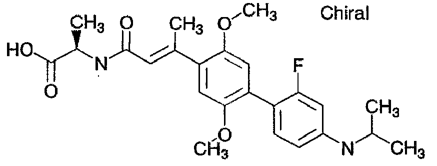 | | |
| 52 | 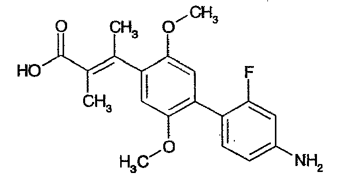 | | |
| 53 | 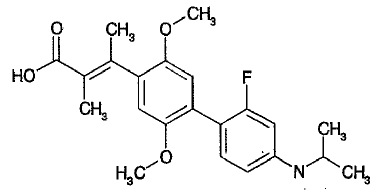 | | |
| 54 | 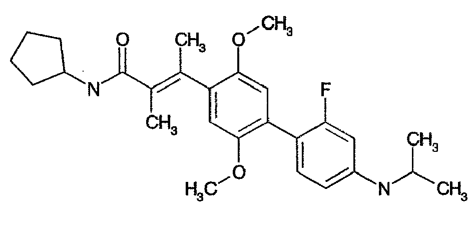 | 1H NMR (CDCl3) δ 1.24 (d, 6H, J = 6.3 Hz), 1.4-1.8 (brm, 8H), 1.74 (s, 3H), 2.06 (s, 3H), 3.62 (m, 1H), 3.73 (s, 3H), 3.77 (s, 3H), 4.35 (m, 1 H), 5.65 (d, 1H, d = 7.8 Hz), 6.10-6.45 (m, 2H), 6.68 (s, 1H), 6.81 (s, 1 H), 7.15 (t, 1H, d = 8.4 Hz) | |
| 55 | 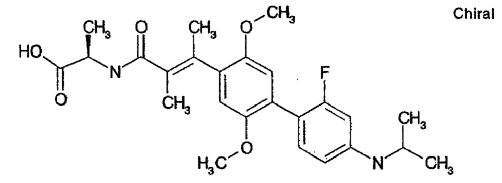 | | |
| 56 | 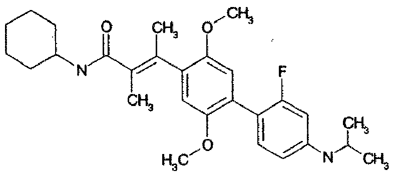 | | |
| 57 | 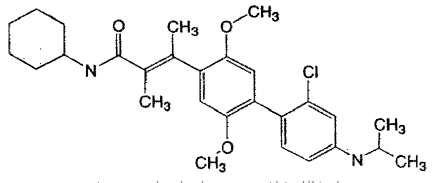 | | |

**[Table 11]**

| | | | |
|---|---|---|---|
| 58 | 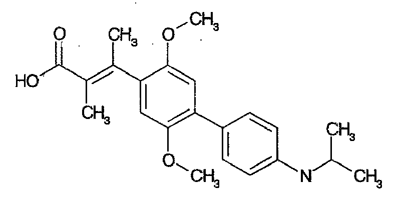 | 1 H NMR (CDCl3) δ 1.26 (d, J = 6.3 Hz, 6H), 1.85 (s, 3H), 2.05 (s, 3H), 2.13 (s, 3H), 3.30 (s, 3H), 3.60 (s, 3H), 6.67 (d, J = 8.4 Hz, 2H), 6.67 (d, J = 8.7 Hz, 2H), 7.82 (s, 1H)ppm | |
| 59 | 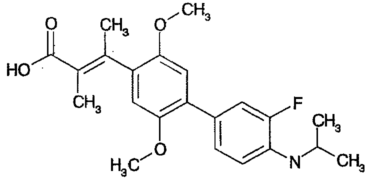 | | |
| 60 | 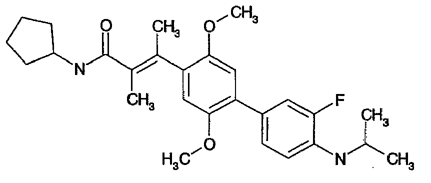 | | |
| 61 | 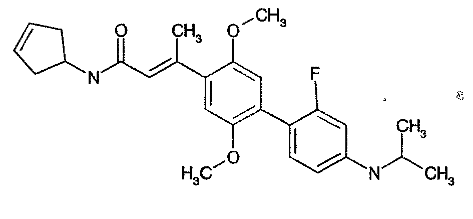 | 1H NMR (DMSO-d6) δ 1.14 (d, 6H, J = 6.3 Hz), 2.17 (dd, 2H, J = 14.7 Hz, 5.1 Hz), 2.40 (d, 3H, J = 1.2 Hz), 2.64 (dd, 2H, J = 14.7 Hz, 8.4 Hz), 3.58 (m, 1 H), 3.67 (s, 3H), 3.72 (s, 3H), 4.40 (m, 1H), 5.72 (s, 2H), 8.81 (d, 1H, J = 7.5 Hz), 5.88 (d, 1 H, 1.2 Hz), 6.30-6.46 (m, 2H), 6.79 (s, 2H), 7.04 (t, 1H, J = 8.4 Hz), 8.09 (d, 1 H, J = 7.2 Hz) | |
| 62 | 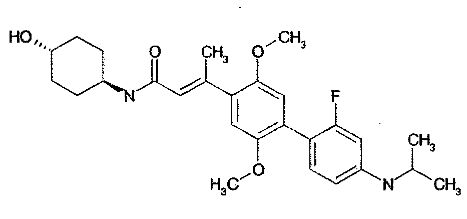 | 1 H NMH (DMSO-d6) δ 1.14 (d, 6H, J = 6.3 Hz), 1.15-1.25 (m, 4H), 1.70-1.90 (m, 4H), 2.39 (s, 3H), 3.50-3.70 (m, 2H), 3.67 (s, 3H), 3.71 (s, 3H), 4.60 (d, 1 H, J = 4.2 Hz), 5.81 (d, 1H, J = 8.1 Hz), 5.85 (s, 1H), 6.30-6.45 (m, 2H), 6.79 (s, 2H), 7.04 (t, 1H, J = 8.7 Hz), 7.55-7.70 (m, 1H), 7.65 (d, 1H, J = 8.1 Hz) | |

**[Table 12]**

| | | | |
|---|---|---|---|
| 63 | 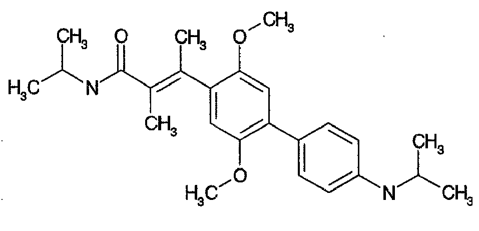 | 1 H NMR (CDCl3) δ 1.25 (d, J = 6.6 Hz. 12H), 1.74 (s, 3H), 2.07 (s, 3H), 3.62-3.70 (m, 1H), 3.73 (s, 3H), 3.79 (s, 3H), 4.17-4.24 (m, 1 H), 5.49 (d, J = 8.1 Hz, 1 H), 6.66 (d, J = 8.1 Hz, 2H), 6.67 (s, 1H), 6.86 (s, 1H), 7.40 (d, J = 8.7 Hz, 2H)ppm | |
| 64 | 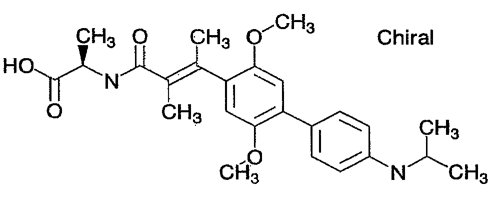 | 1H NMR (CDCl3) δ 1.26 (d, J = 6.6 Hz, 6H), 1.57 (d, J = 7.2 Hz, 3H), 1.76 (s, 3H), 2.03 (s, 3H), 2.12 (s, 3H), 2.13 (s, 3H), 3.70-3.75 (m, 1H), 3.73 (s,3H), 3.78 (s, 3H), 4.69-4.74 (m, 1 H), 6.28 (d, J = 6.9 Hz, 1H), 6.64 (s, 1H), 6.69 (d, J = 8.1 Hz, 2H), 6.89 (s, 1H), 7.39 (d, J = 8.7 Hz, 2H)ppm | |
| 65 | 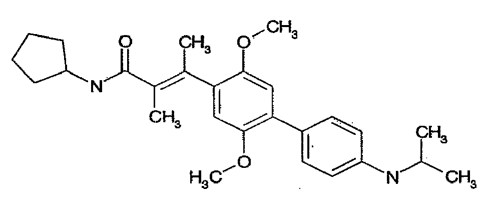 | 1H NMR (CDCl3) δ 1.26 (d, J = 6.3 Hz, 6H), 1.48-1.71 (m, 6H), 175 (s, 3H), 1.98-2.02 (m, 2H), 2.07 (s, 3H), 3.62-3.67 (m, 1H), 3.73 (s, 3H), 3.79 (s, 3H), 4.32-4.39 (m, 1H), 5.62 (d, J = 7.5 Hz, 1H), 6.65-6.83 (m, 3H), 7.26 (s, 1H), 7.40 (d, J = 8.7 Hz, 2H)ppm | |

**[Table 13]**

| Compo und No. | Structure | 1HNMR | mp |
|---|---|---|---|
| 66 | 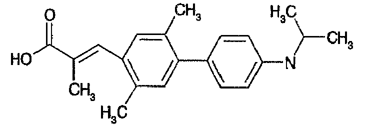 | 1H NMR (CDCl3) δ 1.25 (d, J = 6.0Hz, 6H), 2.07 (s, 3H), 2.29 (s, 3H), 2.30 (s, 3H), 3.67 (m, 1 H), 6.64 (d, J = 8.4Hz, 2H), 7.01-7.18 (m, 4H), 7.92 (s, 1H) ppm | |
| 67 | 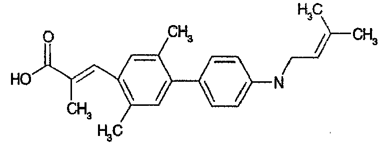 | 1H NMR (CDCl3) δ 1.75 (d, J = 10.8Hz, 6H), 2.07 (s, 3H), 2.30 (s, 6H), 3.75 (d, J = 6.6Hz, 2H), 5.37 (m, 1H), 6.66 (d, J = 8.7Hz, 2H), 7.11-7.19 (m, 4H), 7.92 (s, 1 H) ppm | |
| 68 | 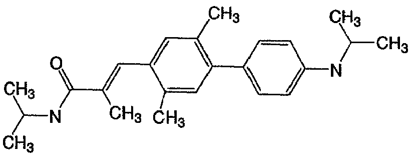 | 1H NMR (CDCl3) δ 1.23 (s, 6H), 1.26 (s, 6H), 2.00 (s, 3H), 2.25 (s, 3H), 2.27 (s, 3H), 3.67 (m, 1H), 4.21 (m, 1H), 5.65 (m, 1H), 6.63 (d, J = 8.4Hz, 2H), 7.03 (s, 1H), 7.07 (s, 1H). 7.15 (d, J = 8.7Hz, 2H), 7.40 (s, 1H) ppm | |
| 69 | 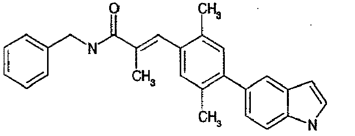 | 1 H NMR (CDCl3) δ 2.06 (s, 3H), 2.28 (s, 6H), 3.67 (m, 1H), 4.61 (d, J = 5.7Hz, 2H), 6.16 (m, 1 H), 6.58 (s, 1H), 7.08-7.58 (m, 12H), 8.22 (s, 1H) ppm | |
| 70 | 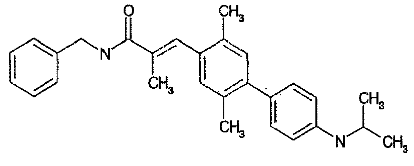 | 1 H NMR (CDCl3) δ 1.24 (d, J = 5.7Hz, 6H), 2.03 (s, 3H), 2.25 (s, 3H), 2.27 (s, 3H), 3.66 (m, 1H), 4.60 (d, J = 5.7Hz, 2H), 6.15 (m, 1 H), 6.63 (d, J = 8.4Hz, 2H), 7.04 (s, 1H), 7.07 (s, 1H), 7.14 (d, J = 8.7Hz, 2H), 7.30-7.38 (m, 5H), 7.49 (s, 1H) ppm | |
| 71 | 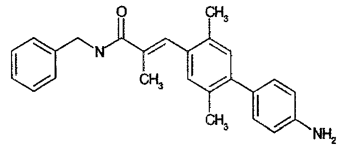 | 1 H NMR (CDCl3) δ 2.03 (s, 3H), 2.25 (s, 6H), 4.60 (d, J = 6.0Hz, 2H), 6.17 (m, 1H), 6.73 (d, J = 8.7Hz, 2H), 7.04 (s, 1H), 7.07 (s, 1H), 7.13 (d, J = 8.4Hz, 2H), 7.30-7.38 (m, 5H), 7.49 (s, 1H) ppm | |
| 72 | 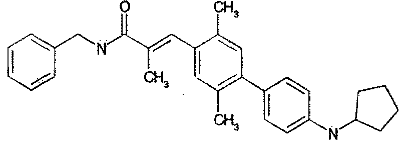 | 1H NMR (CDCl3) δ 1.47-1.80 (m, 6H), 2.03 (m, 5H), 2.25 (s, 3H), 2.27 (s, 3H), 3.81 (m, 1H), 4.60 (d, J = 6.0Hz, 2H), 6.15 (m, 1 H), 6.64 (d, J = 8.4Hz, 2H), 7.04 (s, 1H), 7.08 (s, 1H), 7.14 (d, J = 8.7Hz, 2H), 7.28-7.38 (m, 5H), 7.49 (s, 1H) ppm | |

**[Table 14]**

| | | | |
|---|---|---|---|
| 73 | 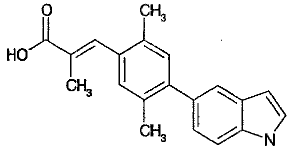 | 1 H NMR (CDCl3) δ 2.09 (s, 3H), 2.31 (s, 3H), 2.32 (s, 3H), 6.60 (m, 1H), 7.17-7.28 (m, 4H), 7.44 (d, J = 8.4Hz, 1 H), 7.60 (s, 1 H), 7.94 (s, 1H), 8.22 (s, 1H) ppm | |
| 74 | 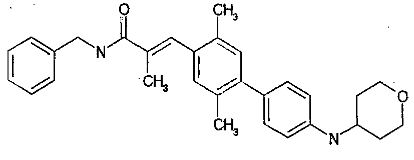 | 1H NMR (CDCl3) δ 1.53 (m, 2H), 2.03-2.10 (m, 5H), 2.25 (s, 3H), 2.27 (s, 3H), 3.53 (m, 3H), 4.02 (m, 2H), 4.60 (d, J = 4.60Hz, 2H), 6.17 (m, 1H), 6.67 (d, J = 8.4Hz, 2H), 7.04 (s, 1H), 7.06 (s, 1H), 7.16 (d, J = 8.4Hz, 2H), 7.28-7.38 (m, 5H), 7.49 (s, 1H) ppm | |
| 75 | 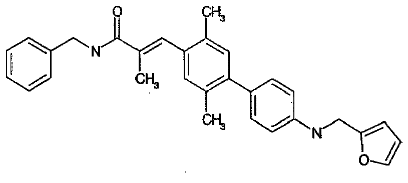 | 1 H NMR (COCl3) δ 2.03 (s, 3H), 2.25 (s, 3H), 2.26 (s, 3H), 4.36 (s, 2H), 4.60 (d, J = 5.7Hz, 2H), 6.15 (m, 1 H), 6.27-6.35 (m, 2H), 6.72 (d, J = 8.7Hz, 2H), 7.03 (s, 1H), 7.07 (s, 1H), 7.12 (d, J = 8.4Hz, 2H), 7.30-7.39 (m, 6H), 7.49 (s, 1H) ppm | |
| 76 | 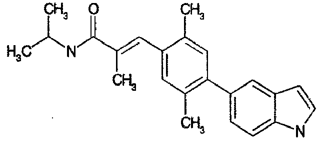 | 1 H NMR (CDCl3) δ 1.25 (d, J = 6.6Hz, 6H), 2.02 (s, 3H), 2.27 (s, 6H), 4.24 (m, 1H), 5.70 (m, 1H), 6.59 (m, 1H), 7.07 (s, 1H), 7.60-7.94 (m, 7H), 8.27 (s, 1H) ppm | |
| 77 | 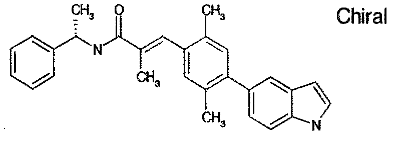 | 1H NMR (CDCl3) δ 1.59 (m, 6H), 2.04 (s, 3H), 2.26 (s, 3H), 2.28 (s, 3H), 5.29 (m, 1H), 6.07 (m, 1H), 6.57 (m, 1H), 7.08-7.58 (m, 10H), 8.24 (s, 1H) ppm | |
| 78 | 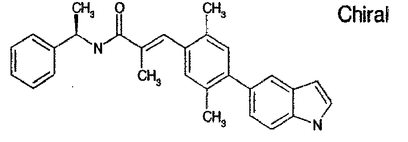 | 1H NMR (CDCl3) δ 1.59 (m, 6H), 2.04 (s, 3H), 2.26 (s, 3H), 2.28 (s, 3H), 5.29 (m, 1H), 6.07 (m, 1H), 6.57 (m, 1H), 7.08-7.58 (m, 10H), 8.24 (s, 1H) ppm | |
| 79 | 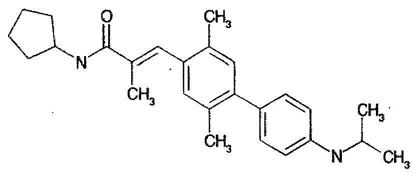 | 1H NMR (CDCl3) δ 1.25 (d, J = 6.3Hz, 6H), 1.43-1.75 (m, 6H), 2.00 (s, 3H), 2.06 (m, 2H), 2.45 (s, 3H), 2.27 (s, 3H), 3.67 (m, 1H), 4.37 (m, 1H), 5.78 (m, 1H), 6.63 (d, J = 8.4Hz, 2H), 7.03 (s, 1H), 7.07 (s, 1H), 7.15 (d, J = 8.4Hz, 2H), 7.40 (s, 1H) ppm | |

**[Table 15]**

| | | | |
|---|---|---|---|
| 80 | 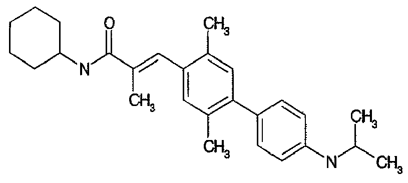 | 1H NMR (CDCl3) δ 1.25 (d, J = 6.3Hz, 6H), 1.43-1.75 (m, 8H), 2.00 (s, 3H), 2.06 (m, 2H), 2.45 (s, 3H), 2.27 (s, 3H), 3.66 (m, 1H), 3.92 (m, 1H), 5.71 (m, 1H), 6.62 (d, J = 8.7Hz, 2H), 7.04 (s, 1H), 7.07 (s, 1H), 7.14 (d, J = 8.7Hz, 2H), 7.40 (s, 1H) ppm | |
| 81 | 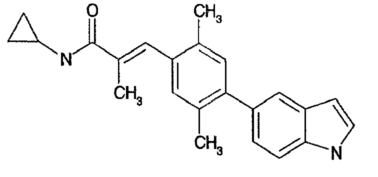 | 1H NMR (CDCl3) δ 0.62 (m, 2H), 0.85 (m, 2H), 2.00 (s, 3H), 2.26 (s, 3H), 2.28 (s, 3H), 2.86 (m, 1H), 5.98 (brs, 1H), 6.58 (m, 1H), 7.06 (s, 1 H), 7.07 (s, 1 H), 7.15-7.44 (m, 6H), 7.58 (s, 1H), 8.24 (s, 1H) ppm | |
| 82 | 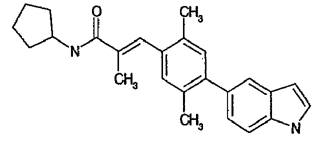 | | 145.5 °C |
| 83 | 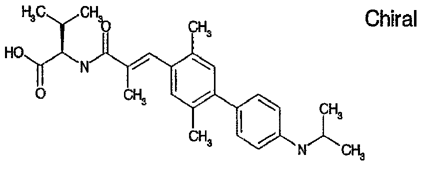 | 1H NMR (CDCl3) δ 0.97 (s, 3H), 1.26 (d, J = 6.3Hz, 6H), 1.57 (d, J = 7.2Hz, 3H), 2.03 (s, 3H), 2.15 (s, 3H), 2.16 (s, 3H), 3.12 (m, 1H),3.65 (m, 1H), 4.49 (m, 1 H), 6.59 (d, J = 8.4Hz, 2H), 6.95 (s, 1 H), 7.00 (s, 1 H), 7.22-7.34 (m, 4H), 7.55 (s, 1H) ppm | |
| 84 | 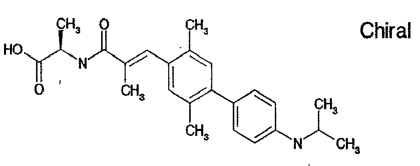 | 1H NMR (CDCl3) δ 1.25 (d, J = 6.3Hz, 6H), 2.04 (s, 3H), 2.15 (s, 3H), 2.17 (s, 3H), 3.69 (m, 1H), 4.73 (m, 1H), 6.55 (d, J = 8.4Hz, 2H), 6.94 (s, 1 H), 7.01 (s, 1H), 7.20-7.28 (m, 4H), 7.48 (s, 1H) ppm | |
| 85 | 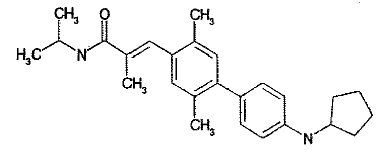 | 1 H NMR (CDCl3) δ 1.25 (d, J = 6.6Hz, 6H), 1.49-1.79 (m, 6H), 2.00 (s, 3H), 2.08 (m, 2H), 2.25 (s, 3H), 2.27 (s, 3H), 3.82 (m, 1H), 4.23 (m, 1H), 5.66 (m, 1H), 6.67 (d, J = 8.4Hz, 2H), 7.04 (s, 1 H), 7.07 (s, 1H), 7.15 (d, J = 8.7Hz, 2H), 7.41 (s, 1H) ppm | |
| 86 | 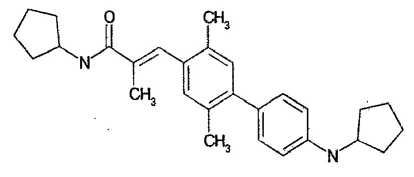 | 1H NMR (CDCl3) δ 1.43-1.79 (m, 12H), 2.00 (s, 3H), 2.08 (m, 4H), 2.25 (s, 3H), 2.27 (s, 3H), 3.82 (m, 1H), 4.34 (m, 1H), 5.78 (m, 1H), 6.67 (d, J = 8.4Hz, 2H), 7.04 (s, 1 H), 7.07 (s, 1 H), 7.15 (d, J = 8.7Hz, 2H), 7.40 (s, 1H) ppm | |

**[Table 16]**

| | | | |
|---|---|---|---|
| 87 | 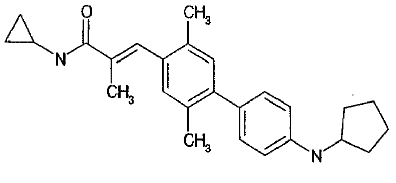 | 1H NMR (CDCl3) δ 0.62 (m, 2H), 0.85 (m, 2H), 1.48-1.78 (m, 6H), 2.01 (s, 3H), 2.08 (m, 2H), 2.26 (s, 3H), 2.28 (s, 3H), 3.80 (m, 1H), 4.21 (m, 1H), 5.65 (m, 1H), 6.69 (d, J = 8.4Hz, 2H), 7.05 (s, 1 H), 7.08 (s, 1 H), 7.17 (d, J = 8.7Hz, 2H), 7.43 (s, 1 H) ppm | |
| 88 | 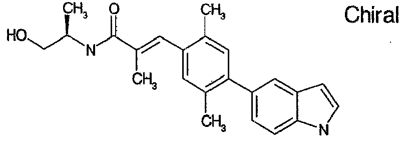 | 1H NMR (CDCl3) δ 1.28 (d, J = 6.9Hz, 3H), 2.05 (s, 3H), 2.27 (s, 6H), 3.70 (m, 1H), 4.11 (m, 1H), 4.29 (m, 1H), 6.04 (m, 1H), 6.59 (brs, 1H), 7.09 (s, 1H), 7.14-7.58 (m, 7H), 8.26 (s, 1H) ppm | |
| 89 | 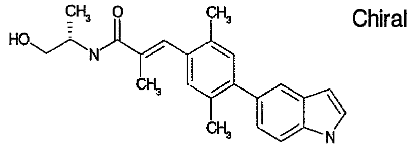 | 1H NMR (CDCl3) δ 1.28 (d, J = 6.9Hz, 3H), 2.05 (s, 3H), 2.27 (s, 6H), 3.70 (m, 1H), 4.11 (m, 1 H), 4.28 (m, 1 H), 6.04 (m, 1H), 6.58 (brs, 1 H), 7.08 (s, 1 H), 7.16-7.59 (m, 7H), 8.27 (s, 1H) ppm | |
| 90 | 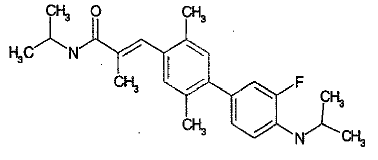 | 1H NMR (CDCl3) δ 1.26(m, 12H), 1.99(s, 3H), 2.20(s, 3H), 2.27(s, 3H), 3.66(m, 1H), 3.76(b, 1H), 4.20(m, 1H), 5.68(d, J=7.5Hz, 1 H), 6.73(t, J=8.4Hz, 1 H), 6.97(d, J=8.4Hz, 1 H), 6.99(d, J=5.4Hz, 1 H), 7.05(s, 1H), 7.25(s, 1H), 7.40(s, 1H)ppm | |
| 91 | 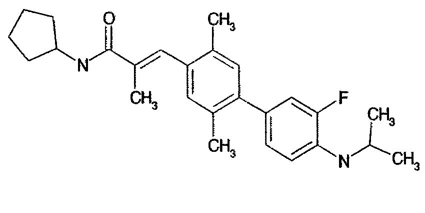 | 1 H NMR (CDCl3) δ 1.26(s, 3H), 1.28(s, 3H), 1.41-1.52(m, 2H), 1.61-1.77(m, 4H), 1.99(s, 3H), 2.04-2.14(m, 2H), 2.24(s, 3H), 2.27(s, 3H), 3.68(m, 1H), 3.76(b, 1 H), 4.33(m, 1 H), 5.80(d, J=7.2Hz, 1 H), 6.72(t, J=8.4Hz, 1 H), 6.97(d, J=8.4Hz, 1H), 6.98(d, J=5.4Hz, 1H), 7.03(s, 1H), 7.05(s, 1 H), 7.40(s,1H)ppm | |
| 92 | 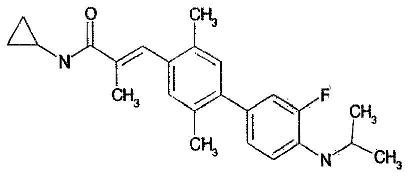 | 1H NMR (CDCl3) δ 0.62 (m, 2H), 0.85 (m, 2H), 1.25 (d, J = 6.6Hz, 6H), 1.98(s, 3H), 2.11(s, 3H), 2.27(s, 3H), 2.85(m, 1H), 3.75(m, 1H), 5.97 (m, 1H), 6.72(t, J=8.1 Hz, 1 H), 6.97(d, J=8.4Hz, 1H), 6.99(d, J=5.4Hz, 1 H), 7.04(s, 1 H), 7.26(s, 1 H), 7.39(s,1H)ppm | |

**[Table 17]**

| | | | |
|---|---|---|---|
| 93 | 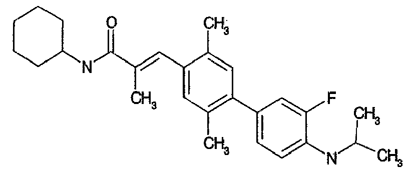 | 1 H NMR (CDCl3) δ 1.25(s, 3H), 1.26(s, 3H), 1.27-1.52(m, 2H), 1.61-1.77(m, 6H), 2.00(s, 3H), 2.05-2.12(m, 2H), 2.24(s, 3H), 2.27(s, 3H), 3.69(m, 1H), 3.76(m, 1H), 4.35(m, 1 H), 5.72(m, 1 H), 6.72(t, J=8.4Hz, 1 H), 6.97(d, J=8.4Hz, 1 H), 6.98(d, J=5.4Hz, 1 H), 7.03(s, 1 H), 7.04(s, 1 H), 7.39(s, 1H)ppm | |
| 94 | 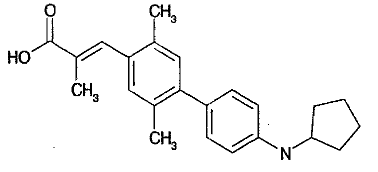 | 1 H NMR (CDCl3) δ 1.50-1.80 (m, 6H), 2.00 (s, 3H), 2.05 (m, 2H), 2.10 (s, 6H), 3.82 (m, 1 H), 6.82 (s, 1H), 7.06 (s, 1H), 7.25 (d, J = 8.1 Hz, 2H), 7.59 (d, J = 8.4Hz, 2H), 7.82 (s, 1H) ppm | |
| 95 | 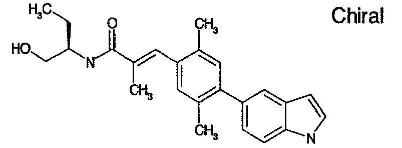 | | |
| 96 | 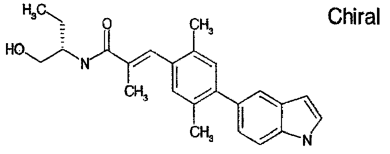 | | |
| 97 | 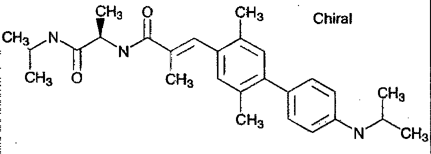 | | |
| 98 | 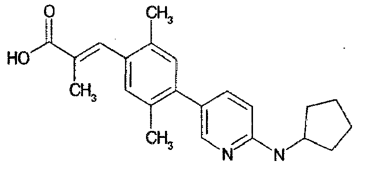 | 1H NMR (CDCl3) δ 1.25-1.80 (m, 6H), 2.03 (s, 3H), 2.04 (m, 2H), 2.10 (s, 6H), 3.92 (m, 1H), 6.53 (d, J=8.4Hz, 1H), 7.05 (s, 1 H), 7.14 (s, 1H), 7.44-7.71 (m, 2H), 7.81 (s, 1H), 7.97 (s, 1H) ppm | |
| 99 | 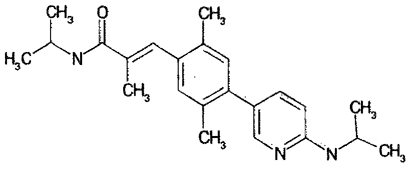 | 1H NMR (CDCl3) δ 1.25 (m, 12H), 1.99 (s, 3H), 2.26(s, 3H), 2.27(s, 3H), 3.89(m, 1 H), 4.23 (m, 1H), 5.67 (m, 1H), 7.04(s, 1H), 7.05(s, 1H), 7.40(s, 2H), 7.50(dd, J = 2.4, 8.7Hz, 1 H), 8.02 (s, 1H)ppm | |

**[Table 18]**

| | | | |
|---|---|---|---|
| 100 | 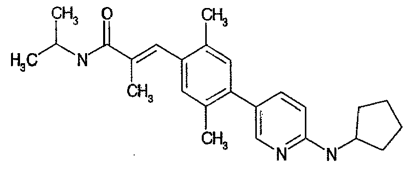 | 1H NMR (CDCl3) δ 1.25 (d, J = 6.6Hz, 6H), 1.63 (m, 6H), 2.03 (s, 3H), 2.04 (m, 2H), 2.25 (s, 6H), 3.93 (m, 1H), 4.25 (m, 1H), 5.66 (m, 1H), 6.48 (d, J=8.7Hz, 1 H), 7.04 (s, 1H), 7.05 (s, 1 H), 7.44-7.71 (m, 2H), 7.97 (s, 1H) ppm | |
| 101 | 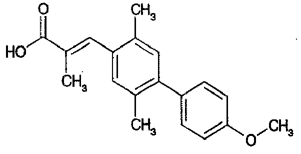 | 1H NMR (CDCl3) δ 2.65 (d, J = 1.5Hz, 3H), 2.27 (s, 3H), 2.29(s, 3H), 3.85(s, 3H), 6.96(d, J = 6.9Hz, 2H), 7.10 (s, 1H), 7.15 (s, 1H), 7.27(d, J = 6.9Hz, 2H), 7.90 (s, 1H)ppm | |
| 102 | 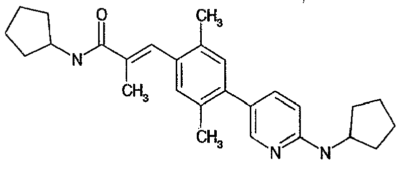 | 1H NMR (CDCl3) δ 1.26-1.81 (m, 12H), 2.03 (s, 3H), 2.07 (m, 4H), 2.10 (s, 6H), 3.99 (m, 1H), 4.23 8m, 1H), 6.53 (d, J=8.4Hz, 1H), 7.05 (s, 1H), 7.14 (s, 1H), 7.44-7.71 (m, 2H), 7.81 (s, 1H), 7.97 (s, 1H) ppm | |
| 103 | 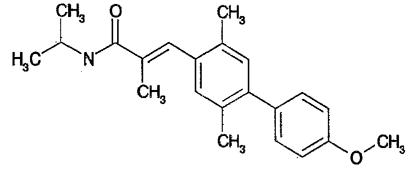 | 1H NMR (CDCl3) δ 1.25 (d, J = 6.9Hz, 6H), 1.99 (s, 3H), 2.25 (s, 3H), 2.26 (s, 3H), 3.85(s, 3H), 4.25 (m, 1 H), 5.66 (m, 1H), 6.92(d, J = 6.9Hz, 2H), 6.98 (s, 1 H), 7.05 (s, 1H), 7.27(d, J = 6.9Hz, 2H), 7.41 (s, 1H)ppm | |
| 104 | 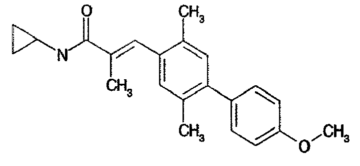 | 1H NMR (CDCl3) δ 0.62 (m, 2H), 0.85 (m, 2H), 1.98 (s, 3H), 2.82 (s, 6H), 3.86(s, 3H), 5.96 (brs, 1 H), 6.95(d, J = 9.0Hz, 2H), 7.03 (s, 1H), 7.06 (s, 1H), 7.27(d, J = 9.0Hz, 2H), 7.39 (s, 1H)ppm | |
| 105 | 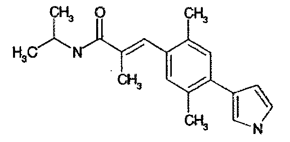 | 1H NMR (CDCl3) δ 1.24 (d, J = 6.9Hz, 6H), 1.99 (s, 3H), 2.40 (s, 3H), 4.25 (m, 1H), 5.65 (m, 1H), 6.42 (s, 1H), 6.83-7.25 (m, 5H), 7.39 (s, 1 H), 8.35 (s, 1H)ppm | |
| 106 | 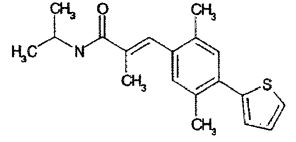 | 1H NMR (CDCl3) δ 1.25 (d, J = 6.6Hz, 6H), 1.98 (s, 3H), 2.25 (s, 3H), 2.39 (s, 3H), 4.21 (m, 1 H), 5.64 (brs, 1H), 7.08-7.33 (m, 5H), 7.39 (s, 1H) ppm | |

**[Table 19]**

| | | | |
|---|---|---|---|
| 107 | 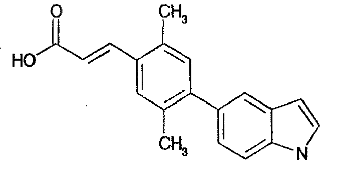 | 1H NMR (CDCl3) δ 2.31 (s, 3H), 2.47 (s, 3H), 6.44 (d, J = 15.6Hz, 1 H), 6.59-6.60 (m, 1 H), 7.17 (d, J = 8.4Hz, 1H), 7.18 (s, 1 H), 7.27 (s, 1H), 7.44 (d, J = 8.4Hz, 1 H), 7.53 (s, 1H), 7.58 (s, 1H), 8.12 (d, J = 15.9Hz, 1H), 8.23 (s, 1 H)ppm | |
| 108 | 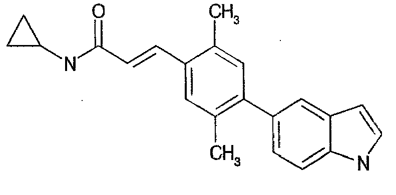 | 1H NMR (CDCl3) δ 0.61 (s, 2H), 0.85-0.87 (m, 2H), 2.28 (s, 3H), 2.44 (s, 3H), 2.88-2.89 (m, 1H), 5.77 (s, 1H), 6.23 (d, J = 15.0Hz, 1H), 6.59 (s, 1H), 7.15-7.17 (m, 2H), 7.28 (s, 1 H), 7.42 (s, 1H), 7.43 (d, J = 8.4Hz, 1 H), 7.58 (s, 1H), 7.95 (d, J = 15.5Hz, 1H), 8.29 (s, 1H)ppm | |
| 109 | 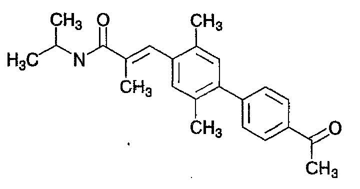 | 1H NMR (CDCl3) δ 1.25 (d, J = 6.6Hz, 6H), 1.99 (s, 3H), 2.27 (s, 3H), 2.28 (s, 3H), 2.65 (s, 3H), 4.21 (m, 1H), 5.66 (brs, 1H), 7.07 (s, 6H), 7.42-7.45 (m, 3H), 8.01 (d, J = 8.4Hz, 2H) ppm | |
| 110 | 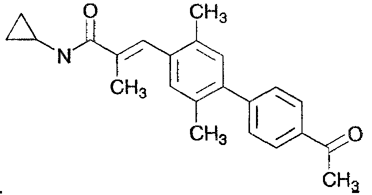 | 1H NMR (CDCl3) δ 0.62 (m, 2H), 0.85 (m, 2H), 1.98 (s, 3H), 2.24 (s, 3H), 2.27 (s, 3H), 2.65 (s, 3H), 2.88 (m, 1H), 5.98 (brs, 1 H), 7.06(s, 1 H), 7.07 (s, 1H), 7.42 (m, 3H), 8.00 (d, J = 8.1 Hz, 2H),ppm | |
| 111 | 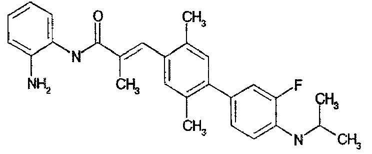 | 1H NMR (CDCl3) δ 1.25 (d, J = 6.6Hz, 6H), 2.15 (s, 3H), 2.28(s, 3H), 2.29(s, 3H), 3.69(m, 1H), 3.76(b, 1H), 6.77(t, J=8.4Hz, 1H), 6.97-7.05(m, 8H), 7.62(s, 2H), 7.66(s,1H)ppm | |
| 112 | 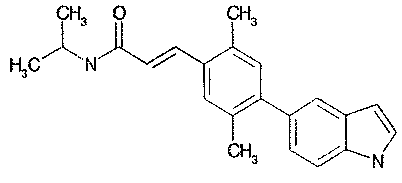 | 1H NMR (CDCl3) δ 1.24 (d, J = 6.6Hz, 6H), 2.28 (s, 3H), 2.44 (s, 3H), 4.21-4.28 (m, 1H), 5.42-5.46 (m, 1H), 6.31 (d, J = 15.3Hz, 1H), 6.59 (s, 1H), 7.15-7.17 (m, 2H), 7.26 (s, 1H), 7.42-7.43 (m, 2H), 7.57 (s, 1H), 7.93 (d, J = 15.6Hz, 1H), 8.25-8.30 (m, 1H)ppm | |

**[Table 20]**

| | | | |
|---|---|---|---|
| 113 | 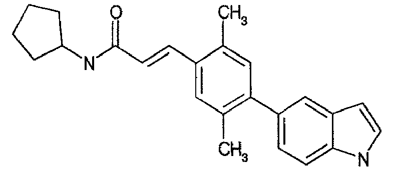 | 1H NMR (COCl3) δ 1.44-1.73 (m, 6H), 2.05-2.09 (m, 2H), 2.28 (s, 3H), 2.43 (s, 3H), 4.34-4.38 (m, 1H), 5.57-5.59 (m, 1 H), 6.32 (d, J = 15.3Hz, 1 H), 6.58 (s, 1 H), 7.14-7.27 (m, 2H), 7.42-7.44 (m, 2H), 7.54 (s, 1H), 7.93 (d, J = 15.0Hz, 1H), 8.28 (s, 1 H)ppm | |
| 114 | 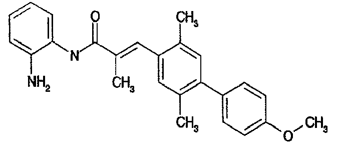 | 1H NMR (CDCl3) δ 2.17 (s, 3H), 2.27 (s, 3H), 2.29 (s, 3H), 3.86(s, 3H), 6.90-7.33 (m, 10H), 7.63(s, 1 H), 7.77 (s, 1 H)ppm | |
| 115 | 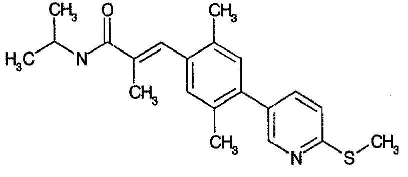 | 1 H NMR (CDCl3) δ 1.25 (d, J = 6.6Hz, 6H), 1.99 (s, 3H), 2.29(s, 3H), 2.30(s, 3H), 2.69(s, 3H), 4.22(m, 1H), 5.67 (m, 1H), 7.02(d, J = 6.6Hz, 2H), 7.40(s, 2H), 7.23-7.27 (m, 3H), 8.44 (s, 1H)ppm | |
| 116 | 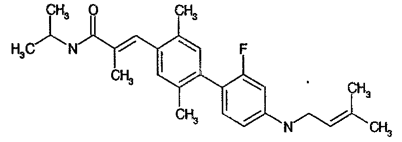 | | 124.4 °C |
| 117 | 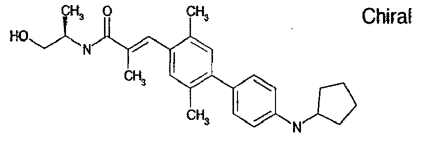 | 1H NMR (CDCl3) δ 1.28 (d, J = 6.9Hz, 3H), 2.05 (s, 3H), 2.27 (s, 6H), 3.70 (m, 1H), 4.11 (m, 1H), 4.29 (m, 1 H), 6.04 (m, 1 H), 6.69 (d, J = 8.4Hz, 2H), 7.05 (s, 1 H), 7.08 (s, 1H), 7.17 (d, J = 8.7Hz, 2H), 7.43 (s, 1 H) ppm | |
| 118 | 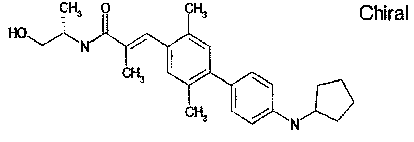 | 1H NMR (COCl3) δ 1.28 (d, J = 6.9Hz, 3H), 2.05 (s, 3H), 2.27 (s, 6H), 3.70 (m, 1H), 4.11 (m, 1H), 4.28 (m, 1H), 6.04 (m, 1H), 6.69 (d, J = 8.4Hz, 2H), 7.05 (s, 1H), 7.08 (s, 1H), 7.17 (d, J = 8.7Hz, 2H), 7.43 (s, 1H) ppm | |
| 119 | 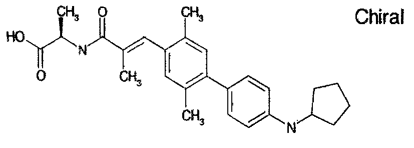 | 1H NMR (CDCl3) δ 1.25 (d, J = 6.3Hz, 6H), 1.49-1.79 (m, 6H), 2.04 (s, 3H), 2.08 (m, 2H), 2.15 (s, 3H), 2.17 (s, 3H), 3.69 (m, 1 H), 4.73 (m, 1H), 6.55 (d, J = 8.4Hz, 2H), 6.91 (s, 1H), 7.03 (s, 1H), 7.20-7.29 (m, 4H), 7.43 (s, 1 H) ppm | |

**[Table 21]**

| | | | |
|---|---|---|---|
| 120 | 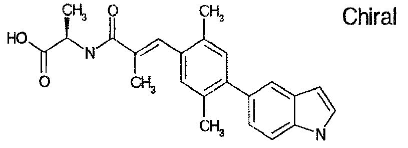 | 1 H NMR (CDCl3) δ 1.59 (d, J = 6.3Hz, 3H), 2.08 (s, 3H), 2.28 (s, 6H), 4.72 (m, 1H), 6.40 (m, 1H), 6.59 (brs, 1H), 7.08 (s, 1H), 7.15-7.44 (m, 4H), 7.55 (s, 2H), 8.23 (s, 1H) ppm | |
| 121 | 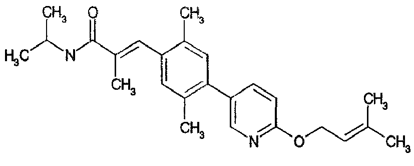 | 1H NMR (CDCl3) δ 1.25 (d, J = 6.6Hz, 6H), 1.80 (d, J = 9.3Hz, 6H),1.99 (s, 3H), 2.26(s, 3H), 2.27(s, 3H), 4.22(m, 1 H), 4.88 (m,2H), 5.69 (m, 2H), 6.81 (d, J = 5.1Hz, 4H), 7.23-7.27 (m, 3H), 8.13 (d, , J = 2.1 Hz, 1H)ppm | |
| 122 | 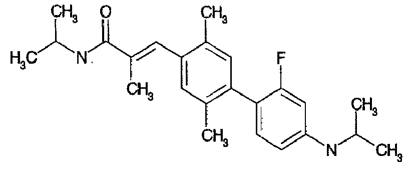 | 1 H NMR (CDCl3) δ 1.24 (s, 6H), 1.26 (s, 6H), 2.00 (s, 3H), 2.19 (s, 3H), 2.24 (s, 3H), 3.63 (m, 1H), 4.22(m, 1H), 5.65(m, 1H), 6.38 (m,2H), 7.00(m, 3H), 7.40 (s, 1H)ppm | |
| 123 | 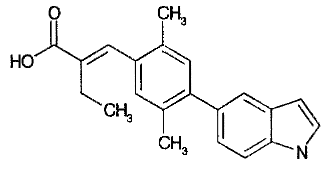 | 1 H NMR (CDCl3) δ 0.23 (t, J = 7.5Hz, 3H), 2.30 (s, 6H), 2.52 (q, J = 7.5Hz, 2H), 6.59 (s, 1 H), 7.16-7.19 (m, 3H), 7.25 (s, 1 H), 7.46 (d, J = 8.4Hz, 1H), 7.60 (s, 1H), 7.90 (s, 1H), 7.82 (s, 1H)ppm | |
| 124 | 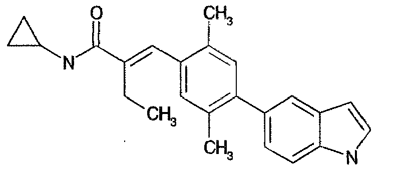 | 1H NMR (CDCl3) δ 0.58-063 (m, 2H), 0.83-0.90 (m, 2H), 1.10 (t, J = 7.5Hz, 3H), 2.25 (s, 3H), 2.27 (s, 3H), 2.46 (q, J = 7.5Hz, 2H), 2.84-2.89 (m, 1H), 5.98 (s, 1 H), 6.58 (s, 1H), 7.05 (s, 1H), 7.13-7.19 (m, 3H), 7.43 (d, J = 8.4Hz, 1H), 7.58 (s, 1H), 8.27 (s, 1H)ppm | |
| 125 | 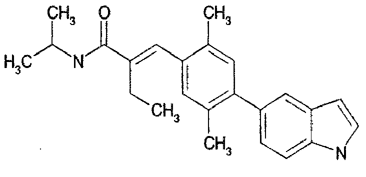 | 1H NMR (CDCl3) δ 1.11 (t, J = 7.5Hz, 3H), 1.26 (d, J = 6.6Hz, 6H), 2.26 (s, 3H), 2.28 (s, 3H), 2.47 (q, J = 7.5Hz, 2H), 4.18-4.30 (m, 1H), 5.68 (d, J = 8.7Hz, 1H), 6.58 (s, 1H), 7.06 (s, 1 H), 7,15-7.19 (m, 3H), 7.25-7.26 (m, 1H), 7.43 (d, J = 8.4Hz, 1 H), 7.59 (s, 1H), 8.27 (s, 1 H)ppm | |

**[Table 22]**

| | | | |
|---|---|---|---|
| 126 | 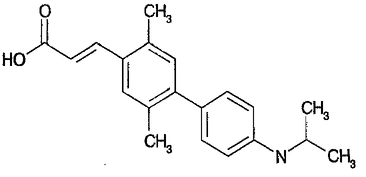 | 1H NMR (CDCl3) δ 1.25 (d, J = 6.3 Hz, 6H), 2.31 (s, 3H), 2.44 (s, 3H), 3.63-3.71 (m, 1 H), 6.41 (d, J = 15.9 Hz, 1 H), 6.63 (d, J = 8.7 Hz, 2H), 7.11 (d, J = 8.4 Hz, 2H), 7.16 (s, 1H), 7.49 (s, 1H), 8.08 (d, J = 15.6 Hz, 1 H)ppm | |
| 127 | 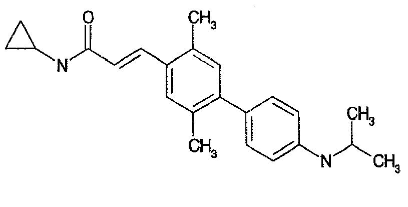 | 1H NMR (CDCl3) δ 0.60 (s, 2H), 0.84-0.88 (m, 2H), 1.26 (d, J = 6.0Hz, 6H), 2.27 (s, 3H), 2.40 (s, 3H), 2.86-2.89 (m, 1H), 3.62-3.78 (m, 1H), 4.04-4.41 (m, 1 H), 5.74 (s, 1H), 6.26 (d, J = 15.3Hz, 1H), 6.65 (s, 1H), 6.67 (s, 1 H), 7.06 (s, 1H), 7.12 (s, 1H), 7.15 (s, 1H), 7.26 (s, 1H), 7.37 (s, 1H), 7.91 (d, J = 15.3Hz, 1H)ppm | |
| 128 | 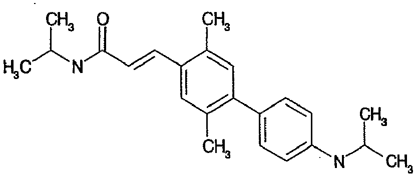 | 1 H NMR (CDCl3) δ 1.24 (d, J = 6.3Hz, 6H), 1.25 (d, J = 6.3Hz, 6H), 2.27 (s, 3H), 2.41 (s, 3N), 3.62-3.71 (m, 1H), 4.20-4.27 (m, 1H), 5.41-5.43 (m, 1H), 6.23 (d, J = 15.3Hz, 1H), 6.65 (d, J = 8.4Hz, 1H), 7.06 (s, 1H), 7.12 (s, 1H), 7.16 (s, 1H), 7.26 (s, 1H), 7.39 (s, 1H), 7.89 (d, J = 15.3Hz, 1H)ppm | |
| 129 | 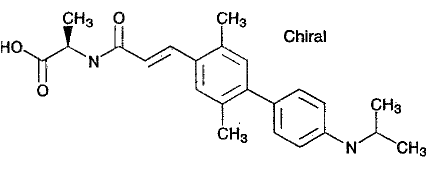 | 1 H NMR (CD3OD) δ 1.31 (d, J = 6.6Hz, 6H), 1.48 (d, J = 7.2Hz, 3H), 2.25 (s, 3H), 2.41 (s, 3H), 3.70-3.79 (m, 1H), 4.50-4.56 (m, 1H), 6.61 (d, J = 15.6Hz, 1H), 7.06 (s, 1H), 7.14 (d, J = 8.1Hz, 1H), 7.16 (s, 1H), 7.33 (s, 1H), 7.35 (d, J = 8.4Hz, 1H), 7.52 (s, 1H), 7.87 (d, J = 15.6Hz, 1 H), 8.47 (d, J = 6.9Hz, 1 H)ppm | |
| 130 | 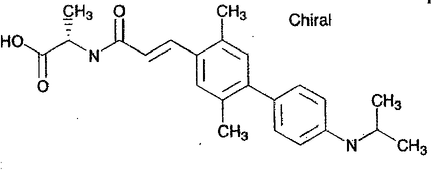 | 1 H NMR (CD3OD) δ 1.27 (d, J = 6.3Hz, 6H), 1.47 (d, J = 7.2Hz, 3H), 2.26 (s, 3H), 2.40 (s, 3H), 3.70-3.74 (m, 1H), 4.51-4.56 (m, 1 H), 6.60 (d, J = 15.6Hz, 1 H), 3 6.95 (d, J = 8.4Hz, 2H), 7.05 (s, 1 H), 7.23 (d, J = 8.7Hz, 2H), 7.87 (d, J = 15.6Hz, 1H), 8.46 (d, J = 7.5Hz, 1 H)ppm | |

**[Table 23]**

| | | | |
|---|---|---|---|
| 131 | 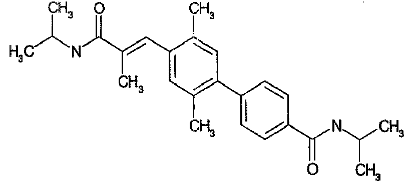 | 1H NMR (DMSO-d6) δ 1.17 (m, 12H), 1.88 (s, 3H), 2.22 (s, 6H), 4.30 (m, 2H), 7.11 (s, 1H), 7.14 (s, 1H), 7.24 (s, 1H), 7.43 (d, J = 8.4Hz, 2H), 7.75 (m, 1H), 7.90 (d, J = 8.4Hz, 2H), 8.25 (m, 1 H)ppm | |
| 132 | 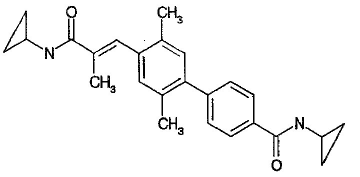 | 1H NMR (DMSO-d6) δ 0.63 (m, 8H), 1.87 (s, 3H), 2.21 (s, 6H), 2.47 (m, 2H), 7.12 (s, 1H), 7.14 (s, 1H), 7.25 (s, 1H), 7.44 (d, J = 8.4Hz, 2H), 7.74 (m, 1H), 7.91 (d, J = 8.4Hz, 2H), 8.27 (m, 1H)ppm | |
| 133 | 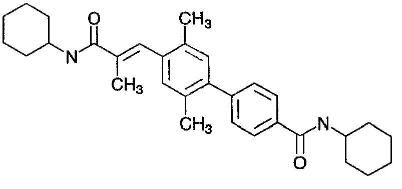 | 1 H NMR (DMSO-d6) δ 1.14-1.74 (m, 16H), 1.87 (s, 3H), 2.23 (s, 6H), 3.79 (m, 2H), 7.12 (s, 1H), 7.13 (s, 1H), 7.25 (s, 1H), 7.41 (d, J = 8.4Hz, 2H), 7.73 (m, 1H), 7.91 (d, J = 8.4Hz, 2H), 8.24 (m, 1H)ppm | |
| 134 | 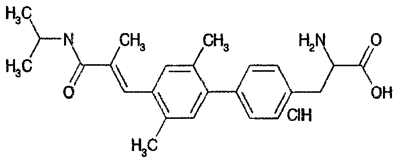 | 1H NMR (DMSO-d6) δ 1.14 (d, 6H, J = 6.6 Hz), 1.89 (s, 3H), 2.21 (s, 3H,), 2.22 (s, 3H), 3.17 (d, 3H, J = 6.3 Hz), 4.03 (m, 1H), 4.24 (brs, 1H), 7.06 (s, 1H), 7.11 (s, 1H), 7.25 (s, 1H), 7.34 (s, 4H), 7.45 (d, 1 H, d = 7.5 Hz), 8.43 (brs, 2H) | |
| 135 | 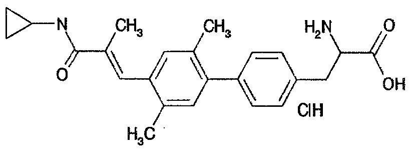 | 1H NMR (DMSO-d6) δ 0.55 (brs, 2H), 0.66 (brs, 2H), 1.88 (s, 3H), 2.22 (s, 6H,), 2.88 (m, 1 H), 3.17 (d, 3H, J = 6.3 Hz), 4.25 (m, 1H), 7.05 (s, 1H), 7.10 (s, 1H), 7.23 (s, 1H), 7.34 (s, 4H), 8.01 (d, 1H, d = 4.2 Hz), 8.43 (brs, 2H) | |
| 136 | 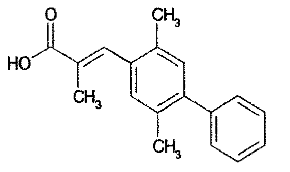 | 1H NMR (CDCl3) δ 2.07 (d, J = 1.5Hz, 3H), 2.27(s, 3H), 2.30(s, 3H), 7.11 (s, 1H), 7.17 (s, 2H), 5.69 (m, 2H), 7.31-7.45 (m, 3H), 7.91 (s, 5H)ppm | |
| 137 | 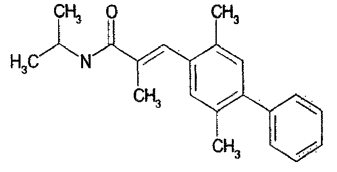 | 1H NMR (CDCl3) δ 1.25 (d, J = 5.4Hz, 6H), 2.00 (d, J = 1.2Hz, 3H), 2.25 (s, 3H), 2.26 (s, 3H), 4.21 (m, 1H), 5.64 (m, 1H), 7.06 (s, 1H), 7.09 (s, 1 H), 7.31-7.45 (m, 6H) ppm | |

**[Table 24]**

| | | | |
|---|---|---|---|
| 138 | 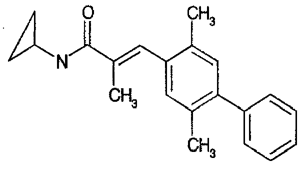 | 1H NMR (CDCl3) δ 0.60 (m, 2H), 0.85 (m, 2H), 2.04 (d, J = 1.2Hz, 3H), 2.24 (s, 3H), 2.26 (s, 3H), 2.88 (m, 1H), 5.69 (brs, 1H), 7.04 (s, 1H), 7.08 (s, 1H), 7.30-7.44 (m, 6H) ppm | |
| 139 | 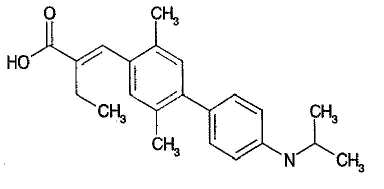 | 1H NMR (CDCl3) δ 1.18 (t, J = 7.5Hz, 3H), 1.26 (d, J = 6.0Hz, 6H), 2.28 (s, 3H), 2.30 (s, 3H), 2.49 (q, J = 7.2Hz, 2H), 3.61-3.72 (m, 1H), 6.68 (d, J = 8.1 Hz, 2H), 7.09 (s, 1H), 7.12 (s, 1H), 7.17 (d, J = 8.4Hz, 2H), 7.27 (s, 1H), 7.87 (s, 1H)ppm | |
| 140 | 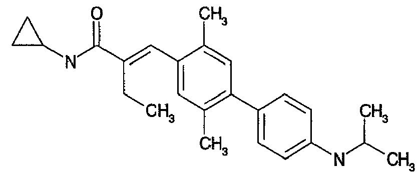 | 1 H NMR (CDCl3) δ 0.60-0.62 (m, 2H), 0.85-0.89 (m, 2H), 1.08 (t, J = 7.5Hz, 3H), 1.26 (d, J = 6.3Hz, 6H), 2.22 (s, 3H), 2.27 (s, 3H), 2.43 (q, J = 7.2Hz, 2H), 2.83-2.96 (m, 1 H), 2.63-2.71 (m, 1 H), 5.96 (s, 1H), 6.66(d, J = 6.8Hz, 2H), 7.01 (s, 1 H), 7.06 (s, 1 H), 7.11 (s, 1H), 7.15 (d, J = 8.7Hz, 2H)ppm. | |
| 141 | 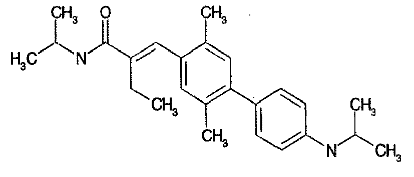 | 1 H NMR (CDCl3) δ 1.09 (t, J = 7.5Hz, 3H), 1.25 (d, J =6.0Hz, 6H), 1.26 (d, J =6.0Hz, 6H), 2.23 (s, 3H), 2.27 (s, 3H), 2.44 (q, J = 7.5Hz, 2H), 3.67-3.69 (m, 1H), 4.17-4.29 (m, 1H), 5.65 (d, J = 7.5Hz, 1H), 6.66(d, J = 8.1Hz, 2H), 7.01 (s, 1H), 7.06 (s, 1H), 7.12 (s, 1H), 7.16 (d, J = 7.8 Hz, 2H)ppm | |
| 142 | 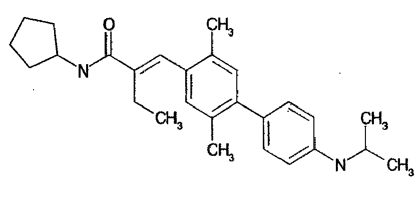 | 1H NMR (CDCl3) δ 1.09 (t, J = 7.5Hz, 3H), 1.26 (d, J = 6.3Hz, 6H), 1.46-1.50 (m, 2H), 1.67-1.71 (m, 4H), 2.05-2.10 (m, 2H), 2.23 (s, 3H), 2.27 (s, 3H), 2.44 (q, J = 7.2Hz, 2H), 3.65-3.71 (m, 1H), 4.31-4.38 (m, 1H), 5.78 (d, J = 7.2Hz, 1 H), 6.67 (d, J = 8.1Hz, 2H), 7.02 (s, 2H), 7.06 (s, 1H), 7.12 (s., 1H), 7.16 (d, J = 8.4Hz, 2H)ppm | |

**[Table 25]**

| | | | |
|---|---|---|---|
| 143 | 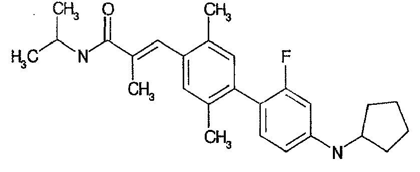 | 1 H NMR (CDCl3) δ 1.25 (d, J = 6.6Hz, 6H), 1.24 (s, 3H), 1.26 (s, 3H), 1.48-1.78(m, 6H), 2.02-2.08(m, 2H), 2.19 (s, 3H), 2.23 (s, 3H), 2.44(q, J=7.8Hz, 2H), 4.22(m, 1 H), 5.65(d, J=7.5Hz, 1 H), 6.34-6.52(m,2H), 6.97-7.04(m, 4H), 7.40 (s, 1H)ppm | |
| 144 | 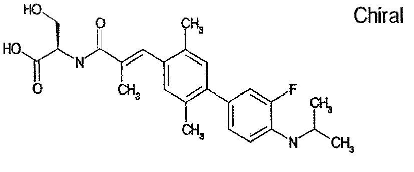 | 1H NMR (DMSO-d6) δ 1.20 (m, 9H), 1.93(s, 3H), 2.21 (s, 3H), 2.25(s, 3H), 3.66(m, 1H), 3.76(b, 2H), 4.38(m, 1 H), 5.02(m, 1 H), 6.78(t, J=8.1 Hz, 1 H), 6.97-7.13(m, 4H), 7.36(s, 1 H), 7.84(d, J=7.5Hz, 1H) ppm | |
| 145 | 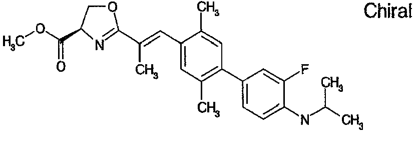 | 1 H NMR (CDCl3) δ 1.27 (d, J = 6.0Hz, 6H), 2.12 (s, 3H), 2.26(s, 3H), 2.28(s, 3H), 3.66(m, 1H), 3.87(s, 3H), 4.61 (m, 2H), 4.91 (m, 1 H), 6.73(t, J=8.7Hz, 1 H), 6.95-6.99(m, 3H), 7.06(s, 1H), 7.10(s, 1H), 7.27(s, 1 H), 7.49(s, 1H)ppm | |
| 146 | 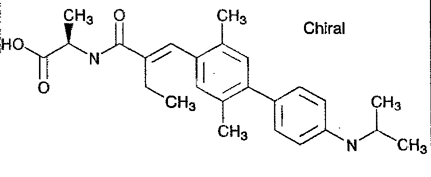 | 1H NMR (CD3OD) δ 1.10 (t, J = 7.5Hz, 3H), 1.22 (d, J = 6.3Hz, 6H), 1.49 (d, J = 7.5Hz, 3H), 2.24 (s, 6H), 2.44 (q, J = 7.5Hz, 2H), 3.61-3.69 (m, 1H), 4.50-4.55 (m, 1H), 6.73 (d, J = 8.7Hz, 2H), 7.03 (s, 1H), 7.06 (s, 1 H), 7.11 (d, J =8.7 Hz, 2H), 7.22 (s, 1 H), 8.25 (d, J = 7.2Hz, 1 H)ppm | |
| 147 | 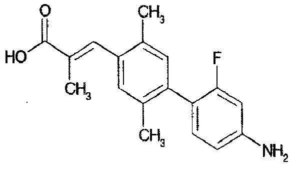 | 1H NMR (CDCl3) δ 2.06 (s, 3H), 2.19 (s, 3H), 2.28 (s, 3H), 6.44-6.52(m,2H), 6.99-7.06(m, 2H), 7.14 (s, 1H), 7.88 (s, 1 H)ppm | |
| 148 | 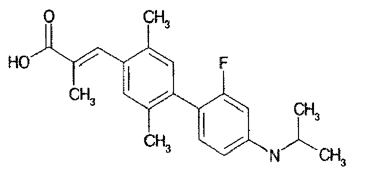 | 1H NMR (CDCl3) δ 1.25 (d, J = 6.3Hz, 6H), 2.05 (s, 3H), 2.20 (s, 3H), 2.28 (s, 3H), 3.46 (m, 2H), 6.33-6.59(m,2H), 6.96-7.16(m, 2H), 7.26 (s, 1H), 7.89 (s, 1H)ppm | |
| 149 | 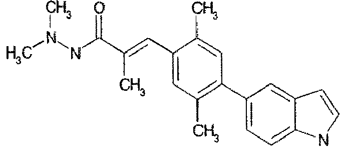 | 1H NMR (CDCl3) δ 2.03 (s, 3H), 2.28 (s, 6H), 2.74 (s, 6H), 6.59 (m, 1 H), 7.08 (s, 1 H), 7.16-7.59 (m, 6H), 8.28 (s, 1 H) ppm | |

**[Table 26]**

| | | | |
|---|---|---|---|
| 150 | 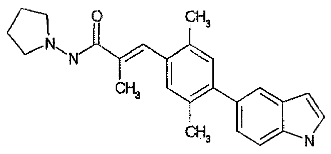 | 1H NMR (CDCl3) δ 1.95 (m, 4H), 2.04 (s, 3H), 2.28 (s, 6H), 3.07 (m, 4H), 6.58 (m, 1H), 7.16 (s, 1H), 7.18-7.59 (m, 6H), 8.26 (s, 1 H) ppm | |
| 151 | 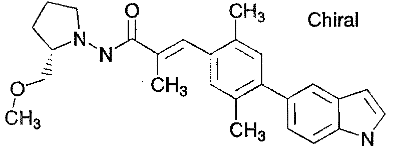 | 1 H NMR (CDCl3) δ 1.95 (m, 4H), 2.04 (s, 3H), 2.28 (s, 6H), 3.10 (m, 3H), 3.38 (s, 3H), 6.59 (m, 1H), 7.09 (s, 1H), 7.16-7.59 (m, 6H), 8.24 (s, 1H) ppm | |
| 152 | 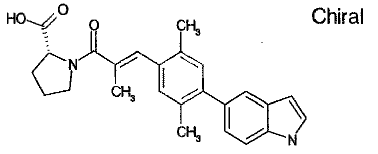 | | |
| 153 | 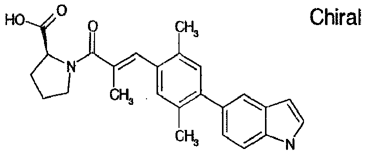 | | |
| 154 | 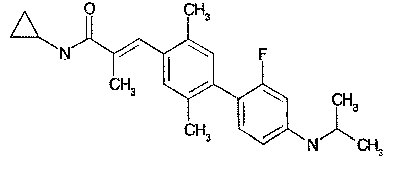 | 1 H NMR (CDCl3) δ 0.60 (m, 2H), 0.85 (m, 2H), 1.25 (d, J = 6.3Hz, 6H),2.00 (s, 3H), 2.19 (s, 3H), 2.24 (s, 3H), 2,89 (m, 1H), 4.23(m, 1H), 5.62(m, 1H), 6.39 (m,2H), 7.02(m, 3H), 7.45 (s, 1H)ppm | |
| 155 | 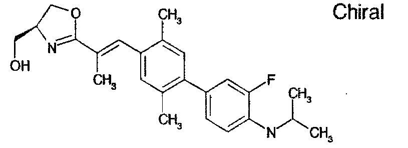 | | |
| 156 | 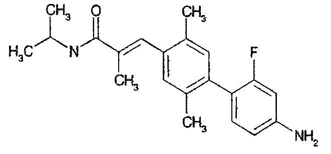 | | |

**[Table 27]**

| | | | |
|---|---|---|---|
| 157 | 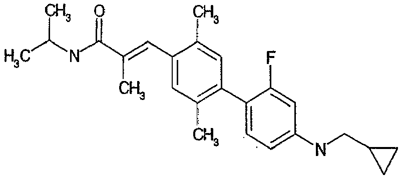 | 1 H NMR (CDCl3) δ 0.28 (m, 2H), 0.58(m, 2H), 1.24 (s, 3H), 1.26 (s, 3H), 2.00 (s, 3H), 2.18 (s, 3H), 2.24 (s, 3H), 2.98(d, J=6.9Hz, 2H), 3.33 (m, 1H), 4.21 (m, 1H), 5.65(m, 1 H), 6.41 (m,2H), 7.02(m, 3H), 7.40 (s, 1 H)ppm | |
| 158 | 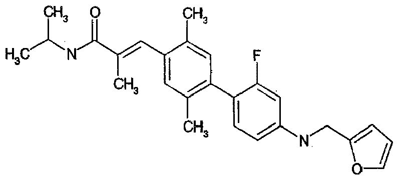 | 1 H NMR (CDCl3) δ 1.24 (s, 3H), 1.26 (s, 3H), 1.99 (s, 3H), 2.17 (s, 3H), 2.24 (s, 3H), 4.23(m, 1H), 4.36 (s, 2H), 5.74(d, J=6.6Hz, 1H), 6.33 (m,2H), 6.53(m, 2H), 7.06(m, 3H), 7.40 (s, 2H)ppm | |
| 159 | 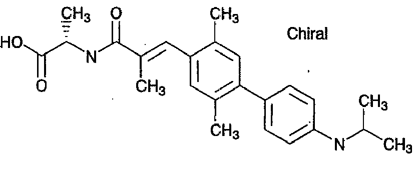 | 1 H NMR (CD3OD) δ 1.25 (d, J = 6.3Hz, 6H), 1.48 (d, J = 6.9Hz, 3H), 2.04 (s, 3H), 2.25 (s, 3H), 2.28 (s, 3H), 3.62-3.71 (m, 1H), 4.38-4.43 (m, 1H), 6.67 (d, J = 8.7Hz, 2H), 7.05 (s, 1H), 7.08 (s, 1H), 7.10(d, J = 8.4Hz, 2H), 7.38 (s, 1H), 7.49 (s, 1H)ppm | |
| 160 | 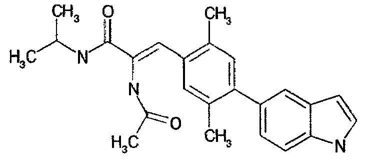 | 1H NMR (DMSO-d6) δ 1.12 (d, 2H, J=6.6 Hz), 1.94 (s, 3H), 2.22 (s, 3H,), 2.30 (s, 3H), 4.00 (m, 1H), 6.45 (s, 1H), 7.00 -7.80 (m, 9H), 9.25 (s, 1H), 11.14 (s, 1H) | |
| 161 | 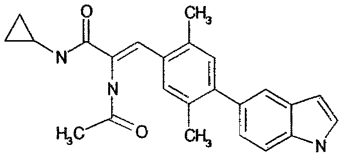 | | |
| 162 | 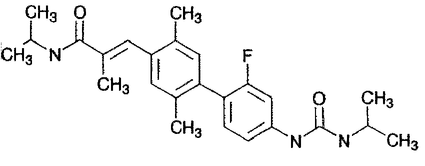 | 1 H NMR (CDCl3) δ 1.21 (d, J = 6.6Hz, 6H), 1.26 (d, J = 6.6Hz, 6H), 1.99 (s, 3H), 2.14 (s, 3H), 2.22 (s, 3H), 4.02 (m, 1H), 4.22 (m, 1H), 5.72 (m, 1H), 6.84-7.34 (m, 6H), 7.41 (s, 1 H)ppm | |
| 163 | 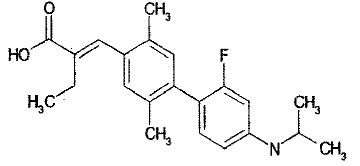 | 1H NMR (CDCl3) δ 1.17 (t, J = 7.5Hz, 3H), 1.25 (d, J = 6.6Hz, 6H), 2.21 (s, 3H), 2.27 (s, 3H), 2.49 (m, 2H), 3.63 (m, 1H), 6.33 (m, 2H), 7.02(m, 2H), 7.12(s, 1H), 7.84 (s, 1 H)ppm | |

**[Table 28]**

| | | | |
|---|---|---|---|
| 164 | 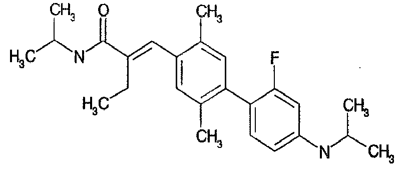 | 1 H NMR (CDCl3) δ 1.26 (t, J = 6.6Hz, 3H), 1.26 (m, 12H), 2.18 (s, 3H), 2.22 (s, 3H), 2.45 (m, 2H), 3.63 (m, 1H), 4.23 (m, 1H), 5.65(m, 1 H), 6.44 (m,2H), 7.00(m, 3H), 7.09 (s, 1H)ppm | |
| 165 | 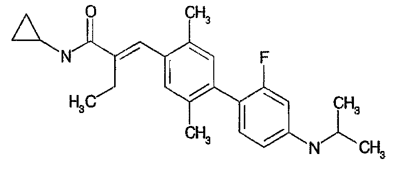 | 1H NMR (CDCl3) δ 0.60 (m, 2H), 0.85 (m, 2H),1.26 (t, J = 6.6Hz, 3H), 1.24 (d, J = 6.6Hz, 6H), 2.18 (s, 3H), 2.21 (s, 3H), 2.44 (m, 2H), 2.87 (m, 1H), 3.62 (m, 1 H), 5.95(brs, 1 H), 6.40 (m, 2H), 7.02(m, 3H), 7.09 (s, 1 H)ppm | |
| 166 | 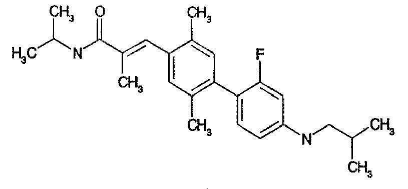 | 1H NMR (CDCl3) δ 1.00(s, 3H), 1.02(s, 3H), 1.24 (s, 3H), 1.26 (s, 3H), 1.93(m, 1 H), 1.99 (s, 3H), 2.18 (s, 3H), 2.96(d, J=6.9Hz, 2H), 4.23(m, 1H), 5.65(m, 1 H), 6.43 (m,2H), 7.02(m, 3H), 7.40 (s, 1H)ppm | |
| 167 | 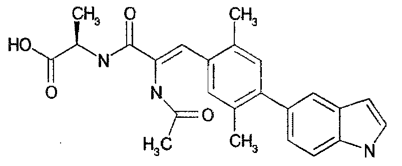 | | |
| 168 | 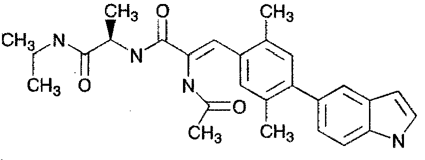 | | |
| 169 | 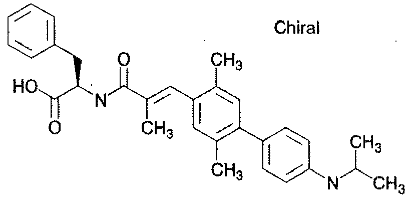 | 1H NMR (CDCl3) δ 1.25 (d, J = 6.0Hz, 6H), 1.93 (s, 3H), 2.21 (s, 3H), 2.26 (s, 3H), 3.18 (dd, J = 13.8, 6.3Hz, 1H), 3.30 (dd, J = 14.1, 5.7Hz, 1 H), 3.62-3.70 (m, 1H), 4.78-4.81 (m, 1H), 6.65 (d, J = 8.4Hz, 2H), 7.02 (s, 1H), 7.06 (s, 1H), 7.14 (d, J = 8.7Hz, 2H), 7.21-7.32 (m, 7H)ppm | |
| 170 | 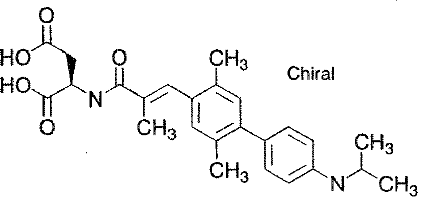 | 1H NMR (CD3OD) δ 1.26 (d, J = 6.3Hz, 6H), 2.05 (s, 3H), 2.25 (s, 3H), 2.27 (s, 3H), 2.91-2.96 (m, 1H), 3.11 (dd, J = 17.4, 4.5Hz, 1H), 3.62-3.69 (m, 1H), 6.68 (d, J = 8.4Hz, 2H), 7.06 (s, 1H), 7.08 (s, 1H), 7.17 (d, J = 8.4Hz, 2H), 7.52 (s, 1H)ppm | |

**[Table 29]**

| | | | |
|---|---|---|---|
| 171 | 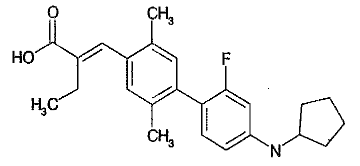 | 1H NMR (CDCl3) δ 1.17 (t, J = 7.5Hz, 3H), 1.46-2.12 (m, 8H), 2.27 (s, 3H), 2.29 (s, 3H), 2.46 (m, 2H), 3.77 (m, 1H), 6.35 (m, 2H), 7.02(m, 2H), 7.12(s, 1 H), 7.85 (s, 1H)ppm | |
| 172 | 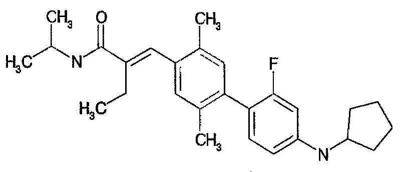 | 1 H NMR (CDCl3) δ 1.09(t, J=7.8Hz, 3H), 1.24 (s, 3H), 1.26 (s, 3H), 1.48-1.78(m, 6H), 2.02-2.08(m, 2H), 2.19 (s, 3H), 2.23 (s, 3H), 2.44(q, J=7.8Hz, 2H), 3.77-3.88(m, 2H), 4.22(m, 1 H), 5.65(d, J=7.5Hz, 1 H), 6.33-6.44(m,2H), 6.97-7.04(m, 3H), 7.11 (s, 1 H)ppm | |
| 173 | 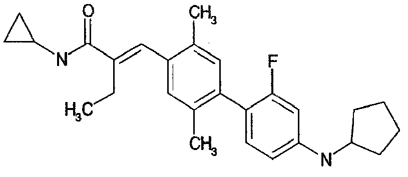 | 1 H NMR (CDCl3) δ 0.60(m, 2H), 0.85(m, 2H), 1.07(t, J=7.5Hz, 3H), 1.26-1.78(m, 6H), 2.02-2.08(m, 2H), 2.18 (s, 3H), 2.22 (s, 3H), 2.43(q, J=7.5Hz, 2H), 2.85(m, 1H), 3.79(m, 1H), 5.95(m, 1 H), 6.34-6.43(m,2H), 6.97-7.04(m, 3H), 7.10 (s, 1H)ppm | |
| 174 | 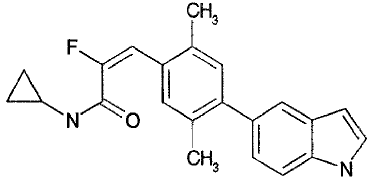 | 1 H NMR (CDCl3) δ 0.46-0.50 (m, 2H), 0.76-0.82 (m, 2H), 2.26 (s, 3H), 2.28 (s, 3H), 2.74-2.79 (m, 1 H), 6.11 (s, 1H), 6.58 (s, 1H), 6.78 (d, J = 23.7Hz, 1 H), 7.13-7.16 (m, 2H), 7.25 (s, 1H), 7.34 (s, 1H), 7.42 (d, J = 8.1Hz, 1H), 7.56 (s, 1H), 8.21 (s, 1H)ppm | |
| 175 | 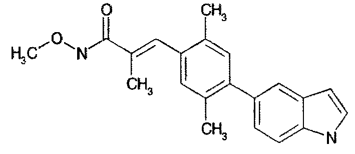 | 1H NMR (CDCl3) δ 2.03 (s, 3H), 2.27 (s, 6H), 3.89 (s, 3H), 4.24 (m, 1H), 6.58 (m, 1H), 7.08 (s, 1 H), 7.15-7.58 (m, 6H), 8.23 (s, 1 H) ppm | |
| 176 | 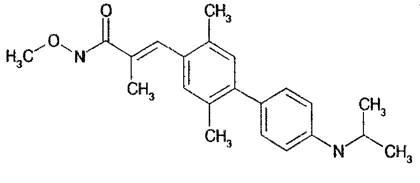 | 1 H NMR (COCl3) δ 1.25 (d, J = 6.3Hz, 6H), 2.01 (s, 3H), 2.24 (s, 3H), 2.27 (s, 3H), 3.67 (m, 1H), 3,88 (s, 3H), 5.65 (m, 1H), 6.73 (d, J = 8.1 Hz, 2H), 7.04 (s, 1 H), 7.07 (s, 1H), 7.15 (d, J = 8.4Hz, 2H), 7.35 (s, 1H), 8.46 (s, 1H) ppm | |

**[Table 30]**

| | | | |
|---|---|---|---|
| 177 | 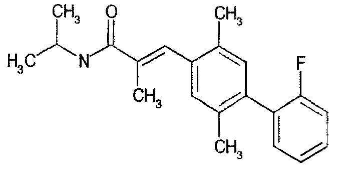 | 1H NMR (CDCl3) δ 1.25 (d, J = 6.6Hz, 6H), 2.00 (d, J = 1.5Hz, 3H), 2.17 (s, 3H), 2.26 (s, 3H), 4.22 (m, 1H), 5.65 (m, 1H), 7.07-7.41 (m,7H) ppm | |
| 178 | 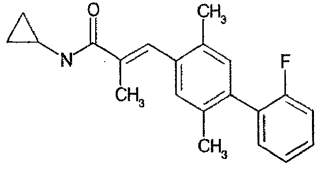 | 1H NMR (CDCl3) δ 0.60 (m, 2H), 0.85 (m, 2H), 2.00 (d, J = 1.5Hz, 3H), 2.17 (s, 3H), 2.26 (s, 3H), 2.86 (m, 1H), 5.96 (m, 1H), 7.07-7.41 (m,7H) ppm | |
| 179 | 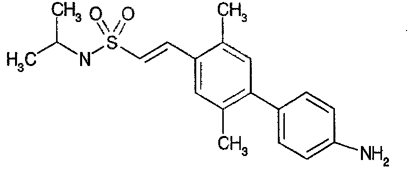 | 1H NMR (CDCl3+CD3OD) δ 1.24 (d, J = 6.6Hz, 1 H), 2.27 (s, 3H), 2.41 (s, 3H), 3.51 - 3.61 (m, 1 H), 6.69 (d, J = 15.3Hz, 1 H), 6.83 (d, J = 8.4Hz, 2H), 7.08 (s, 1H), 7.15 (d, J = 8.4 Hz, 2H), 7.37 (s, 1H), 7.73 (d, J = 15.3Hz, 1H) ppm | |
| 180 | 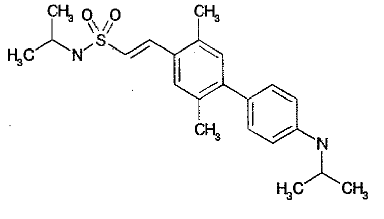 | 1 H NMR (CDCl3) δ 1.25 (d, J = 6.6 Hz, 6H), 1.26 (d, J = 6.3Hz, 6H), 2.29 (s, 3H), 2.41 (s, 3H), 3.53 - 3.72 (m, 2H), 4.13 (d, J = 7.5Hz, 1 H), 6.64 (d, J = 7.2Hz, 2H), 6.70 (s, 1H), 7.08 - 7.16 (m, 3H), 7.36 (s, 1H), 7.74 (d, J = 15.3H, 1 H) ppm | |
| 181 | 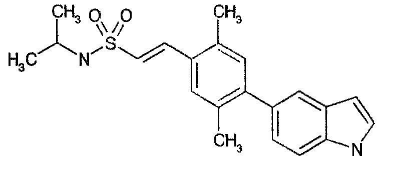 | 1H NMR (CDCl3) δ 1.26 (d, J = 6.6 Hz, 6H), 2.30 (s, 3H), 2.43 (s, 3H), 3.54 - 3.67 (m, 1H), 4.13 (d, J = 7.5 Hz, 1H), 6.57-6.61 (m, 1H), 6.71 (d, J = 15.3 Hz, 1H), 7.12 - 7.19 (m, 2H), 7.25-7.29 (m, 1H), 7.39-7.46 (m,2H), 7.57 (s, 1 H), 7.77 (d, J = 15.3 Hz, 1 H), 8.25 (s, 1H) ppm | |
| 182 | 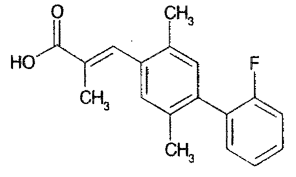 | 1H NMR (COCl3) δ 2.06 (d, J = 1.5Hz, 3H), 2.19 (s, 3H), 2.29 (s, 3H), 6.97-7.38 (m,6H), 7.91 (s, 1H)ppm | |

**[Table 31]**

| | | | |
|---|---|---|---|
| 183 | 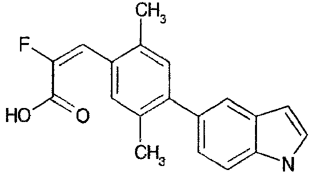 | 1 H NMR (DMSO-d6) δ 2.20 (s, 3H), 2.24 (s, 3H), 6.45 (s, 1H), 6.97-7.18 (m, 4H), 7.34-7.47 (m, 3H), 11.1 (s, 1 H)ppm | |
| 184 | 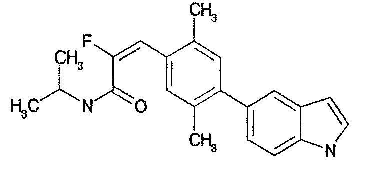 | 1 H NMR (CDCl3) δ 1.12 (d, J = 6.6Hz, 6H), 2.26 (s, 3H), 2.29 (s, 3H), 4.09-4.16 (m, 1H), 5.83-5.85 (m, 1H), 6.58 (s, 1H), 6.78 (d, J = 23.4Hz, 1 H), 7.18 (d, J = 8.4Hz, 1H), 7.24 (s, 1H), 7.25 (s, 1 H), 7.32 (s, 1H), 7.41 (d, J = 8.7Hz, 1H), 7.55 (s, 1H), 8.21 (s, 1H)ppm | |
| 185 | 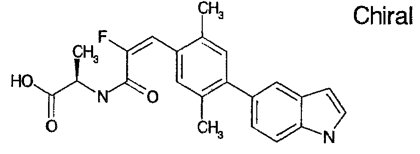 | 1 H NMR (DMSO-d6) δ 1.33 (d, J = 7.2Hz, 3H), 2.28 (s, 3H), 2.24 (s, 3H), 6.45 (s, 1H), 6.85 (d, J = 23.7Hz, 1 H), 7.02-7.06 (m, 2H), 7.24 (s, 1H), 7.35 (s, 1H), 7.36-7.46 (m, 3H), 11.01 (s, 1 H)ppm | |
| 186 | 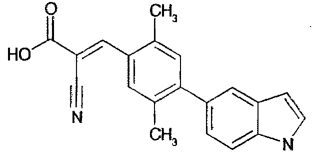 | 1H NMR (COCl3) δ 2.36 (s, 3H), 2.49 (s, 3H), 6.61 (m, 1H), 7.15-7.60 (m,5H), 8.22 (s, 1H), 8.26 (m, 1H), 8.65 (s, 1H), ppm | |
| 187 | 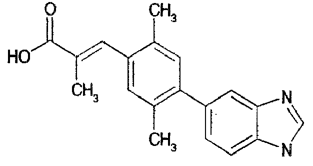 | | 296-298°C |
| 188 | 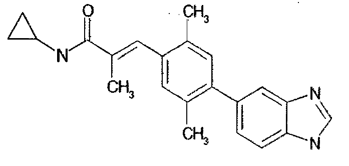 | 1H NMR (CDCl3) δ 0.61 (m, 2H), 0.85 (m, 2H), 2.01 (s,3H), 2.25 (s, 3H), 2.29 (s, 3H), 2.87 (m, 1H), 6.31 (m, 1H), 7.07-7.69 (m, 7H), 8.19 (s, 1H) | |
| 189 | 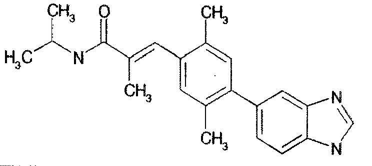 | 1 H NMR (CDCl3) δ 1.25 (d, J = 6.9Hz, 6H), 2.01 (s, 3H), 2.25 (s, 3H), 2.29 (s, 3H), 4.13 (m,1H), 5.68 (m, 1H), 7.08-7.87 (m, 7H), 8.29 (s, 1H) | |

**[Table 32]**

| | | | |
|---|---|---|---|
| 190 | 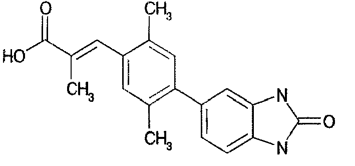 | | 334°C |
| 191 | 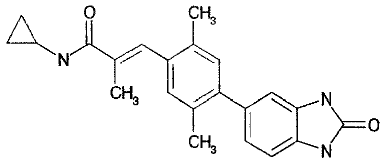 | 1 H NMR (CDCl3) δ 1.25 (d, J = 6.6Hz, 6H), 1.99 (s, 3H), 2.24 (s, 3H), 2.25 (s, 3H), 4.24 (m,1H), 5.68 (m, 1 H), 7.00-7.07 (m, 5H), 7.41 (s, 1H), 8.21 (brs, 2H) | |
| 192 | 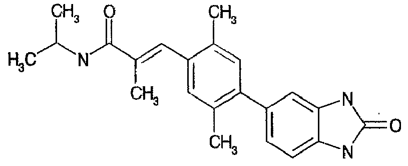 | 1H NMR (CDCl3) δ 1.25 (d, J = 6.6Hz, 6H), 1.99 (s, 3H), 2.24 (s, 3H), 2.25 (s, 3H), 4.24 (m, 1H), 5.68 (m, 1 H), 7.03-7.11 (m, 5H), 7.42 (s, 1 H), 8.90 (brs, 2H) | |
| 193 | 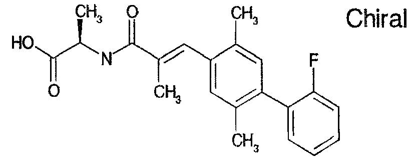 | | |
| 194 | 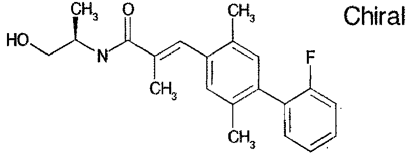 | 1 H NMR (CDCl3) δ 1.28 (d, J = 5.1 Hz, 3H), 2.02 (d, J = 1.5Hz, 3H), 2.17 (s, 3H), 2.26 (s, 3H), 3.67 (m, 2H), 4.21 (m, 1H), 6.02 (m, 1H), 7.07-7.49 (m,7H) ppm | |
| 195 | 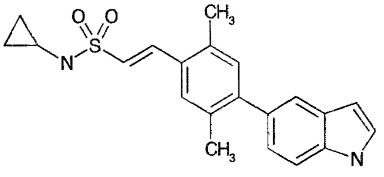 | 1H NMR (CDCl3) δ 0.68 - 0.77 (m, 4H), 2.30 (s, 3H), 2.44 (s, 3H), 2.50 - 2.59 (m, 1H), 4.74 (s, 1H), 6.57 - 6.61 (m, 1 H), 6.73 (d, J = 15.3Hz, 1 H), 7.15 (d.d, J = 8.4 & 1.5Hz, 1 H), 7.19 (s, 1 H), 7.25 - 7.29 (m, 1 H), 7.42 (s, 1H), 7.45 (d, J = 8.4Hz, 1H), 7.57 (s, 1H), 7.84 (d, J = 15.3Hz, 1H), 8.25 (s, 1H) ppm | |
| 196 | 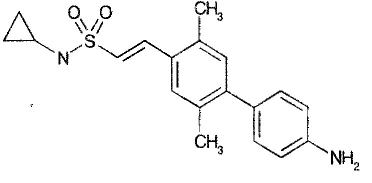 | 1 H NMR (CDCl3) δ 0.66 - 0.78 (m, 4H), 2.28 (s, 3H), 2.42 (s, 3H), 2.50 - 2.59 (m, 1H), 4.74 (s, 1H), 6.70 (d, J = 15.3Hz, 1H), 6.71 - 6.78 (m, 2H), 7.05 - 7.16 (m, 3H), 7.38 (s, 1H), 7.80 (d, J = 15.3Hz, 1H) ppm | |

**[Table 33]**

| | | | |
|---|---|---|---|
| 197 | 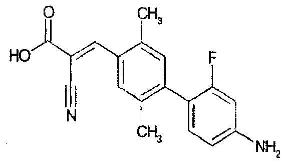 | 1 H NMR (DMSO-d6) δ 2.08 (s, 3H), 2.37 (s, 3H), 6.47 (m, 2H), 6.98 (t, J = 8.4Hz, 1 H), 7.16 (s, 1H), 7.90 (s, 1H), 8.47 (s, 1H)ppm | |
| 198 | 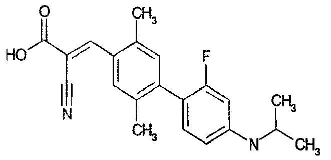 | 1H NMR (DMSO-d6) δ 1.24 (d, J = 6.6Hz, 6H), 2.28 (s, 3H), 2.45 (s, 3H), 6.44 (m, 2H), 7.02 (t, J = 8.4Hz, 1H), 7.17 (s, 1 H), 8.19 (s, 1H), 8.63 (s, 1H)ppm | |
| 199 | 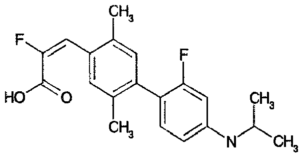 | 1 H NMR (DMSO-d6) δ 1.25 (d, J = 6.3Hz, 6H), 2.13 (s, 3H), 2.25 (s, 3H), 3.55-3.66 (m, 1 H), 6.34-6.45 (m, 2H), 6.91-7.15 (m, 4H)ppm | |
| 200 | 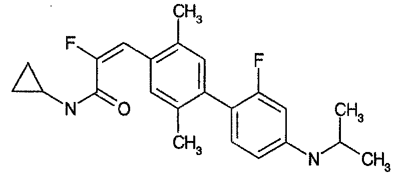 | 1H NMR (CDCl3) δ 0.40-0.46 (m, 2H), 0.74-0.80 (m, 2H), 2.17 (s, 3H), 2.25 (s, 3H), 2.71-2.77 (m, 1H), 3.52-3.64 (m, 1 H), 6.05 (s, 1 H), 6.34-6.44 (m, 2H), 6.75 (d, J = 23.1 Hz, 1H), 6.69-7.04 (m, 2H), 7.26-7.31 (m, 1H)ppm | |
| 201 | 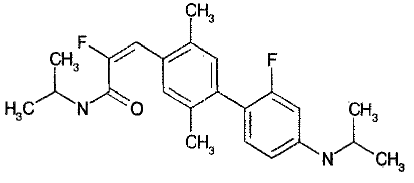 | 1 H NMR (CDCl3) δ 1.08(d, J = 6.6 Hz, 6H), 1.25 (d, J = 6.3 Hz, 6H), 2.17 (s, 3H), 2.26 (s, 3H), 3.58-3.64 (m, 1H), 4.05-4.16 (m, 1H), 5.76 (s, 1H), 6.33-6.42 (m, 2H), 6.74 (d, J = 23.3 Hz, 1 H), 6.96-7.05 (m, 2H), 7.26-7.28 (m, 1H)ppm | |
| 202 | 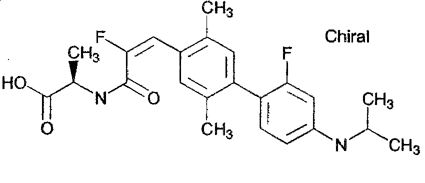 | 1H NMR (CDCl3) δ 1.25 (d, J = 6.3 Hz, 6H), 1.43 (d, J = 6.9 Hz, 3H), 2.17 (s, 3H), 2.27 (s, 3H), 3.58-3.66 (m, 1 H), 4.56-4.65 (m, 1H), 6.23-6.42 (m, 2H), 6.58 (d, J=6.3 Hz, 1H), 6.84 (d, J = 22.2 Hz, 1H),6.96-7.05 (m, 2H), 7.26-7.28 (m, 1H)ppm | |
| 203 | 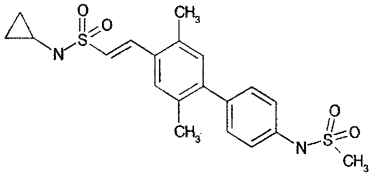 | 1 H NMR (CDCl3+CD3OD) δ 0.67-0.75 (m, 4H), 2.62 (s, 3H), 2.43 (s, 3H), 2.48-2.56 (m, 1H), 3.05 (s, 3H), 6.73 (d, J = 15.0Hz, 1H), 7.09 (s, 1 H), 7.29 (s, 4H), 7.41 (s, 1H), 7.80 (d, J= 15.0Hz, 1H) ppm | |

**[Table 34]**

| | | | |
|---|---|---|---|
| 204 | 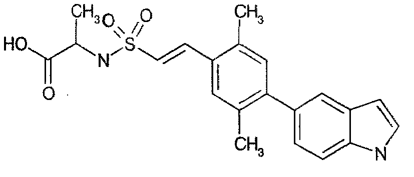 | 1 H NMR (CDCl3+CD3OD) δ 1.51 (d, J = 7.4 Hz, 3H), 2.29 (s, 3H), 2.42 (s, 3H), 3.96-4.07 (m, 1 H), 6.57 (s, 1H), 6.70 (d, J = 7.4 Hz, 3H), 7.12 (d.d, J = 8.4 & 1.5 Hz, 1H),7.17 (s, 1H), 7.30 (s, 2H), 7.39 (s, 1H), 7.44 (d, J = 8.4 Hz, 1H), 7.55 (s, 1H), 7.75 (d, J = 15.3 Hz, 1 H), 9.04 (s, 1H) ppm | |
| 205 | 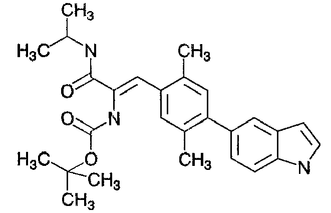 | | |
| 206 | 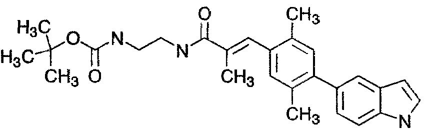 | 1H NMR (CDCl3) δ 1.45 (s, 9H), 2.28 (d, 3H, J = 1.2 Hz), 2.22 (s, 6H), 3.40 (m, 2H), 3.50 (m, 2H), 4.00 (m, 1H), 6.59 (m, 1H), 7.07 - 7.60 (m, 7H), 8.23 (s, 1H) | |
| 207 | 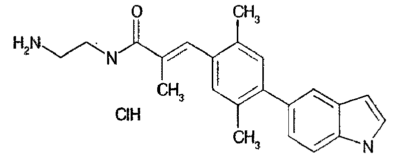 | 1H NMR (DMSO-d6) δ 1.91 (s, 3H), 2.23 (s, 6H), 2.90 (m, 2H), 3.47 (m, 2H), 6.80 - 7.90 (m, 7H), 8.10 (br, 2H), 8.30 (m, 1H) | |
| 208 | 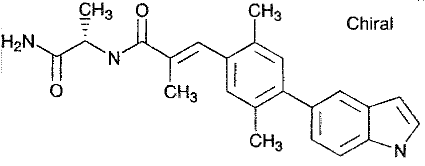 | 1H NMR (CDCl3) δ 1.51 (d, J = 7.2Hz, 3H), 2.59 (s, 3H), 2.27 (s, 3H), 2.28 (s, 3H), 4.68 (m, 1H), 5.49 (brs, 2H), 6.43 (brs, 1H), 6.58 (m, 1 H), 7.08 (s, 1H), 7.15-7.58 (m, 6H), 8.29 (s, 1 H) ppm | |
| 209 | 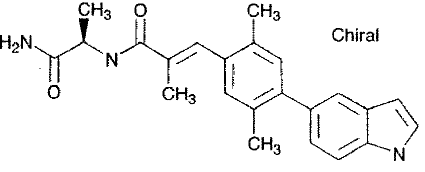 | 1H NMR (CDCl3) δ 1.51 (d, J = 6.9Hz, 3H), 2.59 (s, 3H), 2.27 (s, 3H), 2.28 (s, 3H), 4.68 (m, 1H), 5.49 (brs, 2H), 6.43 (brs, 1H), 6.58 (m, 1H), 7.08 (s, 1H), 7.15-7.58 (m, 6H), 8.23 (s, 1H) ppm | |

**[Table 35]**

| | | | |
|---|---|---|---|
| 210 | 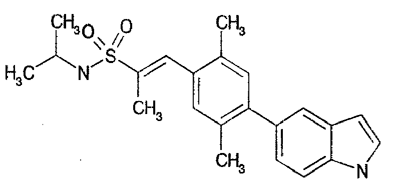 | 1H NMR (CDCl3 + CD3OD) δ 1.25 (d, J = 6.6Hz, 6H), 2.18 (d, J = 1.2Hz, 3H), 2.30 (s, 3H), 3.48 - 3.58 (m, 1 H), 6.57 (d.d, J = 3.0 & 0.9Hz, 1 H), 7.10 (s, 1H), 7.15 (d.d, J = 8.4 & 1.5Hz, 1H), 7.19 (s, 1H), 7.27 (d, J = 3.6Hz, 1 H), 7.45 (d, J = 8.4Hz, 1 H), 7.56- 7.58 (m, 1H), 7.64 (s, 1H), ppm | |
| 211 | 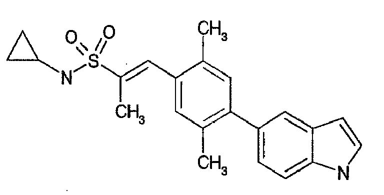 | 1H NMR (CDCl3) δ 0.67 - 0.75 (m, 4H), 2.19 (d, J = 1.2Hz, 3H), 2.30 (s, 3H), 2.32 (s, 3H), 2.46-2.54 (m, 1 H), 4.72 (s, 1 H), 6.59 (s, 1H), 7.13 (s, 1H), 7.17 (d.d, J = 8.1 & 1.5Hz, 1 H), 7.25 - 7.29 (m, 1 H), 7.44 (d, J = 8.1Hz, 1 H), 7.58-7.60 (m, 1H), 7.74 (s, 1H), 8.25 (s, 1H)ppm | |
| 212 | 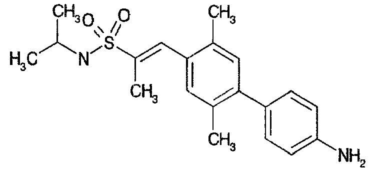 | 1H NMR (CDCl3) δ 1.25 (d, J = 6.6Hz, 6H), 2.16 (d, J = 1.5Hz, 1H), 2.76 (s, 6H), 3.52 -- 3.63 (m, 1 H), 4.11 (d, J = 7.5Hz, 1 H), 6.75 (d, J = 8.4 Hz, 2H), 7.06 (s, 1H), 7.09 (s, 1H), 7.13 (d, J = 8.4Hz, 2H), 7.64 (s, 1H), ppm | |
| 213 | 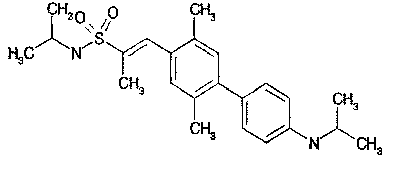 | 1H NMR (CDCl3) δ 1.25 (d, J = 6.6Hz, 6H), 1.26 (d, J = 6.0Hz, 6H), 2.17 (d, J = 1.5Hz, 1 H), 2.28 (s, 3H), 2.29 (s, 3H), 3.50-3.62 (m, 1 H), 3.62 -- 3.72 (m, 1H), 4.09 (d, J = 7.8Hz, 1 H), 6.66 (d, J = 8.7Hz, 2H), 7.06 (s, 1H), 7.10 (s, 1H), 7.15 (d, J = 8.7Hz, 2H), 7.64 (s, 1H) ppm | |
| 214 | 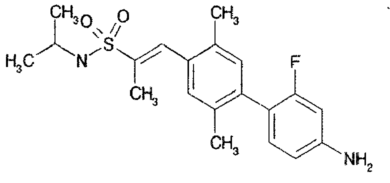 | 1H NMR (CDCl3) δ 1.25 (d, J = 6.6Hz, 6H), 2.16 (d, J = 1.5Hz, 3H), 2.19 (s, 3H), 2.27 (s, 3H), 3.49 - 3.62 (m, 1H), 4.10 (d, J = 7.8Hz, 2H), 6.48 - 6.60 (m, 2H), 6.98.- 7.09 (m, 3H), 7.63 (s, 1 H) ppm | |
| 215 | 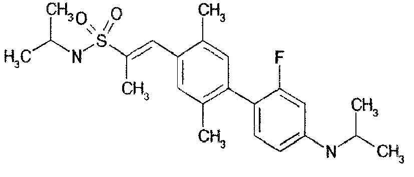 | 1H NMR (CDCl3) δ 1.25 (d, J = 6.6Hz, 6H), 1.26 (d, J = 6.3Hz, 6H), 2.16 (d, J = 1.2Hz, 3H), 2.20 (s, 3H), 2.27 (s, 3H), 3.48-3.69 (m, 2H), 4.09 (d, J = 7.5Hz, 1H), 6.33 - 6.47 (m, 2H), 7.01 (t, J = 2.4Hz, 1H), 7.08 (s, 2H), 7.63 (s, 1H) ppm | |

**[Table 36]**

| | | | |
|---|---|---|---|
| 216 | 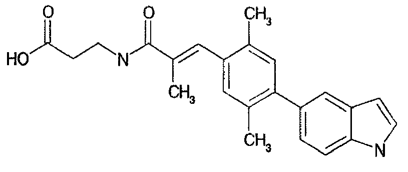 | 1 H NMR (DMSO-d6) δ 1.91 (s, 3H), 2.22 (s, 3H), 2.23 (s, 3H), 3.30-3.50 (br, 4H), 6.45 (m, 1H), 7.00-1.10 (dd, 1H, J = 8.4, 1.5 Hz), 7.11 (s, 2H), 7.30 (s, 1 H), 7.38 (t, 1 H, 2.7 Hz), 7.43 (d, 1H, J = 8.4Hz), 7.48 (m, 1H), 8.07 (t, 1H, J = 5.4 Hz), 11.14 (s, 1H) | |
| 217 | 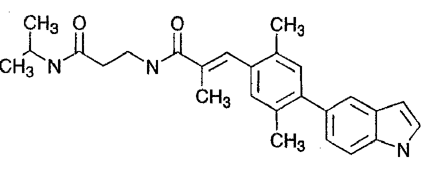 | 1H NMR (DMSO-d6) δ 1.05 (d, 6H, J = 6.6 Hz), 1.91 (d, 3H, J = 1.5Hz), 2.22 (s, 3H), 2.23 (s, 3H), 2.30 (t, 2H), 3.39 (t, 2H), 3.90 (m, 1 H), 6.45 (m, 1 H), 7.06 (dd, 1 H, J = 8.4, 1.5Hz), 7.10 (d, 2H, J = 4.5 Hz), 7.29 (s, 1H), 7.38 (t, 1 H, J = 2.4 Hz), 7.43 (d, 1 H, J = 8.4 Hz), 7.48 (s, 1 H), 7.77 (d, 1H, J = 7.8 Hz), 8.03 (t, 1H, J = 5.4 Hz), 11.15 (s, 1H) | |
| 218 | 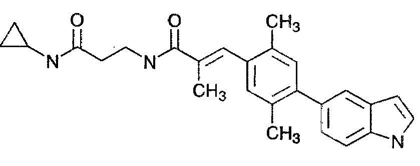 | 1H NMR (DMSO-d6) δ 0.35-0.45 (m, 2H), 0.55-0.65 (m, 2H), 1.91 (s, 3H), 2.22 (s, 3H), 2.23 (s, 3H), 2.29 (t, 3H), 2.62 (m, 1H), 3.38 (m, 2H), 6.45 (s, 1H), 7.06 (dd, 1 H, J = 8.4, 1.5 Hz), 7.10 (d, 2H, J = 3.6 Hz), 7.28 (s, 1 H), 7.34 (t, 1H, 2.7 Hz), 7.43 (d, 1 H, J = 8.4 Hz), 7.48 (s, 1H), 7.97 (d, 1 H, J = 3.9 Hz), 8.05 (t, 1H, J = 5.7 Hz), 11.10 (s, 1H) | |
| 219 | 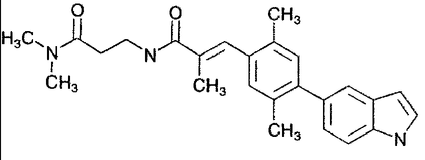 | | |
| 220 | 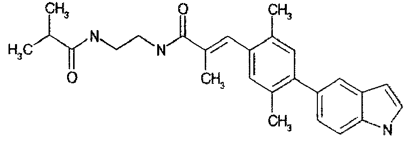 | | |
| 221 | 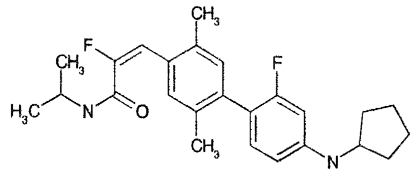 | 1H NMR (CDCl3) δ 1.08 (d, J = 6.6 Hz, 6H), 1.48-1.75 (m, 6H), 1.96-2.05 (m, 2H), 2.17 (s, 3H), 2.26 (s, 3H), 3.73-3.78 (m, 1H), 4.05-4.16 (m, 1 H), 5.74-5.79 (m, 1H), 6.33-6.43 (m, 2H), 6.75 (d, J = 22.8 Hz, 1H), 6.95-7.05 (m, 2H), 7.28 (s, 1H)ppm | |

**[Table 37]**

| | | | |
|---|---|---|---|
| 222 | 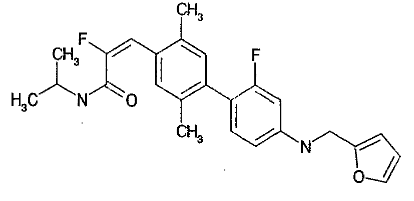 | 1H NMR (CDCl3) δ 1.09 (d, J = 6.6 Hz, 6H), 2.16 (s, 3H), 2.26 (s, 3H), 4.05-4.16 (m, 1H), 4.32 (s, 2H), 5.67 (d, J = 4.5 Hz, 1H), 6.28-6.36 (m, 2H), 6.42-6.53 (m, 2H), 6.74 (d, J = 23.4 Hz, 1 H), 6.99-7.04 (m, 2H), 7.28 (s,1H), 7.39 (s, 1H)ppm | |
| 223 | 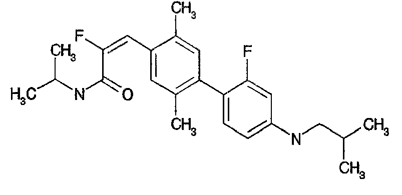 | 1 H NMR (COCl3) δ 1.01 (d, J = 6.6 Hz,6H), 1.08 (d, J = 6.3 Hz, 6H), 2.17 (s, 3H), 2. 26 (s, 3H), 2.95 (d, J = 6.9 Hz, 2H), 4.09-4.16 (m, 1H), 5.75 (d, J = 5.4 Hz, 1H), 6.34-6.45 (m, 2H), 6.75 (d, J = 23.1 Hz, 1H), 6.96-7.05 (m, 2H), 7.28 (s, 1H) ppm | |
| 224 | 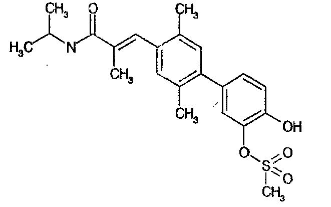 | | |
| 225 | 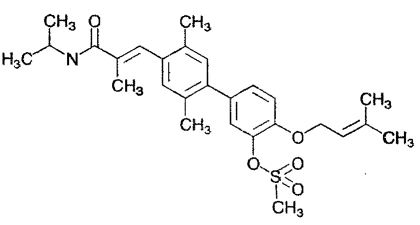 | | |
| 226 | 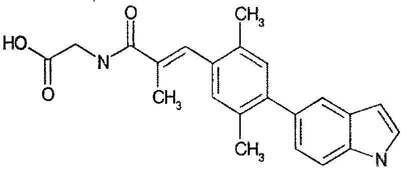 | 1 H NMR (DMSO-d6) δ 1.95 (d, 3H, J = 1.2 Hz), 2.23 (s, 3H), 2.24 (s, 3H), 3.85 (d, 2H, J = 5.7 Hz), 6.45 (t, 1 H, J = 2.1 Hz), 7.07 (dd, 1H, J = 1.8 Hz, 8.4 Hz), 7.12 (d, 2H, J = 3.3 Hz), 7.34-7.52 (m, 4H), 8.34 (t, 1H, J = 5.4 Hz), 11.15 (s, 1H), 12.6 (br, 1H) | |
| 227 | 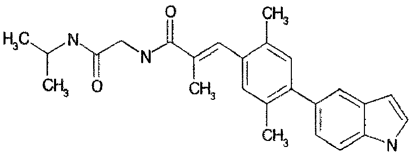 | | |

**[Table 38]**

| | | | |
|---|---|---|---|
| 228 | 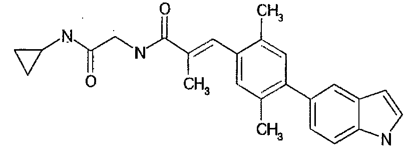 | | |
| 229 | 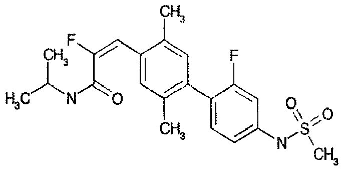 | 1 H NMR (CDCl3) δ 1.14 (d, J = 6.6 Hz, 6H), 2.23 (s, 3H), 2.28 (s, 3H), 3.08 (s, 3H), 5.93 (s, 1H), 6.74 (d, J = 23.7 Hz, 1H), 6.94-7.08 (m, 3H), 7.15-7.20 (m, 1 H), 7.32 (s, 1 H)ppm | |
| 230 | 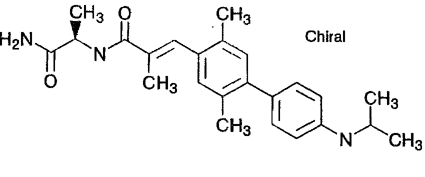 | 1 H NMR (CDCl3) δ 1.25 (d, J = 6.3Hz, 6H), 1.50 (d, J = 6.9Hz, 3H), 2.03 (s, 3H), 2.25 (s, 3H), 2.27 (s, 3H), 3.67 (m, 1H), 4.68 (m, 1H), 5.48 (brs, 2H), 6.43 (brs, 1H), 6.53 (m, 1H), 6.65 (d, J = 8.4Hz, 2H), 7.04 (s, 1 H), 7.08 (s, 1H), 7.14 (d, J = 8.4Hz, 1H), 7.51 (s, 1H) | |
| 231 | 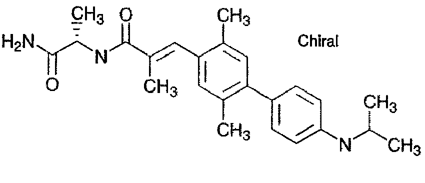 | 1H NMR (CDCl3) δ 1.25 (d, J = 6.3Hz, 6H), 1.50 (d, J = 6.9Hz, 3H), 2.04 (s, 3H), 2.25 (s, 3H), 2.27 (s, 3H), 3.67 (m, 1H), 4.68 (m, 1H), 5.46 (brs, 2H), 6.39 (brs, 1H), 6.51 (m, 1 H), 6.66 (d, J = 8.4Hz, 2H), 7.04 (s, 1 H), 7.08 (s, 1 H), 7.15 (d, J = 8.4Hz, 1H), 7.51 (s, 1H) | |
| 232 | 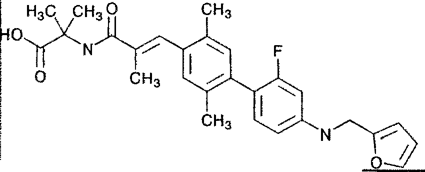 | ¹H NMR (CDCl3) δ 1.70 (s, 6H), 2.03 (s, 3H), 2.18 (s, 3H), 2.25 (s, 3H), 4.35 (s, 2H), 6.28-6.36 (m, 3H), 6.44-6.55 (m, 2H), 7.01-7.07 (m, 3H), 7.39-7.70 (m, 4H) | |
| 233 | 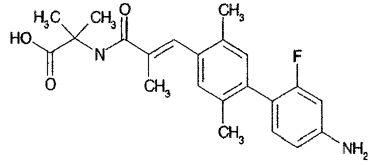 | 1 H NMR (CDCl3) δ 1.70 (s, 6H), 2.03 (s, 3H), 2.18 (s, 3H), 2.25 (s, 3H), 4.20 (m, 1H), 6.40-6.53 (m, 3H), 6.98-7.05 (m, 3H), 7.44-7.70 (m, 2H) | |

**[Table 39]**

| | | | |
|---|---|---|---|
| 234 | 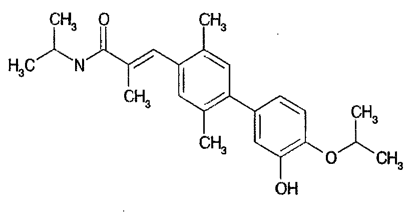 | 1H NMR (CDCl3) δ 1.25 (d, J = 6.6Hz, 6H), 1.40 (d, J = 6.0Hz, 6H), 1.99 (d, J = 1.2Hz, 3H), 2.25 (s, 3H), 2.26 (s, 3H), 4.15-4.28 (m, 1H), 4.57 - 4.67 (m, 1H), 5.66 (d, J = 8.1 Hz, 1 H), 5.73 (s, 1H), 6.78 (d.d, J = 8.4 & 2.1 Hz, 1 H), 6.89 (d, J = 8.4Hz, 1H), 6.93 (d, J = 2.1Hz, 1H), 7.03 (s, 1H), 7.07 (s, 1H), 7.40 (s, 1H) ppm | |
| 235 | 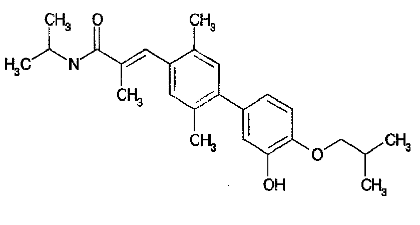 | 1H NMR (CDCl3) δ 1.07 (d, J = 6.6Hz, 6H), 1.25 (d, J = 6.6Hz, 6H), 1.99 (d, J = 1.5Hz, 3H), 2.09 - 2.23 (m, 1H), 2.25 (s, 3H), 2.26 (s, 3H), 3.85 (d, J = 6.6Hz, 3H), 4.16 - 4.28 (m, 1 H), 5.66 (d, J = 8.1 Hz, 1 H), 5.68 (s, 1H), 6.78 (d.d, J = 8.4 & 2.1 Hz, 1H), 6.88 (d, J = 8.4Hz, 1 H), 6.93 (d, J = 2.1Hz, 1 H), 7.03 (s, 1 H), 7.07 (s, 1H), 7.40 (s, 1H) ppm | |
| 236 | 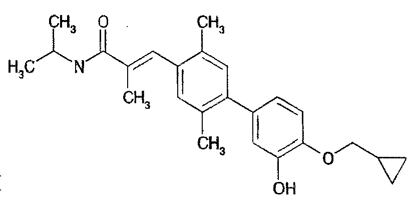 | 1H NMR (CDCl3) δ 0.33 - 0.41 (m, 2H), 0.63 - 0.72 (m, 2H), 1.25 (d, J = 6.6Hz, 6H), 1.26-1.39 (m, 1H), 1.99 (d, J = 1.2Hz, 3H), 2.25 (s, 6H), 3.91 (d, J = 6.6Hz, 2H), 4.15 - 4.29 (m, 1 H), 5.66 (d, J = 7.5Hz, 1 H), 6.77 (d.d, J = 8.4 & 2.1Hz, 1 H), 6.86 (d, J = 8.4Hz, 1 H), 6.93 (d, J = 2.1Hz, 1H), 7.03 (s, 1H), 7.06 (s, 1 H), 7.40 (s, 1H) 6.89 (d, J = 8.4Hz, 1 H), 6.93 (d, J = 2.1 Hz, 1 H), 7.03 (s, 1H), 7.07 (s, 1H), 7.40 (s, 1H) ppm | |
| 237 | 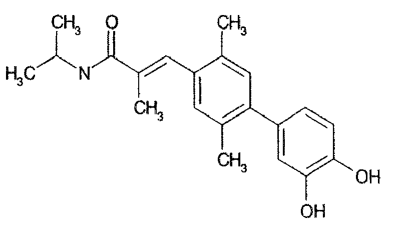 | 1H NMR (CDCl3+CD3OD) δ 1.25 (d, J = 6.6Hz, 6H), 1.99 (d, J = 1.5Hz, 3H), 1.24 (s, 3H), 1.25 (s, 3H), 4.11 - 4.25 (m, 1H), 6.70 (d.d, J = 8.1 & 2.1 Hz, 1 H), 6.83 (d, J := 2.1 Hz, 1 H), 6.87 (d, J = 8.1 Hz, 1 H), 7.03 (s, 1H), 7.06 (s, 1H), 7.38 (s, 1H) ppm | |

**[Table 40]**

| | | | |
|---|---|---|---|
| 238 | 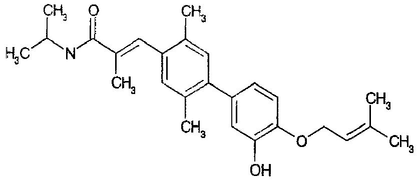 | 1H NMR (CDCl3) δ 1.25 (d, J = 6.6Hz, 6H), 1.76 (s, 3H), 1.82 (s, 3H), 1.99 (d, J = 1.2Hz, 3H), 2.25 (s, 3H), 2.26 (s, 3H), 4.16-4.28 (m, 1H), 4.61 (d, J = 6.9Hz, 1H), 5.48 - 5.56 (m, 1H), 5.66 (d, J = 6.6Hz), 6.79 (d.d, J = 8.1 & 2.1Hz, 1H), 6.91 (d, J = 8.1Hz, 1H), 6.93 (d, J = 2.1Hz, 1 H), 7.03 (s, 1H), 7.07 (s, 1H), 7.40 (s, 1 H) ppm | |
| 239 | 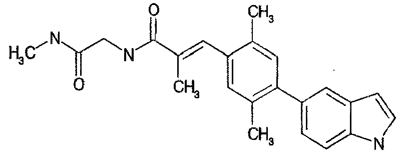 | | |
| 240 | 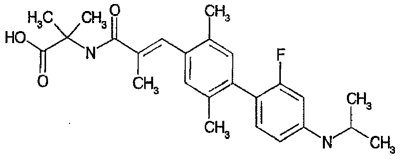 | 1H NMR (CDCl3) δ 1.27 (d, J = 6.9Hz, 6H), 1.69 (s, 6H), 2.03 (s, 3H), 2.19 (s, 3H), 2.24 (s, 3H), 3.64 (m, 1H), 6.44-6.51 (m, 3H), 6.96-7.09 (m, 3H), 7.44-7.71 (m, 3H), 7.39-7.70 (m, 2H) | |
| 241 | 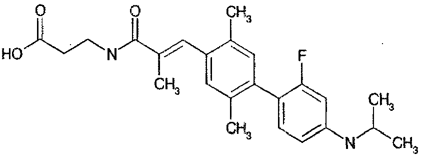 | 1 H NMR (CDCl3) δ 1.25 (d, J = 6.3 Hz, 6H), 2.00 (s, 3H), 2.18 (s, 3H), 2.32 (s, 3H), 2.70-2.74 (m, 2H), 3.58-3.71 (m, 2H), 4.09-4.16 (m, 1 H), 6.34-6.43 (m, 2H), 6.57 (s, 1H), 6.97-6.70 (m, 3H), 7.46 (s, 1 H)ppm | |
| 242 | 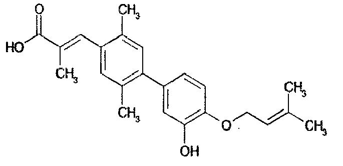 | | |
| 243 | 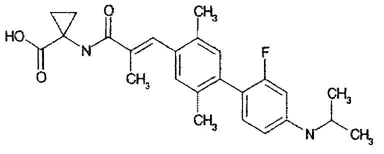 | 1H NMR (DMSO-d6) δ 1.09-1.28 (m, 8H), 1.88 (s, 3H), 2.11 (s, 3H), 2.19 (s, 3H), 3.63 (m, 1 H), 5.83 (d, J = 8.1 Hz, 1H), 6.35-6.44 (m, 3H), 6.94-7.09 (m, 4H), 7.29 (s, 1H), 8.43 (s, 1H) | |
| 244 | 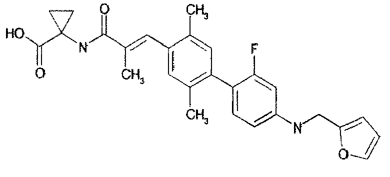 | 1H NMR (DMSO-d6) δ 1.28 (m, 2H), 1.88 (s, 3H), 2.10 (s, 3H), 2.18 (s, 3H), 4.28 (d, J = 5.8 Hz, 2H), 6.35-6.55 (m, 4H), 6.99-7.07 (m, 3H), 7.28 (s, 1H), 7.60 (s, 1 H), 8.43 (s, 1 H) | |

**[Table 41]**

| | | | |
|---|---|---|---|
| 245 | 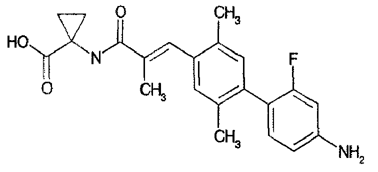 | 1 H NMR (CDCl3) δ 1.67 (m, 2H), 2.01 (s, 3H), 2.17 (s, 3H), 2.24 (s, 3H), 6.48-6.58 (m, 2H), 6.99-7.06 (m, 3H), 7.44-7.67 (m, 2H) | |
| 246 | 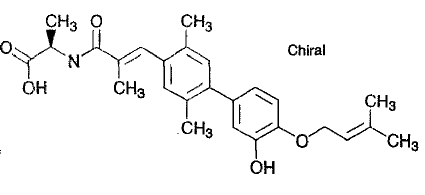 | 1 H NMR (CDCl3) δ 1.58 (d, J = 7.2Hz, 3H), 1.77 (s, 3H), 1.82 (s, 3H), 2.05 (s, 3H), 2.26 (s, 3H), 2.63 (s, 3H), 4.62 (d, J = 6.6Hz, 2H), 4.65 - 4.77 (m, 1H), 5.48-5.56 (m, 1H), 5.73 (brs, 1 H), 6.39 (d, J = 6.6Hz, 1H), 6.78 (d.d, J = 8.1 & 2.1 Hz, 1 H), 6.91 (d, J = 6.6Hz, 1H), 6.93 (s, 1 H), 7.05 (s, 1H), 7.08 (s, 1H), 7.56 (s, 1 H) ppm | |
| 247 | 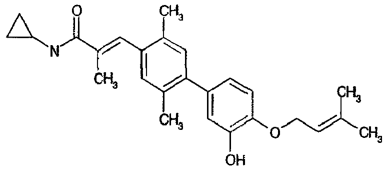 | | |
| 248 | 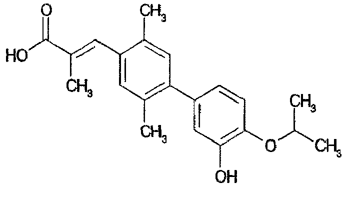 | 1 H NMR (COCl3) δ 1.41 (d, J = 5.7Hz, 6H), 2.06 (d, J = 1.5Hz, 3H), 2.28 (s, 6H), 4.58 - 4.68 (m, 1H), 5.75 (brs, 1H), 6.78 (d.d, J = 8.4 & 2.4Hz, 1 H), 6.91 (d, J = 6.6Hz, 1 H), 6.94 (s, 1 H), 7.10 (s, 1H), 7.14 (s, 1H), 7.90 (s, 1H) ppm | |
| 249 | 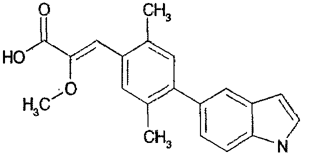 | 1 H NMR (CDCl3) δ 2.32 (s, 3H), 2.40 (s, 3H), 3.79 (s, 3H), 6.59 (s, 1H), 7.16-7.26 (m, 3H), 7.39 (s, 1 H), 7.43 (d, J = 8.4 Hz, 1H), 7.59 (s, 1H), 7.89 (s, 1H), 8.22 (s, 1H)ppm | |
| 250 | 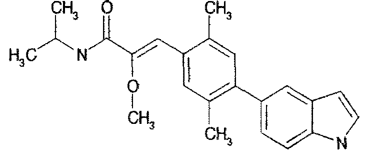 | 1 H NMR (CDCl3) δ 1.25 (d, J = 6.6 Hz, 6H), 2.29 (s, 3H), 2.36 (s, 3H), 3.59 (s, 3H), 4.11-4.23 (m, 1H), 6.59 (s, 2H), 7.15-7.27 (m, 4H), 7.43 (d, J = 8.4 Hz, 1 H), 7.59 (s, 2H), 8.23 (s, 1 H)ppm | |
| 251 | 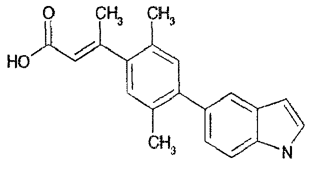 | 1H NMR (CDCl3) δ 2.27 (s, 3H), 2.31 (s, 3H), 2.53 (d, 3H, J = 1.5 Hz), 5.90 (m, 1H), 6.59 (m,1H), 7.02 (s, 1 H), 7.18 (d, 1H, J = 8.4 Hz), 7.18 (m, 1H), 7.41 (d, 1H, J = 8.4 Hz), 7.58 (m, 1 H), 8.22 (s, 1 H) | |

**[Table 42]**

| | | | |
|---|---|---|---|
| 252 | 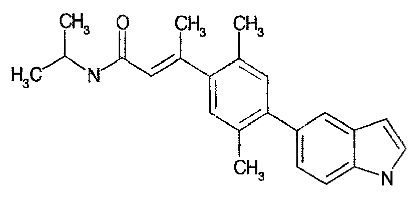 | 1H NMR (CDCl3) δ 1.22 (d, 6H, J = 6.8 Hz), 2.26 (s, 3H), 2.29 (s, 3H), 2.49 (d, 3H, J = 1.4 Hz), 4.19 (m, 1H), 5.23 (d, 1H, J = 7.7 Hz), 5.65 (q, 1H, J = 1.4 Hz), 6.58 (ddd, 1H, J = 3.0, 2.0, 0.8 Hz), 6.99 (s, 1H), 7.12 (s, 1H), 7.16 (dd, 1H, J = 8.4, 1.7 Hz), 7.26 (1H, m), 7.42 (d, 1H, J = 8.4 Hz), 7.57 (d, 1H, 1.7 Hz), 8.24 (brs, 1H) | |
| 253 | 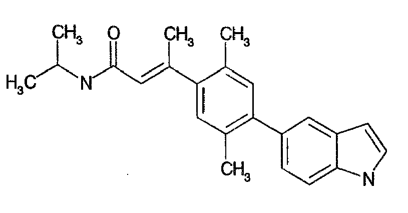 | 1 H NMR (CDCl3) δ 1.22 (d, 6H, J = 6.8 Hz), 2.26 (s, 3H), 2.29 (s, 3H), 2.49 (d, 3H, J = 1.4 Hz), 4.19 (m, 1H), 5.23 (d, 1H, J = 7.7 Hz), 5.65 (q, 1H, J = 1.4 Hz), 6.58 (ddd, 1 H, J = 3.0, 2.0, 0.8 Hz), 6.99 (s, 1H), 7.12 (s, 1H), 7.16 (dd, 1 H, J = 8.4, 1.7 Hz), 7.26 (1 H, m), 7.42 (d, 1 H, J = 8.4 Hz), 7.57 (d, 1H, 1.7 Hz), 8.24 (brs, 1 H) | |
| 254 | 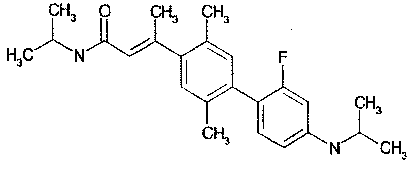 | 1H NMR (CDCl3) δ 1.21 (d, 6H, J = 6.6 Hz), 1.25 (d, 6H, J = 6.3 Hz), 2.17 (s, 3H), 2.26 (s, 3H), 2.46 (d, 3H, J = 1.4 Hz), 3.63 (m, 1 H), 4.18 (m, 1H), 5.26 (d, 1H, J = 7.8 Hz), 5.62 (1H, q, J = 1.4 Hz), 6.35 (dd, 1H, J = 12.4, 2.2 Hz), 6.40 (dd, 1H, 8.4, 2.2 Hz), 6.96 (1H, s), 7.00 (t, 1H, J = 8.4 Hz), 7.01 (s, 1H) | |
| 255 | 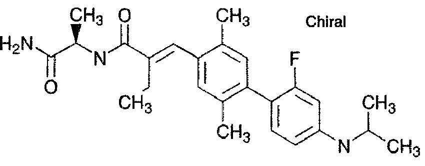 | | 159-160°C |
| 256 | 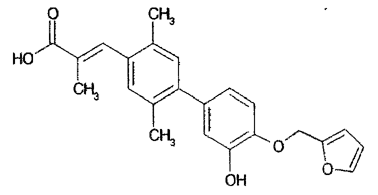 | 1 H NMR (CDCl3) δ 2.06 (s, 3H), 2.27 (s, 3H), 2.29 (s, 3H), 5.10 (s, 2H), 5.70 (s, 1H), 6.39 - 6.43 (m, 1H), 6.47 (d, J := 3.6 Hz, 1 H), 6.81 (d.d, J = 8.4 & 2.1Hz, 1 H), 6.93 (d, J = 2.1Hz, 1 H), 7.03 (d, J = 8.1 Hz, 1H), 7.09 (s, 1 H), 7.14 (s, 1H), 7.47 - 7.51 (m, 1H), 7.89 (s, 1H) ppm | |

**[Table 43]**

| | | | |
|---|---|---|---|
| 257 | 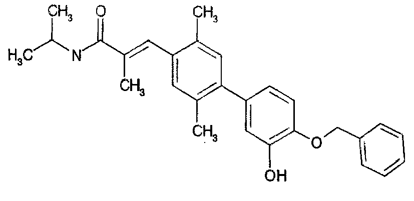 | 1H NMR (CDCl3) δ 1.25 (d, J = 6.6Hz, 6H), 1.99 (d, J = 1.2Hz, 3H), 2.25 (s, 3H), 2.26 (s, 3H), 4.15 - 4.28 (m, 1H), 5.15 (s, 2H), 5.66 (d, J = 8.1Hz, 1H), 5.71 (s, 1 H), 6.80 (d.d, J = 8.4 & 2.1 Hz, 1 H), 6.93 - 6.99 (m, 2H), 7.04 (s, 1H), 7.07 (s, 1H), 7.35-7.48 (m, 6H) ppm | |
| 258 | 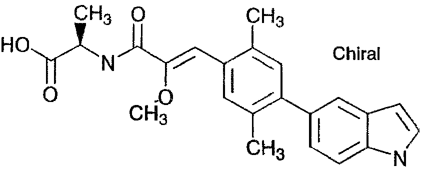 | 1H NMR (CDCl3) δ 1.58 (d, J = 7.2 Hz, 3H), 2.30 (s, 3H), 2.37 (s, 3H), 3.64 (s, 3H), 4.09-4.16 (m, 1 H), 4.68-4.73 (m, 1 H), 6.59 (s, 1 H), 7.16-7.28 (m, 5H), 7.43 (d, J = 8.4 Hz, 1 H), 7.60 (d, J = 8.1 Hz, 1H), 8.22 (s, 1 H)ppm | |
| 259 | 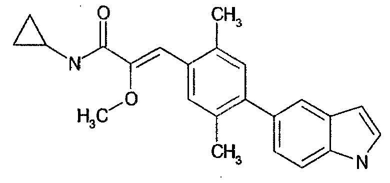 | 1 H NMR (CDCl3) δ 0.61-0.64 (m, 2H), 0.86-0.88 (m, 2H), 2.29 (s, 3H), 2.36 (s, 3H), 3.56 (s, 3H), 4.07-4.16 (m, 1H), 6.58 (s, 1H), 6.81 (s, 1H), 7.14-7.16 (m, 2H), 7.24-7.26 (m, 2H), 7.43 (d, J = 8.4 Hz, 1H), 7.57 (s, 1H), 7.59 (s, 1H), 8.23 (s, 1 H)ppm | |
| 260 | 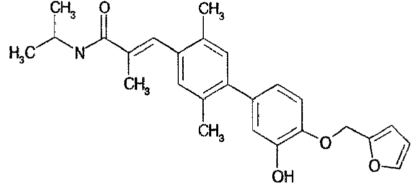 | 1H NMR (CDCl3) δ 1.25 (d, J= 6.3Hz, 6H), 2.00 (s, 3H), 2.25 (s, 6H), 4.15-4.28 (m, 1H), 5.09 (s, 2H), 5.67 (d, J = 7.8 Hz, 1 H), 5.72 (s, 1 H), 6.42 (s, 1H), 6.47 (s, 1H), 6.80 (d, J = 8.4Hz, 1H), 6.93 (s, 1 H), 6.98 - 7.11 (m, 3H), 7.41 (s, 1H), 7.49 (s, 1H) ppm | |
| 261 | 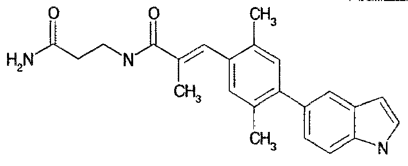 | 1H NMR (CDCl3) δ 2.02 (m, 3H), 2.25 (s, 3H), 2.27 (s, 3H), 2.57 (t, 2H, 6.3 Hz), 3.69 (m, 2H), 6.57 (m, 1H), 6.90 (m, 1 H), 7.07 (s, 1H), 7.10-7.20 (m, 2H), 7.25 (m, 1H), 7.40-7.60 (m, 3H), 8.50 (br, 1H) | |
| 262 | 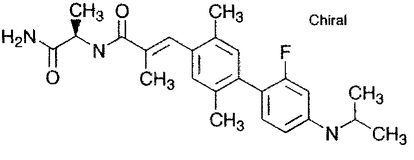 | 1H NMR (CDCl3) δ 1.25 (d, J = 6.3 Hz, 6H), 1.51 (d, J = 6.9 Hz, 3H), 2.03 (s, 3H), 2.19 (s, 3H), 2.24 (s, 3H), 3.63 (m, 1H), 4.69 (m, 1H), 5.48 (brs, 1H), 6.34-6.54 (m, 4H), 6.98-7.05 (m, 3H), 7.51 (s, 1H) | |

**[Table 44]**

| | | | |
|---|---|---|---|
| 263 | 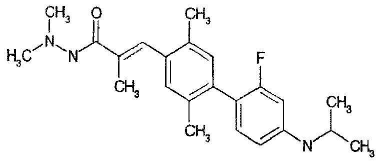 | 1H NMR (CDCl3) δ 1.25 (d, J = 6.3 Hz, 6H), 2.00 (s, 3H), 2.19 (s, 3H), 2.24 (s, 3H), 2.72 (s, 6H), 3.62 (m, 1H), 3.64 (m, 2H), 6.38 (m, 2H), 6.98-7.05 (m, 4H), 7.31 (s, 1H) | |
| 264 | 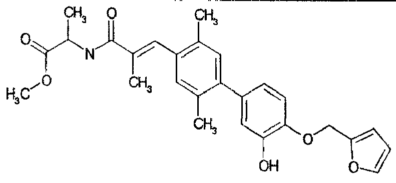 | | |
| 265 | 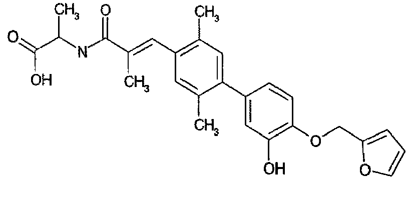 | 1 H NMR (CDCl3+CD3OD) δ 1.50 (d, J = 6.9Hz, 3H), 2.02 (s, 3H), 2.23 (s, 6H), 4.47 - 4.58 (brs, 1 H), 5.08 (s, 2H), 6.38 -6.44 (m, 1H), 6.48 (d, J = 3.3Hz, 1 H), 6.78 (d.d, J = 8.1 & 2.1 Hz, 1 H), 6.90 (d, J = 2.1 Hz, 1H), 6.98-7.06 (m, 3H), 7.50 (d.d, J = 2.1 & 0.9Hz, 1 H) ppm | |
| 266 | 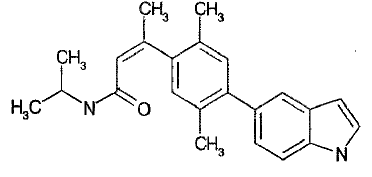 | 1H NMR (CDCl3) δ 0.81 (d, 6H, J=6.3Hz),2.14(d,3H,J= 1.5 Hz), 2.23 (s, 3H), 2.27 (s, 3H), 3.92 (m, 1H), 5.98 (s, 1H), 6.58 (br, 1H), 6.98 (s, 1H), 7.11 (m, 1H), 7.20 (s, 1H), 7.26-7.30 (m, 1H), 7.40 (d, 1H, J=8.1 Hz), 7.54 (s, 1H), 8.38 (s, 1H) | |
| 267 | 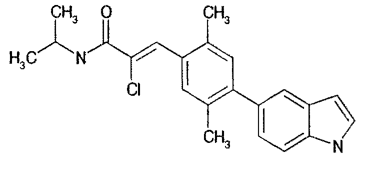 | 1H NMR (CDCl3) 1.28 (d, J = 6.6 Hz, 6H), 2.31 (s, 3H), 2.35 (s, 3H), 4.11-4.26 (m, 1H), 6.58-6.64 (m, 2H), 7.16-7.19 (m, 2H), 7.26 (s, 1 H), 7.44 (d, J = 7.8 Hz, 1H), 7.60 (s, 2H), 8.17 (s, 1H), 8.23 (s, 1H)ppm | |
| 268 | 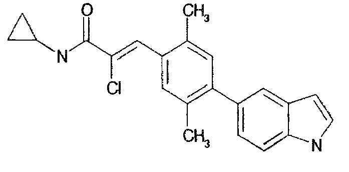 | 1H NMR (CDCl3) δ 0.65-0.68 (m, 2H), 0.88-0.93 (m, 2H), 2.30 (s, 3H), 2.45 (s, 3H), 2.87-2.90 (m, 1H), 6.59 (s, 1 H), 6.87 (s, 1H), 7.14-7.18 (m, 3H), 7.43 (d, J = 8.4 Hz, 1 H), 7.56-7.45 (m, 2H), 8.19 (s, 1H), 8.23 (s, 1H)ppm | |

**[Table 45]**

| | | | |
|---|---|---|---|
| 269 | 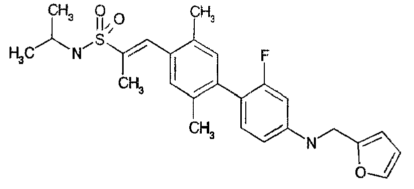 | 1 H NMR (COCl3) δ 1.25 (d, J = 6.6Hz, 6H), 2.16 (d, J = 1.5Hz, 3H), 2.19 (s, 3H), 2.27 (s, 3H), 3.50 - 3.62 (m, 1 H), 4.11 (d, J = 7.8 Hz, 1 H), 4.35 (s, 2H), 6.29 (d, J = 3.6 Hz, 1H), 6.33 - 6.37 ( m, 1 H), 6.43 - 6.55 (m, 2H), 6.98 - 7.05 (m, 3H), 7.40 (s, 1 H), 7.63 (s, 1H) ppm | |
| 270 | 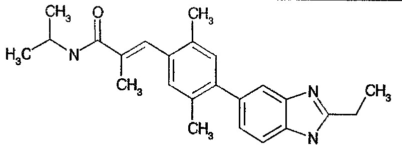 | 1 H NMR (COCl3) δ 1.32 (t, J = 7.5 Hz, 3H), 1.95 (s, 3H), 2.27 (s, 3H), 2.28 (s, 3H), 2.85 (m, 1H), 7.12-7.29 (m, 3H), 7.45 (s, 1 H), 7.56 (d, J = 8.4 Hz, 1H), 8.01 (s, 1H). | |
| 271 | 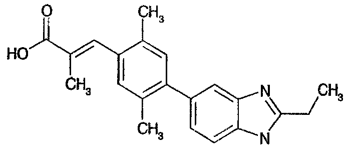 | 1 H NMR (d6-DMSO) δ 1.34 (t, J = 7.5 Hz, 3H), 1.94 (s, 3H), 2.24 (s, 6H), 2.73 (m, 1 H), 7.10-7.19 (m, 3H), 7.41 (s, 1H), 7.52 (d, J = 8.1 Hz, 1H), 7.66 (s, 1 H). | |
| 272 | 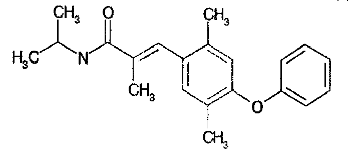 | 1 H NMR (COCl3) δ 1.24 (d, J= 6.6 Hz, 6H), 1.98 (s, 3H), 2.18 (s, 3H), 2.20 (s, 3H), 4.21 (m, 1 H), 5.64 (d, J = 7.2 Hz, 1 H), 6.74 (s, 1H), 6.91-7.09 (m, 4H), 7.26-7.36(m, 3H) ppm | |
| 273 | 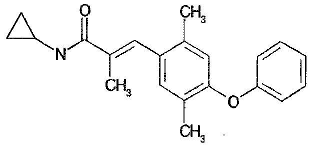 | 1H NMR (CDCl3) δ 0.58-0.61 (m, 2H), 0.84-0.87 (m, 2H), 1.96 (s, 3H), 2.17 (s, 3H), 2.20 (s, 3H), 2.85 (m, 1H), 5.94 (m, 1 H), 6.73 (s, 1H), 6.91-7.09 (m, 4H), 7.29-7.35 (m, 3H) | |
| 274 | 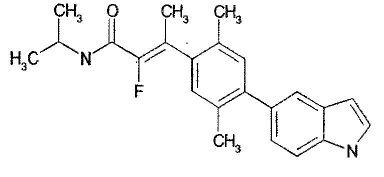 | 1H NMR (CDCl3) δ 0.90 (d, 6H, J = 6.3 Hz), 2.10 (d, 3H, J = 4.2 Hz), 2.23 (s, 3H), 2.26 (s, 3H), 4.00 (m, 1 H), 5.51 (br, 1H), 6.57 (m, 1H), 6.98 (s, 1H), 6.95-7.20 (m, 2H), 7.25 (s, 1H), 7.42 (d, 1H, J = 8.4 Hz), 7.56 (s, 1H), 8.27 (br, 1H) | |
| 275 | 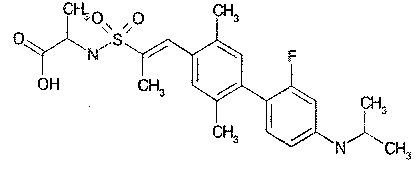 | 1 H NMR (CDCl3 + CD3OD) δ 1.27 (d, J = 6.6Hz, 6H), 1.51 (d, J = 7.2Hz, 3H), 2.16 (d, J = 1.2Hz, 3H), 2.19 (s, 3H), 2.26 (s, 3H), 3.58 - 3.68 (m, 1H), 3.81 (q, J = 7.2Hz, 1H), 6.40 - 6.56 (m, 2H), 7.02 (d, J = 8.4Hz, 1H), 7.08 (s, 2H), 7.58 (s, 1H)ppm | |

**[Table 46]**

| | | | |
|---|---|---|---|
| 276 | 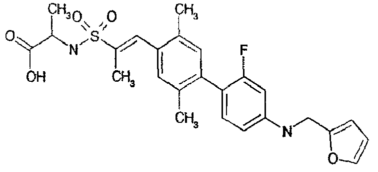 | | |
| 277 | 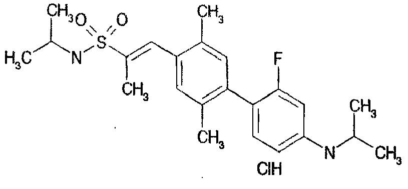 | 1 H NMR (DMSO) δ 1.12 (d, J = 6.6Hz, 6H), 1.20 (d, J = 6.3Hz, 6H), 2.06 (s, 3H), 2.13 (s, 3H), 3.25-3.37 (m,1H), 3.56-3.68 (m, 1H), 6.79 (brs, 2H), 7.09 (s, 1 H), 7.17 (s, 2H), 7.41 (s, 1 H),7.42 (d, J = 6.9Hz, 1 H) ppm | |
| 278 | 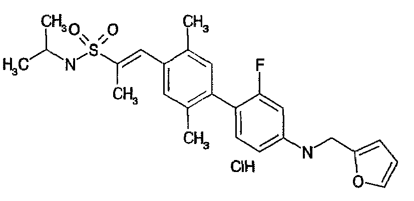 | 1 H NMR (DMSO) δ 1.11 (d, J = 6.6Hz, 6H), 2.05 (d, J = 1.2Hz, 3H), 2.12 (s, 3H), 2.21 (s, 3H), 3.22 - 3.36 (m, 1H), 4.29 (s, 2H), 6.36 (d.d, J = 3.3 & 0.6Hz, 1 H), 6.41 (d.d, J = 3.3 & 1.8Hz, 1H), 6.46-6.58 (m, 2H), 6.99 (t, J = 8.7Hz, 1 H), 7.05 (s, 1H), 7.15 (s, 1H), 7.39 (s, 1 H), 7.40 (d, J = 6.9Hz, 1 H), 7.59 - 7.62 (m, 1 H) ppm | |
| 279 | 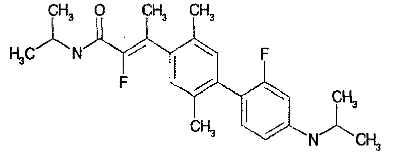 | | |
| 280 | 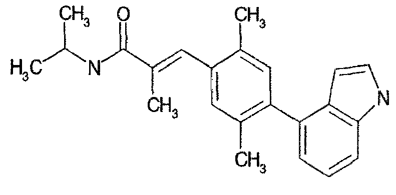 | 1H NMR (CDCl3) δ 1.26 (d, J = 6.6 Hz, 6H), 2.04 (d, J=1.5Hz, 3H), 2.17 (s, 3H), 2.27 (s, 3H), 4.22 (m, 1H), 5.68 (m, 1H), 6.30 (m, 1 H), 7.01 (d, J = 7.2 Hz, 1 H), 7.11 (s, 1H), 7.20-7.27 (m, 3H), 7.40 (d, J = 8.1 Hz, 1H), 7.45 (s, 1 H), 8.28 (s, 1 H)ppm | |
| 281 | 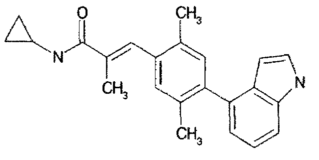 | | 102-103°C |

**[Table 47]**

| | | | |
|---|---|---|---|
| 282 | 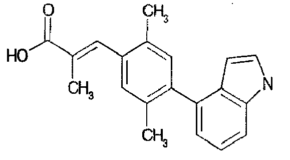 | 1H NMR (CDCl3) δ 2.11 (d, J = 1.2 Hz, 3H), 2.19 (s, 3H), 2.32 (s, 3H), 6.31 (m, 1H), 7.02 (dd, J = 0.9, 7.2 Hz, 1 H), 7.21-7.28 (m, 4H), 7.97(dd, J = 0.9, 7.2 Hz, 1 H), 8.24 (s, 1 H)ppm | |
| 283 | 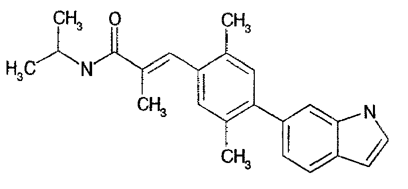 | 1H NMR (CDCl3) δ 1.25 (d, J = 6.6 Hz, 6H), 2.02 (d, J = 1.2 Hz, 3H), 2.27 (s, 3H), 2.28 (s, 3H), 4.22 (m, 1H), 5.66 (m, 1H), 6.59 (m, 1 H), 7.09-7.15 (m, 3H), 7.24 (m, 1H), 7.35 (s, 1H), 7.43 (s, 1H), 7.66 (d, J = 8.4 Hz, 1 H), 8.23 (s, 1H)ppm | |
| 284 | 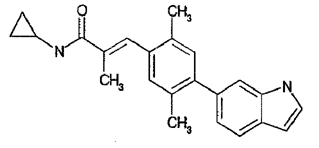 | | 146-149°C |
| 285 | 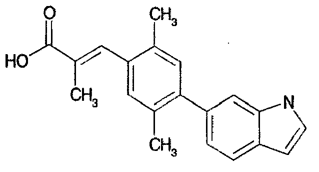 | 1 H NMR (CDCl3) δ 2.09 (d, J = 1.5 Hz, 3H), 2.31 (s, 3H), 2.32 (s, 3H), 6.60 (m, 1H), 7.11 (dd, J = 1.2, 4.8 Hz, 1H), 7.18 (s, 2H), 7.26 (m, 1H), 7.35 (s, 1 H), 7.67 (d, J = 8.4 Hz, 1 H), 7.93 (s, 1 H), 8.20 (s, 1 H)ppm | |
| 286 | 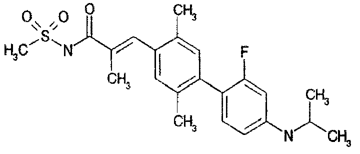 | 1 H NMR (CDCl3) δ 1.26 (d, J = 6.0 Hz, 6H), 2.06 (s, 3H), 2.19 (s, 3H), 2.25 (s, 3H), 3.43 (s, 3H), 3.64 (m, 1H), 6.41 (m, 2H), 7.02 (m, 4H), 7.58 (s, 1H), 8.11 (s, 1 H)ppm | |
| 287 | 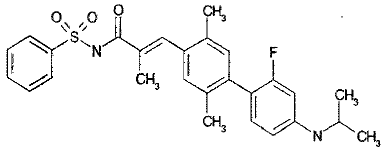 | 1H NMR (CDCl3) δ 1.25 (m, 6H), 1.99 (s, 3H), 2.17 (s, 3H), 2.21 (s, 3H), 3.62(m, 1 H), 6.38 (m, 2H), 7.02 (m, 3H), 7.58 (m, 5H), 8.15 (s, 1 H), 8.18 (s, 1 H), 8.35 (br s, 1H)ppm | |
| 288 | 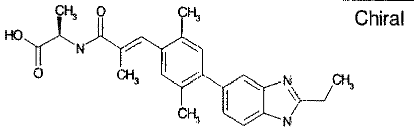 | | 186°C |
| 289 | 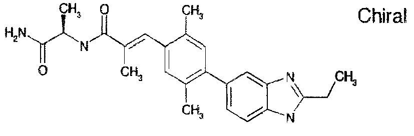 | 1H NMR (CDCl3) δ 1.44-1.50 (m, 5H), 2.05 (s, 3H), 2.25 (s, 3H), 2.26 (s, 3H), 2.97 (m, 2H), 4.62 (m, 1 H), 5.75 (brs, 2H), 6.88-7.59 (m, 7H)ppm | |

**[Table 48]**

| | | | |
|---|---|---|---|
| 290 | 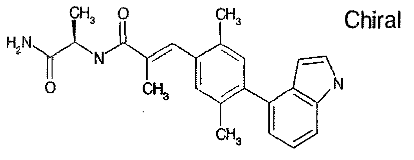 | 1H NMR (CDCl3) δ 1.48 (d, J = 7.2 Hz, 3H), 2.07 (d, J = 1.5 Hz, 3H), 2.17 (s, 3H), 2.28 (s, 3H), 4.63 (m, 1H), 6.29 (m, 1H), 7.00 (d, J = 6.3 Hz, 1 H), 7.11 (s, 1 H), 7.21-7.29 (m, 4H), 7.40 (d, J = 7.2 Hz, 1 H), 7.56 (s, 1 H)ppm | |
| 291 | 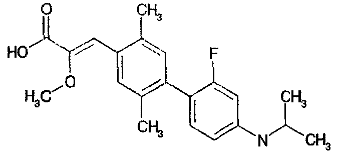 | 1H NMR (DMS-d6) δ 1.15 (d, J = 6.3 Hz, 6H), 2.12 (s, 3H), 2.28 (s, 3H), 3.54-3.60 (m, 1H), 3.69 (s, 3H), 6.38-6.47 (m, 2H), 6.94-7.03 (m, 3H), 7.76 (s, 1H)ppm | |
| 292 | 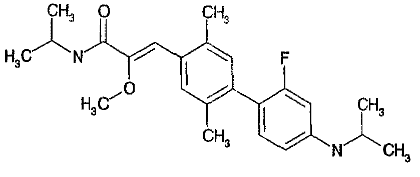 | 1H NMR (CDCl3) δ 1.24 (d, J = 6.6 Hz, 6H), 1.25 (d, J = 6.3 Hz, 6H), 2.19 (s, 3H), 2.33 (s, 3H), 3.55 (s, 3H), 3.58-3.67 (m, 1 H), 4.13-4.23 (m, 1 H), 6.33-6.42 (m, 2H), 6.59 (d, J = 7.8 Hz, 1 H), 6.98-7.03 (m, 2H), 7.20 (s, 1H), 7.56 (s, 1 H)ppm | |
| 293 | 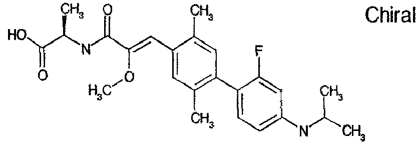 | 1H NMR (CDCl3) δ 1.26 (d, J = 6.3 Hz, 6H), 1.57 (d, J = 7.2 Hz, 3H), 2.19 (s, 3H), 2.33 (s, 3H), 3.61 (s, 3H), 4.66-4.71 (m, 1 H), 6.35-6.45 (m, 2H), 6.98-7.03 (m, 2H), 7.22-7.27 (m, 2H), 7.58 (s, 1H)ppm | |
| 294 | 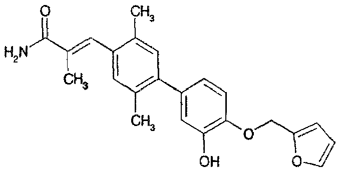 | | |
| 295 | 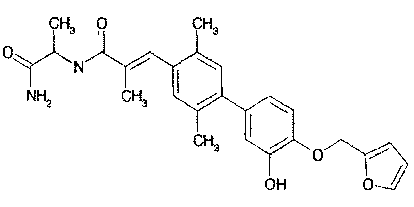 | 1H NMR (CDCl3) δ 1.50 (d, J = 6.9Hz, 3H), 2.03 (s, 3H), 2.25 (s, 6H), 4.59 - 4.72 (m, 1 H), 5.09 (s, 2H), 5.43 (brs, 1H), 5.72 (s, 1 H), 6.28 (s, 1 H), 6.36 - 6.52 (m, 2H), 6.80 (d.d, J = 8.4 & 2.1 Hz, 1 H), 6.92 (d, J = 2.1 Hz, 1 H), 7.02 (d, J = 8.4Hz, 1H), 7.04 (s, 1H), 7.07 (s, 1H), 7.47-7.53 (m, 2H) ppm | |

**[Table 49]**

| | | | |
|---|---|---|---|
| 296 | 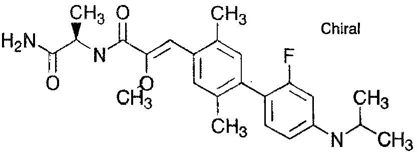 | 1H NMR (CDCl3) δ 1.27 (d, J = 6.6 Hz, 6H), 1.49 (d, J = 7.2 Hz, 3H), 2.20 (s, 3H), 2.33 (s, 3H), 3.59 (s, 3H), 3.59-3.65 (m, 1 H), 4.09-4.13 (m, 1H), 4.60-4.62 (m, 1H), 5.41 (s, 1 H), 6.36-6.44 (m, 2H), 6.98-7.04 (m, 2H), 7.21-7.27 (m, 2H), 7.56 (s, 1 H)ppm | |
| 297 | 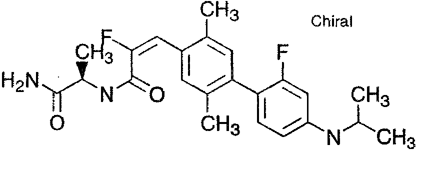 | 1H NMR (CDCl3) δ 1.25 (d, J = 6.6 Hz, 6H), 1.34 (d, J = 6.9 Hz, 3H), 2.16 (s, 3H), 2.24 8s, 3H), 3.57-3.66 (m, 1H), 4.09-4.16 (m, 1H), 4.63-4.68 (m, 1H), 5.05 (s, 1H), 6.34-6.42 (m, 2H), 6.79 (d, J = 23.2 Hz, 1H), 6.89-7.04 (m, 4H), 7.20 (s, 1H)ppm | |
| 298 | 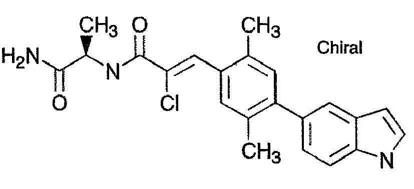 | 1H NMR (CDCl3) δ 1.54 (d, J = 6.9 Hz, 3H), 2.31 (s, 3H), 2.35 (s, 3H), 4.21-4.29 (m, 1H), 4.59-4.62 (m, 1H), 5.48 8s, 1 H), 6.14 (s, 1H), 6.59 (s, 1H), 7.16-7.35 (m, 4H), 7.44 (d, J = 8.4 Hz, 1 H), 7.59 (s, 1 H), 7.65 (s, 1H), 8.19 (s, 1H), 8.25 (s, 1 H)ppm | |
| 299 | 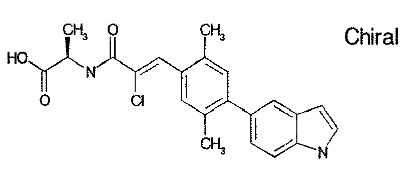 | 1 H NMR (CDCl3) δ 1.61 (d, J = 7.2 Hz, 3H), 2.31 (s, 3H), 2.35 (s, 3H), 4.69-4.78 (m, 1H), 6.60 (s, 1 H), 7.16-7.33 (m, 4H), 7.44 (d, J = 8.4 Hz, 1 H), 7.59 (s, 1 H), 7.65 (s, 1H), 8.20 (s, 1H), 8.23 (s, 1H)ppm | |
| 300 | 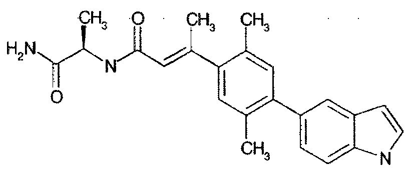 | 1 H NMR (COCl3) δ 1.46 (d, 3H, J = 6.9 Hz), 2.26 (s, 3H), 2.28 (s, 3H), 2.50 (s, 3H), 4.62 (q, 1 H, J = 7.2 Hz), 5.74 (s, 1H), 6.03 (d, 1H, J = 7.2 Hz), 6.32 (s, 1H), 6.58 (s, 1H), 6.98 (s, 1 H), 7.13 (s, 1H), 7.15 (dd, 1H, J = 8.7 Hz, 1.5 Hz), 7.42 (d, 1H, J = 8.4 Hz), 7.57 (s, 1H), 8.23 (s, 1 H) | |
| 301 | 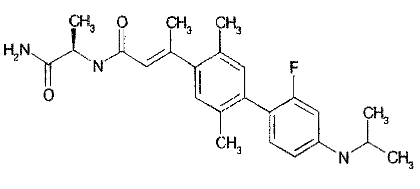 | 1H NMR (CDCl3) δ 1.27 (d, 6H, J=6.6Hz), 1.45 (d, 3H, J = 6.9 Hz), 2.17 (s, 3H), 2.25 (s, 3H), 2.46 (s, 3H), 3.62 (m, 1H), 4.62 (m, 1 H), 5.41 (s, 1 H), 5.71 (s, 1H), 6.06 (d, 1 H, J = 4.2Hz), 6.30-6.50 (m, 2H), 6.95 (s, 1H), 6.98 (m, 1H), 7.02 (s, 1H), 7.25-7.80 (m, 2H) | |

**[Table 50]**

| | | | |
|---|---|---|---|
| 302 | 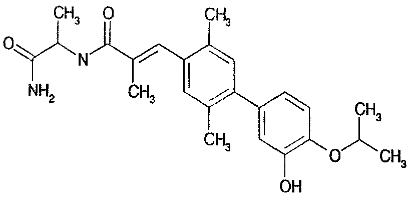 | 1H NMR (CDCl3) δ 1.40 (d, J = 6.3Hz, 6H), 1.51 (d, J = 8.4Hz, 3H), 2.03 (d, J = 1.2Hz, 3H), 2.25 (s, 3H), 2.26 (s, 3H), 4.50-4.72 (m, 2H), 5.42 (brs, 1 H), 5.74 (s, 1H), 6.26 (s, 1H), 6.47 (d, J = 6.9Hz, 1 H), 6.77 (d.d, J = 8.1 & 2.1Hz, 1 H), 6.86 - 6.97 (m, 2H), 7.04 (s, 1H), 7.08 (s, 1H), 7.27 - 7.48 (m, 1H) 7.51 (s, 1H), ppm | |
| 303 | 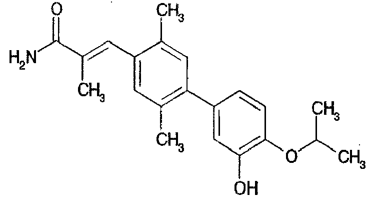 | 1H NMR (CDCl3) δ 1.41 (d, J = 6.0Hz, 6H), 2.03 (d, J = 1.2Hz, 3H), 2.26 (s, 3H), 2.27 (s, 3H), 4.54 - 4.69 (m, 1H), 5.72 (brs, 1 H), 6.78 (d.d, J = 8.1 & 2.1 Hz, 1H), 6.89 (d,J=8.1 Hz, 1H), 6.93 (d,J=2.1 Hz, 1 H), 7.06 (s, 1 H), 7.08 (s, 1 H), 7.27 - 7.47 (m, 1H) 7.51 (s, 1H), ppm | |
| 304 | 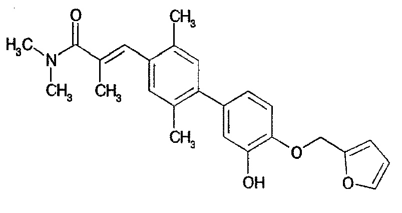 | 1H NMR (CDCl3) δ 2.01 (s, 3H), 2.25 (s, 3H), 2.26 (s, 3H), 3.10 (s, 6H), 5.09 (s, 2H), 5.73 (s, 1H), 6.39 - 6.48 (m, 1H), 6.56 (d, J = 1.5Hz, 1 H), 6.80 (d.d, J = 8.4 & 2.1Hz, 1H), 6.93 (d, J= 2.1 Hz, 1 H), 7.02 (d, J = 8.4 Hz, 1H), 7.06 (s, 1H), 7.12 (s, 1H), 7.49 (d.d, J = 2.1 & 0.9Hz, 1H) ppm | |
| 305 | 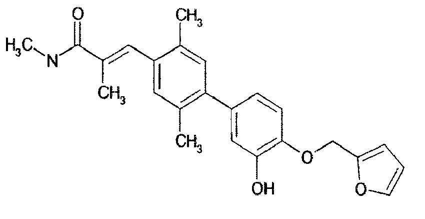 | 1 H NMR (CDCl3) δ 2.01 (s, 3H), 2.25 (s, 6H), 2.96 (d, J = 4.8Hz, 3H), 3.10 (brs, 1H), 5.09 (s, 2H), 5.72 (brs, 1H),5.89(brs, 1H), 6.36 - 6.50 (m, 2H), 6.80 (d.d, J = 8.4 & 2.1 Hz, 1 H), 6.93 (d. J = 2.1 Hz, 1 H), 7.02 (d, J = 8.4 Hz, 1H), 7.04 (s, 1H), 7.07 (s, 1H), 7.42 (s, 1H), 7.49 (d.d, J = 2.7 & 2.1 Hz, 1 H) ppm | |
| 306 | 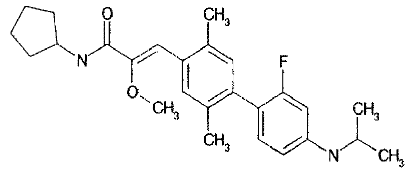 | 1H NMR (CDCl3) δ 1.27 (d, J = 6.6 Hz, 6H), 1.49-1.76 (m, 4H), 2.03-2.10 (m, 2H), 2.19 (s, 3H), 2.33 (s, 3H), 3.56 (s, 3H), 4.28-4.35 (m, 1 H), 6.27-6.45 (m, 2H), 6.70 (d, J = 7.8 Hz, 1 H), 6.99-7.04 (m, 2H), 7.20 (s, 1H), 7.56 (s, 1H)ppm | |

**[Table 51]**

| | | | |
|---|---|---|---|
| 307 | 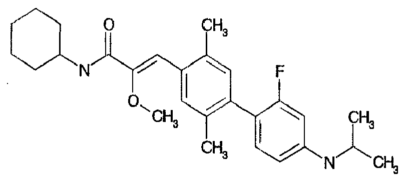 | 1 H NMR (COCl3) δ 1.26 (d, J = 6.3 Hz, 6H), 1.37-1.77 (m, 8H), 1.97-2.05 (m, 2H), 2.19 (s, 3H), 2.34 (s, 3H), 3.56 (s, 3H), 3.59-3.74 (m, 1 H), 3.85-3.93 (m, 1 H), 6.36-6.45 (m, 2H), 6.65 (d, J = 7.8 Hz, 1H), 6.99-7.04 (m, 2H), 7.20 (s, 1H), 7.57 (s, 1H)ppm | |
| 308 | 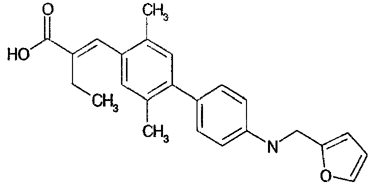 | 1H NMR (CDCl3) δ 1.18 (t, J = 7.5 Hz, 3H), 2.27 (s, 3H), 2.28 (s, 3H), 2.49 (q, J = 7.5 Hz, 2H), 6.29-6.36 (m, 2H), 6.76 (d, J = 8.7 Hz, 1H), 7.08 (s, 1H), 7.12 (s, 1 H), 7.19 (d, J = 8.7 Hz, 1H), 7.39-7.40 (m, 1H), 7,86 (s, 1H)ppm | |
| 309 | 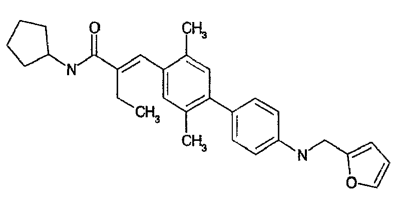 | 1 H NMR (CDCl3) δ 1.09 (t, J = 7.5 Hz, 3H), 1.48-1.73 (m, 6H), 2.08-2.10 (m, 2H), 2.23 (s, 3H), 2.26 (s, 3H), 2.44 (q, J = 7.2 Hz, 2H), 4.25-4.30 (m, 1 H), 4.37 (s, 2H), 5.78 (d, J = 6.9 Hz, 1 H), 6.30-6.35 (m, 2H), 6.77 (d, J = 8.7 Hz, 1 H), 7.02 (s, 1H), 7.05 (s, 1 H), 7.12 (s, 1H), 7.18 (d, J = 8.7 Hz, 1H), 7.39 (s, 1H)ppm | |
| 310 | 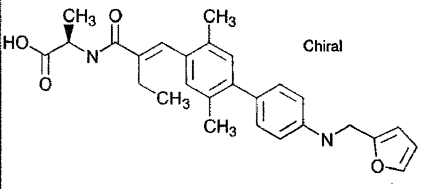 | 1H NMR (CDCl3) δ 1.26 (t, J = 7.2 Hz, 3H), 1.51 (d, J = 7.5 Hz, 3H), 2.18 8s, 3H), 2.22 (s, 3H), 2.43 (q, J = 7.5 Hz, 2H), 4.34 (s, 2H), 4.56-4.58 (m, 1H), 6.25-6.33 (m, 2H), 6,74 (d, J = 8.4 Hz, 2H), 7.00 (d, J = 9.0 Hz, 2H), 7.12 (d, J = 8.4 Hz, 2H), 7.26 8s, 1H), 7.38 (s, 1 H)ppm | |
| 311 | 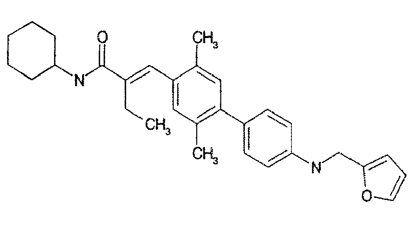 | 1H NMR (CDCl3) δ 1.09 (t, J = 7.5 Hz, 3H), 1.39-1.77 (m, 6H), 1.99-2.05 (m, 2H), 2.23 (s, 3H), 2.26 (m, 3H), 2.44 (q, J = 7.5 Hz, 2H), 3.91-3.94 (m, 1H), 4.37 (s, 2H), 5.70 (d, J = 8.1 Hz, 1H), 6.23-6.35 (m, 2H), 6.75-6.77 (m, 2H), 7.03 (d, J = 8.1 Hz, 2H), 7.11 (s, 1 H), 7.19 (d, J = 8.1 Hz, 2H), 7.39 (s, 1H)ppm | |

**[Table 52]**

| | | | |
|---|---|---|---|
| 312 | 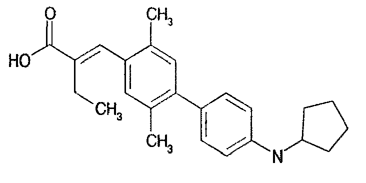 | 1 H NMR (CDCl3) δ 1.19 (t, J = 7.5 Hz, 3H), 1.53-1.78 (m, 6H), 2.01-2.11 (m, 2H), 2.28 (s, 3H), 2.30 (s, 3H), 2.50 (q, J = 7.2 Hz, 2H), 3.79-3.87 (m, 1H), 6.65 (d, J = 8.4 Hz, 2H), 7.10-7.17 (m, 4H), 7.87 (s, 1 H)ppm | |
| 313 | 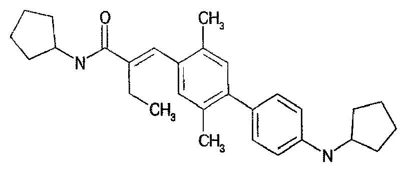 | 1 H NMR (CDCl3) δ 1.09 (t, J = 7.5 Hz, 3H), 1.46-1.78 (m, 6H), 2.05-2.08 (m, 2H), 2.23 (s, 3H), 2.27 (s, 3H), 2.44 (q, J = 7.5 Hz, 2H), 3.79-3.85 (m, 1H), 4.31-4.28 (m, 1 H), 5.78 (d, J = 7.2 Hz, 1 H), 6.65 (d, J = 8.5 Hz, 2H), 7.02-7.16 (m, 5H)ppm | |
| 314 | 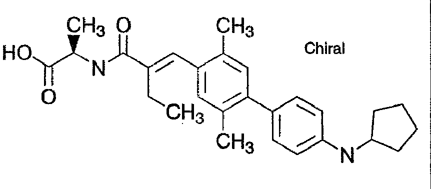 | 1H NMR (CDCl3) δ 1.16 (t, J = 7.2 Hz, 3H), 1.58 (d, J = 7.2 Hz, 3H), 1.51-1.76 (m, 6H), 2.00-2.11 (m, 2H), 2.24 (s, 3H), 2.27 (s, 3H), 2.48 (q, J = 7.5 Hz, 2H), 3.79-3.87 (m, 1 H), 4.67-4.77 (m, 1H), 6.45 (s, 1H), 6.69 (d, J = 8.4 Hz, 2H), 7.03 (s, 1H), 7.07 (s, 1 H), 7.16 (d, J = 8.7 Hz, 2H), 7.30 (s, 1 H)ppm | |
| 315 | 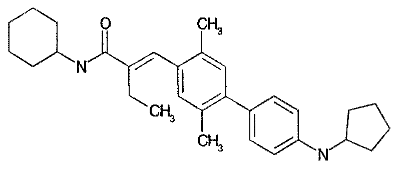 | 1H NMR (CDCl3) δ 1.09 (t, J = 7.5 Hz, 3H), 1.37-1.76 (m, 6H), 2.02-2.08 (m, 2H), 2.23 (s, 3H), 2.27 (s, 3H), 2.45 (q, J = 7.2 Hz, 2H), 3.80-3.86 (m, 1H), 3.91-3.93 (m, 1H), 5,70 (d, J = 8.4 Hz, 1 H), 6.66 (d, J = 8.4 Hz, 2H), 7.02 (s, 1H), 7.06 (s, 1H), 7.11 (s, 1H), 7.15 (d, J = 8.4 Hz, 2H)ppm | |
| 316 | 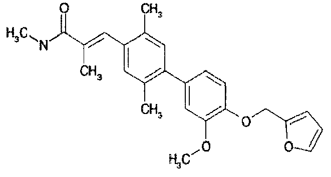 | | |
| 317 | 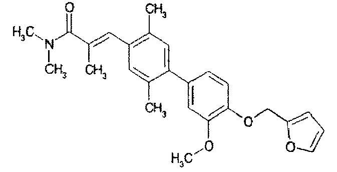 | | |

**[Table 53]**

| | | | |
|---|---|---|---|
| 318 | 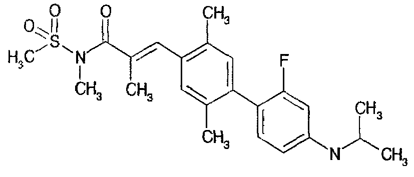 | 1 H NMR (CDCl3+CD3OD) δ 1.27 (d, J = 6.3Hz, 6H), 2.08 (d, J = 1.8Hz, 3H), 2.20 (s, 3H), 2.26 (s, 3H), 3.32 (s, 3H), 3.36 (s, 3H), 3.54 - 3.70 (m, 1 H), 6.34 - 6.52 (m, 2H), 7.06 - 6.94 (m, 2H), 7.07 (s, 1H), 7.13 (s, 1H) ppm | |
| 319 | 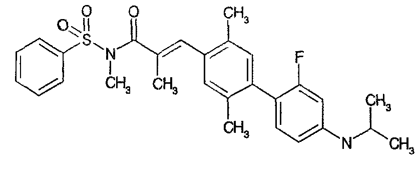 | 1H NMR (CDCl3) δ 1.27 (d, J = 6.3Hz, 6H), 2.03 (d, J = 1.5Hz, 3H), 2.21 (s, 6H), 3.31 (s, 3H), 3.58 - 3.69 (m, 1 H), 6.34 -- 6.58 (m, 2H), 6.84 - 7.16 (m, 4H), 7.17 - 7.67 (m, 3H), 7.73 - 7.80 (m, 1H), 7.93-7.99 (m,1 H) ppm | |
| 320 | 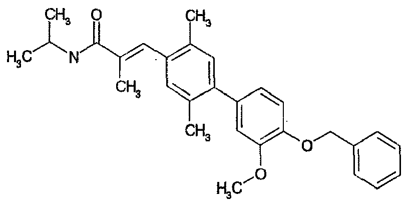 | 1H NMR (COCl3) δ 1.25 (d, J = 6.6Hz, 6H), 1.99 (d, J = 1.2Hz, 3H), 2.25 (s, 6H), 3.90 (s, 3H), 4.15 - 4.29 (m, 6H), 5.2.0 (s, 2H), 5.66 (d, J = 7.8Hz, 1 H), 6.81 (d.d, J = 8.1 & 2.1 Hz, 1 H), 6.87 (d, J = 2.1 Hz, 1H), 6.93 (d, J = 8.1Hz, 1H), 7.05 (s, 1 H), 7.08 (s, 1H), 7.28 - 7.51 (m, 6H) ppm | |
| 321 | 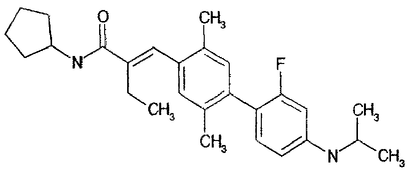 | 1 H NMR (CDCl3) δ 1.09 (t, J = 7.5 Hz, 3H), 1.46-1.73 (m, 6H), 2.05-2.12 (m, 2H), 2.18 (s, 3H), 2.23 (s, 3H), 2.44 (q, J = 7.5 Hz, 2H), 3.61-3.67 (m, 1H), 4.31-4.38 (m, 1H), 5.78 (d, J = 8.4 Hz, 1H), 6.35-6.44 (m, 2H), 6.98-7.02 (m, 3H), 7.11 (s, 1 H)ppm | |
| 322 | 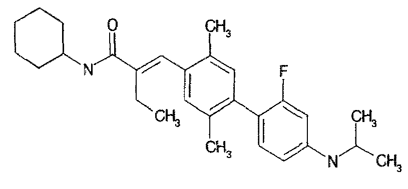 | 1 H NMR (CDCl3) δ 1.09 (t, J = 7.5 Hz, 3H), 1.20-1.72 (m, 8H), 2.00-2.11 (m, 2H), 2.18 (s, 3H), 2.23 (s, 3H), 2.43 (q, J = 7.5 Hz, 2H), 3.59-3.63 (m, 1H), 3.90-3.93 (m, 1H), 5,71 (d, J = 9.0 Hz, 1H), 6.37-6.45 (m, 2H), 6.99-7.05 (m, 3H), 7.11 (s, 1H)ppm | |
| 323 | 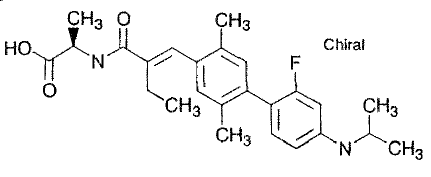 | 1 H NMR (CDCl3) δ 1.12 (t, J = 7.5 Hz, 3H), 1.27 (d, J = 6.0 Hz, 6H), 1.58 (d, J = 7.2 Hz, 3H), 2.19 (s, 3H), 2.26 (s, 3H), 2.45 (q, J = 7.8 Hz, 2H), 3.59-3.67 (m, 1 H), 4.70-4.74 (m, 1 H), 6.40-6.49 (m, 3H), 6.99-7.04 (m, 3H), 7.29 (s, 1H)ppm | |

**[Table 54]**

| | | | |
|---|---|---|---|
| 324 | 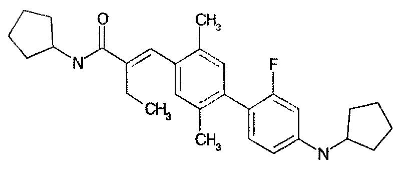 | 1H NMR (CDCl3) δ 1.09 (t, J = 7.8 Hz, 3H), 1.44-1.77 (m, 12H), 2.00-2.12 (m, 4H), 2.18 (s, 3H), 2.23 (s, 3H), 2.41 (q, J = 7.5 Hz, 2H), 3.77-3.83 (m, 1H), 4..29-4.38 (m, 1H), 5.77 (d, J = 6.9 Hz, 1H), 6.41-6.47 (m, 2H), 7.03 (s, 2H), 7.11 (s, 1 H)I, 7.16 (s, 1H)ppm | |
| 32.5 | 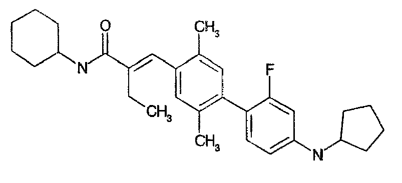 | 1 H NMR (CDCl3) δ 1.09 (t, J = 7.5 Hz, 3H), 1.20-1.76 (m, 14H), 2.02-2.11 (m, 4H), 2.18 (s, 3H), 2.24 (s, 3H), 2.44 (q, J = 7.5 Hz, 2H), 3.75-3.83 (m, 1H), 3.91-3.93 (m, 1H), 5.7 (d, J = 7.5 Hz, 1 H), 6.40-6.48 (m, 2H), 6.99-7.03 (m, 3H), 7.11 (s, 1H)ppm | |
| 326 | 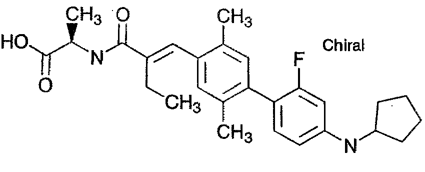 | 1 H NMR (CDCl3) δ 1,12 (t, J = 7.5 Hz, 3H), 1.52-1.78 (m, 6H), 2.00-2.12 (m, 2H), 2.19 (s, 3H), 2.24 (s, 3H), 2.48 (q, J = 7.5 Hz, 2H), 3.74-3.83 (m, 1 H), 4.68-4.77 (m, 1 H), 6.34-6.43 (m, 3H), 6.97-7.04 (m, 3H), 7.29 (s, 1 H)ppm | |
| 327 | 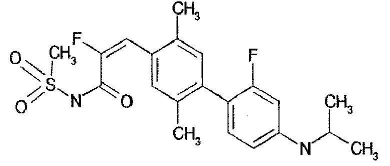 | 1H NR (CDCl3) δ 1.26 (d, J = 6.3 Hz, 6H), 2.19 (s, 3H), 2.28 (s, 3H), 3.33 (s, 3H), 3.58-3.67 (m, 1H), 6.36-6.45 (m, 2H), 6.97-7.11 (m, 4H), 3.74 (s, 1H)ppm | |
| 328 | 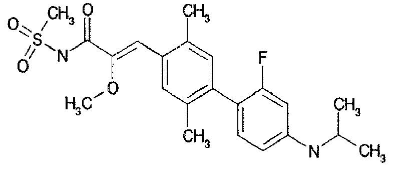 | 1 H MMR (CDCl3) δ 1.26 (d, J = 6.3 Hz, 6H), 2.21 (s, 3H), 2.33 (s, 3H), 4.40 8s, 3H), 3.62 (s, 3H), 6.34-6.44 (m, 2H), 6.97-7.06 (m, 2H), 7.26 (s, 1H), 7.33 (s, 1 H), 7.53 (s, 1H)ppm | |
| 329 | 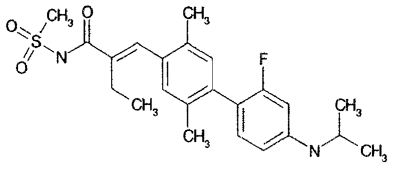 | 1H NMR (CDCl3) δ 1.14 (t, J = 7.5 Hz, 3H), 1.27 (d, J = 6.3 Hz, 6H), 2.20 (s, 3H), 2.24 (s, 3H), 2.49 (q, J = 7.5 Hz, 2H), 3.43 (s, 3H), 3.63-3.66 (m, 1H), 6.38-6.47 (m, 2H), 6.98-7.10 (m, 3H), 7.40 (s, 1H), 8.21 (s, 1 H)ppm | |

**[Table 55]**

| | | | |
|---|---|---|---|
| 330 | 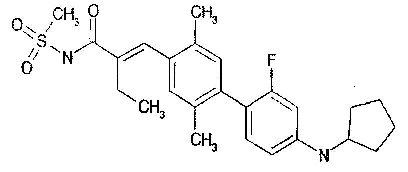 | 1 H MNR (CDCl3) δ 1.14 (t, J = 7.5 Hz, 3H), 1.56-1.79 (m, 6H), 2.05-2.13 (m, 2H), 2.19 (s, 3H), 2.23 (s, 3H), 2.49 (q, J = 7.5 Hz, 2H), 3.43 (s, 3H), 3.77-3.81 (m, 1H), 6.61 (s, 2H), 7.04-7.07 (m, 3H), 7.39 (s, 1H), 8.14 (s, 1 H)ppm | |
| 331 | 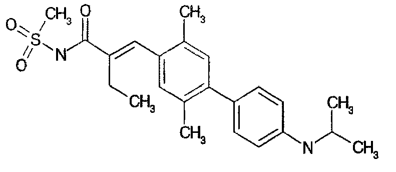 | 1 H NMR (CDCl3) δ 1.14 (t, J = 7.5 Hz, 3H), 1.28 (d, J = 6.3 Hz, 6H), 2.25 (s, 3H), 2.28 (s, 3H), 2.50 (q, J = 7.5 Hz, 2H), 3.75-3.83 (m, 1H), 6.73 (bs, 2H), 7.04 (s, 1H), 7.09 (s, 1H), 7.17 (d, J = 8.4 Hz, 2H), 7.27 (s, 1H), 7.41 (s. 1 H)ppm | |
| 332 | 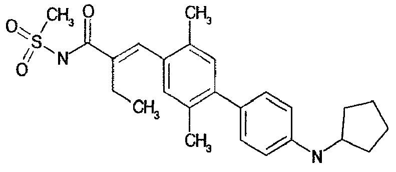 | 1 H NMR (CDCl3) δ 1.14 (t, J = 7.2 Hz, 3H), 1.56-1.77 (m, 6H), 2.05-2.06 (m, 2H), 2.25 (s, 3H), 2.28 (s, 3H), 2.50 (q, J = 7.8 Hz, 2H), 3.43 (s, 3H), 3.82-3.85 (m, 1 H), 6.73 (bs, 2H), 7.03-7.08 (m, 2H), 7.16 (d, J = 7.8 Hz, 2H), 7.41 (s, 1H)ppm | |
| 333 | 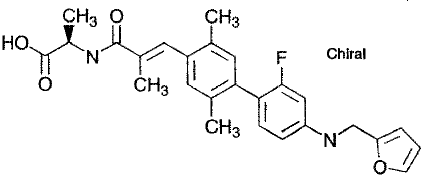 | 1H NMR (CDCl3) δ 1.59 (d, J = 6.9Hz, 3H), 2.08 (s, 3H), 2.27 (s, 3H), 2.28 (s, 3H), 4.22 - 4.29 (m, 1 H), 4.35 (s, 2H), 5.79 (d, J = 8.1 Hz, 1 H), 6.27 (d.d, J = 3.3 & 0.6Hz, 1 H), 6.32 (d.d, J = 3.3 & 2.1 Hz, 1H), 6.40 - 6.58 (m, 2H), 7.06 (t, J = 8.1 Hz, 2H), 7.02 (s, 1 H), 8.40 (d.d, J = 1.8 & 0.9Hz, 1H) ppm | |
| 334 | 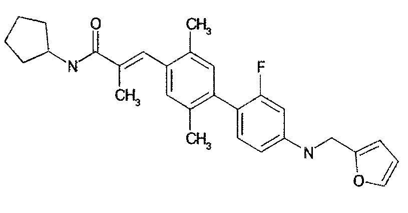 | 1H NMR (CDCl3) δ 1.38 - 1.80 (m, 6H), 1.99 (d, J = 1.2Hz, 3H), 2.03 - 2.13 (m, 2H), 2.17 (s, 3H), 2.24 (s, 3H), 4.27 - 4.39 (m, 1 H), 4.35 (s, 2H), 5.79 (d, J = 7.8Hz, 1H), 6.29 (d.d, J = 3.3 & 0.6Hz, 1 H), 6.35 (d.d, J = 3.3 & 2.1Hz, 1H), 6.42 - 6.54 (m, 2H), 7.04 (t, J = 8.1 Hz, 2H), 7.04 (s, 1H), 6.40 (d.d, J = 1.8 & 0.9Hz, 1H) ppm | |

**[Table 56]**

| | | | |
|---|---|---|---|
| 335 | 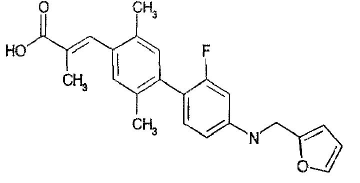 | 1H NMR (CDCl3) δ 1.45 - 1.82 (m, 6H), 1.96 - 2.12 (m, 2H), 2.06 (s, 3H), 2.21 (s, 3H), 2.28 (s, 3H), 3.74 - 3.84 (m, 1H), 6.32 - 6.45 (m, 2H), 7.01 (t, J = 8.4Hz, 1H), 7.08 (s, 1H), 7.15 (s, 1H), 7.90 (s, 1 H) ppm | |
| 336 | 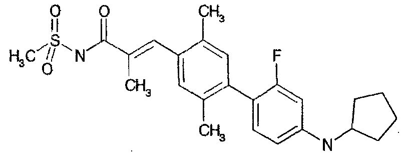 | 1H NMR (CDCl3) δ 1.42 - 1.84 (m, 6H), 1.96 - 2.12 (m, 2H), 2.06 (d, J = 1.5Hz, 1H), 2.20 (s, 1H), 2.26 (s, 1H), 3.43 (s, 3H), 3.63 - 3.84 (m, 1 H), 6.32 - 6.45 (m, 2H), 6.95 - 7.23 (m, 1 H), 7.06 (s, 1H), 7.08 (s, 1H), 7.59 (s, 1H), 8.23 (s, 1H) ppm | |
| 337 | 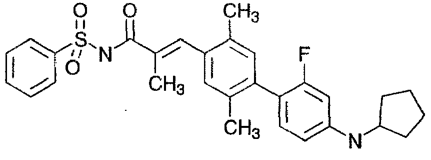 | 1H NMR (CDCl3+CD3OD) δ 1.75 - 2.14 (m, 8H), 1.95 (d, J = 1.2Hz, 3H), 2.11 (s, 3H), 2.22 (s, 3H), 3.74 - 3.85 (m, 1 H), 7.00 (s, 1H), 7.04 (s, 1H), 7.29 - 7.71 (m, 7H), 8.13 (d, J = 1.2Hz, 1H), 8.16 (s, 1H) ppm | |
| 338 | 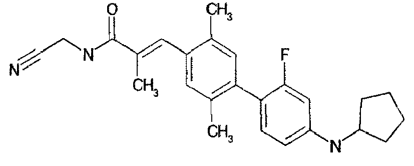 | 1H NMR (CDCl3) δ 1.43 - 1.80 (m, 6H), 1.88 - 2.11 (m, 5H), 2.20 (s, 3H), 2.25 (s, 3H), 3.74-3.84 (m, 1H), 4.34 (d, J = 5.7Hz, 2H), 6.20 (t, J = 5.7Hz, 1H), 6.31 - 6.47 (m, 2H), 6.95 - 7.10 (m, 3H), 7.52 (s, 1H) ppm | |
| 339 | 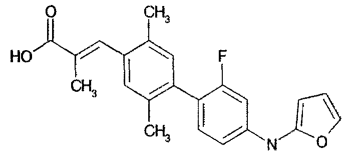 | | |
| 340 | 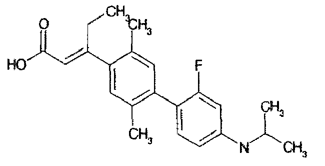 | | |
| 341 | 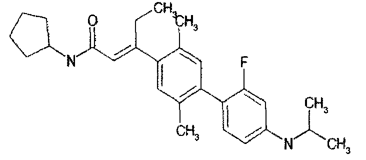 | | |

**[Table 57]**

| | | | |
|---|---|---|---|
| 342 | 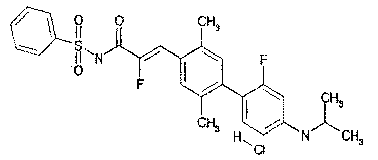 | 1 H NMR (CDCl3) δ 1.51 (d, J = 6.3 Hz, 6H), 2. 07 (s, 3H), 2.16 (s, 3H), 3.67-3.74 (m, 1 H), 6.88-6.96 (m, 2H), 7.23-7.33 (m, 3H), 7.47-7.69 8m, 4H), 8.07 (d, J = 8.1 Hz, 2H), 8.47 (s, 1 H)ppm | |
| 343 | 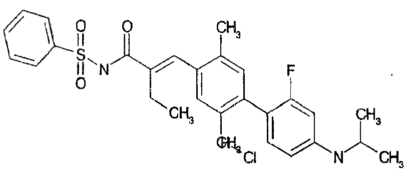 | 1H NMR (CDCl3) δ 0.99 (t, J = 7.5 Hz, 3H), 1.50 (d, J = 6.3 Hz, 6H), 2.09 (s, 3H), 2.17 (s, 3H), 2.38 (q, J =7.5 Hz, 2H), 3.69-3.73 (m, 1H), 6.96 (s, 1H), 6.99 (s, 1H), 7.26-7.33 (m, 2H), 7.46-7.71 (m, 5H), 8.17 (d, J = 7.5 Hz, 2H), 8.67 (s, 1H)ppm | |
| 344 | 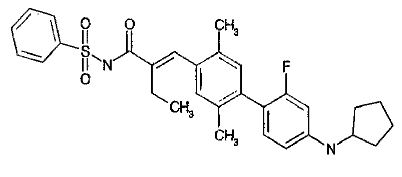 | 1 H NMR (CDCl3) δ 1.00 (t, J = 7.5 Hz, 3H), 1.54-1.76 (m, 6H), 2.17 (s, 3H), 2.20 (s, 3H), 2.40 (q, J = 7.5 Hz, 2H), 3.74-3.80 (m, 1H), 6.39-6.48 (m,2H), 6.97-7.03 (m, 3H), 7.33 (s, 1 H), 7.56-7.70 (m, 3H), 8.16 (d, J = 8.1 Hz, 2H), 8.46 (s, 1 H)ppm | |
| 345 | 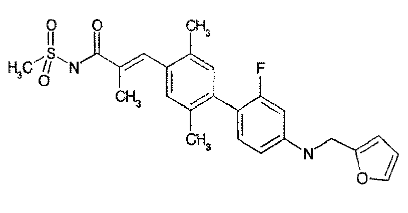 | 1 H NMR (CDCl3) δ 2.06 (d, J = 1.2Hz, 3H), 2.19 (s, 3H), 2.26 (s, 3H), 3.42 (s, 3H), 4.24 (brs, 1H), 4.35 (s, 2H), 6.29 (d.d, J = 6.6 & 2.7Hz, 1 H), 6.36 (d.d, J = 6.6 & 1.8Hz, 1 H), 6.41 - 6.54 (m, 2H), 7.01 (d, J = 8.4Hz, 1 H), 7.06 (s, 1H), 7.08 (s, 1H), 7.40 (d.d, J = 1.8 & 0.9Hz, 1 H), 7.58 (s, 1 H), 8.25 (s, 1H) ppm | |
| 346 | 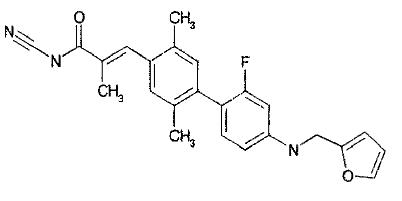 | 1H NMR (CDCl3) δ 2.10 (d, J = 1.2Hz, 3H), 2.16 (s, 3H), 2.64 (s, 3H), 4.35 (s, 2H), 6.29 (d.d, J = 6.6 & 0.6Hz, 1 H), 6.36 (d.d, J = 6.6 & 1.8Hz, 1 H), 6.45 (d.d, J = 12.3 & 2.4Hz, 1 H), 6.51 (d.d, J = 8.4 & 2.1 Hz, 1 H), 7.01 (d, J = 8.4Hz, 1 H), 7.07 (s, 1H), 7.08 (s, 1 H), 7.40 (d.d, J = 1.8 & 0.6Hz, 1 H), 7.65 (s, 1 H) ppm | |

**[Table 58]**

| | | | |
|---|---|---|---|
| 347 | 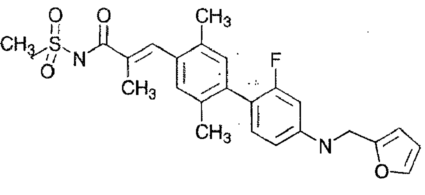 | 1H NMR (d6-DMSO) δ 1.22 (t, J = 7.5Hz, 3H), 1.90 (d, J = 1.2Hz, 3H), 2.11 (s, 3H), 2.22 (s, 3H), 3.28 (q, J = 7.5Hz, 2H), 4.29 (d, J = 6.6Hz, 2H), 6.35 (d, J = 3.3Hz, 1 H), 6.41 (d.d, J = 3.3 & 2.1 Hz, 1 H), 6.46 - 6.58 (m, 2H), 6.95 - 7.40 (m, 2H), 7.11 (s, 1H), 7.49 (s, 1H), 7.60 (d.d, J = 1.8 & 0.9Hz, 1 H) ppm | |
| 348 | 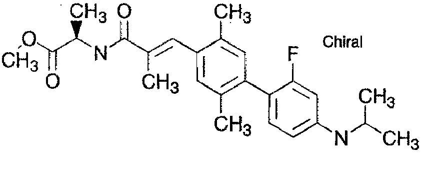 | 1 H NMR (CDCl3) δ 1.23 (d, J = 6.3 Hz, 6H), 1.50 (d, J = 7.2 Hz, 3H), 2.01 (s, 3H), 2.21 (s, 3H), 2.23 (s, 3H), 3.61 (m, 1H), 3.80 (s, 3H), 4.80 (m, 1H), 5.21 (brs, 1H), 6.31-6.64 (m, 4H), 6.92-7.15 (m, 3H), 7.57 (s, 1H) | |
| 349 | 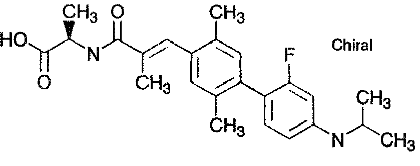 | 1H NMR (CDCl3) δ 1.25 (d, J = 6.3 Hz, 6H), 1.51 (d, J = 6.9 Hz, 3H), 2.03 (s, 3H), 2.19 (s, 3H), 2.24 (s, 3H), 3.63 (m, 1H), 4.69 (m, 1H), 5.48 (brs, 1H), 6.34-6.54 (m, 4H), 6.98-7.05 (m, 3H), 7.51 (s, 1H) | |
| 350 | 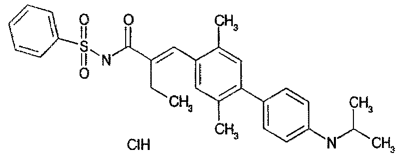 | 1H NMR (CD3OD) δ 1.10 (t, J = 7.2 Hz, 3H), 1.39 (d, J = 6.6 Hz, 6H), 2.24 (s, 3H), 2.26 (s, 3H), 2.40 (q, J = 7.2 Hz, 2H), 3.79-3.86 (m, 1H), 7.11 (s, 1H), 7.15 (s, 1H), 7.51-7.60 (m, 9H), 7.72 (s, 1H)ppm | |
| 351 | 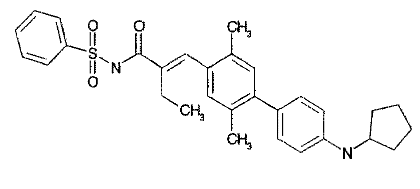 | 1H NMR (CDCl3) δ 1.00 (t, J = 7.8 Hz, 3H), 1.56-1.76 (m, 6H), 2.00-2.05 (m, 2H), 2.20 (s, 3H), 2.25 (s, 3H), 2.41 (q, J = 7.2 Hz, 2H), 3.79-3.83 (m, 1H), 6.85 (d, J = 8.1 Hz, 2H), 6.98 (s, 1 H), 7.06 (s, 1H), 7.14 (d, J = 8.1 Hz, 2H), 7.34 (s, 1H), 7.53-7.61 (m, 3H), 8.16 (d, J = 8.0 Hz, 2H)ppm | |
| 352 | 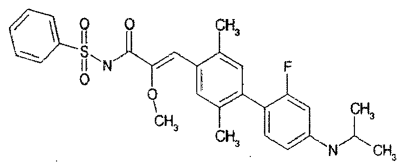 | 1H NMR (CDCl3) δ 1.29 (d, J = 6.3 Hz, 6H), 2.17 (s, 3H), 2.27 (s, 3H), 3.60 (s, 3H), 6.44-6.54 (m, 2H), 7.02 (s, 1 H), 7.49-7.71 (m, 7H), 8.14 (d, J = 8.1 Hz, 2H)ppm | |

**[Table 59]**

| | | | |
|---|---|---|---|
| 353 | 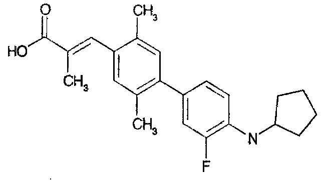 | 1H NMR (CDCl3) δ 1.49-1.82 (m, 6H), 1.90-2.15 (m, 2H), 2.07 (s, 3H), 2.29 (s, 6H), 3.79-3.89 (m, 1H), 6.72 - 6.79 (m, 1 H), 6.93 - 7.02 (m, 1H), 7.09 (s, 1 H) 7.14 (s, 1 H), 7.90 (s, 1 H) ppm | |
| 354 | 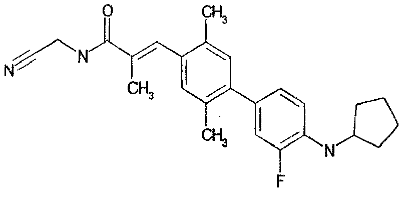 | 1H NMR (CDCl3) δ 1.48-1.87 (m, 6H), 1.96 - 2.14 (m, 2H), 2.04 (s, 3H), 2.25 (s, 3H), 2.28 (s, 3H), 3.79 - 3.89 (m, 1H), 4.34 (d, J=5.4Hz, 3H), 6.23 (t, J=5.4Hz, 1H), 6.73 - 6.80 (m, 1 H), 6.94-7.00 (m, 1H), 7.04 (s, 1 H) 7.08 (s, 1H), 7.52 (s, 1H) ppm | |
| 355 | 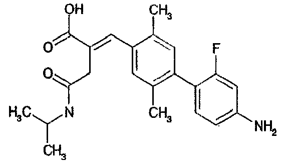 | | 169-173°C |
| 356 | 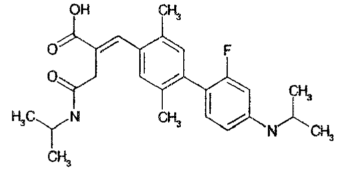 | | 201-203°C |
| 357 | 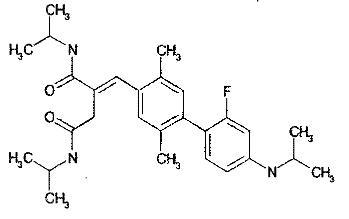 | | 193-196°C |
| 358 | 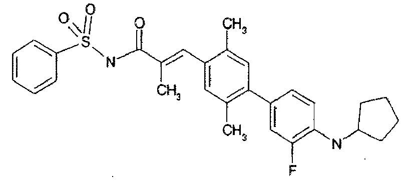 | 1H NMR (CDCl3) δ 1.48 - 1.82 (m, 6H), 1.90 - 2.15 (m, 2H), 1.98 (d, J = 1.2Hz, 3H), 2.22 (s, 3H), 2.25 (s, 3H), 3.75 - 3.84 (m, 1 H), 6.70 - 6.80 (m, 1H), 6.91 - 6.94 (m, 1 H), 6.95 - 6.98 (m, 1H), 6.99 (s, 1H), 7.06 (s, 1 H), 7.47 - 7.71 (m, H), 8.16 (s, 1 H), 8.18 (s, 1H), 8.40 (brs, 1H) ppm | |

**[Table 60]**

| | | | |
|---|---|---|---|
| 359 | 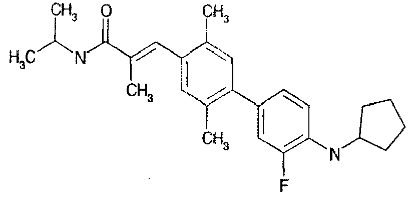 | 1H NMR (CDCl3) δ 1.25 (d, J = 6.6Hz, 6H), 1.50-1.85 (m, 6H), 1.98-2.13 (m, 2H), 1.99 (d, J = 1.5Hz, 3H), 2.25 (s, 3H), 2.27 (s, 3H), 3.79-3.89 (m, 1H), 4.16-4.29 (m, 1H), 5.66 (d, J = 7.2Hz, 1H), 6.74-6.83 (m, 1H), 6.97-7.02 (m, 1H), 7.03 (s, 1H), 7.06 (s, 1H) ppm | |
| 360 | 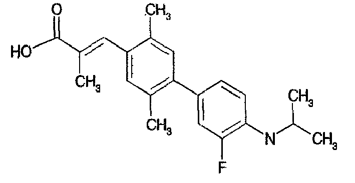 | | |
| 361 | 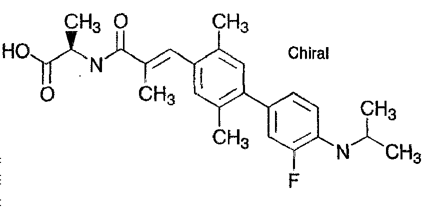 | 1H NMR (CDCl3) δ 1.28 (d, J = 6.6Hz, 6H), 2.28 (d, J = 5.7Hz, 3H), 2.05 (d, J = 1.5Hz, 3H), 2.26 (s, 3H), 2.28 (s, 3H), 3.61-3.76 (m, 1 H), 4.65 - 4.77 (m, 1 H), 6.40 (d, J = 6.6Hz), 6.69-6.78 (m, 1 H), 6.96 (s, 1H), 6.97 - 7.02 (m, 1H), 7.04 (s, 1H), 7.07 (s, 1H), 7.55 (s, 1H) ppm | |
| 362 | 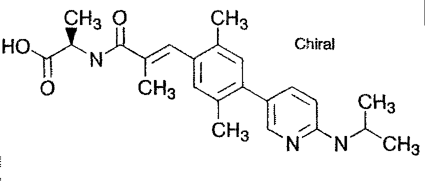 | 1H NMR (CDCl3) δ 1.37 (d, J = 6.3Hz, 6H), 1.57 (d, J = 6.9Hz, 3H), 2.03 (s, 3H) 2.25 (s, 6H), 3.69 - 3.82 (m, 1 H), 4.50 - 4.62 (m, 1H), 6.69 (d, J = 9.0Hz, 1 H), 7.00 (s, 1H), 7.04 (d, J = 6.6Hz, 1H), 7.09 (s, 1 H), 7.42 (s, 1H), 7.69 (d, J = 9.0Hz, 1H), 7.79 (s, 1 H) ppm | |
| 363 | 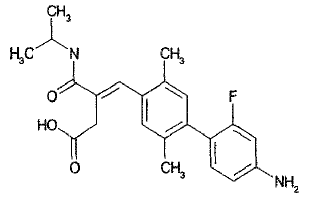 | 1H NMR (d6-DMSO) δ 1.14 (d, J = 6.6 Hz, 6H), 2.08 (s, 3H), 2.20 (s, 3H), 3.27 (s, 2H), 4.00 (m, 1H), 5.48 (brs, 1H), 6.36-6.46 (m, 2H), 6.93 (t, J = 8.4 Hz, 1H), 7.01 (s, 1H), 7.04 (s, 1H), 7.35 (s, 1H), 7.93 (d, J = 7.8 Hz, 1H). | |

**[Table 61]**

| | | | |
|---|---|---|---|
| 364 | 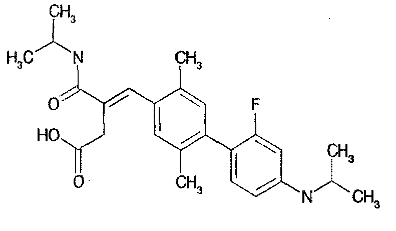 | 1H NMR (d6-DMSO) δ 1.14 (d, J = 6.6 Hz, 3H), 1.15 (d, J = 6.3 Hz, 3H), 2.09 (s, 3H), 2.20 (s, 3H), 3.16 (s, 2H), 3.54 (m, 1H), 3.98 (m, 1H), 4.09 (brs, 1H), 5.84 (d, J = 7.8 Hz, 1H), 6.35-6.46 (m, 2H), 6.97 (t, J = 8.4 Hz, 1H), 7.01 (s, 1H), 7.05 (s, 1 H), 7.95 (d, J = 8.1 Hz, 1H), 12.3 (brs, 1H). | |
| 365 | 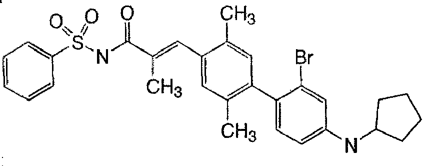 | | |
| 366 | 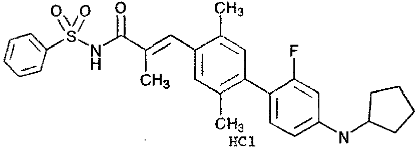 | | |
| 367 | 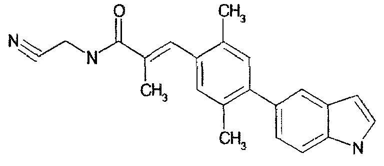 | | 180°C |
| 368 | 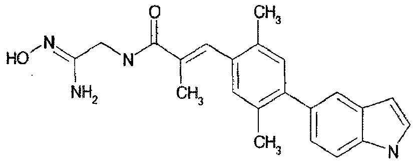 | | 175.5-177.5 °C |
| 369 | 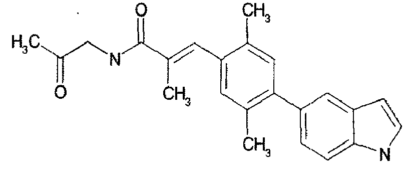 | | 195-196°C |
| 370 | 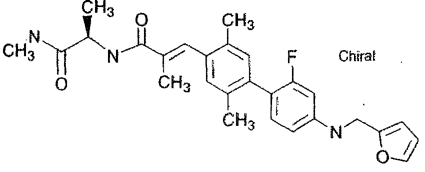 | | |

**[Table 62]**

| | | | |
|---|---|---|---|
| 371 | 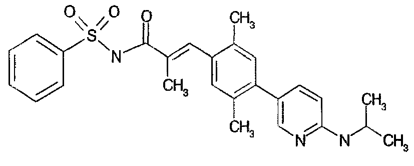 | 1H NMR (CDCl3) δ 1.27 (d, J = 6.3Hz, 6H), 1.98 (d, J = 1.2Hz, 3H), 2.19 (s, 3H), 2.23 (s, 3H), 3.80-3.90 (m, 1 H), 6.45 (d, J = 8.4Hz, 1H), 7.00 (s, 1H), 7.05 (s, 1H), 7.40-7.56 (m, 4H), 7.72 (s, 1 H), 7.98 (d, J = 2.7Hz, 1 H), 8.03 (s, 1H), 8.05 (s, 1H) ppm | |
| 372 | 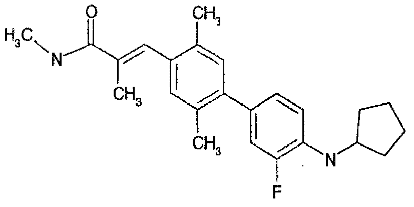 | 1H NMR (CDCl3) δ 1.48-1.85 (m, 6H), 1.96 - 2.13 (m, 2H), 2.01 (d, J = 1.2Hz, 3H), 2.24 (s, 3H), 2.27 (s, 3H), 2.96 (d, J = 4.8Hz,), 3.77 - 3.90 (m, 1H), 3.93 (s, 1H), 5.92 (d, J = 4.8Hz, 1 H), 6.75 (t, J = 8.7Hz, 1 H), 6.95 (s, 1 H), 6.99 (s, 1H), 7.04 (s, 1 H), 7.06 (s, 1H), 7.42 (s, 1H) ppm | |
| 373 | 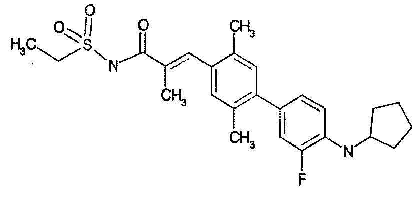 | 1H NMR (CDCl3) δ 1.46 (t, J = 7.5Hz, 3H), 1.48 - 1.85 (m, 6H), 1.96 - 2.13 (m, 2H), 2.07 (s, 3H), 2.27 (s, 3H), 2.28 (s, 3H), 3.61 (q, J = 7.5Hz, 2H), 3.79-3.89 (m, 1H), 6.76 (t, J = 8.4Hz, 1H), 6.93 - 7.00 (m, 2H), 7.06 (s, 1H), 7.09 (s, 1 H), 7.58 (s, 1 H), 8.08 (s, 1 H) ppm | |
| 374 | 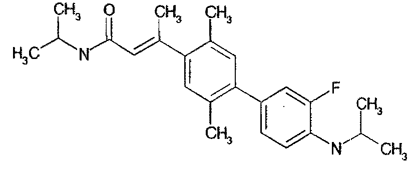 | 1H NMR (CDCl3) δ 1.21 (d, 6H, J = 6.3 Hz), 1.27 (d, 6H, J = 6.0 Hz), 2.25(s, 3H), 2.27 (s, 3H), 2.47 (s, 3H), 3.68 (m, 1H), 4.19 (m, 1 H), 5.29 (d, 1H, J = 7.2 Hz), 5.62 (s, 1H), 6.74 (t, 1H, J = 8.4 Hz), 6.95 (s, 1H), 6.96-7.03 (m, 3H) | |
| 375 | 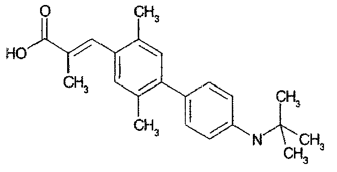 | | 204-205°C |
| 376 | 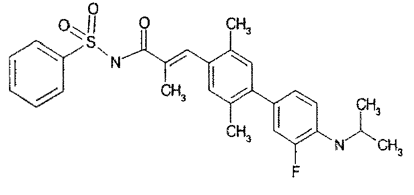 | 1 H NMR (CDCl3+CD3OD) δ 1.26 (d, J = 6.0Hz, 6H), 1.98 (d, J = 1.5Hz, 3H), 2.19 (s, 3H), 2.24 (s, 3H), 3.62-3.74 (m, 1 H), 6.69 (t, J = 8.4Hz, 1H), 6.94 (s, 1 H), 6.96 - 6.99 (m, 1 H), 7.03 (s, 2H), 7.31 - 7.75 (m, 4H), 8.04 (s, 1H), 8.07 (s, 1H) ppm | |

**[Table 63]**

| | | | |
|---|---|---|---|
| 377 | 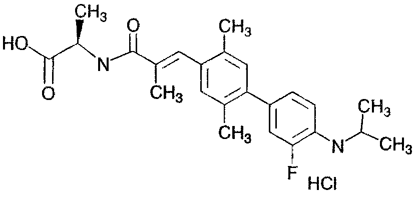 | | |
| 378 | 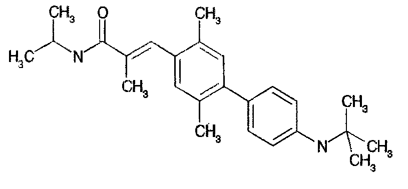 | 1 H NMR (CDCl3) δ 1.25 (d, J = 6.6 Hz, 6H), 1.39 (s, 9H), 2.00 (s, 3H), 2.25 (s, 3H), 2.28 (s, 3H), 4.20 (m, 1H), 5.66 (d, J = 7.8 Hz, 1 H), 6.78 (d, J = 8.7 Hz, 2H), 7.04 (s, 1H), 7.08 (s, 1H), 7.13 (d, J = 8.4 Hz, 2H), 7.41 (s, 1 H). | |
| 379 | 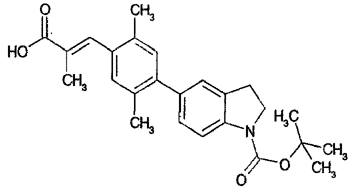 | | 193-195°C |
| 380 | 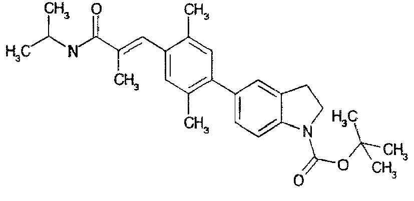 | 1H NMR (CDCl3) δ 1.25 (d, J = 6.6 Hz, 6H), 1.58 (s, 9H), 1.99 (d, J = 1.2 Hz, 3H), 2.25 (s, 6H), 3.14 (t, J = 9.0 Hz, 2H), 4.03 (t, J = 8.4 Hz, 2H), 4.21 (m, 1 H), 5.66 (d, J = 7.8 Hz, 1 H), 7.04 (s, 1H),7.05 (s, 1 H), 7.11-7.89 (m, 4H). | |
| 381 | 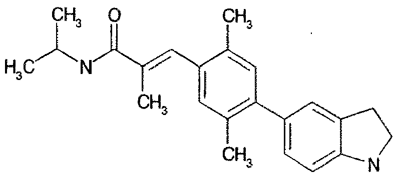 | 1H NMR (CDCl3) δ 1.25 (d, J = 6.6 Hz, 6H), 2.00 (d, J = 1.2 Hz, 3H), 2.25 (s, 3H), 2.27 (s, 3H), 3.08 (t, J = 8.4 Hz, 2H), 3.62 (t, J = 8.4 Hz, 2H), 4.23 (m, 1H), 5.66 (d, J = 7.5 Hz, 1 H), 6.69 (d, J = 8.1 Hz, 1 H), 6.99 (d, J = 8.1 Hz, 1H), 7.03 (s, 1H), 7.07 (s, 1 H), 7.10 (S, 1H), 7.41 (s, 1H). | |
| 382 | 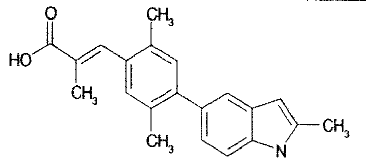 | 1H NMR (d6-DMSO) δ 1.93 (s, 3H), 2.23 (s, 6H), 2.40 (s, 3H), 6.13 (brs, 1 H), 6.94-6.98 (m, 2H), 7.11 (s, 1H), 7.17 (s, 1H), 7.28-7.33 (m, 2H), 7.66 (s, 1H). | |

**[Table 64]**

| | | | |
|---|---|---|---|
| 383 | 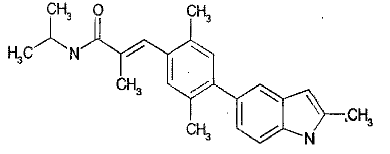 | | 163-165°C |
| 384 | 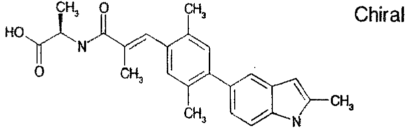 | | 125-128°C |
| 385 | 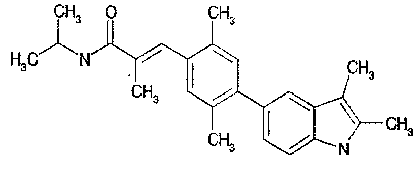 | 1 H NMR (CDCl3) δ 1.25 (d, J = 6.6 Hz, 6H), 2.02 (d, J = 1.2 Hz, 3H), 2.23 (s, 3H), 2.27 (s, 6H), 2.39 (s, 3H), 4.24 (m, 1 H), 5.67 (d, J = 7.5 Hz, 1H), 7.07 (s, 1H), 7.08 (dd, J = 1.8, 8.4 Hz, 1 H), 7.17 (s, 1 H), 7.28 (d, J = 8.4 Hz, 1H), 7.40 (brs, 1 H), 7.43 (s, 1 H). | |
| 386 | 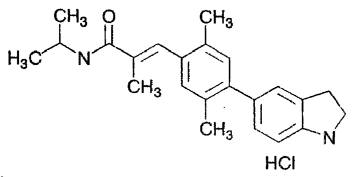 | | 227-230°C |
| 387 | 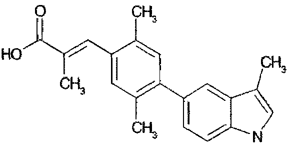 | | 177-180°C |
| 388 | 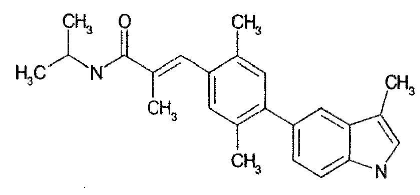 | 1H NMR (CDCl3) δ 1.25 (d, J = 6.6 Hz, 6H), 2.02 (d, J = 1.2 Hz, 3H), 2.28 (s, 6H), 2.34 (s, 3H), 4.22 (m, 1H), 5.68 (d, J = 7.2 Hz, 1H), 7.01 (s, 1H), 7.08 (s, 1H), 7.14-7.26 (m, 2H), 7.37 (d, J = 8.4 Hz, 1H), 7.44 (s, 1H), 7.51 (s, 1 H), 7.97 (s, 1 H). | |
| 389 | 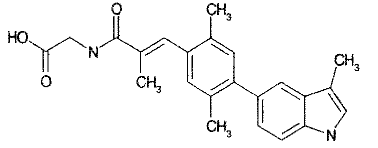 | | 200-203°C |

**[Table 65]**

| | | | |
|---|---|---|---|
| 390 | 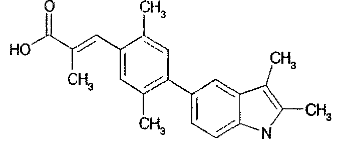 | | 167-170°C |
| 391 | 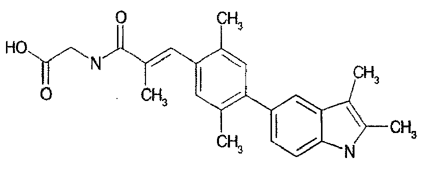 | 1H NMR (d6-DMSO) δ 1.92 (s, 3H), 2.14 (s, 3H), 2.16 (s, 3H), 2.25 (s, 3H), 2.32 (s, 3H), 3.85 (d, J = 6.0 Hz), 6.96 (dd, J = 1.8, 8.1 Hz, 1H), 7.12 (s, 1H), 7.13 (S, 1 H), 7.25 (d, J = 8.1 Hz, 1H), 7.28 (s, 1H), 7.38 (s, 1H), 10.70 (s, 1H), 12.55 (s, 1H). | |
| 392 | 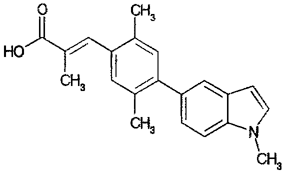 | | 219-221°C |
| 393 | 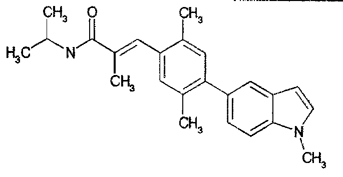 | | 151-153°C |
| 394 | 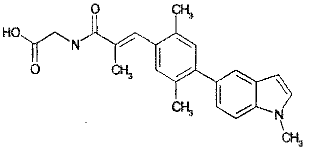 | | 172-174°C |
| 395 | 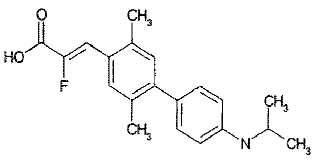 | 1 H NMR (d6-DMSO) δ 1.15 (d, J = 6.3Hz, 6H), 2.24 (s, 3H), 2.32 (s, 3H), 3.49 - 3.64 (m, 1 H), 6.61 (d, J = 8.7Hz, 2H), 7.07 (d, J = 35.7Hz, 1 H), 7.08 (s, 1 H), 7.08 (d, J = 8.7Hz, 2H), 7.57 (s, 1H) ppm | |
| 396 | 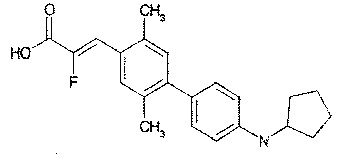 | 1 H NMR (CDCl3+CD3OD) δ 1.45-1.86 (m, 6H), 2.00-2.12 (m, 2H), 2.30 (s, 3H), 2.39 (s, 3H), 3.78-3.89 (m, 1H), 6.72 (d, J = 8.7 Hz, 2H), 7.09 (s, 1H), 7.17 (d, J = 8.7 Hz, 2H), 7.18 (d, J = 35 Hz, 1H), 7.72 (s, 1H) ppm | |

**[Table 66]**

| | | | |
|---|---|---|---|
| 397 | 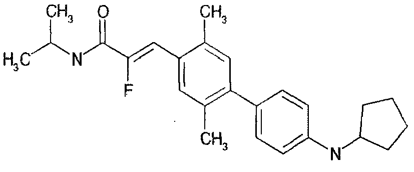 | 1 H NMR (COCl3) δ 1.26 (d, J = 6.3 Hz, 6H), 1.45-1.83 (m, 6H), 1.99-2.11 (m, 2H), 2.92 (s, 3H), 2.38 (s, 3H), 3.77-3.87 (m, 1 H), 4.16-4.29 (m, 1 H), 6.14-6.23 (m, 1 H), 6.65 (d, J = 8.4 Hz, 2H), 6.14 (s, 1H) ppm | |
| 398 | 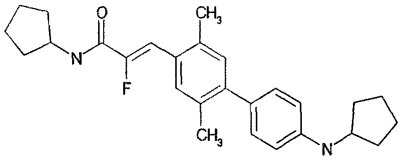 | 1 H NMR (CDCl3) δ 1.42 - 1.83 (m, 12H), 1.90 - 2.14 (m, 4H), 2.28 (s, 3H), 2.37 (s, 3H), 3.76-3.87 (m, 1H), 4.27 - 4.41 (m, 1 H), 6.26 - 6.34 (m, 1H), 6.69 (d, J = 8.4Hz, 2H), 7.04 - 7.21 (m, 4H), 7.60 (s, 1H) ppm | |
| 399 | 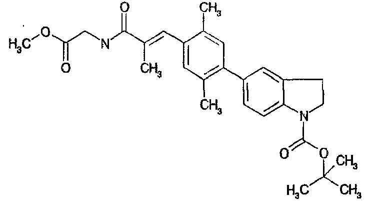 | 1 H NMR (COCl3) δ 1.59 (s, 9H), 2.06 (d, J = 1.2 Hz, 3H), 2.26 (s, 6H), 3.14 (t, J = 8.7 Hz, 2H), 3.81 (s, 3H), 4.03 (t, J = 8.7 Hz, 2H), 4.20 (d, J = 5.1 Hz, 2H), 6.41 (t, J = 5.1 Hz, 1H), 7.06-7.87 (m, 5H). | |
| 400 | 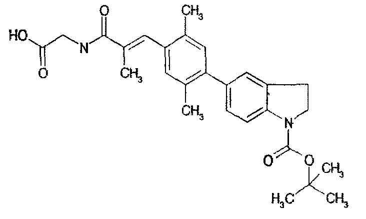 | 1 H NMR (COCl3) δ 1.58 (s, 9H), 2.06 (s, 3H), 2.26 (s, 6H), 3.14 (t, J = 8.7 Hz, 2H), 4.03 (t, J = 8.7 Hz, 2H), 4.25 (d, J = 5.4 Hz, 2H), 6.52 (t, J = 5.4 Hz, 1 H), 7.06-7.87 (m, 5H). | |
| 401 | 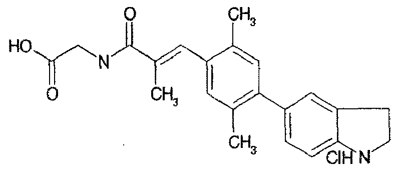 | | 218-225°C |
| 402 | 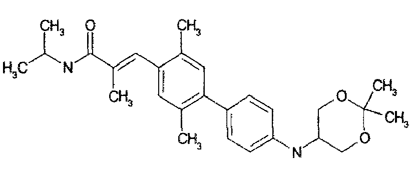 | 1H NMR (CDCl3) δ 1.25 (d, J = 6.9 Hz, 6H), 1.47 (s, 3H), 1.50 (s, 3H), 2.05 (s, 3H), 2.25 (s, 3H), 2.26 (s, 3H), 3.49 (brt, 1H), 3.81 (dd, J = 3.9, 12.0 Hz, 1H), 4.08-4.26 (m, 5H), 5.65 (d, J = 8.1 Hz, 1H), 6.68 (d, J = 8.4 Hz, 2H), 7.04 (s, 1H), 7.06 (s, 1H), 7.16 (d, J = 8.7 Hz, 2H), 7.41 (s, 1H). | |

**[Table 67]**

| | | | |
|---|---|---|---|
| 403 | 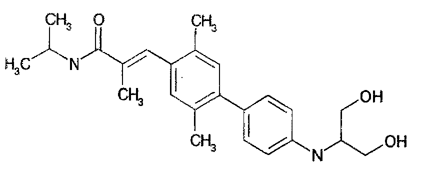 | 1H NMR (CDCl3) δ 1.25 (d, J = 7.2 Hz, 6H), 2.00 (d, J = 1.2 Hz, 3H), 2.24 (s, 3H), 2.26(s, 3H), 3.64 (m, 1H), 3.91 (d, J = 4.5 Hz, 4H), 4.23 (m, 1H), 5.69 (d, J = 7.8 Hz, 1H), 6.72 (d, J = 8.7 Hz, 2H), 7.04 (s, 1H), 7.06 (s, 1H), 7.16 (d, J = 8.7 Hz, 2H), 7.41 (s, 1H). | |
| 404 | 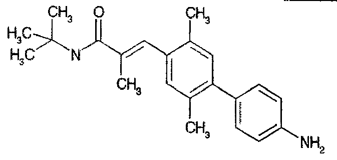 | | 124-126°C |
| 405 | 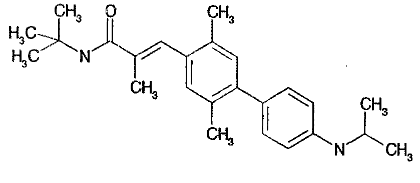 | 1H NMR (CDCl3) δ 1.25 (d, J = 6.3 Hz, 6H), 1.45 (s, 9H), 1.98 (d, J = 1.2 Hz, 3H), 2.25 (s, 3H), 2.27 (s, 3H), 3.67 (m, 1H), 5.68 (s, 1H), 6.63 (d, J = 8.4 Hz, 2H), 7.03 (s, 1 H), 7.07 (s, 1H), 7.15 (d, J = 8.4 Hz, 2H), 7.35 (s, 1H). | |
| 406 | 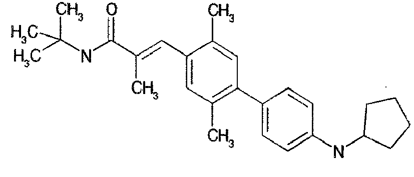 | 1 H NMR (CDCl3) δ 1.45(s, 9H), 1.45-2.13 (m, 10H), 1.98 (d, J = 1.5 Hz, 3H), 2.25 (s, 3H), 2.27 (s, 3H), 3.82 (m, 1H), 5.69 (s, 1H), 6.64-6.67 (m, 2H), 7.03 (s, 1H), 7.07 (s, 1 H), 7.13-7.27 (m, 2H), 7.36 (s, 1H). | |
| 407 | 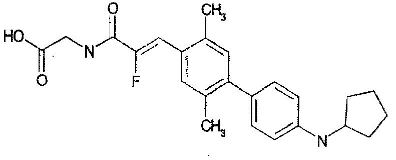 | 1 H NMR (CDCl3+CD3OD) δ 1.48 - 1.83 (m, 6H), 1.98 - 2.12 (m, 2H), 2.30 (s, 3H), 2.38 (s, 3H), 4.17 (d, J = 4.8Hz, 2H), 6.69 (d, J = 8.7Hz, 2H), 7.07-7.24 (m, 4H), 7.65 (s, 1H) ppm | |
| 408 | 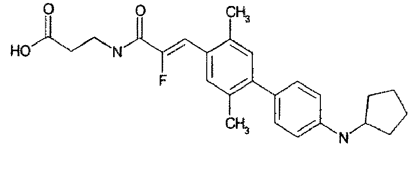 | 1 H NMR (CDCl3+CD3OD) δ 1.48 - 1.82 (m, 6H), 1.98 - 2.22 (m, 2H), 2.28 (s, 3H), 2.38 (s, 3H), 2.64 (t, J = 6.0Hz, 2H), 3.62 - 3.71 (m, 1 H), 3.77 - 3.87 (m, 1H), 6.74 (d, J = 8.4Hz, 2H), 7.04-7.23 (m, 4H), 7.62 (s, 1H) ppm | |

**[Table 68]**

| | | | |
|---|---|---|---|
| 409 | 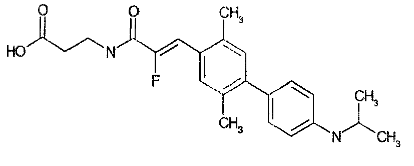 | 1 H NMR (CDCl3+CD3OD) δ 1.28 (d, J = 6.3Hz, 6H), 2.28 (s, 3H), 2.38 (s, 3H), 2.64 (t, J = 5.7Hz, 2H), 3.60-3.68 (m, 3H), 6.80 (d, J = 7.8Hz, 2H), 7.08 (s, 1 H), 7.12 (d, J = 38.7Hz, 1H), 7.20 (d, J = 7.2Hz, 1 H), 7.62 (s, 1 H) ppm | |
| 410 | 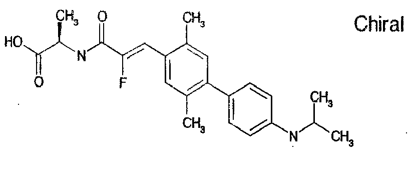 | 1 H NMR (CDCl3) δ 1.27 (d, J = 6.3Hz, 6H), 1.58 (d, J = 7.2Hz, 3H), 2.26 (s, 6H), 2.36 (s, 6H), 3.61 - 3.74 (m, 1H), 4.65 - 4.77 (m, 1H), 6.75 (d, J = 8.4Hz, 2H), 6.93-7.01 (m, 1H), 7.07 (s, 1H), 7.15 (d, J = 38.7Hz, 1 H), 7.17 (d, J = 8.4Hz, 2H), 7.62 (s, 1H) ppm | |
| 411 | 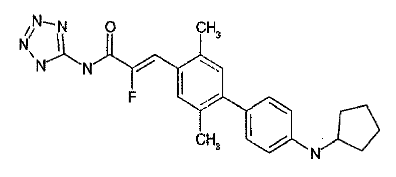 | 1 H NMR (d6-DMSO) δ 1.40-1.76 (m, 6H), 1.87 - 2.01 (m, 2H), 2.27 (s, 3H), 2.38 (s, 3H), 3.67 - 3.77 (m, 1H), 6.62 (d, J = 8.7Hz, 2H), 7.10 (d, J = 8.7Hz, 2H), 7.11 (s, 1H), 7.27 (d, J = 37.2 Hz, 1H), 7.61 (s, 1H) ppm | |
| 412 | 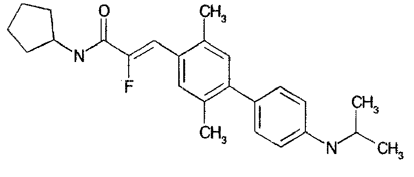 | 1H NMR (CDCl3) δ 1.25 (d, J = 6.6Hz, 6H), 1.43 - 1.81 (m, 6H), 2.02 - 2.16 (m, 2H), 2.29 (s, 3H), 2.38 (s, 3H), 3.60 - 3.73 (m, 1 H), 4.27 - 4.41 (m, 1H), 6.30 (d, J = 4.8Hz, 1 H), 6.63 (d, J = 8.7Hz, 1H), 7.09 (s, 1 H), 7.13 (d, J = 39.3Hz, 1H), 7.15 (d, J = 8.7Hz, 2H), 7.61 (s, 1H) ppm | |
| 413 | 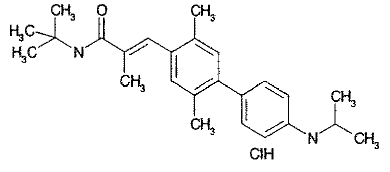 | | 235-236°C |
| 414 | 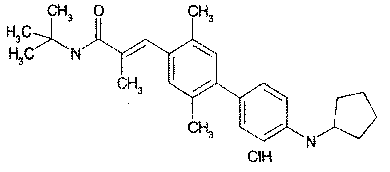 | | 227-229°C |

**[Table 69]**

| Compou nd No. | Structure | 1H-NMR | mp |
|---|---|---|---|
| 415 | 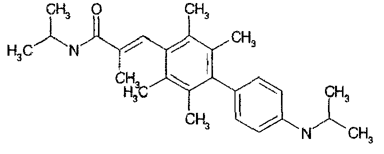 | | 192.8 |
| 416 | 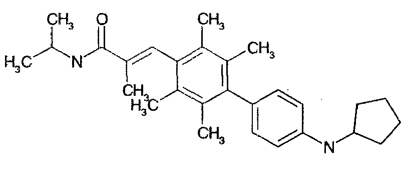 | 1H NMR(CDCl3) δ 1.25 (s, 3H), 1.27 (s, 3H), 1.49-1.82 (m, 6H), 1.70 (s, 3H), 1.96 (s, 6H), 2.01-2.09 (m, 2H), 2.12 (s, 6H), 3.79-3.87 (m, 1 H), 4.19-4.30 (m, 1 H), 5.66 (d, J = 7.8 Hz, 1H), 6.68 (d, J = 8.5 Hz, 2H), 6.90 (d, J = 7.8 Hz, 2H), 7.45 (s, 1 H) | |
| 417 | 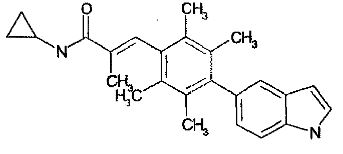 | 1H NMR(CDCl3) δ 0.60-0.65 (m, 2H), 0.84-0.91 (m, 2H), 1.71 (s, 3H), 1.93 (s, 6H), 2.13 (s, 6H), 2.86-2.92 (m, 1H), 5.99 (s, 1H), 6.56-6.57 (m, 1 H), 6.92-6.96 (m, 1 H), 7.36 (s, 1H), 7.43 (s, 1H), 7.46 (s, 1H), 8.24 (s, 1H) | |
| 418 | 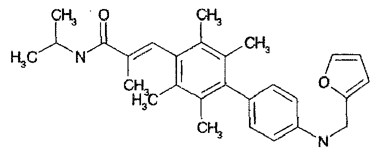 | | 143.4 |
| 419 | 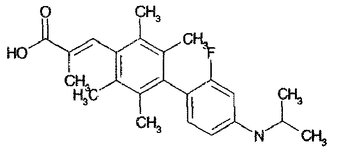 | 1H NMR(CDCl3) δ 1.26 (s, 3H), 1.28 (s, 3H), 1.73(s, 3H), 2.00 (s, 6H), 2.13 (s, 3H), 3.60-3.68 (m, 1H), 6.43 (t, J = 10.1 Hz, 2H), 6.85 (t, J = 8.2 Hz, 1 H), 7.90 (s, 1 H) | |
| 420 | 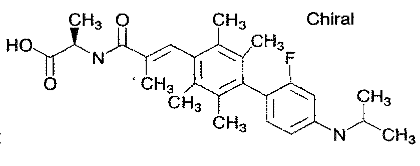 | 1H NMR(CDCl3) δ 1.27 (s, 3H), 1.29 (s, 3H), 1.59 (d, J = 7.0 Hz, 3H), 1.98 (s, 6H), 2.11 (s, 6H), 3.60-3.68 (m, 1 H), 4.68-4.75 (m, 1 H), 6.38-6.51 (m, 3H), 6.85 (t, J = 8.1 Hz, 1 H), 7.59 (d, J = 10.8 Hz, 1H) | |
| 421 | 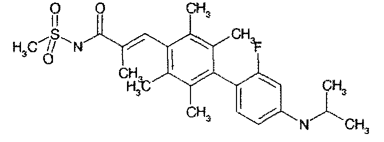 | | 238.4 |

**[Table 70]**

| | | | |
|---|---|---|---|
| 422 | 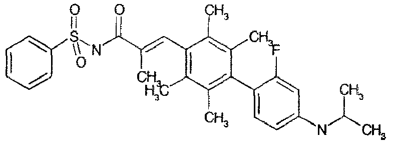 | | 203.9 |
| 423 | 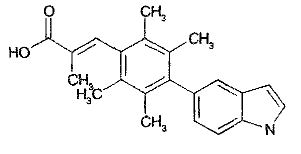 | 1H NMR(CDCl3) δ 1.77 (d, J = 1.2 Hz, 3H), 1.95 (s, 6H), 2.15 (s, 6H), 6.57-6.58 (m, 1 H), 6.95 (dd, J = 1.4 Hz, 6.9 Hz, 1H), 7.25-7.27 (m, 1H), 7.37 (s, 1H), 7.45 (d, J = 8.2 Hz, 1H), 7.95 (s, 1H), 8.21 (s, 1H) | |
| 424 | 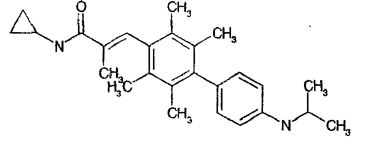 | | 201.8 |
| 425 | 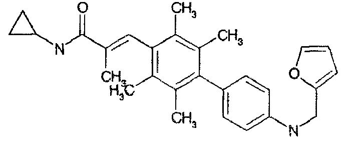 | | 173.6 |
| 426 | 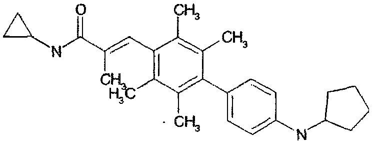 | | 206.3 |
| 427 | 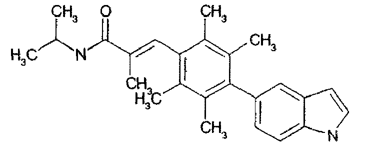 | 1H NMR(CDCl3) δ 1.26 (s, 3H), 1.28 (s, 3H), 1.72 (s, 3H), 1.94 (s, 6H), 2.14 (s, 6H), 4.20-4.31 (m, 1H), 5.68 (d, J = 8.0 Hz, 1H), 6.56-6.57 (m, 1 H), 6.92-6.96 (m, 1 H), 7.37 (s, 1H), 7.43-7.48 (m, 2H), 8.23 (s, 1H) | |
| 428 | 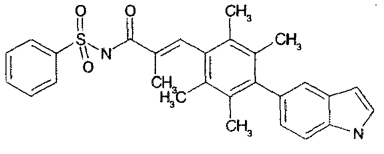 | 1H NMR (CDCl3) δ 1.71 (s, 3H), 1.91 (s, 6H), 2.06 (s, 6H), 6.56 (s, 1H), 6.88-6.93 (m, 1H), 7.34 (s, 1H), 7.44 (d, J = 8.1 Hz, 1H), 7.58-7.72 (m, 4H), 8.19-8.21 (m, 3H), 8.43 (s, 1 H) | |

**[Table 71]**

| | | | |
|---|---|---|---|
| 429 | 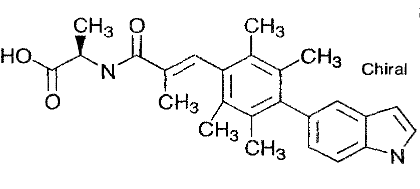 | 1H NMR(CDCl3) δ 1.61 (d, J = 7.2 Hz, 3H), 1.78 (s, 3H), 1.94 (s, 6H), 2.13 (s, 6H), 4.71-4.76 (m, 1H), 6.40 (d, J = 6.1 Hz, 1H), 6.57 (s, 1 H), 6.94 (d, J = 8.0 Hz, 1 H), 7.36 (s, 1H), 7.45 (d, J = 8.3 Hz, 1H), 7.64 (s, 1H), 8.21 (s, 1 H) | |
| 430 | 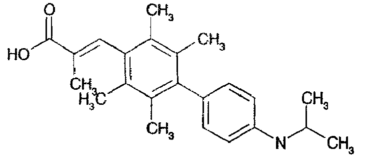 | 1H NMR(DMSO) δ 1.16 (d, J = 6.3 Hz, 6H), 1.56 (s, 3H), 1.89 (s, 6H), 2.04 (s, 6H), 3.51-3.59 (m, 1H), 5.40 (bs, 1H), 6.60 (d, J = 8.7 Hz, 2H), 6.76 (t, J = 9.0 Hz, 2H), 7.59 (s, 1 H), 12.57 (bs, 1H), | |
| 431 | 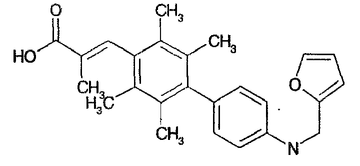 | 1H NMR(CDCl3) δ 1.74 (d, J = 1.2 Hz, 3H), 1.95 (s, 6H), 2.13 (s, 6H), 4.39 (s, 2H), 6.32-6.36 (m, 2H), 6.79 (d, J = 8.1 Hz, 2H), 6.95 (d, J = 8.7 Hz, 2H), 7.40-7.41 (m, 1H), 7.91 (s, 1H) | |
| 432 | 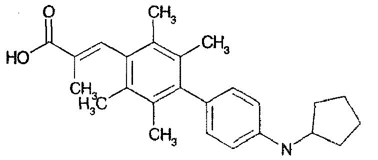 | 1H NMR(CDCl3) δ 1.40-2.09 (m, 8H), 1.74 (s, 3H), 1.97 (s, 6H), 2.13 (s, 6H), 3.80-3.88 (m, 1H), 6.67 (d, J = 8.4 Hz, 2H), 6.90 (d, J = 8.6 Hz, 2H), 7.91 (s, 1 H) | |
| 433 | 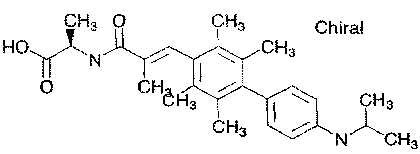 | | 132.8 |
| 434 | 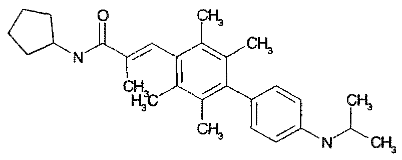 | | 186.3 |
| 435 | 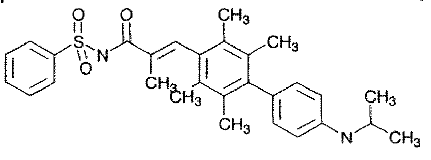 | | 217.2 |

**[Table 72]**

| | | | |
|---|---|---|---|
| 436 | 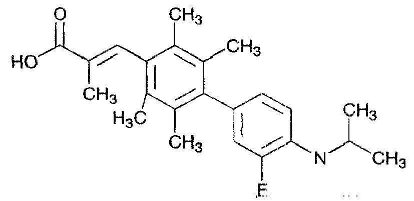 | | 231.2 |
| 437 | 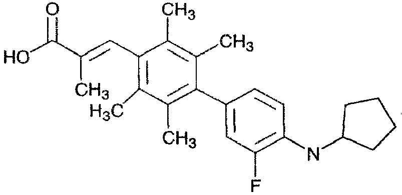 | | 267.2 |
| 438 | 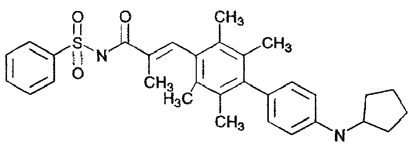 | | 233 |
| 439 | 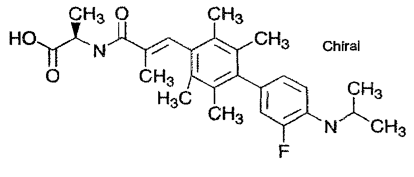 | 1 H NMR(CDCl3) δ 1.31 (d, J = 6.3 Hz, 6H), 1.60 (d, J = 7.2 Hz, 3H), 1.75 (d, J = 1.2 Hz, 3H), 1.94 (s, 6H), 2.11 (s, 6H), 3.68-3.73 (m, 1H), 4.71-4.76 (m, 1 H), 6.42 (d, J = 6.3 Hz, 1H), 6.73-6.78 (m, 2H), 7.59 (s, 1 H) | |
| 440 | 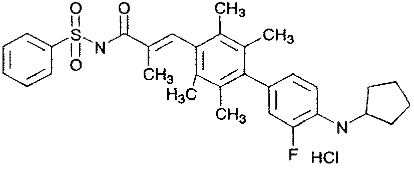 | | 193.7 |
| 441 | 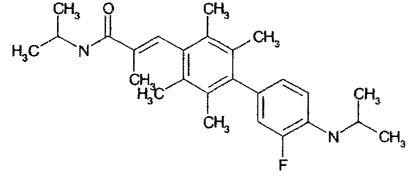 | | 235.6 |

**[Table 73]**

| | | | |
|---|---|---|---|
| 442 | 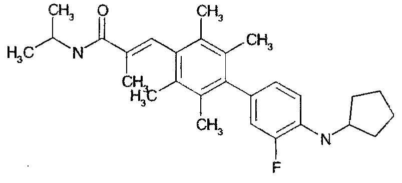 | | 176.1 |
| 443 | 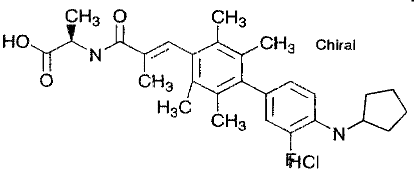 | 1H NMR(CDCl3) δ 1.59 (d, J = 7.2 Hz, 3H), 1.74 (s, 3H), 1.84 (s, 6H), 2.09 (s, 6H), 4.00 (t, J = 6.3 Hz, 1 H), 4.77 (t, J = 6.9 Hz, 1H), 6.51 (d, J = 6.6 Hz, 1H), 6.94-7.03 (m, 2H), 7.55 (d, J = 9.0 Hz, 1H), 7.97 (s, 1H) | |
| 444 | 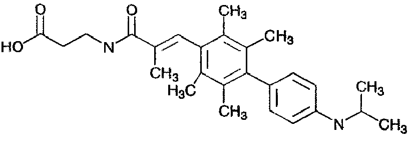 | 1H NMR(CDCl3) δ 1.27 (s, 3H), 1.29 (s, 3H), 1.70 (d, J = 1.1 Hz, 3H), 1.93 (s, 6H), 2.09 (s, 6H), 2.73 (t, J = 6.1 Hz, 2H), 3.64-3.70 (m, 3H), 6.59 (t, J = 6.0 Hz, 1H), 6.72 (d, J = 8.5 Hz, 2H), 6.90 (d, J = 8.9 Hz, 2H), 7.49 (s, 1 H) | |
| 445 | 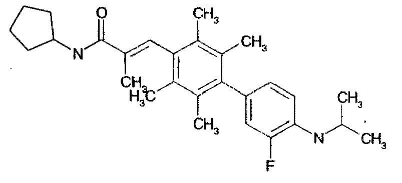 | | 223.5 |
| 446 | 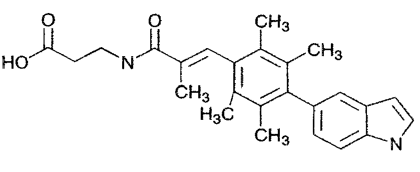 | 1H NMR(DMSO) δ 1.58 (s, 3H), 1.86 (s, 6H), 2.06 (s, 6H), 3.28-3.44 (m, 4H), 6.41 (s, 1 H), 6.75-6.80 (m, 1H), 7.20 (d, J = 6.7 Hz, 1H), 7.27 (s, 1H), 7.35-7.38 (m, 1H), 7.44 (d, J = 8.3 Hz, 1H), 8.09 (t, J = 5.5 Hz, 1 H), 11.1 (s, 1H) | |
| 447 | 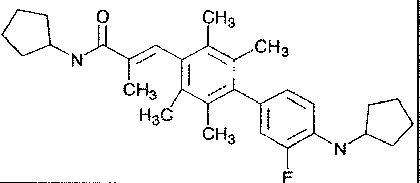 | 1 H NMR(CDCl3) δ 1.44-1.80 (m, 14H), 1.69(s,3H), 1.94(s,3H), 2.05-2.08 (m, 4H), 2.11 (s, 6H), 3.83-3.85 (m, 1H), 4.32-4.39 (m, 1H), 5.79 (d, J = 7.0 Hz), 6.74-6.78 (m, 2H), 6.88-6.90 (m, 1H), 7.45 (d, J = 3.9 Hz, 1H) | |

**[Table 74]**

| | | | |
|---|---|---|---|
| 448 | 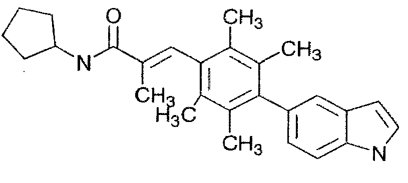 | 1H NMR(CDCl3) δ 1.43-1.54 (m, 2H), 1.64-1.77 (m, 4H), 1.73 (s, 3H), 1.94 (s, 6H), 2.07-2.11 (m, 2H), 2.14 (s, 6H), 4.33-4.40 (m, 1H), 5.81 (d, J = 7.0 Hz, 1 H), 6.56-6.58 (m, 1 H), 6.93-6.97 (m, 1H), 7.37 (s, 1 H), 7.43-7.48 (m, 2H), 8.23 (s, 1H) | |
| 449 | 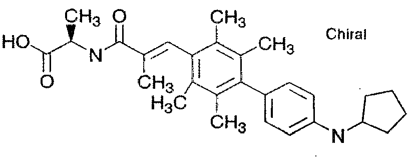 | 1H NMR(CDCl3) δ 1.59 (d, J = 7.2 Hz, 3H), 1.65-2.07 (m, 8H), 1.75 (s, 3H), 1.95 (s, 6H), 2.11 (s, 6H), 3.82-3.86 (m, 1H), 4.68-4.75 (m, 1H), 6.41 (d, J = 6.4 Hz, 1H), 6.76 (d, J = 8.4 Hz, 2H), 6.91 (d, J = 8.4 Hz, 2H), 7.60 (s, 1 H) | |
| 450 | 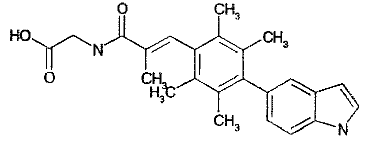 | | 196.5 |
| 451 | 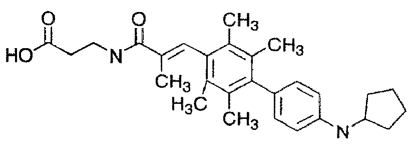 | 1H NMR(CDCl3) δ 1.51-2.07 (m, 8H), 1.71 (s, 3H), 1.95 (s, 6H), 2.10 (s, 6H), 2.74 (t, J = 5.8 Hz, 2H), 3.67-3.73 (m, 2H), 3.79-3.88 (m, 1 H), 6.57 (t, J = 5.6 Hz, 1 H), 6.68 (d, J = 8.7 Hz, 2H), 6.89 (d, J = 8.5 Hz, 2H), 7.50 (s, 1H) | |
| 452 | 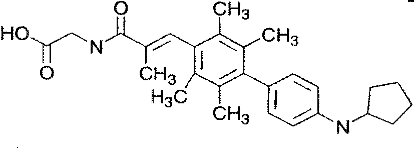 | 1H NMR(CDCl3) δ 1.54-2.08 (m, 8H), 1.77 (s, 3H), 1.96 (s, 6H), 2.12 (s, 6H), 3.83-3.89 (m, 1 H), 4.25 (m, 2H), 6.56 (m, 1H), 6.70 (d, J = 7.8 Hz, 2H), 6.91 (d, J = 7.6 Hz, 2H), 7.59 (s, 1H) | |
| 453 | 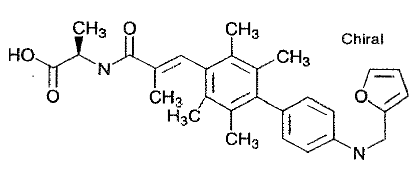 | 1H NMR(CDCl3) δ 1.60 (d, J = 7.2 Hz, 3H), 1.75 (d, J = 0.9 Hz, 3H), 1.94 (s, 6H), 2.11 (s, 6H), 4.38 (s, 2H), 4.71-4.76 (m, 1 H), 6.30-6.37 (m ,2H), 6.41 (d, J = 6.6 Hz, 1H), 6.79 (d, J = 8.7 Hz, 2H), 6.94 (d, J = 8.4 Hz, 2H), 7.41 (d, 0.9 Hz, 1H), 7.61 (s, 1H) | |
| 454 | 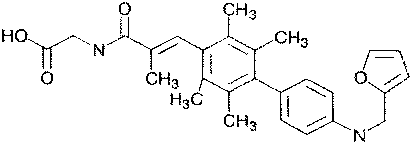 | | 231.8 |

**[Table 75]**

| | | | |
|---|---|---|---|
| 455 | 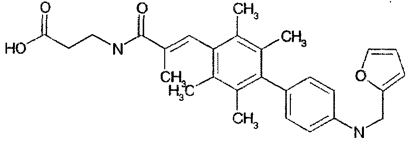 | | 151.7 |
| 456 | 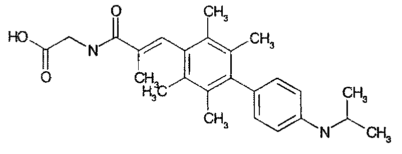 | | 208 |
| 457 | 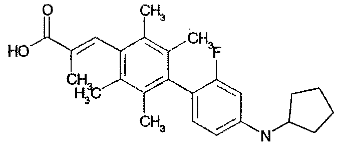 | 1H NMR(CDCl3) δ 1.52-2.16 (m, 8H), 1.73 (s, 3H), 1.99 (s, 6H), 2.13 (s, 6H), 3.76-3.84 (m, 1H), 6.42-6.49 (m, 2H), 6.5 (dd, J = 7.9 Hz, 8.5 Hz, 1 H), 7.90 (s, 1 H) | |
| 458 | 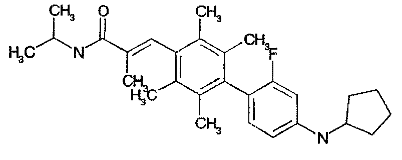 | 1H NMR(CDCl3) δ 1.26 (d, J = 6.6 Hz, 6H), 1.60-2.15 (m, 8H), 1.69 (s, 3H), 1.98 (s, 6H), 2.12 (s, 6H), 3.75-3.84 (m, 1 H), 4.18-4.27 (m, 1 H), 5.65 (d, J = 7.8 Hz, 1 H), 6.42-6.50 (m, 2H), 6.85 (t, J = 8.4 Hz, 1 H), 7.44 (d, J = 18.6 Hz, 1H) | |
| 459 | 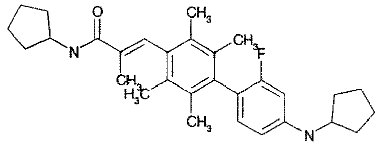 | 1H NMR(CDCl3) δ 1.46-2.15 (m, 16H), 1.69 (s, 3H), 1.98 (s, 6H), 2.12 (s, 6H), 3.75-3.83 (m, 1 H), 4.32-4.39 (m, 1H), 5.79 (d, J = 7.6 Hz), 6.44-6.52 (m, 2H), 6.86 (dd, J = 6.9 Hz, 8.5 Hz, 1 H), 7.45 (d, J = 19.4 Hz, 1H) | |
| 460 | 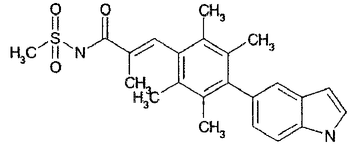 | | 258.6 |
| 461 | 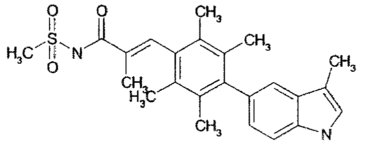 | 1 H NMR(CDCl3) δ 1.78 (s, 3H), 1.95 (s, 6H), 2.12 (s, 6H), 2.32-2.33 (m, 3H), 3.45 (s, 3H), 6.92 (dt, J = 8.1 Hz, 1.8 Hz, 1 H), 7.02 (s, 1H), 7.28 (s, 1H), 7.40 (d, J = 8.4 Hz, 1H), 7.65 (s, 1H), 7.94 (s, 1H), 8.24 (s, 1H) | |

**[Table 76]**

| | | | |
|---|---|---|---|
| 462 | 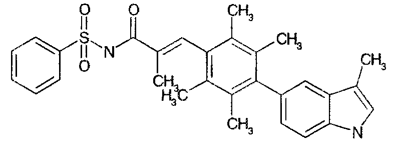 | 1 H NMR(CDCl3) δ 1.71 (s, 3H), 1.93 (s, 6H), 2.07 (s, 6H), 2.31 (s, 3H), 6.89-6.93 (m, 1H), 7.02 (s, 1 H), 7.27 (s, 1 H), 7.38 (d, J = 8.2 Hz, 1 H), 7.58-7.64 (m, 3H), 7.67-7.72 (m, 1H), 7.93 (m, 1H), 8.19-8.22 (m, 2H), 8.38 (s, 1H) | |
| 463 | 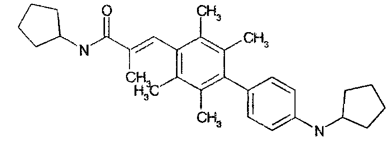 | | 201.8 |
| 464 | 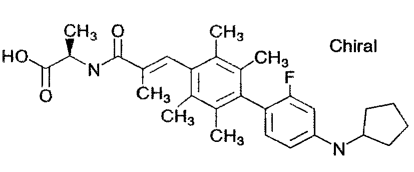 | 1H NMR(CDCl3) δ 1.51-2.15 (m, 8H), 1.59 (d, J = 7.2 Hz, 3H), 1.75 (s, 3H), 1.98 (s, 6H), 2.11 (s, 6H), 3.76-3.84 (m, 1 H), 4.68-4.77 (m, 1H), 6.38-6.48 (m, 3H), 6.84 (t, J =8.1 Hz, 1H), 7.59 (d, J = 10.5 Hz, 1H) | |
| 465 | 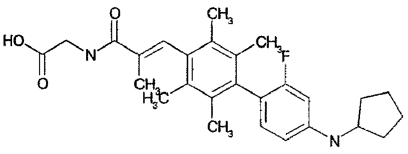 | 1H NMR(CDCl3) δ 1.50-2.07 (m, 8H), 1.76 (s, 3H), 1.98 (s, 6H), 2.11 (s, 6H), 3.75-3.83 (m, 1 H), 4.26 (d, J = 5.0 Hz, 2H), 6.37-6.45 (m, 2H), 6.83 (t, J = 8.2 Hz, 1H), 7.58 (d, J = 9.9 Hz) | |
| 466 | 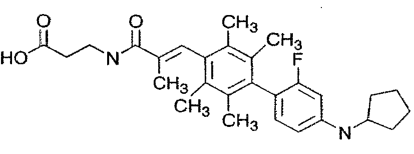 | 1H NMR(CDCl3) δ 1.51-2.14 (m, 8H), 1.70 (s, 3H), 1.97 (s, 6H), 2.10 (s, 6H), 2.74 (t, J = 5.8 Hz, 2H), 3.67-3.73 (m, 2H), 3.78-3.82 (m, 1 H), 6.40-6.47 (m, 2H), 6.56.. 6.60 (m, 1H), 6.83 (t, J = 8.4 Hz, 1H), 7.49 (d, J = 13.4 Hz, 1H) | |
| 467 | 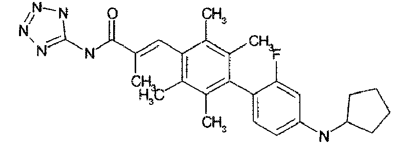 | 1H NMR(DMSO) δ 1.46-2.16 (m, 8H), 1.68 (s, 3H), 1.92 (s, 6H), 2.09 (m, 6H), 3.70-3.72 (m, 1 H), 5.96-5.98 (m, 1 H), 6.37-6.48 (m, 2H), 6.72-6.81 (m, 1H), 7.64 (s, 1 H), 12.10 (brs, 1H) | |
| 468 | 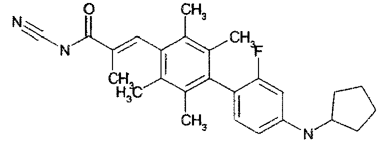 | | 241.6 |

**[Table 77]**

| | | | |
|---|---|---|---|
| 469 | 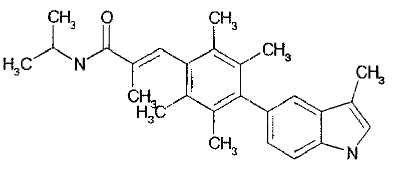 | 1H NMR(CDCl3) δ 1.27 (d, J = 6.6 Hz, 6H), 1.72 (s, 3H), 1.95 (s, 6H), 2.14 (s, 6H), 2.31-2.33 (m, 3H), 4.19-4.31 (m, 1H), 5.67 (d, J =8.1 Hz, 1H), 6.92-6.96 (m, 1H), 7.01 (s, 1H), 7.29 (s, 1H), 7.38 (d, J = 8.4 Hz, 1 H), 7.47 (bs, 1 H), 7.93 (bs, 1 H) | |
| 470 | 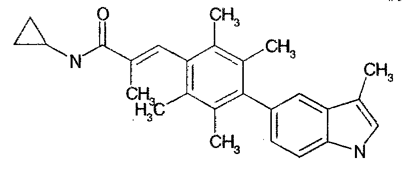 | 1H NMR(CDCl3) δ 0.60-0.65 (m, 2H), 0.84-0.90 (m, 2H), 1.71 (s, 3H), 1.94 (s,6H), 2.13 (s, 6H), 2.32 (s, 3H), 2.84-2.91 (m, 1 H), 5.99 (bs, 1 H), 6.92-6.95 (m, 1 H), 7.01 (s, 1 H), 7.29 (s, 1H), 7.38 (d, J = 8.4 Hz, 1 H), 7.46 (bs, 1 H), 7.94 (bs, 1H) | |
| 471 | 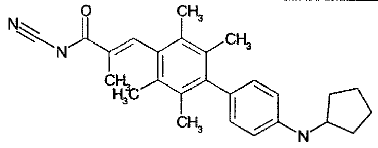 | | 242.4 |
| 472 | 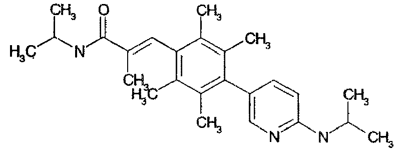 | | 227.4 |
| 473 | 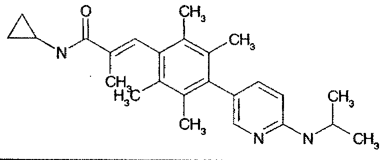 | | 220.6 |
| 474 | 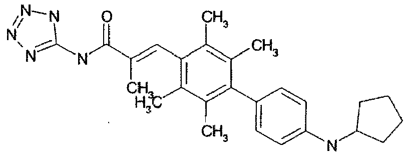 | | 294.5 |
| 475 | 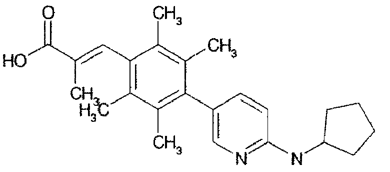 | | 270 |

**[Table 78]**

| | | | |
|---|---|---|---|
| 476 | 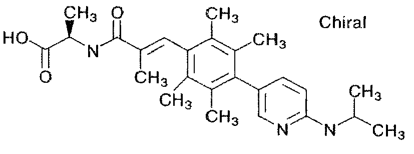 | | 223 |
| 477 | 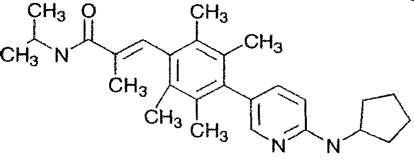 | | 201.5 |
| 478 | 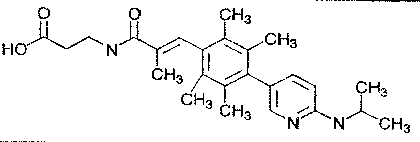 | | 202.5 |
| 479 | 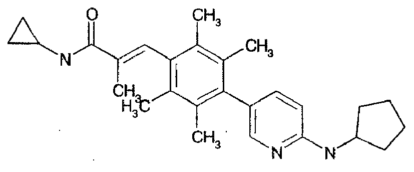 | | 197.6 |
| 480 | 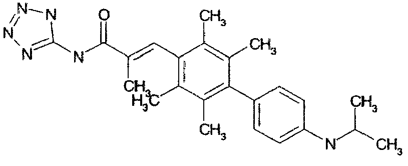 | | 296.3 |
| 481 | 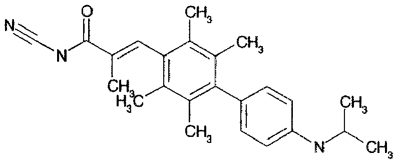 | | 289.2 |
| 482 | 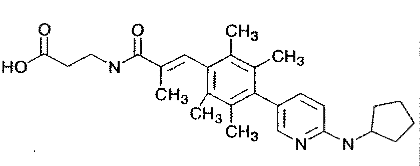 | 1H NMR(DMSO) δ 1.43-1.95 (m, 8H), 1.56 (s, 3H), 1.91 (s, 6H), 2.05 (s, 6H), 3.34-3.43 (m, 4H), 4.12-4.14 (m, 1H), 6.51-6.54 (m, 2H), 7.06-7.13 (m, 1H), 7.24 (s, 1 H), 7.66 (dd, J = 2.4 Hz, 7.8 Hz, 1 H), 8.08 (bs, 1H), 12.25 (bs, 1H) | |

**[Table 79]**

| Compou nd No. | Structure | 1HNMR | mp |
|---|---|---|---|
| 483 | 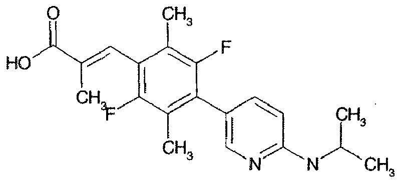 | 1H NMR (CDCl3) δ 1.32 (s, 3H), 1.34 (s, 3H), 1.86 (m, 3H), 2.12 (d, J=2.4Hz, 3H), 2.16 (d, J=2.4Hz, 3H), 3.83 (m, 1H), 6.54 (d, J=8.7Hz, 1 H), 7.44 (m,1H), 7.53(s, 1H), 7.91 (s, 1H)ppm | |
| 484 | 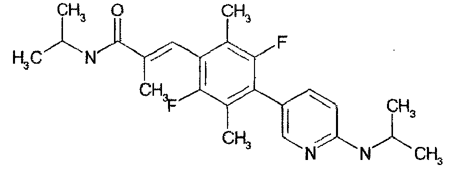 | | 162.4 °C |
| 485 | 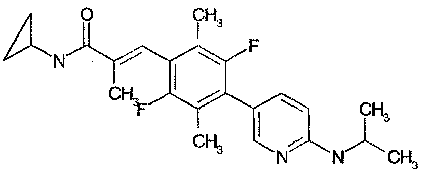 | | 178.1 °C |
| 486 | 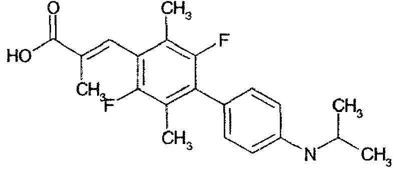 | 1H NMR (CDCl3) δ 1.25 (s, 3H), 1.27 (s, 3H), 1.88 (m, 3H), 2.10 (d, J=3.0Hz, 3H), 2.16 (d, J=3.0Hz, 3H), 3.68 (m, 1 H), 6.68 (d, J=8.7Hz, 2H), 7.07 (d, J = 8.7 Hz, 2H), 7.62 (s, 1H)ppm | |
| 487 | 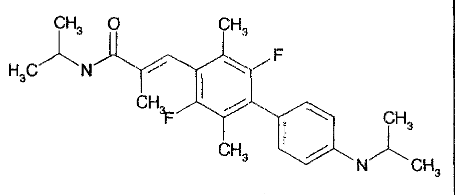 | 1 H NMR (CDCl3) δ 1.24 (s, 6H), 1.27 (s, 6H), 1.88(m, 3), 2.08 (d, J=3.0Hz, 3H), 2.13(d, J=3.0Hz, 3H), 3.67 (m, 1 H), 4.22(m, 1 H), 5.70(d, J=8.4Hz, 1 H), 6.70 (d, J=7.8Hz, 2H), 6.75(s, 1 H), 7.07 (d, J = 7.8Hz, 2H) ppm | |
| 488 | 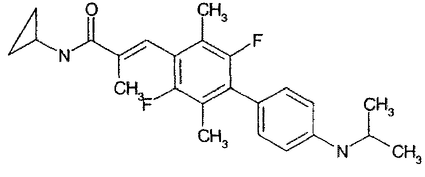 | 1 H NMR (CDCl3) δ 0.61 (m, 2H), 0.86(m, 2H), 1.25(s, 3H), 1.27(s, 3H), 1.82 (m, 3H), 2.05 (d, J=3.0Hz, 3H), 2.12 (d, J=3.0Hz, 3H), 2.86(m, 1H), 3.70(m, 1H), 6.02(s, 1H), 6.69 (d, J=8.4Hz, 2H), 7.02 (d, J = 7.8 Hz, 2H), 7.10 (s, 1 H)ppm | |

**[Table 80]**

| | | | |
|---|---|---|---|
| 489 | 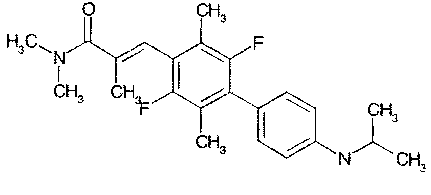 | 1 H NMR (CDCl3) δ 1.25 (s, 3H), 1.27 (s, 3H), 1.88(m, 3), 2.08 (d, J=3.0Hz, 3H), 2.13(d, J=3.0Hz, 3H), 3.67 (m, 1 H), 3.99(s, 6H), 5.70(d, J=8.4Hz, 1 H), 6.70 (d, J=7.8Hz, 2H), 6.75(s, 1H), 7.07 (d, J = 7.8Hz, 2H) ppm | |
| 490 | 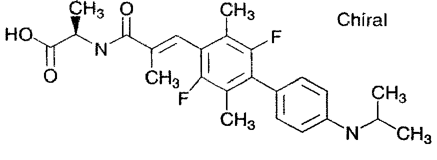 | 1H NMR (DSMO) δ 1.20(s, 3H), 1.22(s, 3H), 1.37(d, J=7.5Hz, 3H), 1.74 (s, 3H), 2.03(d, J=2.7Hz, 3H), 2.11 (d, J=2.7Hz, 3H), 3.65(m, 1H), 4.35(m, 1H), 6.95(b, 2H), 7.09 (s, 1H),7.18(b, 2H), 8.30(d, J=7.5Hz, 1 H)ppm | |
| 491 | 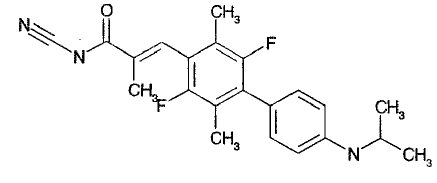 | 1H NMR (CDCl3) δ 1.26(s, 3H), 1.28(s, 3H), 1.90 (m, 3H), 2.09(d, J=2.7Hz, 3H), 2.13(d, J=2.7Hz, 3H), 3.67(m, 1 H), 6.72(d, J=8.7Hz,2H), 7.06 (d, J = 8.7Hz,2H), 7.32 (s, 1 H)ppm | |
| 492 | 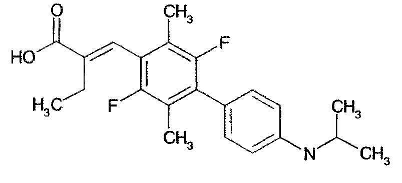 | 1H NMR (CDCl3) δ 1.06(t,J=7.5Hz, 3H), 1.27 (s, 3H), 1.29 (s, 3H), 2.09 (d, J=2.7Hz, 3H), 2.14(d, J=2.7Hz, 3H), 2.27(q, J=7.5Hz, 2H), 3.68 (m, 1 H), 6.75 (d, J=7.2Hz, 2H), 7.09 (d, J = 7.2Hz, 2H), 7.50 (s, 1H)ppm | |
| 493 | 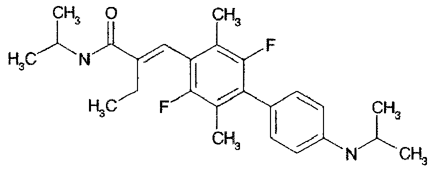 | 1 H NMR (CDCl3) δ 1.01 (t,J=7.2Hz, 3H), 1.24 (s, 6H), 1.27 (s, 6H), 2.08 (d, J=3.0Hz, 3H), 2.13(d, J=3.0Hz, 3H), 2.24(q, J=7.2Hz, 2H), 3.67 (m, 1 H), 4.22(m, 1H), 5.70(d, J=8.4Hz, 1 H), 6.70 (d, J=7.8Hz, 2H), 6.75(s, 1H), 7.07 (d, J = 7.8Hz, 2H) ppm | |
| 494 | 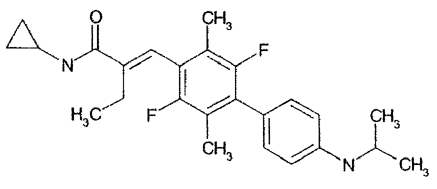 | 1H NMR (CDCl3) δ 0.61 (m, 2H), 0.86(m, 2H), 1.24 (s, 6H), 1.27 (s, 6H), 2.08 (d, J=3.0Hz, 3H), 2.13(d, J=3.0Hz, 3H), 2.24(q, J=7.2Hz, 2H), 3.67 (m, 1H), 4.22(m, 1H), 5.70(d, J=8.4Hz, 1 H), 6.71 (d, J=8.1 Hz, 2H), 6.77(s, 1 H), 7.08 (d, J = 8.2Hz, 2H) ppm | |

**[Table 81]**

| | | | |
|---|---|---|---|
| 495 | 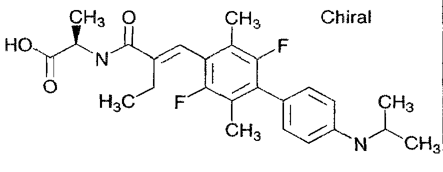 | 1 H NMR (CDCl3) δ 1.25 (s, 3H), 1.27(m, 3H), 1.28 (s, 3H), 1.58(d, J=7.2Hz, 3H), 2.07(m, 3H), 2.11 (m, 3H), 2.28(m, 2H), 3.68 (m, 1 H), 3.75(m, 1 H), 6.42(m, 1 H), 6.75 (d, J=8.1 Hz, 2H), 6.93(s, 1 H), 7.07 (d, J = 8.1 Hz, 2H) ppm | |
| 496 | 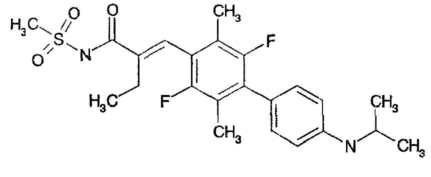 | 1H NMR (CDCl3) δ 1.04(t,J=7.5Hz, 3H), 1.25 (s, 3H), 1.27 (s, 3H), 2.09 (d, J=2.7Hz, 3H), 2.13(d, J=2.7Hz, 3H), 2.29(q, J=7.5Hz, 2H), 3.43(s, 3H), 3.68 (m, 1H), 6.68 (d, J=8.4Hz, 2H), 7.05(s, 1 H), 7.06 (d, J = 8.4Hz, 2H) ppm | |
| 497 | 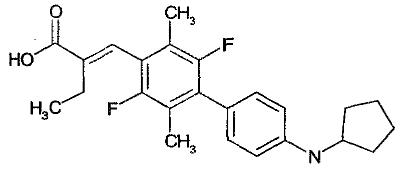 | 1 H NMR (CDCl3) δ 1.06(t,J=7.5Hz, 3H), 1.50-1.79(m, 6H), 2.05 (m, 2H), 2.10(d, J=2.7Hz, 3H), 2.14(d, J=2.7Hz, 3H), 2.28(q, J=7.5Hz, 2H), 3.83(m, 1H), 6.68(d, J=8.4Hz, 2H), 7.07(d, J=8.4Hz, 2H), 7.50(s, 1H)ppm | |
| 498 | 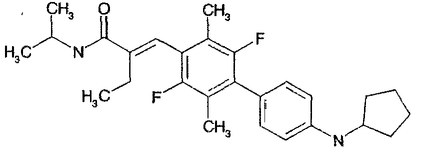 | 1H NMR (CDCl3) δ 1.01 (t,J=7.5Hz, 3H), 1.25(s, 3H),1.27(s, 3H), 1.56-1.78(m, 6H), 2.04 (m, 2H), 2.07(d, J=2.7Hz, 3H), 2.12(d, J=2.7Hz, 3H), 2.24(q, J=7.5Hz, 2H), 3.83(m, 1 H), 4.23(m, 1H), 6.75(s, 1 H), 6.83(b, 2H), 7.09(d, J=8.7Hz, 2H)ppm | |
| 499 | 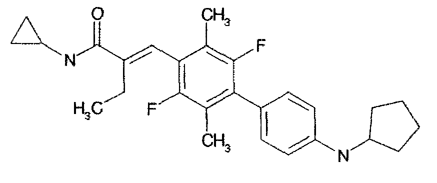 | 1 H NMR (CDCl3) δ 0.60(m, 2H), 0.85(m, 2H), 1.00(t,J=7.5Hz, 3H), 1.26-1.81 (m, 6H), 2.04 (m, 2H), 2.07(d, J=2.4Hz, 3H), 2.11 (d, J=2.4Hz, 3H), 2.25(q, J=7.5Hz, 2H), 2.86(m, 1 H), 3.82(m, 1H), 6.00(s, 1 H), 6.72(d, J=9.0Hz, 2H), 7.07(d, J=9.0Hz, 2H)ppm | |

**[Table 82]**

| | | | |
|---|---|---|---|
| 500 | 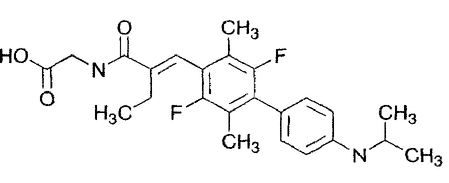 | 1H NMR (DMSO-d6) δ 0.91 (t, J = 8.4Hz, 3H), 1.16 (d, J = 6.3Hz, 6H), 2.04 (d, J = 2.4Hz, 3H), 2.10 (d, J = 2.1 Hz, 3H), 2.14 (m, 2H), 3.56 (m, 1 H), 3.85 (s, 2H), 5.62 (s, 1H), 6.61 (d, J = 8.4Hz, 2H), 6.84 (s, 1H), 7.00 (d, J = 8.4Hz, 2H), 8.48 (m, 1 H) ppm | |
| 501 | 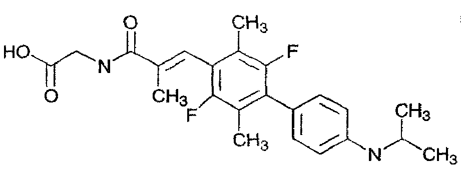 | 1H NMR (DMSO-d6) δ 1.16 (d, J = 5.4Hz, 6H), 1.74 (s, 3H), 2.04 (d, J = 2.4Hz, 3H), 2.10 (d, J = 2.1Hz, 3H), 3.57 (m, 1H), 3.85 (d, J = 5.7Hz, 2H), 5.64 (m, 1H), 6.63 (d, J = 8.4Hz, 2H), 7.00 (d, J = 8.7Hz, 2H), 7.10 (s, 1H), 8.45 (s, 1 H) ppm | |
| 502 | 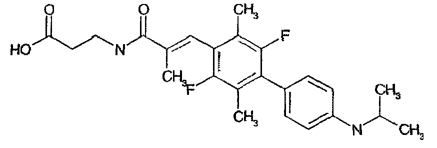 | | 103-104°C |
| 503 | 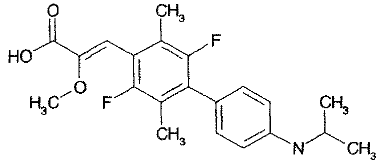 | 1H NMR (CDCl3) δ 1.26 (d, J = 6.0 Hz, 6H), 2.10 (d, J = 2.7 Hz, 3H), 2.20 (d, J = 2.7 Hz, 3H), 3.68 (m, 1 H), 3.69 (s, 3H), 6.68 (d, J = 8.7 Hz, 2H), 7.04-7.08 (m, 3H). | |
| 504 | 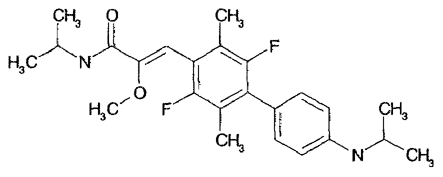 | | 149-150°C |
| 505 | 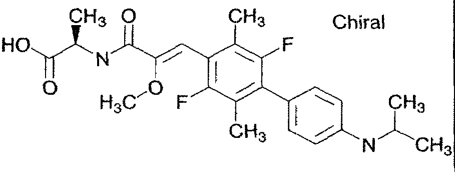 | 1H NMR (CDCl3) δ 1.26 (d, J = 6.3 Hz, 6H), 1.57 (d, J = 7.2 Hz, 3H), 2.09 (d, J = 2.4 Hz, 3H), 2.19 (d, J = 2.1 Hz, 3H), 3.53 (s, 3H), 3.69 (m, 1H), 4.69 (m, 1H), 6.69 (d, J = 8.4 Hz, 2H), 6.93 (s, 1 H), 7.06 (d, J = 8.4 Hz, 2H), 7.28 (d, J = 7.8 Hz, 1 H). | |
| 506 | 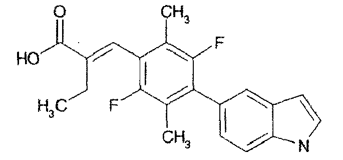 | 1H NMR (CDCl3) δ 1.09 (t, J = 7.5Hz, 3H), 2.09 (d, J = 2.7Hz, 3H), 2.17 (d, J = 2.4Hz, 3H), 2.30 (m, 2H), 6.60 (s, 1H), 7.09 (d, J= 8.4Hz, 1 H), 7.26-7.64 (m, 4H) ppm | |

**[Table 83]**

| | | | |
|---|---|---|---|
| 507 | 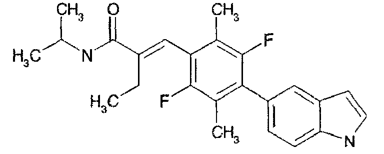 | | 162-163°C |
| 508 | 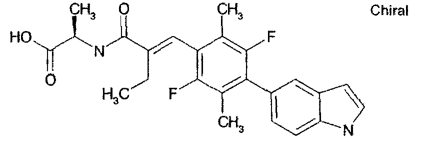 | | 156-157°C |
| 509 | 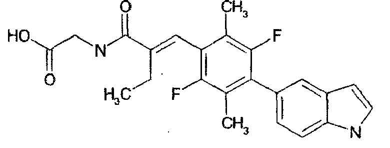 | | 166-168°C |
| 510 | 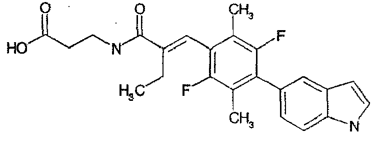 | | 104-108°C |
| 511 | 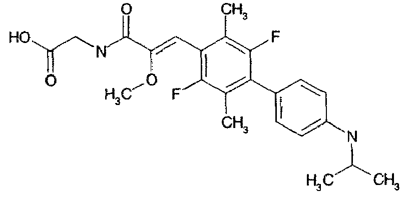 | | 182-185°C |
| 512 | 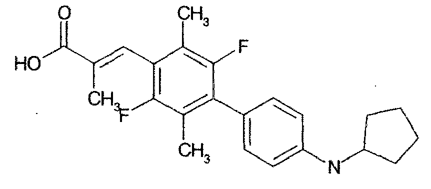 | 1 H NMR (DMSO-d6) δ 1.44-1.71 (m, 9H), 1.88-2.04 (m, 2H), 2.04 (s, 3H), 2.09 (s, 3H), 3.71 (m, 1H), 5.84 (m, 1 H), 6.63 (d, J = 8.4 Hz, 2H), 6.99 (d, J = 8.4Hz, 2H), 7.37 (s, 1H)ppm | |
| 513 | 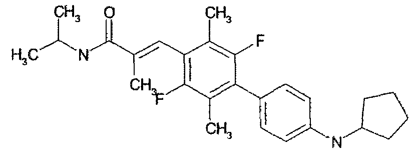 | | 137-138°C |
| 514 | 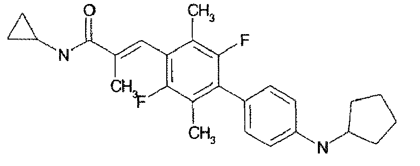 | | 162-164°C |

**[Table 84]**

| | | | |
|---|---|---|---|
| 515 | 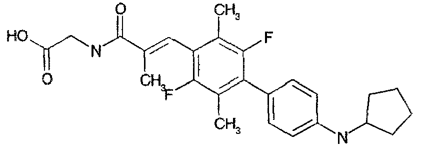 | 1H NMR (DMSO-d6) δ 1.48-1.74 (m, 12H), 1.92 (m, 2H), 2.04 (s, 3H), 2.10 (s, 3H), 3.17 (m, 2H), 3.85 (m, J = 8.7 Hz, 3H), 5.82 (m, 1 H), 6.63 (d, J = 9.0 Hz, 2H), 6.99 (d, J = 8.4Hz, 2H), 7.10 (s, 1 H), 8.45 (m, 1 H), 12.59 (brs, 1H)ppm | |
| 516 | 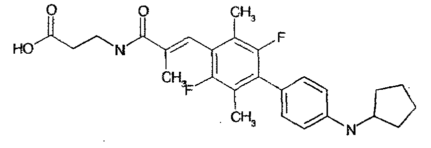 | | 149°C |
| 517 | 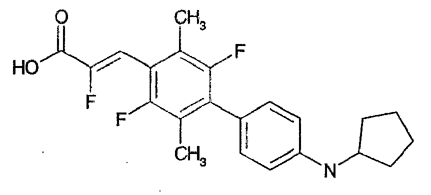 | 1H NMR (DMSO-d6) δ 1.40-2.05 (m, 8H), 2.04 (d, 3H, J = 2.7 Hz), 2.16 (s, 3H), 3.72 (m, 1 H), 6.63 (d, 2H, J = 8.7 Hz), 6.99 (d, 2H, J = 8.7 Hz), 7.02 (d, 1H, J = 35.4 Hz) | |
| 518 | 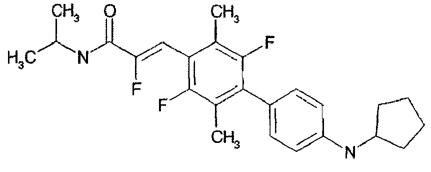 | 1 H NMR (CDCl3) δ 1.26 (d, 6H, J = 6.6 Hz), 1.40-2.10 (m, 8H), 2.09 (s, 3H), 2.20 (s, 3H), 6.19 (d, 1H, J = 5.1 Hz), 6.66 (d, 2H, J = 8.1 Hz), 6.96 (d, 1H, J = 41.1 Hz), 7.04 (d, 2H, J = 8.4 Hz) | |
| 519 | 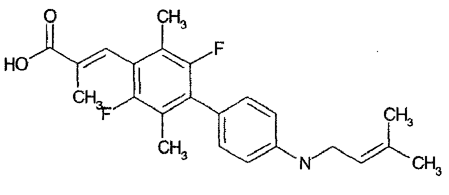 | 1 H NMR (CDCl3) δ 1.73 (s, 3H), 1.77 (s, 3H), 1.88 (s, 3H), 2.11 (d, J = 2.7Hz, 3H), 2.16 (d, J = 2.4Hz, 3H), 3.75 (d, J = 5.7Hz, 2H), 5.38 (m, 1H), 6.71 (d, J = 8.4Hz, 2H), 7.09 (d, J = 8.1 Hz, 2H), 7.62 (s, 1H)ppm | |
| 520 | 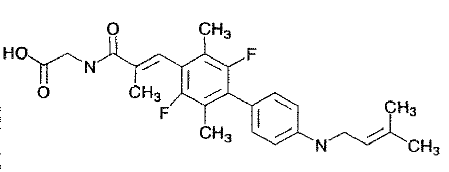 | 1 H NMR (DMSO-d6) δ 1.70 (s, 3H), 1.72 (s, 3H), 1.74 (s, 3H), 2.03 (d, J = 2.4Hz, 3H), 2.09 (d, J = 2.1Hz, 3H), 3.64 (d, J = 6.0Hz, 2H), 3.84 (d, J = 6.0Hz, 2H), 5.29 (m, 1H), 5.85 (m, 1H), 6.63 (d, J = 8.4Hz, 2H), 7.00 (d, J = 8.1Hz, 2H), 7.09 (s, 1H), 8.42 (m, 1 H)ppm | |

**[Table 85]**

| | | | |
|---|---|---|---|
| 521 | 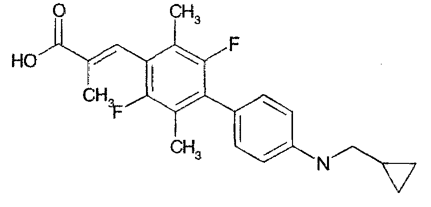 | 1 H NMR (CDCl3) δ 0.26 (m, 2H), 0.56 (m, 2H), 1.12 (m, 1 H), 1.88 (s, 3H), 2.09-2.16 (m, 6H), 3.01 (d, J = 6.9Hz, 1 H), 3.32 (d, J = 6.0Hz, 2H), 3.21 (s, 3H), 3.49 (s, 3H), 5.71 (m, 1 H), 6.62 (d, J = 8.4Hz, 2H), 7.08 (d, J = 8.1 Hz, 2H), 7.62 (s, 1 H)ppm | |
| 522 | 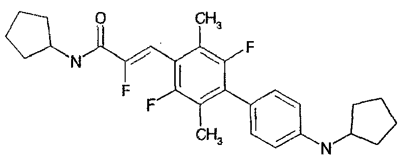 | 1 H NMR (CDCl3) δ 1.40-2.20 (m, 16H), 2.08 (d, 3H, J = 2.7 Hz), 2.19 (s, 3H), 3.90 (m, 1H), 4.35 (m, 1 H), 6.30 (br, 1H), 6.78 (br, 2H), 6.98 (d, 1H, J = 48.0 Hz), 7.08 (d, 2H, J = 8.1 Hz) | |
| 523 | 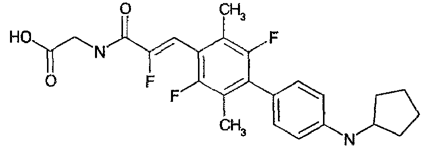 | 1 H NMR (CD3OD) δ 1.56-1.78 (m, 6H), 1.96-2.04 (m, 2H), 2.08 (s, 3H), 2.20 (s, 3H), 3.80-3.87 (m, 2H), 4.05-4.11 (m, 1H), 6.73 (d, J = 9.0 Hz. 2H), 6.88 (s, 1 H), 7.00 (d, J = 8.7 Hz., 2H)ppm | |
| 524 | 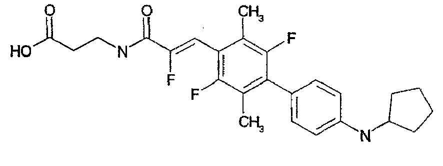 | 1H NMR (CD3OD) δ 1.51-1.77 (m, 8H), 1.99-2.07 (m, 2H), 2.07 (s, 3H), 2.19 (s, 3H), 2.62 (t, J = 6.9 Hz, 2H), 3.54-3.63 (m, 2H), 3.80-3.86 (m, 1H), 6.71 (d, J = 8.7 Hz, 2H), 6.84 (s, 1 H), 6.99 (d, J = 8.7 Hz, 2H), 8.63 8s, 1 H)ppm | |

**[Table 86]**

| Compo und No. | Structure | 1HNMR | mp |
|---|---|---|---|
| 525 | 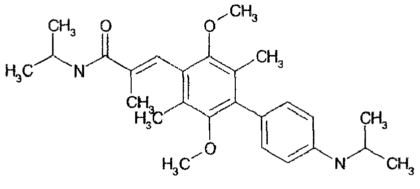 | 1H NMR (CDCl3) δ 1.25 (d, J = 6.9Hz, 6H), 1.27 (d, J = 6.9Hz, 6H), 1.81 (s, 3H), 2.03 (s, 3H), 2.12 (s, 3H), 3.29 (s, 3H), 3.58 (s, 3H), 3.61 (m, 1H), 4.24 (m, 1 H), 5.68 (d, J = 8.4Hz, 1 H), 6.75 (m, 2H), 7.10 (d, J = 8.1Hz, 2H), 7.32 (s, 1H)ppm | |
| 526 | 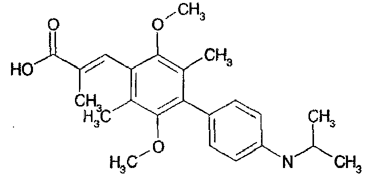 | 1H NMR (DMSO-d6) δ 1.16 (d, J = 6.3Hz, 6H), 1.67 (s, 3H), 1.95 (m, 1 H), 2.03 (s, 3H), 3.21 (s, 3H), 3.49 (s, 3H), 3.58 (m, 1H), 6.60 (d, J = 8.7Hz, 2H), 6.94 (d, J = 8.7Hz, 2H), 7.51 (s, 1H), 12.50 (brs, 1H)ppm | |
| 527 | 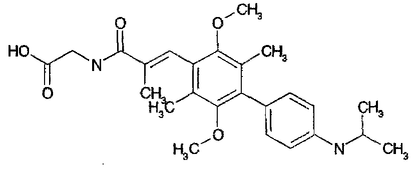 | 1H NMR (DMSO-d6) δ 1.16 (d, J = 6.0Hz, 6H), 1.70 (s, 3H), 1.94 (s, 3H), 2.03 (s, 3H), 3.22 (s, 3H), 3.49 (s, 3H), 3.57 (m, 1H), 3.74 (s, 2H), 6.60 (d, J = 8.4Hz, 2H), 6.95 (d, J = 8.4Hz, 2H), 7.22 (s, 1H), 8.10 (s, 1H) ppm | |
| 528 | 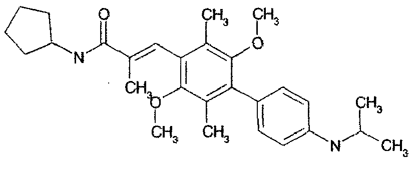 | 1H NMR (CDCl3) δ 1.26 (d, J = 6.3 Hz, 6H), 1.46-1.74 (m, 6H), 2.04 (s, 3H), 2.12 (m, 3H), 3.29 (s, 3H), 3.56 (s, 3H), 3.66-3.70 (m, 1H), 4.31-4.36 (m, 1H), 5.82 (d, J = 7.2 Hz, 1 H), 6.68 (d, J = 8.4 Hz, 2H), 7.08 (d, J = 8.4 Hz, 2H), 7.34 (s, 1H)ppm | |
| 529 | 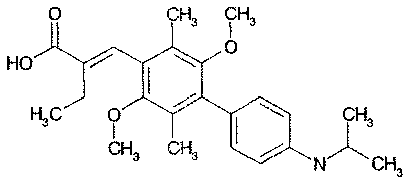 | 1H NMR (CDCl3) δ 1.05 (t, J = 7.5 Hz, 3H), 1.26 (d, J = 6.3 Hz, 6H), 2.05 (s, 3H), 2.24 (s, 3H), 2.23 (q, J = 7.2 Hz, 2H), 3.30 (s, 3H), 3.61 (s, 3H), 3.64-3.73 (m, 1H), 6.66 (d, J = 8.4 Hz, 2H), 7.08 (d, J = 8.4 Hz, 2H), 7.71 (s, 1H) ppm | |

**[Table 87]**

| | | | |
|---|---|---|---|
| 530 | 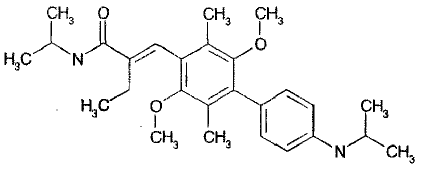 | 1 H NMR (CDCl3) δ 1.00 (t, J = 7.8 Hz, 3H), 1.25 (d, J = 6.6 Hz, 6H), 1.26 (d, J = 6.3 Hz, 6H), 2.03 (s, 3H), 2.13 (s, 3H), 2.25 (q, J = 7.8 Hz, 2H), 3.29 (s, 3H), 3.61 (s, 3H), 3.66-3.72 (m, 1H), 4.21-4.28 (m, 1 H), 5.66 (d, J = 7.8 Hz, 1H), 6.68 (d, J = 8.1 Hz, 2H), 7.00 (s, 1H), 7.08 (d, J = 7.8 Hz, 2H)ppm | |
| 531 | 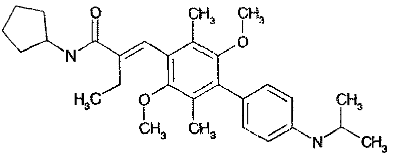 | 1H NMR (CDCl3) δ 1.00 (t, J = 7.5 Hz, 3H), 1.26 (d, J = 6.3 Hz, 6H), 1.44-1.74 (m, 6H), 2.02-2.08 (m, 2H), 2.04 (s, 3H), 2.14 (s, 3H), 2.25 (q, J = 7.5 Hz, 2H), 3.29 (s, 3H), 3.61 (s, 3H), 3.68-3.70 (m, 1 H), 4.37 (m, 1 H), 5.79 (d, J = 7.8 Hz, 1H), 6.67 (d, J = 8.4 Hz, 2H), 7.01 (s, 1H), 7.07 (d, J = 8.1 Hz, 2H)ppm | |
| 532 | 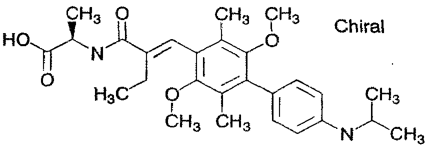 | 1H NMR (CDCl3) δ 1.02 (t, J = 7.8 Hz, 3H), 1.28 (d, J = 6.6. Hz, 6H), 1.26 (d, J = 7.2 Hz, 3H), 2.03 (s, 3H), 2.12 (s, 3H), 2.28 (q, J = 7.8 Hz, 2H), 3.28 (s, 3H), 3.60 (s, 3H), 3.63-3.71 (m, 1H), 4.71-4.76 (m, 1H), 6.04 (s, 1H), 6.75 (s, 2H), 7.09 (d, J = 8.4 Hz, 2H), 7.17 (s, 1H)ppm | |
| 533 | 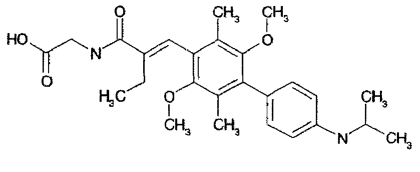 | 1H NMR (CDCl3) δ 1.02 (t, J = 7.2 Hz, 3H)), 1.27 (d, J = 6.3 Hz, 6H), 2.03 (s, 3H), 2.12 (s, 3H), 2.29 (q, J = 7.5 Hz, 2H), 3.28 (s, 3H), 3.62 (s, 3H), 3.66-3.73 (m, 1H), 4.24-4.26 (s, 2H), 6.71-6.74 (m, 3H), 7.09 (d, J = 8.7 Hz, 2H), 7.15 (s, 1H)ppm | |
| 534 | 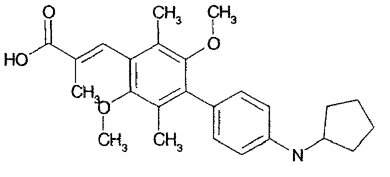 | 1H NMR (CDCl3) δ 1.51-1.74 (m, 6H), 1.85 (s, 3H), 2.01-2.08 (m, 2H), 2.05 (s, 3H), 2.14 (s, 3H), 3.30 (s, 3H), 3.59 (s, 3H), 3.84-3.86 (m, 1H), 6.69 (d, J = 8.1 Hz, 2H), 7.08 (d, J = 8.4 Hz, 2H), 7.81 (s, 1H)ppm | |

**[Table 88]**

| | | | |
|---|---|---|---|
| 535 | 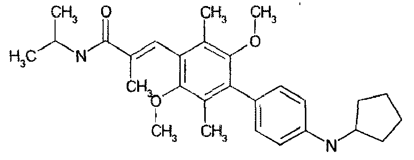 | 1H NMR (CDCl3) δ 1.25 (d, J = 6.6 Hz, 6H), 1.58-1.76 (m, 6H), 1.81 (s, 3H), 2,04-2.11 (m, 2H), 2.05 (s, 3H), 2.12 (s, 3H), 3.29 (s, 3H), 3.58 (s, 3H), 3.82-3.86 (m, 1H), 4.16-4.26 (m, 1H), 5.69 (d, J = 7.8 Hz., 1H), 6.70 (d, J = 7.5 Hz, 2H), 7.08 (d, J = 7.5 Hz, 2H), 7.33 (s, 1H)ppm | |
| 536 | 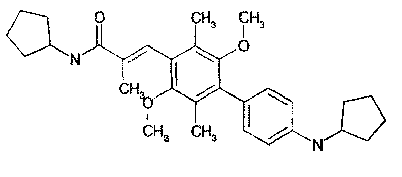 | 1H NMR (CDCl3) δ 1,46-1.77 (m, 12H), 1.81 (s, 3H), 2.04-2.12 (m, 4H), 2.05 (s, 3H), 2.12 (s, 3H), 3.29 (s, 3H), 3.58 (s, 3H), 3.82-3.85 (m, 1H), 4.31-4.38 (m, 1 H), 5.82 (d, J = 7.5 Hz, 1H), 6.70 (d, J = 7.5 Hz, 2H), 7.08 (d, J = 8.4 Hz, 2H), 7.33 (s, 1 H)ppm | |
| 537 | 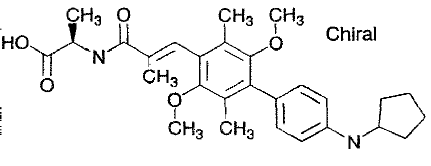 | 1H NMR (CDCl3) δ 1.52-1.76 (m, 6H), 1.83 (s, 3H), 2.05 (s, 3H), 2.98 (s, 3H), 3.26 (s, 3H), 3.54 (s, 3H), 3.81-3.85 (m, 1H), 4.59-4.64 (m, 1 H), 6.65 (d, J = 8.7 Hz, 2H), 6,63-6.70 (m, 1H), 7.04 (d, J = 8.1 Hz, 2H), 7.44 (s, 1 H)ppm | |
| 538 | 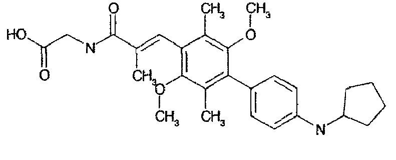 | 1 H NMR (CDCl3) δ 1.55-1.77 (m, 6H), 1.87b (s, 3H), 2.03-2.07 (m, 2H), 2.03 (s, 3H), 2.12 (s, 3H), 3.29 (s, 3H), 3.59 (s, 3H), 3.82-3.90 (m, 1H), 4,25 (d, J = 7.5 Hz, 2H), 6.59 (s, 1H), 6.73 (d, J = 7.5 Hz, 2H), 7.08 (d, J = 8.4 Hz, 2H), 7.46 (s, 1H)ppm | |
| 539 | 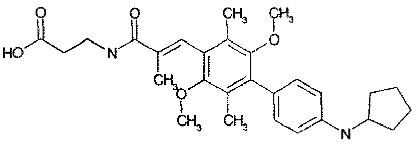 | 1H NMR (CDCl3) δ 1.53-1.78 (m, 6H), 1.81 (s, 3H), 2.03-2.10 (m, 2H), 2.03 (s, 3H), 2.13 (s, 3H), 2.73 (t, J = 6.0 Hz, 2H), 3.28 (s, 3H), 3.57 (s, 3H), 3.63-3.72 (m, 2H), 3.82-3.86 (m, 1 H), 6.68-6.70 (m, 3H), 7.06 (d, J = 8.7 Hz, 2H), 7.37 (s, 1 H)ppm | |
| 540 | 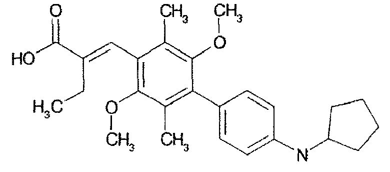 | 1H NMR (CDCl3) δ 1.05 (t, J = 7.5 Hz, 3H), 1.53-1.76 (m, 6H), 2.01-2.13 (m, 2H), 2.05 (s, 3H), 2.14 (s, 3H), 2.28 (q, J = 7.5 Hz, 2H), 3.28 (s, 3H), 3.61 (s, 3H), 3.83-3.86 (m, 1 H), 6..69 (d, J = 8.4 Hz, 2H), 7.08 (d, J = 8.4 Hz, 2H), 7.70 (s, 1H) ppm | |

**[Table 89]**

| | | | |
|---|---|---|---|
| 541 | 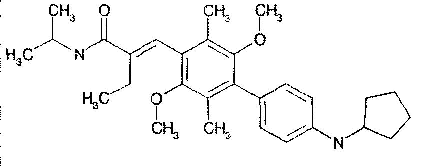 | 1H NMR (CDCl3) δ 1.01 (t, J = 7.8 Hz, 3H), 1.26 (d, J = 6.6 Hz, 6H), 1.56-1.78 (m, 6H), 2.03-2.10 (m, 2H), 2.04 (s, 3H), 2.14 (m, 3H), 2.26 (q, J = 7.8 Hz, 2H), 3.82-3.86 (m, 1 H), 4.22-4.29 (m, 1 H), 5.66 (d, J = 7.8 Hz, 1 H), 6.73 (s, 2H), 7.01 (s, 1H), 7.11 (d, J = 8.1 Hz, 2H)ppm | |
| 542 | 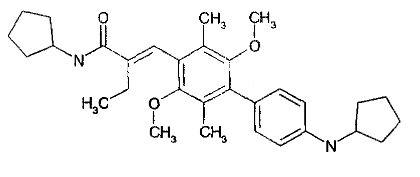 | 1H NMR (CDCl3) δ 1.00 (t, J = 7.5 Hz, 3H), 1.46-1.80 (m, 12H), 2.03-2.12 (m, 4H), 2.03 (s, 3H), 2.20 (s, 3H), 2.25 (q, J = 7.5 Hz, 2H), 3.29 (s, 3H), 3.60 (s, 3H), 3.81-3.86 (m, 1H), 4.32-4.39 (m, 1H), 5.78 (d, J = 6.3 Hz, 1H), 6.70 (d, J = 8.1 Hz, 2H), 7.01 (s, 1H), 7.08 (d, J = 8.4 Hz, 2H)ppm | |
| 543 | 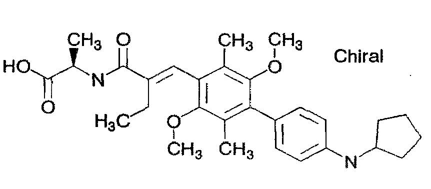 | 1 H NMR (COCl3) δ 1.03 (t, J = 7.5 Hz, 3H), 1.24-1.67 (m, 6H), 1.59 (d, J = 7.2 Hz, 3H), 2.03-2.09 (m, 2H), 2.03 (s, 3H), 2.13 (s, 3H), 2.29 (q, J = 7.5 Hz, 2H), 3.29 (s, 3H), 3.61 (s, 3H), 3.79-3.84 (m, 1H), 4.72-4.76 (m, 1 H), 6.41 (s, 1 H), 6.78 (s, 1 H), 7.10 (d, J = 8.4 Hz, 2H), 7.17 (s, 1 H), 7.52-7.55 (m, 2H)ppm | |
| 544 | 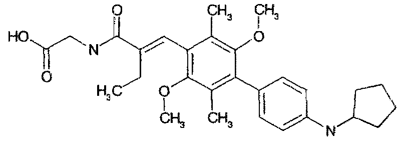 | 1H NMR (COCl3) δ 1.02 (t, J = 7.5 Hz, 3H), 1.44-1.76 (m, 6H), 2.05-2.14 (m, 2H), 2.03 (s, 3H), 2.11 (s, 3H), 3.28 (s, 3H), 3.61 (S, 3H), 3,81-3,86 (m, 1 H), 4 21 (s, 1 H), 6.66 (d, J = 8.4 Hz, 2H), 6.80 (s, 1H), 7.06 (d, J = 8.4 Hz, 2H), 7.16 (s, 1H)ppm | |
| 545 | 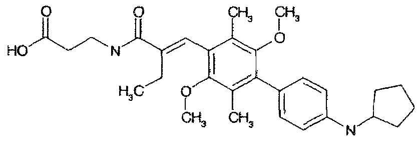 | 1H NMR (CDCl3) δ 0.98 (t, J = 7.5 Hz, 3H), 1.54-1.77 (m, 6H), 2.02-2.11 (m, 2H), 2.02 (s, 3H), 2.11 (s, 3H), 2.25 (q, J = 7.5 Hz, 2H), 2.73 (t, J = 6.0 Hz, 2H), 3.28 (s, 3H), 3.59 (s, 3H), 3.71 (q, J = 6.0 Hz, 2H), 3.80-3.86 (m, 1H), 6.61-6.62 (m, 1H), 6.69 (d, J = 8.7 Hz, 2H), 7.06 (d, J = 8.7 Hz, 2H), 7.08 8s, 1 H)ppm | |

**[Table 90]**

| | | | |
|---|---|---|---|
| 546 | 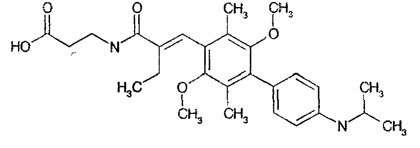 | 1H NMR (CDCl3) δ 0.99 (t, J = 8.2 Hz, 3H), 1.27 (d, J = 6.3 Hz, 6H), 2.02 (s, 3H), 2.11 (s, 3H), 2.25 (q, J = 7.5 Hz, 2H), 2.27 (t, J = 6.3 Hz, 2H), 3.28 (s, 3H), 3.62 (s, 3H), 3.67 (q, J = 6.6 Hz, 2H), 6.63 (s, 1H), 6.70 (d, J = 7.5 Hz, 2H), 7.06 (s, 1 H), 7.08 (d, J = 8.7 Hz, 2H)ppm | |
| 547 | 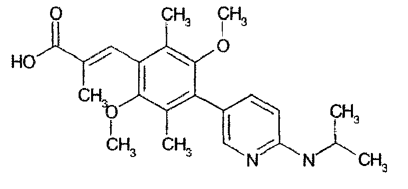 | 1H NMR (CDCl3) δ 1.35 (d, J = 6.3 Hz, 6H), 1.84 (s, 3H), 2.08 (s, 3H), 2.14 (s, 3H), 3.35 (s, 3H), 3.60 (s, 3H), 3.76-3.85 (m, 1H), 6.57 (d, J = 8.7 Hz, 1H), 7.52 (d, J = 8.7 Hz, 1H), 7.71 (s, 1 H), 7.91 (s, 1H)ppm | |
| 548 | 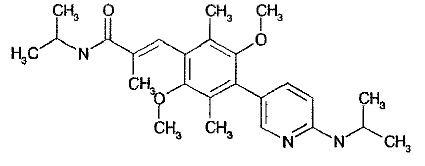 | 1H NMR (CDCl3) δ 1.26 (d, J = 6.6 Hz, 6H), 1.31 (d, J = 6.3 Hz, 6H), 1.81 (s, 3H), 2.07 (s, 3H), 2.13 (s, 3H), 3.87-3.89 (m, 1H), 4.20-4.25 (m, 1H), 5.69 (d, J = 7.5 Hz, 1 H), 6.50 (d, J = 8.4 Hz, 1H), 7.33 (s, 1H), 7.44 (d, J = 7.8 Hz, 1 H), 7.96 (s, 1 H)ppm | |
| 549 | 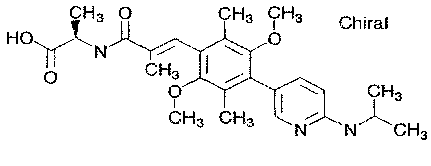 | 1 H NMR (CDCl3) δ 1.38 (d, J = 6.3 Hz, 6H), 1.57 (d, J = 6.9 Hz, 3H), 1.84 (s, 3H), 2.06 (s, 3H), 2.12 (s, 3H), 3.34 (s, 3H), 3.57 (s, 3H), 3.74-3.81 (m, 1H), 4.53-4.60 (m, 1 H), 6.70 (d, J = 9.6 Hz, 1 H), 7.07 (d, J = 6.6 Hz, 1H), 7.39 (s, 1H), 7.66 (d, J = 9.6 Hz, 1H), 7.75 (s, 1H)ppm | |
| 550 | 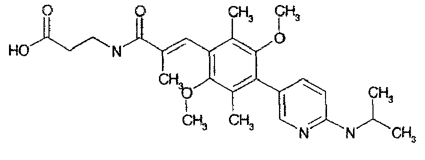 | 1H NMR (CDCl3) δ 1.39 (d, J = 6.0 Hz, 6H), 1.81 (s, 3H), 1.99 (s, 3H), 2.11 (s, 3H), 2.58-2.60 (m, 2H), 3.33 (s, 3H), 3.55 (s, 3H), 3.61-3.77 (m, 2H), 4.09-4.15 (m, 1H), 6.72 (d, J = 9.3 Hz, 1H), 7.30 (s, 1H), 7.54 (s, 1H), 7.68 (d, J = 9.0 Hz, 1H), 7.94 (s, 1 H)ppm | |
| 551 | 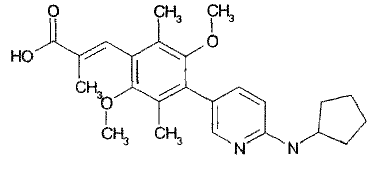 | 1H NMR (CDCl3) δ 1.26 (bs, 6H), 1.83 (s, 3H), 2.08-2.14 (m, 2H), 2.08 (s, 3H), 2.14 (s, 3H), 3.34 (s, 3H), 3.60 (s, 3H), 3.90-3.95 (m, 1H), 6.60 (d, J = 9.0 Hz, 1H), 7.53 (d, J = 8.4 Hz, 1 H), 7.70 (s, 1H), 7.89 (s, 1 H)ppm | |

**[Table 91]**

| | | | |
|---|---|---|---|
| 552 | 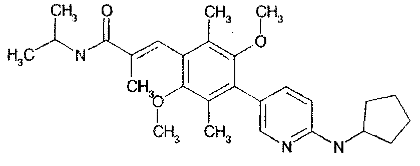 | 1H NMR (CDCl3) δ 1.25 (d, J = 6.6 Hz, 6H), 1.57-1.76 (m, 6H), 1.81 (s, 3H), 2.07-2.13 (m, 2H), 2.07 (s, 3H), 2.13 (s, 3H), 3.33 (s, 3H), 3.59 (s, 3H), 4.00-4.03 (m, 1H), 4.20-4.25 (m, 1H), 5.69 (d, J = 6.9Hz, 1H), 6.50 (d, J = 8.4Hz, 1H), 7.33 (s, 1 H), 7.20 (d, J = 8.4 Hz, 1H), 7.98 (s, 1 H)ppm | |
| 553 | 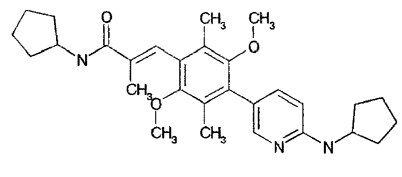 | 1H NMR (CDCl3) δ 1.43-1.78 (m, 6H), 1.81 (s, 3H), 2.05-2.09 (m, 2H), 2.07 (s, 3H), 2.12 (s, 3H), 3.33 (s, 3H), 3.59 (s, 3H), 3.99-4.05 (m, 1 H), 4.31-4.38 (m, 1H), 4.68 (s, 1H), 5.72 (d, J = 8.1 Hz, 1H), 6.48 (d, J = 8.4 Hz, 1 H), 7.34 (s, 1H), 7.43 (d, J = 8.4 Hz, 1H), 7.99 (s, 1 H)ppm | |
| 554 | 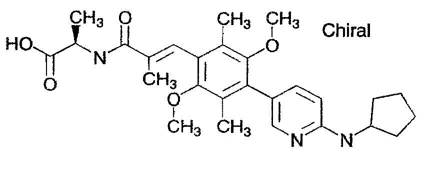 | 1H NMR (CDCl3) δ 1.57 (d, J = 6.9 Hz, 3H), 1.67-1.79 (m, 6H), 1,84 (s, 3H), 2.06-2.17 (m, 2H), 2.06 (s, 3H), 2.12 (s, 3H), 3,34 (s, 3H), 3,57 (s, 3H), 3.85-3.89 (m, 1H), 4.55-4.60 (m, 1H), 6.74 (d, J = 9.0 Hz, 1H), 7.09 (d, J = 6.0 Hz, 1H), 7.39 (s, 1H), 7.66 (d, J = 8.7 Hz, 1 H), 7.72 (s, 1 H)ppm | |
| 555 | 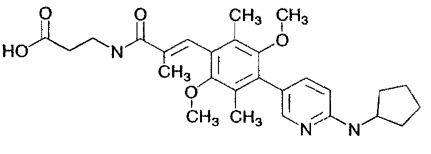 | 1 H NMR (CDCl3) δ 1.68-1.88 (m, 6H), 1.80 (s, 3H), 2.05-2.10 (m, 2H), 2.05 (s, 3H), 2.10 (s, 3H), 2.58 (t, J = 6.0 Hz, 2H), 3,31 (s, 3H), 3.54 (s, 3H), 3.63-3.65 (m, 2H), 3.78-3.87 (m, 1H), 4.11-4.16 (m, 1 H), 6.73 (d, J = 9.3 Hz, 1 H), 7.52 (s, 1H), 7.65 (d, J = 9.0 Hz, 1 H), 8.00 (s, 1 H)ppm | |
| 556 | 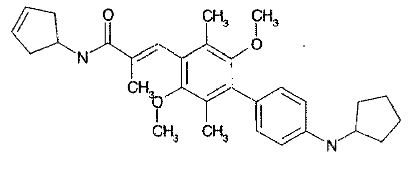 | 1H NMR (CDCl3) δ 1.57-1.63 (m, 6H), 1.81 (s, 3H), 2.02-2.11 (m, 2H), 2.02 (s, 3H), 2.11 (s, 3H), 2.29 (dd, J = 14.7, 3,6 Hz, 2H), 2.89 (dd, J = 15.2, 7.8 Hz. 2H), 3.28 (s, 3H), 3.58 (s, 3H), 3.83 (s, 1H), 4.70-4.74 (m, 1H), 5.77 (s, 1H), 6.03 (d, J = 7.8 Hz, 1H), 6.87 (s, 1H), 7.12 (s, 2H), 7.34 (s, 1H)ppm | |

**[Table 92]**

| | | | |
|---|---|---|---|
| 557 | 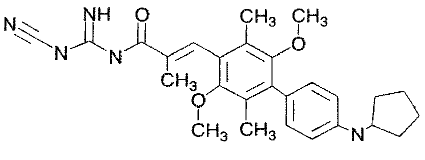 | 1 H NMR (CDCl3) δ 1.25-2.77 (m, 6H), 2.04-2.11 (m, 2H), 2.04 (s, 3H), 2.11 (s, 3H), 3.29 (s,3H), 3.56 (s, 3H), 3.81-3.86 (m, 1H), 6.29 (s, 1H), 6.75 (s, 2H), 7.06 (d, J = 8.4 Hz, 2H), 7.56 (s, 1 H), 8.28(s, 1 H), 9.31 (s, 1 H).p | |
| 558 | 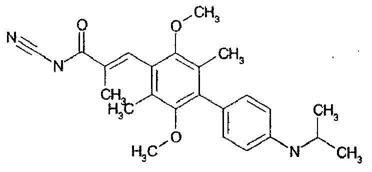 | 1 H NMR (DMSO-d6) δ 1.67 (d, J = 6.3 Hz, 6H), 1.71 (s, 3H), 1.95 (s, 3H), 2.04 (s, 3H), 3.22 (s, 3H), 3.50 (s, 3H), 3.56 (m, 1 H), 6.67 (d, J = 8.7 Hz, 2H), 6.98 (d, J = 8.4Hz, 2H), 7.40 (s, 1 H)ppm | |
| 559 | 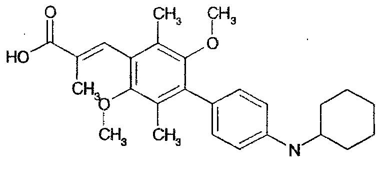 | 1H NMR (CDCl3) δ 1.19-1.46 (m, 8H), 1.66-1.82 (m, 2H), 1.85 (s, 3H), 2.05 (s, 3H), 2.13 (s, 3H), 3.28-3.34 (m, 1H), 3.30 (s, 3H), 3.59 (s, 3H), 6.68 (d, J = 8.4 Hz, 2H), 7.06 (d, J = 8.7 Hz, 2H), 7.81 (s, 1H).ppm | |
| 560 | 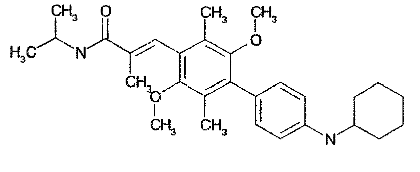 | 1 H MMR (CDCl3) δ 1.25 (d, J = 6.6 Hz, 6H), 1.33-1.77 (m, 10H), 1.81 (s, 3H), 2.04 (s, 3H), 2.11 (s, 3H), 3.29-3.33 (m,1H),3.29(s, 3H), 3.58 (s, 3H), 4.20-4.26 (m, 1 H), 5.69 (d, J = 9.0 Hz., 1 H), 6.71 (s, 2H), 7.08 (d, J = 8.1 Hz, 2H), 7.32 (s, 1 H) ppm | |
| 561 | 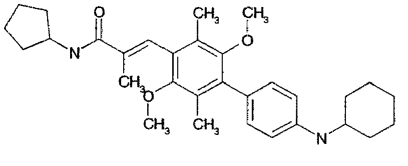 | 1H NMR (CDCl3) δ 1.21-1.50 (m, 12H), 1.65-1.77 (m, 4H), 1.81 (s, 3H), 2.03 (s, 3H), 2.05-2.12 (m, 2H), 2.13 (s, 3H), 3.19-3.29 (m, 1 H), 3.29 (s, 3H), 3.58 (s, 3H), 4.29-4.46 (m, 1H), 5.82 (d, J = 7.8 Hz, 1H), 6.72 (s, 2H), 7.08 (d, J = 7.8 Hz, 2H), 7.33 (s, 1 H)ppm | |
| 562 | 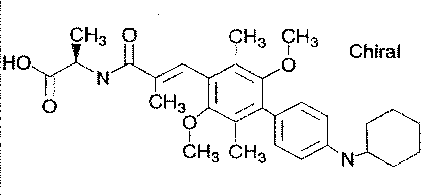 | 1 H NMR (CDCl3) δ 1.01.41 (m, 8H), 1.50 (d, J = 7.2 Hz, 3H), 1.78-1.83 (m, 2H), 1.86 (s, 3H), 2.03 (s, 3H), 2.12 (s, 3H), 3.28-3.39 (m, 1H), 3.29 (s, 3H), 3.58 (s, 3H), 4.69-4.74 (m, 1H), 6.53 (s, 1H), 6.72 (d, J = 8.1 Hz, 2H), 7.08 (d, J = 8.7 Hz, 2H), 7.46 (s, 1H)ppm | |

**[Table 93]**

| | | | |
|---|---|---|---|
| 563 | 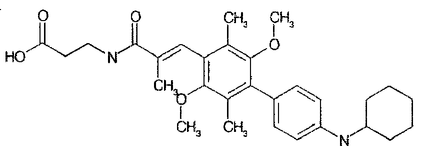 | 1H NMR (CDCl3) δ 1.19-1.41 (m, 8H), 1.66-1.77 (m, 2H), 1.81 (s, 3H), 2.01 (s, 3H), 2.10 (s, 3H), 2.79 (t, J = 6.0 Hz, 2H), 3.28-3.29 (m, 1 H), 3.28 (s, 3H), 3.56 (s, 3H), 3.68-3.70 (m, 1 H), 6.67-6.69 (m, 3H), 7.05 (d, J = 8.1 Hz, 2H), 7.37 (s, 1H) ppm | |
| 564 | 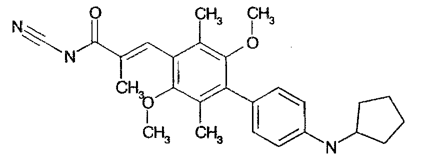 | 1H NMR (DMSO-d6) δ 1.45-1.64 (m, 6H), 1.70 (s, 3H), 1.95-2.04 (m, 2H), 1.96 (s, 3H), 2.04 (s, 3H), 3.21 (s, 3H), 3.49 (s, 3H), 3.71 (bs, 1H), 6.20 (bs, 1H), 6.62 (d, J = 8.4 Hz, 2H), 6.95 (d, J = 8.4 Hz, 2H), 7.39 (s, 1 H), 7.96 (s, 1H)ppm | |
| 565 | 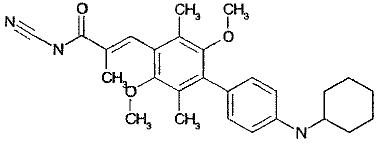 | 1H NMR (DMSO-d6) δ 1.17-1.70 (m, 10H), 1.70 (s, 3H), 1.95 (s, 3H), 2.04 (m, 3H), 3.22 (s, 3H), 3.49 (s, 3H), 6.60 (bs, 2H), 6.97 (bs, 2H), 7.40 (bs, 2H)ppm | |
| 566 | 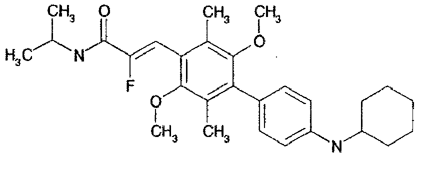 | 1H NMR (CDCl3) δ 1.26 (d, J = 6.3 Hz, 6H), 1.37-1.78 (m, 10H), 2.05 (s, 3H), 2.19 (s, 3H), 3.30 (s, 3H), 3.66 (s, 3H), 4.11-4.16 (m, 1H), 4.22-4.27 (m, 1H), 6.18 (bs, 1H), 6.72 (bs, 2H), 7.04-7.09 (m, 3H)ppm | |
| 567 | 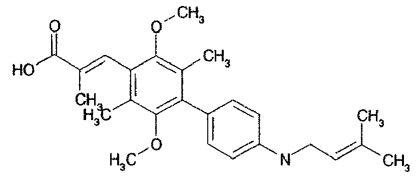 | 1H NMR (DMSO-d6) δ 1.67 (s, 3H), 1.68 (s, 3H), 1.73 (s, 3H), 1.94 (s, 3H), 2.03 (s, 3H), 3.21 (s, 3H), 3.49 (s, 3H), 3.63 (d, J = 6.3 Hz, 2H), 5.31 (m, 2H), 5.68 (m, 1 H), 6.61 (d, J = 8.7 Hz, 2H), 6.95 (d, J = 8.4Hz, 2H), 7.51 (s, 1H), 12.58 (brs, 1H)ppm | |
| 568 | 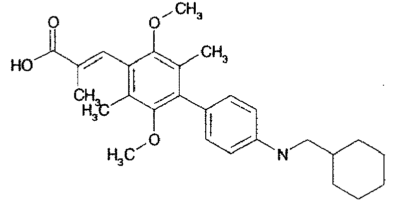 | 1 H NMR (DMSO-d6) δ 0.94-1.26 (m, 5H), 1.56-1.82 (m, 7H), 1.84 (m, 2H), 1.95 (s, 3H), 2.03 (s, 3H), 2.89 (m, 2H), 3.21 (s, 3H), 3.49 (s, 3H), 5.68 (m, 1H), 6.60 (d, J = 8.4 Hz, 2H), 6.94 (d, J = 8.4Hz, 2H), 7.51 (s, 1 H), 12.54 (brs, 1H)ppm | |

**[Table 94]**

| | | | |
|---|---|---|---|
| 569 | 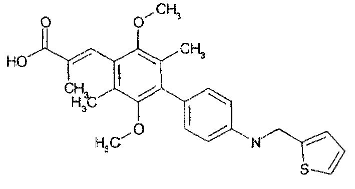 | 1H NMR (DMSO-d6) δ 1.66 (s, 3H), 1.93 (s, 3H), 2.02 (s, 3H), 3.19 (s, 3H), 3.48 (s, 3H), 4.27 (s, 2H), 6.32 (m, 1H), 6.69 (d, J = 8.4 Hz, 2H), 6.97-7.00 (m, 3H), 7.08-7.50 (m, 3H) ppm | |
| 570 | 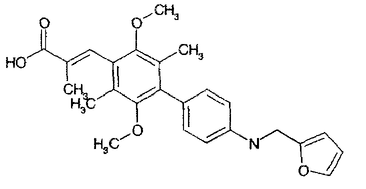 | 1H NMR (DMSO-d6) δ 1.66 (s, 3H), 1.93 (s, 3H), 2.03 (s, 3H), 3.20 (s, 3H), 3.49 (s, 3H), 4.26 (s, 2H), 6.17 (m, 1H), 6.34-6.40 (m, 2H), 6.70 (d, J = 8.1 Hz, 2H), 6.96 (d, J = 8.7Hz, 2H), 7.49 (s, 1 H), 7.60 (m, 1 H) ppm | |
| 571 | 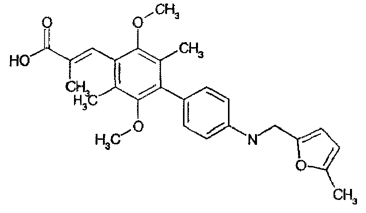 | 1 H NMR (DMSO-d6) δ 1.67 (s, 3H), 1.94 (s, 3H), 2.03 (s, 3H), 2.25 (s, 3H), 3.21 (s, 3H), 3.49 (s, 3H), 4.21 (s, 2H), 5.99-6.21 (m, 3H), 6.70 (d, J = 8.7 Hz, 2H), 6.96 (d, J = 8.7Hz, 2H), 7.51 (s, 1H), 12.54 (brs, 1H)ppm | |
| 572 | 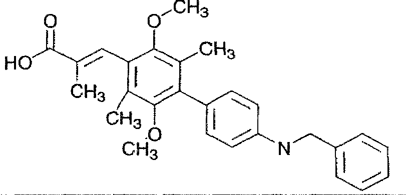 | 1H NMR (DMSO-d6) δ 1.66 (s, 3H), 1.92 (s, 3H), 2.02 (s, 3H), 3.19 (s, 3H), 3.48 (s, 3H), 4.28 (m, 2H), 6.28 (m, 1H), 6.64 (d, J = 8.7 Hz, 2H), 6.94 (d, J = 8.7Hz, 2H), 7.21-7.42 (m, 5H), 7.49 (s, 1H), ppm | |
| 573 | 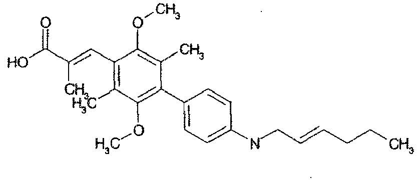 | 1 H NMR (DMSO-d6) δ 0.86 (t, J = 7.5 Hz, 3H), 1.35 (m, 2H), 1.67 (s, 3H), 1.94 (s, 3H), 2.03 (s, 3H), 2.05 (m, 2H), 3.21 (s, 3H), 3.49 (s, 3H), 3.65 (m, 2H), 5.51-5.79 (m, 3H), 6.62 (d, J = 8.4 Hz, 2H), 6.95 (d, J = 8.4Hz, 2H), 7.51 (s, 1H), 12.50 (brs, 1H)ppm | |
| 574 | 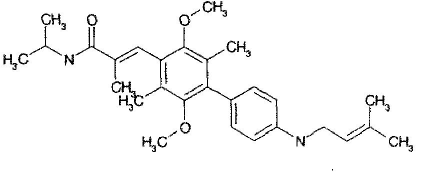 | 1 H NMR (CDCl3) δ 1.25 (d, J = 6.6Hz, 12H), 1.71 (s, 3H), 1.77 (s, 3H), 1.81 (s, 3H), 2.03 (s, 3H), 2.12 (s, 3H), 3.29 (s, 3H), 3.58 (s, 3H), 3.76 (d, J = 6.3Hz, 2H), 4.24 (m, 1H), 5.39 (m, 1H), 5.69 (d, J = 7.8Hz, 1 H), 6.76 (d, J = 8.7Hz, 2H), 7.04 (d, J = 8.4Hz, 2H), 7.33 (s, 1H)ppm | |

**[Table 95]**

| | | | |
|---|---|---|---|
| 575 | 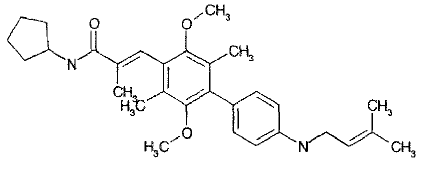 | 1 H NMR (CDCl3) δ 1.43-1.76 (m, 12H), 1.80 (s, 3H), 2.00-2.13 (m, 8H), 3.28 (s, 3H), 3.58 (s, 3H), 3.76 (d, J = 7.2Hz, 2H), 4.35 (m, 1H), 5.40 (m, 1H), 5.82 (d, J = 7.2Hz, 1H), 6.77 (m, 2H), 7.11 (d, J = 5.1 Hz, 2H), 7.33 (s, 1H) ppm | |
| 576 | 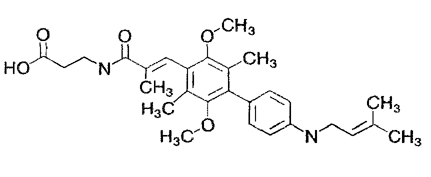 | 1 H NMR (DMSO-d6) δ 1.67 (s, 3H), 1.70 (s, 3H), 1.73 (s, 3H), 1.94 (s, 3H), 2.02 (s, 3H), 3.20 (s, 3H), 3.22-3.42 (m, 4H), 3.48 (s, 3H), 3.64 (m, 2H), 5.53 (m, 2H), 5.66 (m, 1H), 6.60 (d, J = 8.4 Hz, 2H), 6.94 (d, J = 8.4Hz, 2H), 7.15 (s, 1 H), 8.04 (m, 1H)ppm | |
| 577 | 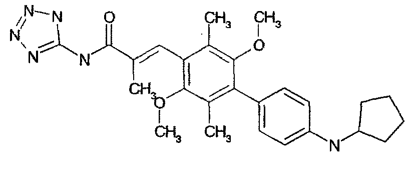 | 1 H NMR (DMSO-d6) δ 1.47-1.70 (m, 6H), 1.81 (s, 3H), 1.96-2.17 (m, 2H), 1.96 (s, 3H), 2.00 (s, 3), 3.23 (s, 3H), 3.53 (s, 3H), 3.67-3.77 (m, 1H), 6.62 (d, J = 8.7 Hz, 2H), 6.98 (d, J = 8.1 Hz, 2H), 7.35 (s, 1H), 7.54 (s, 1H), 12.13 (s, 1 H)ppm | |
| 578 | 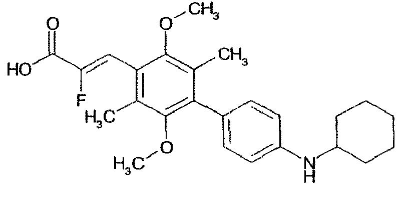 | 1H NMR (CDCl3+CD3OD) δ 1.13-1.47 (m, 4H), 1.62-1.86 (m, 3H), 2.00-2.18 (m, 3H), 2.04 (s, 3H), 2.20 (d, J = 3.0 Hz, 3H), 3.23-3.36 (m, 1 H), 3.31 (s, 3H), 3.68 (s, 3H), 6.72 (d, J = 8.7 Hz, 2H), 7.07 (d, J = 8.7 Hz, 2H), 7.20 (d, J = 36 Hz, 1H) ppm | |
| 579 | 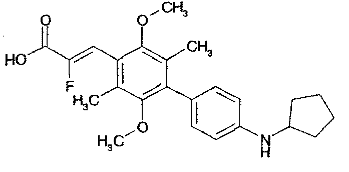 | 1 H NMR (CDCl3+CD3OD) δ 1.52-1.83 (m, 6H), 1.97-2.10 (m, 2H), 2.03 (s, 3H), 2.20 (d, J = 3.0 Hz, 3H), 3.31 (s, 3H), 3.68 (s, 3H), 3.77-3.87 (m, 1H), 6.82 (d, J = 8.7 Hz, 2H), 7.10 (d, J = 8.7 Hz, 2H), 7.19 (d, J = 35 Hz, 1H) ppm | |

**[Table 96]**

| | | | |
|---|---|---|---|
| 580 | 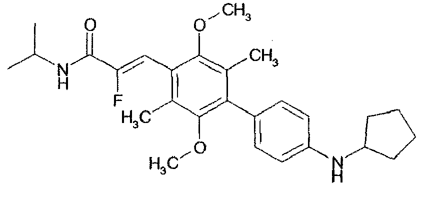 | 1 H NMR (CDCl3) δ 1.26 (d, J = 6.6 Hz, 6H), 1.46-1.82 (m, 6H), 1.97-2.13 (m, 2H), 2.05 (s, 3H), 2.19 (s, 3H), 3.30 (s, 3H), 3.66 (s, 3H), 3.79-3.90 (m, 1 H), 4.16-4.30 (m, 1 H), 6.13-6.21 (broad, 1 H), 6.67 (d, J = 8.4 Hz, 2H), 7.06 (d, J = 8.4 Hz, 2H), 7.11 (d, J=39 Hz, 1 H) ppm | |
| 581 | 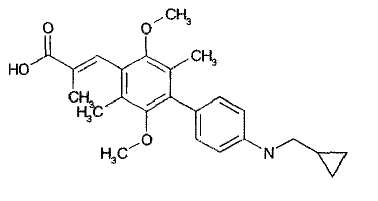 | 1 H NMR (DMSO-d6) δ 0.25 (m, 2H), 0.50 (m, 2H), 1.07 (m, 1H), 1.66 (s, 3H), 1.94 (s, 3H), 2.03 (s, 3H), 2.91 (d, J = 6.9Hz, 2H), 3.21 (s, 3H), 3.49 (s, 3H), 5.71 (m, 1H), 6.63 (d, J = 8.4Hz, 2H), 6.95 (d, J = 8.4Hz, 2H), 7.50 (s, 1H)ppm | |
| 582 | 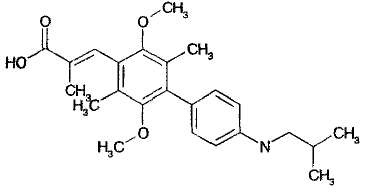 | 1 H NMR (DMSO-d6) δ 0.96 (d, J = 6.9Hz, 6H), 1.66 (s, 3H), 1.82 (m, 1 H), 1.95 (s, 3H), 2.03 (s, 3H), 2.84 (d, J = 6.3Hz, 2H), 3.21 (s, 3H), 3.49 (s, 3H), 5.70 (m, 1H), 6.61 (d, J = 8.7Hz, 2H), 6.94 (d, J = 8.4Hz, 2H), 7.51 (s, 1 H), 12.50 (brs, 1 H)ppm | |
| 583 | 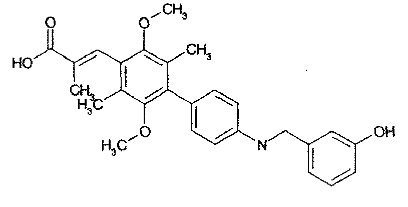 | 1H NMR (DMSO-d6) δ 1.66 (s, 3H), 1.93 (s, 3H), 2.02 (s, 3H), 3.20 (s, 2H), 3.48 (s, 3H), 4.20 (s, 2H), 6.23 (m, 1 H), 6.61-6.81 (m, 5H), 6.94 (d, J = 8.4Hz, 2H), 7.13 (t, J = 8.1 Hz, 1 H), 7.52 (s, 1 H), 9.33 (brs, 1 H), 12.53 (brs, 1H)ppm | |
| 584 | 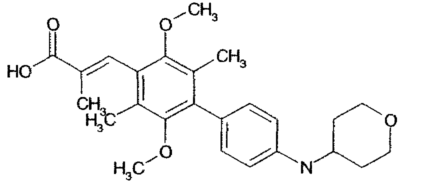 | 1H NMR (DMSO-d6) δ 1.42 (m, 2H), 1.66 (s, 3H), 1.87 (m, 2H), 1.95 (s, 3H), 2.03 (s, 3H), 3.21 (s, 3H), 3.43 (m, 3H), 3.49 (s, 3H), 3.89 (m, 2H), 5.60 (m, 2H), 6.65 (d, J = 8.7 Hz, 2H), 6.95 (d, J = 8.4Hz, 2H), 7.51 (s, 1H), 12.57 (brs, 1 H)ppm | |
| 585 | 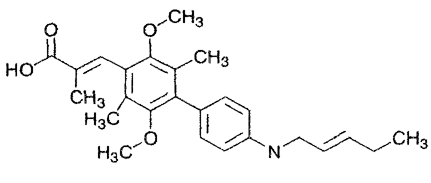 | 1 H NMR (DMSO-d6) δ 0.97 (t, J = 7.2 Hz, 3H), 1.67 (s, 3H), 1.94 (s, 3H), 2.03 (s, 3H), 2.05 (m, 2H), 3.21 (s, 3H), 3.49 (s, 3H), 3.65 (m, 2H), 5.51-5.78 (m, 2H), 6.61 (d, J = 7.8 Hz, 2H), 6.95 (d, J = 8.1Hz, 2H), 7.50 (s, 1 H), 12.55 (brs, 1H)ppm | |

**[Table 97]**

| | | | |
|---|---|---|---|
| 586 | 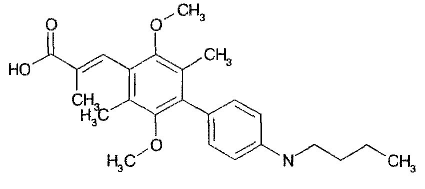 | 1 H NMR (DMSO-d6) δ 0.94 (t, J = 7.2 Hz, 3H), 1.35-1.59 (m, 4H), 1.66 (s, 3H), 1.94 (s, 3H), 2.03 (s, 3H), 3.01 (m, 2H), 3.21 (s, 3H), 3.49 (s, 3H), 5.60 (m, 1H), 6.59 (d, J = 8.4 Hz, 2H), 6.95 (d, J = 8.1Hz, 2H), 7.49 (s, 1H), 12.65 (brs, 1H)ppm | |
| 587 | 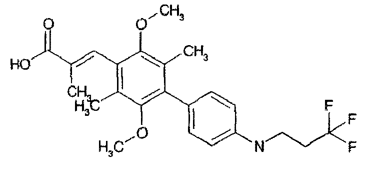 | 1H NMR (DMSO-d6) δ 1.67 (s, 3H), 1.94 (s, 3H), 2.03 (s, 3H), 2.49-2.65 (m, 4H), 3.21 (s, 3H), 3.49 (s, 3H), 5.83 (m, 1H), 6.65 (d, J = 8.7 Hz, 2H), 6.85 (d, J = 8.4Hz, 2H), 7.51 (s, 1H), 12.65 (brs, 1 H)ppm | |
| 588 | 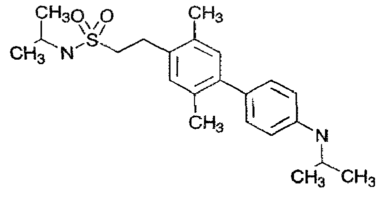 | 1H NMR (CDCl3) δ 1.23 (d, J = 6.6 Hz, 6H), 1.25 (d, J = 6.3 Hz, 6H), 2.25 (s, 3H), 2.31 (s, 3H), 3.05-3.17 (m, 2H), 3.18-3.29 (m, 2H), 3.58-3.72 (m, 2H), 3.93 (d, J = 7.5 Hz, 1 H), 6.62 (d, J = 8.7 Hz, 1H), 6.98 (s, 1H), 7.03 (s, 1H), 7.05 (s, 1H) 7.11 (d, J = 8.7 Hz, 2H). | |
| 589 | 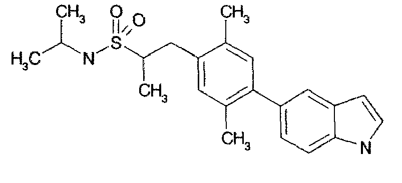 | 1H NMR (CDCl3) δ 1.23 (d, J = 8.4 Hz, 6H), 1.37 (d, J = 6.9 Hz, 3H), 2.26 (s, 3H), 2.34 (s, 3H), 2.73 (d.d, J=13.5 & 10.8Hz, 1H), 3.18-3.31 (m, 1 H), 3.44 (d.d, J = 13.5 & 3.6 Hz, 1 H), 3.62-3.75 (m, 1H), 3.83 (d, J = 8.1 Hz, 1H), 6.58 (s, 1H), 7.03 (s, 1H), 7.11-7.17 (m, 2H), 7.24-7.28 (m, 1 H), 7.42 (d, J = 8.4 Hz, 1H), 7.55 (s, 1H) | |
| 590 | 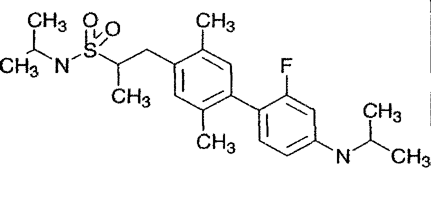 | 1H NMR (CDCl3) δ 1.20 (d, J = 6.6 Hz, 6H), 1.24 (d, J = 6.6 Hz, 6H), 1.35 (d, J=6.3 Hz, 3H), 2.17 (s, 3H), 2.31 (s, 3H), 2.71 (d.d, J = 13.8 & 10.5 Hz, 1H), 3.14-3.28 (m, 1H), 3.41 (d.d, J = 13.5 & 3.9 Hz, 1H), 3.55-3.74 (m, 2H), 3.82 (d, J = 8.1 Hz, 1 H), 6.30-6.42 (m, 2H), 6.94-7.03 (m, 3H). | |

**[Table 98]**

| | | | |
|---|---|---|---|
| 591 | 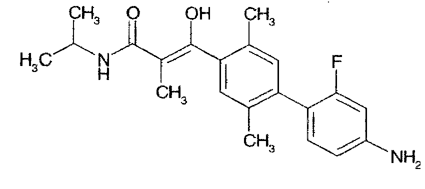 | 1 H NMR (CDCl3) δ 1.12 (d, J = 6.6 Hz, 3H), 1.14 (d, J = 6.6 Hz, 3H), 1.49 (d, J = 7.2 Hz, 3H), 2.22 (s, 3H), 2.45 (s, 3H), 3.86 (brs, 2H), 4.05 (m, 1H), 4.20 (q, J = 7.2 Hz, 1H), 6.28 (brd, J = 8.7 Hz, 1H), 6.42-6.56 (m, 2H), 6.98 (t, J = 8.7 Hz, 1 H), 7.09 (s, 1H), 7.59 (s, 1 H). | |
| 592 | 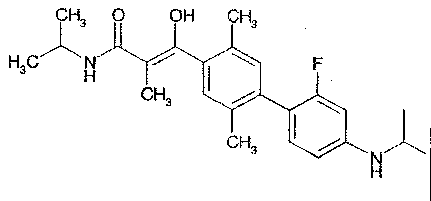 | 1H NMR (CDCl3) δ 1.12 (d, J = 6.6Hz,3H),1.14(d,J=6.6Hz, 3H), 1.26 (d, J = 6.3 Hz, 6H), 1.49 (d, J = 6.9 Hz, 3H), 2.23 (s, 3H), 2.45 (s, 3H), 3.63 (sept, J = 6.3 Hz, 1H), 4.06 (m, 1H), 4.22 (q, J = 7.2 Hz, 1 H), 6.28 (brd, J = 8.1 Hz, 1H), 6.35-6.45 (m, 2H), 6.99 (t, J = 8.4 Hz, 1H), 7.10 (s, 1 H), 7.59 (s, 1 H). | |
| 593 | 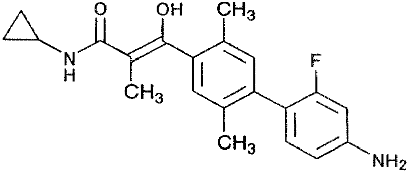 | | 189-191°C |
| 594 | 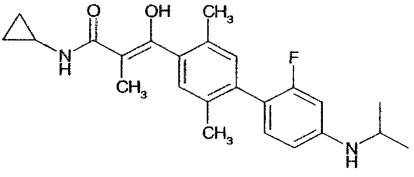 | | 153-156°C |
| 595 | 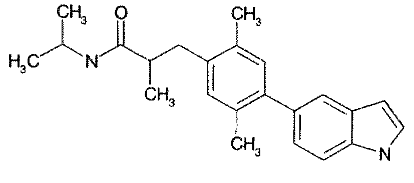 | 1 H NMR (CDCl3) δ 0.97 (d, J = 6.6 Hz, 3H), 1.08 (d, J = 6.3 Hz, 3H), 1.24 (d, J = 6.9 Hz, 3H), 2.22 (s, 3H), 2.31 (s, 3H), 2.36 (m, 1H), 2.71 (dd, J = 6.0, 13.5 Hz, 1H), 2.93 (dd, J = 9.0, 13.8 Hz, 1H), 4.01 (m, 1H), 4.92 (d, J = 7.8 Hz, 1H), 6.57 (t, J = 2.1 Hz, 1H), 7.01 (s, 1H), 7.09 (s, 1H), 7.12 (dd, J = 1.5, 8.4 Hz, 1H), 7.25 (d, J = 2.7 Hz, 1H), 7.41 (d, J = 8.4 Hz, 1 H), 7.53 (d, J = 0.9 Hz, 1 H), 8.27 (s, 1H). | |

**[Table 99]**

| Compo und No. | Structure | Compou nd No. | Structure |
|---|---|---|---|
| 596 | 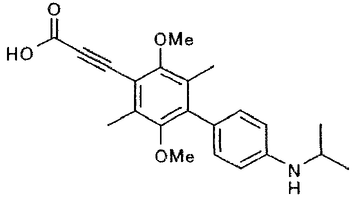 | 602 | 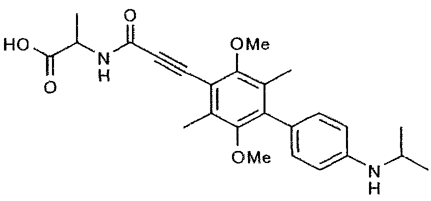 |
| 597 | 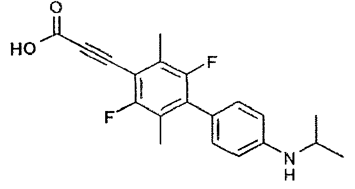 | 603 | 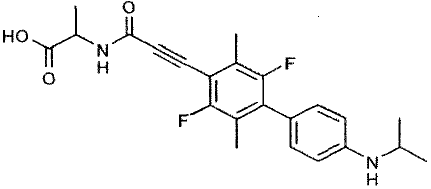 |
| 598 | 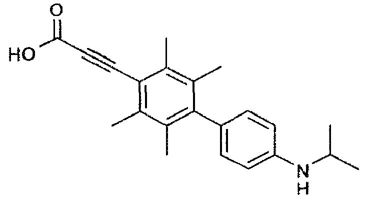 | 604 | 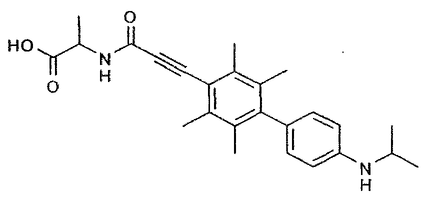 |
| 599 | 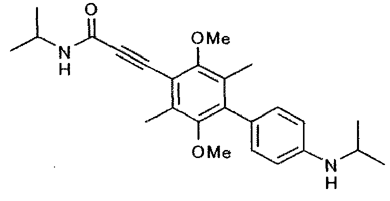 | 605 | 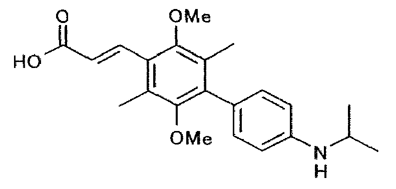 |
| 600 | 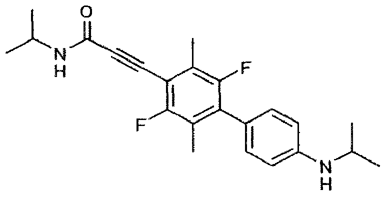 | 606 | 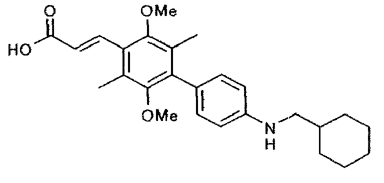 |
| 601 | 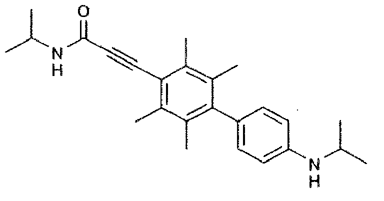 | 607 | 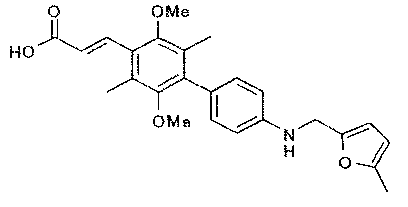 |

**[Table 100]**

| Compo und No. | Structure | Compou nd No. | Structure |
|---|---|---|---|
| 608 | 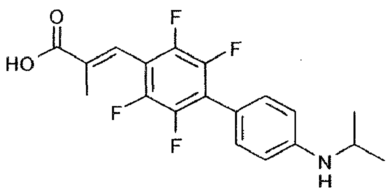 | 614 | 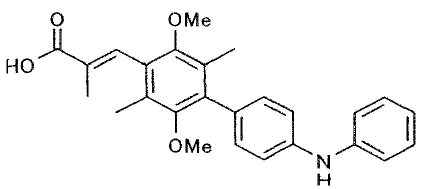 |
| 609 | 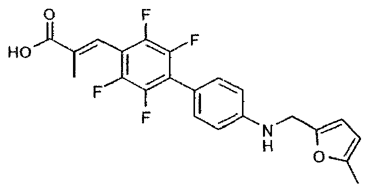 | 615 | 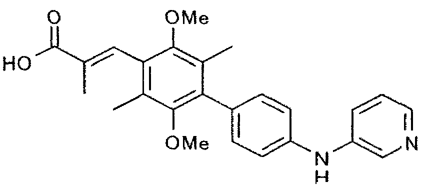 |
| 610 | 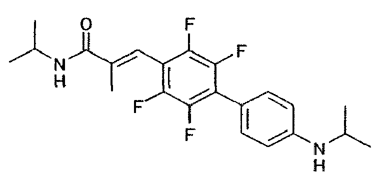 | 616 | 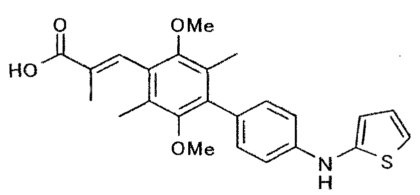 |
| 611 | 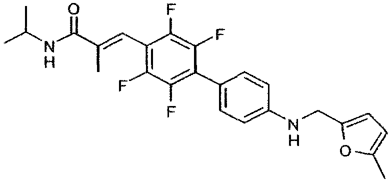 | 617 | 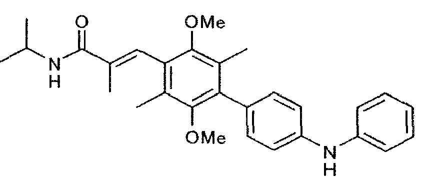 |
| 612 | 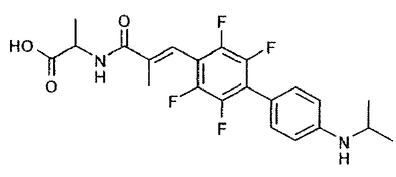 | 618 | 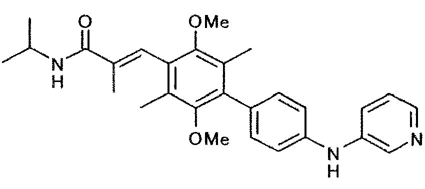 |
| 613 | 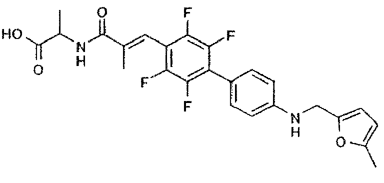 | 619 | 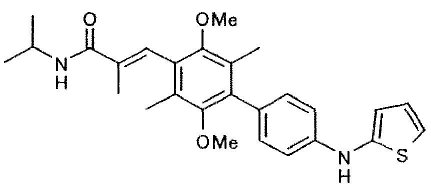 |

### Test Example 1 Method of testing dihydrooroate dehydrogenase inhibition

Human U-937 cell (human histiocytic lymphoma cell line) was sonicated in a homogenizing buffer (20 mM Tris-HCl, pH 7.4, containing 2 mM EDTA and protease inhibitor cocktail), and this was centrifuged at 4°C and 1,200 × g for 15 minutes to remove cell debris. Further, ultracentrifugation at 4°C and 120,000 × g for 60 minutes afforded a mitochondrial/microsomal fraction. The resulting fraction was quantitated for a protein, and prepared to 10 mg/ml, which was freezing-stored in a -40°C in a refrigerator until measurement. To 150 µl of the reaction solution (50 mM Tris-HCl, pH 7.4, containing 0.1%Triton X-100, 1 mM KCN, 100 µM coenzyme Q10, 200 µM DCIP), were added 10 µl of a dilution series of the compound and 0.2 mg of a mitochondrial/microsomal fraction, followed by pre-incubation at 37°C for 30 minutes. Then, 20 µl of a 5mM DHO solution (final concentration 500 µM), which is a substrate, was added, this was incubated at 37°C for 120 minutes, and an absorbance at a measurement wavelength of 620 nm was measured. A rate of suppression of a compound at each concentration relative to a change in an absorbance due to the enzyme reaction was obtained, and a concentration indicating 50% inhibition (IC50 value) was calculated to assess the inhibition activity of the compound.
Results are shown in the following Table.

**[Table 101]**

| | | | | | | | |
|---|---|---|---|---|---|---|---|
| Compound No. | IC50 (ng/ml) | | 299 | 3.8 | | 426 | 0.42 |
| | | | 309 | 1.8 | | 427 | 4.9 |
| 24 | 3.1 | | 313 | 4.8 | | 434 | 1 |
| 27 | 3.5 | | 321 | 2.7 | | 463 | 4.1 |
| 28 | 3.3 | | 324 | 2.1 | | 488 | 4 |
| 41 | 3.7 | | 325 | 4.7 | | 518 | 3.1 |
| 42 | 4.6 | | 334 | 1.1 | | 528 | 1.5 |
| 116 | 1.5 | | 347 | 2.9 | | 530 | 4.3 |
| 122 | 4 | | 348 | 2.6 | | 536 | 4 |
| 154 | 3 | | 349 | 3.7 | | 574 | 4.2 |
| 158 | 3 | | 383 | 4.9 | | 575 | 4.6 |
| 165 | 4 | | 397 | 2.7 | | | |
| 173 | 3 | | 414 | 3.9 | | | |
| 201 | 2 | | 415 | 2.4 | | | |
| 252 | 4.7 | | 416 | 1.1 | | | |
| 253 | 4.7 | | 417 | 2.5 | | | |
| 267 | 2.8 | | 418 | 1.2 | | | |
| 268 | 2.2 | | 422 | 3.3 | | | |
| 281 | 1.8 | | 424 | 1 | | | |
| 292 | 1.1 | | 425 | 0.39 | | | |

### Test Example 2 Effect of suppression of IgE antibody production to anti-ovalbumin (OVA)

### 1) Animal

BALB/c mice (female, 8 to 10 week old) purchased from Japan Charles River (Kanagawa) and Wistar rats (female, 8 to 10 week old) purchased from Japan SLC (Sizuoka) were used.

### 2) Immunizing method

BALB/c mice were immunized by intraperitoneally injecting 0.2 ml of a solution obtained by suspending 2 µg of ovalbumin (OVA) and an aluminum hydroxide gel (1 mg) in a brine. After ten days, blood was taken from a heart, the serum was separated, and an IgE antibody value was measured.

### 3) Compound

The compounds of the present invention were suspended in 0.5% methylcellulose, and the suspension was orally administered at 0.1 ml per mouse (dose 10 or 40 mg/kg). Administration was performed from immunization date to the day before blood collection for consecutive 10 days.

### 4) Measurement of anti-OVA IgE antibody value (PCA titer)

The resulting mouse serum was prepared into a 2-fold dilution series with a brine, and each 50 µl of this was subcutaneously injected into a back of a pre-shaved Wistar rat. After 24 hours, 0.5ml of a brine containing 1 mg of OVA and 5 mg of an Evans Blue dye was intravenously injected to induce a passive skin anaphylactic reaction (PCA). After 30 minutes, a maximum dilution rate of the serum exhibiting PCA positive reaction in which a pigment spot was of a diameter of not smaller than 5 mm was determined, and Log₂ of the dilution rate was adopted as a PCA titer. For example, certain serum exhibited PCA positive reaction until 2⁷-fold dilution, and an anti-OVA IgE antibody value of the mouse was determined to be 7.

### Test Example 3 Effect of suppressing antibody production using human lymphocyte

### 1. Experimental method

### 1) Human peripheral blood

Human peripheral blood was collected with a plastic syringe containing heparin (final concentration 1.5%) from a vein of an adult healthy male, and subjected to collection of lymphocyte immediately after blood collection.

### 2) Medium

To the RPMI medium (Sigma) were added 10% of fetal bovine serum, (HyClone Lab.) which had been immobilized at 56°C for 30 minutes, penicillin (100 units/ml) and streptomycin (100 µg/ml) (Invitrogen), which was used.

### 3) Compound

The present compound was dissolved in dimethyl sulfoxide (Nakalai Tesque) to 2 mg/ml and, thereafter, the solution was diluted with a medium to a final concentration of 0.01 pg/ml to 10 µg/ml.

### 4) Human lymphocyte

Human peripheral blood was mixed with an equal amount of PBS-10mM EDTA, 10 ml of this was overlaid on a tube containing 3 ml of Ficoll-Paque Plus (Pharmacia Biotech), followed by centrifugation at room temperature and 300 × g for 30 minutes to obtain a lymphocyte layer. The collected cell suspension was centrifugation-washed with a sterilized Hanks's balanced salt solution (Invitrogen), passed through a nylon mesh, and centrifugation-washed with a medium, which was used in an experiment as human lymphocyte.

### 5) Inducement of IgE antibody production due to B cell stimulation

Human lymphocyte was seeded on a 96-well culturing plate (Sumitomo Bakelite) to 2 × 10⁵ cells per well, and the compound, an anti-human CD40 antibody (Pharmingen, 2 µg/ml), and human recombinant interleukin-4 (IL-4) (PEPROTECH, 0.1 µg/ml) were added, followed by culturing at 37°C under the presence of 5% CO₂ (0.2ml/well). After cultured for 9 days, an amount of an antibody produced in the supernatant was quantitated by a specific ELISA method.

### 6) Quantitation of IgE antibody

For quantitating IgE, a commercially available kit, MESACUP IgE Test (Medical & Biological Laboratories Co., Ltd.) was used. An experimental method was according to the manual, an experiment was performed duplicately, and an average was obtained.
Results of Test Examples 2 and 3 are shown below.

**[Table 102]**

| | | | | | | | | |
|---|---|---|---|---|---|---|---|---|
| Compound No. | Test Example 2 | | Test Example 3 | | 40 | 40 | 1.3 | 3.7 |
| | | | | | 526 | 40 | 2.7 | 0.9 |
| | dose (mg/kg) | PCA | IC50 (ng/ml) | | 420 | 40 | 0< | 39 |
| | | | | | 347 | 40 | 2.0 | 60 |
| 76 | 10 | 4.3 | 1.6 | | 459 | 10 | 2.7 | 0.8 |
| 80 | 10 | 5.3 | 4.6 | | 397 | 20 | 0< | 0.3 |
| 122 | 10 | 4.7 | 0.5 | | 559 | 10 | 4 | 7.2 |
| 484 | 40 | 0< | 9.3 | | 562 | 40 | 0< | 8.4 |
| 486 | 10 | 0< | 13.5 | | 408 | 40 | 0< | 8 |
| 142 | 10 | 0.0 | 8.4 | | 571 | 10 | 1.3 | 6.4 |
| 493 | 10 | 4.0 | 9.4 | | 573 | 10 | 0< | 6.6 |
| 495 | 10 | 4.7 | 41 | | 585 | 10 | 1.0 | 4.8 |
| 201 | 10 | 5.3 | 0.6 | | | | | |
| 252 | 40 | 0< | 75 | | | | | |

**Preparation Example 1 Tablet**

| | |
|---|---|
| Compound 1 | 5 mg |
| Starch | 15 mg |
| Lactose | 15 mg |
| Crystalline cellulose | 19 mg |
| Polyvinyl alcohol | 3 mg |
| Distilled water | 30 ml |
| Calcium stearate | 3 mg |

Components other than calcium stearate are uniformly mixed, ground, granulated, and dried to obtain granules having a suitable size. Then, calcium stearate is added, and this is compressed and molded to obtain tablets.

### Industrial applicability

As apparent from the above Test Examples, the compounds of the present invention are useful as an antibody production suppressing agent, a DHODH inhibitor, an anti-allergic agent, an immunosuppressing agent and/or an anti-cancer agent.

## Claims

1. A compound represented by the formula (I): 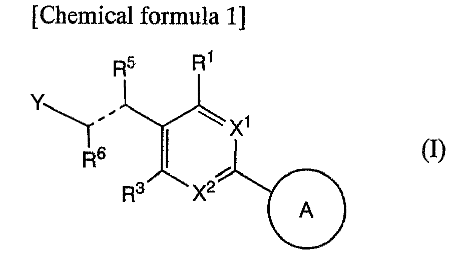 wherein X¹ is N or CR², X² is N or CR⁴,
R¹, R², R³ and R⁴ are each independently hydrogen, halogen, optionally substituted lower alkyl, optionally substituted lower alkoxy, optionally substituted acyl, carboxy, or optionally substituted lower alkoxycarbonyl, provided that all of R¹ to R⁴ are not simultaneously hydrogen, 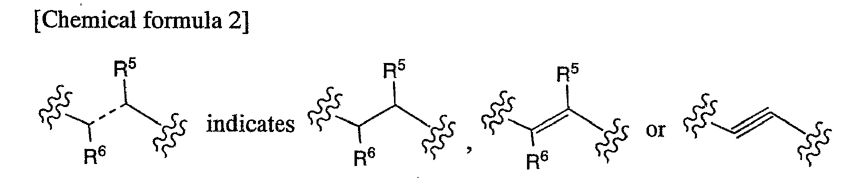 wherein R⁵ and R⁶ are each independently hydrogen, halogen, hydroxy, optionally substituted lower alkyl, optionally substituted lower alkoxy, optionally substituted amino, or cyano, and when a broken line is a single bond, then R⁵ may be oxo,
Y is: 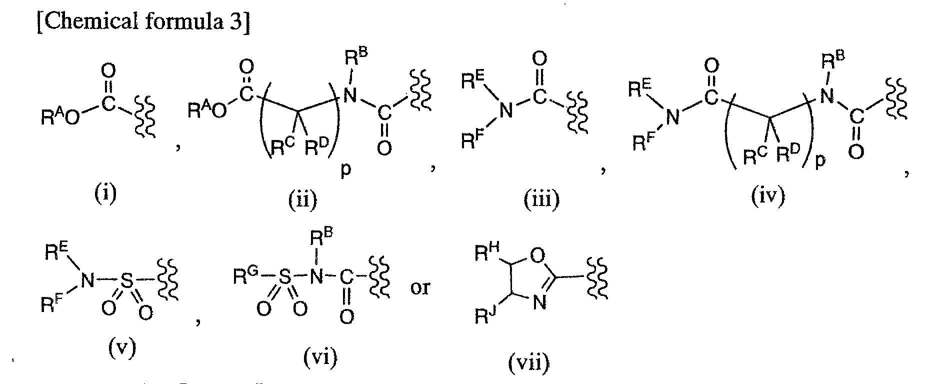 wherein R^{A}, R^{B} and R^{E} are each independently hydrogen or lower alkyl,
R^{C} and R^{D} are each independently hydrogen, optionally substituted lower alkyl, and R^{C} and R^{D} may be taken together to form a carbocycle containing an adjacent carbon atom, R^{F} is hydrogen, optionally substituted lower alkyl, optionally substituted lower alkoxy, optionally substituted cycloalkyl, optionally substituted cycloalkenyl, optionally substituted amino, optionally substituted amidino, cyano, optionally substituted aryl, or optionally substituted heterocycle,
R^{G} is optionally substituted lower alkyl, optionally substituted aryl, or optionally substituted heterocycle,
R^{H} and R^{J} are each independently hydrogen, optionally substituted lower alkyl, carboxy, or optionally substituted lower alkoxycarbonyl,
p is 1 or 2,
ring A is: 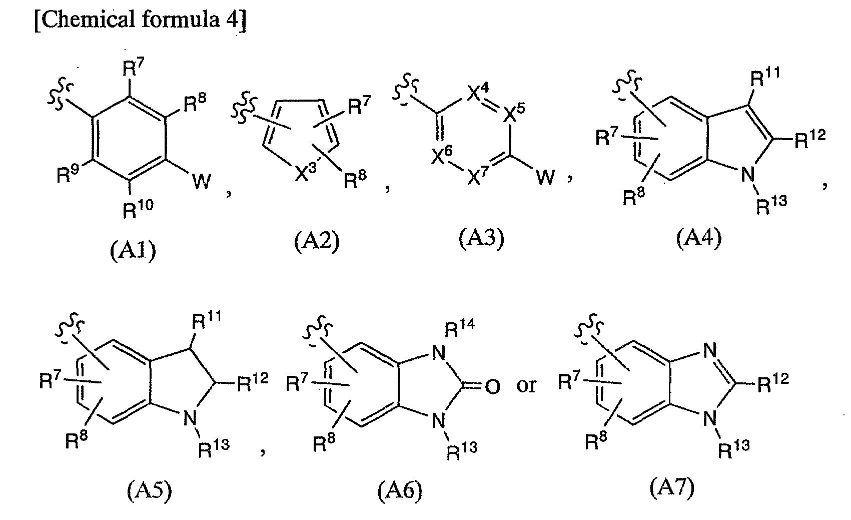 wherein X³ is O, S or NR¹³,
X⁴ is CR⁷ or N, X⁵ is CR⁸ or N, X⁶ is CR⁹ or N, X⁷ is CR¹⁰ or N, provided that at least one of X⁴ to X⁷ is N, and at least one of X⁴ to X⁷ is other than N, R⁷ to R¹² are each independently hydrogen, halogen, hydroxy, lower alkyl, lower alkenyl, lower alkoxy, carboxy, lower alkoxycarbonyl, acyl, acyloxy, lower alkylsulfonyloxy or arylsulfonyloxy,
R¹³ and R¹⁴ are each independently hydrogen, lower alkyl, lower alkoxycarbonyl or aryl(lower)alkyl,
W is hydrogen, optionally substituted lower alkyl, NR¹⁵R¹⁶, OR¹⁷, SR¹⁸, COR¹⁹ or CONR²⁰R²¹,
when ring A is (A1), and R⁵ is lower alkyl, then W is NR¹⁵R¹⁶, SR¹⁸, COR¹⁹ or CONR²⁰R²¹,
R¹⁵ and R¹⁶ are each independently hydrogen, optionally substituted lower alkyl, optionally substituted lower alkenyl, optionally substituted cycloalkyl, optionally substituted carbamoyl, optionally substituted lower alkylsulfonyl, optionally substituted arylsulfonyl, optionally substituted aryl, or optionally substituted heterocycle,
R¹⁷, R¹⁸, R¹⁹, R²⁰ and R²¹ are each independently hydrogen, optionally substituted lower alkyl, optionally substituted lower alkenyl, optionally substituted cycloalkyl, optionally substituted aryl, or optionally substituted heterocycle,
or a pharmaceutically acceptable salt, or a solvate thereof.

2. The compound according to claim 1, wherein ring A is: 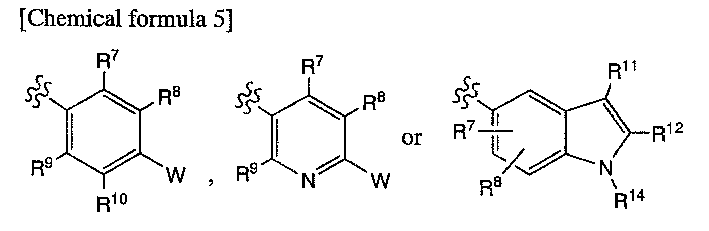 or a pharmaceutically acceptable salt, or a solvate thereof.

3. The compound according to claim 1, wherein ring A is: 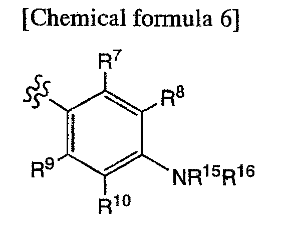 or a pharmaceutically acceptable salt, or a solvate thereof.

4. The compound according to any one of claims 1 to 3, wherein X¹ is CR², and X² is CR⁴, or a pharmaceutically acceptable salt, or a solvate thereof.

5. The compound according to any one of claims 1 to 4, wherein: 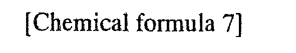 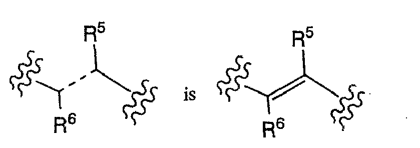 or a pharmaceutically acceptable salt, or a solvate thereof.

6. A compound represented by the formula (I'): 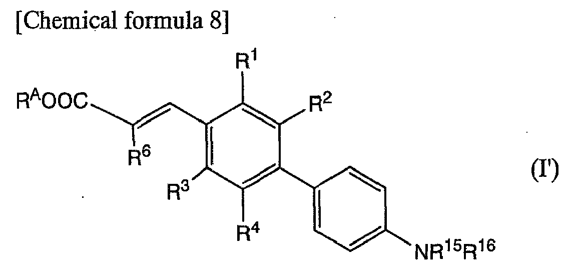 wherein R^{A} is hydrogen or lower alkyl, R⁶ is hydrogen, halogen or lower alkyl, R¹, R², R³ and R⁴ are each independently hydrogen, halogen, lower alkyl or lower alkoxy, and R¹⁵ and R¹⁶ are each independently hydrogen, optionally substituted lower alkyl or lower alkenyl,
or a pharmaceutically acceptable salt, or a solvate thereof.

7. The compound according to claim 1, 4 or 5, wherein Y is (i), ring A is (A1) or (A3), and W is NR¹⁵R¹⁶, or a pharmaceutically acceptable salt, or a solvate thereof.

8. The compound according to claim 6, wherein R^{A} is a hydrogen, ring A is (A1), R⁵ and R⁶ are each independently hydrogen, halogen or lower alkyl, R¹, R², R³ and R⁴ are each independently hydrogen, lower alkyl or lower alkoxy, R⁷, R⁸, R⁹ and R¹⁰ are each independently hydrogen or halogen, and R¹⁵ and R¹⁶ are each independently hydrogen, optionally substituted lower alkyl, lower alkenyl or cycloalkyl, or a pharmaceutically acceptable salt, or a solvate thereof.

9. The compound according to claim 1, 4 or 5, wherein Y is (i), ring A is (A4), (A5), (A6), or (A7), both of R⁷ and R⁸ are hydrogen, R¹¹ and R¹² are each independently hydrogen or lower alkyl, and R¹³ and R¹⁴ are each independently hydrogen, lower alkyl or lower alkoxycarbonyl, or a pharmaceutically acceptable salt, or a solvate thereof.

10. The compound according to claim 1, 4 or 5, wherein Y is (ii), ring A is (A1) or (A3), W is NR¹⁵R¹⁶, and R¹⁵ is optionally substituted lower alkyl, lower alkenyl or cycloalkyl, or a pharmaceutically acceptable salt, or a solvate thereof.

11. The compound according to claim 10, wherein ring A is (A1), and R⁷, R⁸, R⁹ and R¹⁰ are each independently hydrogen, halogen, lower alkyl, or lower alkoxy, or a pharmaceutically acceptable salt, or a solvate thereof.

12. The compound according to claim 1, 4 or 5, wherein Y is (ii), ring A is (A4), (A5) or (A7), both of R⁷ and R⁸ are hydrogen, R¹¹ and R¹² are each independently hydrogen or lower alkyl, and R¹³ is hydrogen or lower alkoxycarbonyl, or a pharmaceutically acceptable salt, or a solvate thereof.

13. The compound according to claim 1, 4 or 5, wherein Y is (iii), ring A is (A1) or (A3), and W is NR¹⁵R¹⁶, or a pharmaceutically acceptable salt, or a solvate thereof.

14. The compound according to claim 13, wherein R^{F} is hydrogen, optionally substituted lower alkyl, optionally substituted lower alkoxy, optionally substituted cycloalkyl, cyano, optionally substituted amino, optionally substituted aryl, or optionally substituted heterocycle, or a pharmaceutically acceptable salt, or a solvate thereof.

15. The compound according to claim 13, wherein R^{F} is optionally substituted lower alkyl, or optionally substituted cycloalkyl, or a pharmaceutically acceptable salt, or a solvate thereof.

16. The compound according to claim 13, wherein R¹⁵ is hydrogen, optionally substituted lower alkyl, lower alkenyl, cycloalkyl, lower alkylcarbamoyl, lower alkylsulfonyl or a heterocycle, or a pharmaceutically acceptable salt, or a solvate thereof.

17. The compound according to claim 13, wherein R¹⁵ is lower alkyl, lower alkenyl or cycloalkyl, or a pharmaceutically acceptable salt, or a solvate thereof.

18. The compound according to claim 1, 4 or 5, wherein Y is (iii), ring A is(A2), (A4), (A5), (A6) or (A7), and R^{F} is optionally substituted lower alkyl, optionally substituted lower alkoxy, optionally substituted amino, optionally substituted cycloalkyl, or optionally substituted heterocycle, or a pharmaceutically acceptable salt, or a solvate thereof.

19. The compound according to claim 18, wherein ring A is (A4), all of R⁷, R⁸ and R¹³ are hydrogen, and R¹¹ and R¹² are each independently hydrogen or lower alkyl, or a pharmaceutically acceptable salt, or a solvate thereof.

20. The compound according to claim 18, wherein ring A is (A4), and R^{F} is optionally substituted lower alkyl, or optionally substituted cycloalkyl, or a pharmaceutically acceptable salt, or a solvate thereof.

21. The compound according to claim 1, 4 or 5, wherein Y is (iv), and ring A is (A1), (A4) or (A7), or a pharmaceutically acceptable salt, or a solvate thereof.

22. The compound according to claim 21, wherein ring A is (A1), and W is NR¹⁵R¹⁶, or a pharmaceutically acceptable salt, or a solvate thereof.

23. The compound according to claim 1, 4 or 5, wherein Y is (v), ring A is (A1) or (A4), and R^{F} is optionally substituted lower alkyl, or optionally substituted cycloalkyl, or a pharmaceutically acceptable salt, or a solvate thereof.

24. The compound according to claim 1, 4 or 5, wherein Y is (vi), ring A is (A1), (A3) or (A4), and R^{G} is optionally substituted lower alkyl, or optionally substituted aryl, or a pharmaceutically acceptable salt, or a solvate thereof.

25. The compound according to claim 24, wherein ring A is (A1), and W is NR¹⁵R¹⁶, or a pharmaceutically acceptable salt, or a solvate thereof.

26. The compound according to claim 1, 4 or 5, wherein Y is (vii), and ring A is (A1), or a pharmaceutically acceptable salt, or a solvate thereof.

27. The compound according to claim 1, wherein Y is (i), (ii), or (iii), R^{F} is optionally substituted lower alkyl, or optionally substituted cycloalkyl, 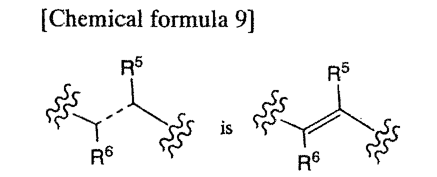
X is CR², X² is CR⁴, R¹ to R⁴ are each independently hydrogen, fluoro, methyl or methoxy (provided that the case where 3 or more selected from R¹ to R⁴ are hydrogen is excluded), ring A is (A1), and W is lower alkylamino, lower alkenylamino, cycloalkylamino, cycloalkyl(lower)alkylamino, or furyl(lower)alkyl optionally substituted with lower alkyl, or a pharmaceutically acceptable salt, or a solvate thereof.

28. The compound according to claim 1, wherein Y is (i), (iv), (v), or (vi), or Y is (ii) and p is 1, or Y is (iii) and R^{F} is optionally substituted lower alkyl, optionally substituted cycloalkyl, optionally substituted cycloalkenyl, optionally substituted aryl, or cyano, 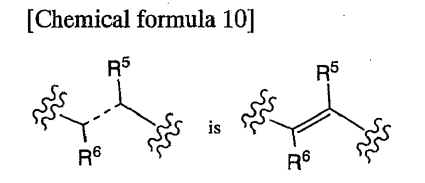
X¹ is CR², ring A is (A1), and W is NR¹⁵R¹⁶, or a pharmaceutically acceptable salt, or a solvate thereof.

29. The compound according to claim 1, wherein Y is (i), (ii), or (iii), R^{F} is optionally substituted lower alkyl, or optionally substituted cycloalkyl, 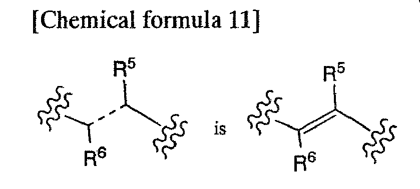
X¹ is CR², X² is CR⁴, R¹ to R⁴ are each independently hydrogen, fluoro, methyl or methoxy (provided that the case where 3 or more selected from R¹ to R⁴ are hydrogen is excluded), ring A is (A1), and W is lower alkylamino, lower alkenylamino, cycloalkylamino, cycloalkyl(lower)alkylamino, or furyl(lower)alkyl optionally substituted with lower alkyl, or a pharmaceutically acceptable salt, or a solvate thereof.

30. The compound according to claim 1, wherein Y is (i), (iv), (v) or (vi), or Y is (ii) and p is 1, or Y is (iii), and R^{F} is optionally substituted lower alkyl, optionally substituted cycloalkyl, optionally substituted cycloalkenyl, optionally substituted aryl, or cyano, 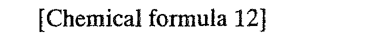 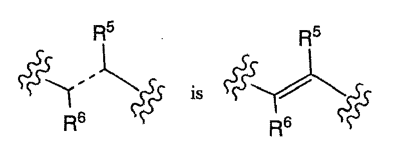
X¹ is CR², ring A is (A1), and W is NR¹⁵R¹⁶, or a pharmaceutically acceptable salt, or a solvate thereof.

31. A pharmaceutical composition comprising a compound as defined in any one of claims 1 to 30, or a pharmaceutically acceptable salt, or a solvate thereof.

32. A pharmaceutical composition for use as antibody production suppressor comprising a compound as defined in any one of claims 1 to 30, or a pharmaceutically acceptable salt, or a solvate thereof.

33. A pharmaceutical composition for use as dihydroorotate dehydrogenase inhibitor comprising a compound as defined in any one of claims 1 to 30, or a pharmaceutically acceptable salt, or a solvate thereof.
